(19) Europäisches Patentamt
European Patent Office
Office européen des brevets

(11) EP 1 546 336 B1

(12) **EUROPEAN PATENT SPECIFICATION**

(45) Date of publication and mention of the grant of the patent:
**28.12.2011 Bulletin 2011/52**

(21) Application number: **03779082.1**

(22) Date of filing: **18.09.2003**

(51) Int Cl.:
*C12N 15/29* (2006.01) *C12N 15/82* (2006.01)
*A01H 5/00* (2006.01)

(86) International application number:
**PCT/US2003/030292**

(87) International publication number:
**WO 2004/031349 (15.04.2004 Gazette 2004/16)**

(54) **POLYNUCLEOTIDES AND POLYPEPTIDES IN PLANTS**
POLYNUKLEOTIDE UND POLYPEPTIDE IN PFLANZEN
POLYNUCLEOTIDES ET POLYPEPTIDES CHEZ DES PLANTES

(84) Designated Contracting States:
**AT BE BG CH CY CZ DE DK EE ES FI FR GB GR HU IE IT LI LU MC NL PT RO SE SI SK TR**

(30) Priority: **18.09.2002 US 411837 P**
**17.12.2002 US 434166 P**
**24.04.2003 US 465809 P**

(43) Date of publication of application:
**29.06.2005 Bulletin 2005/26**

(60) Divisional application:
**10176341.5 / 2 272 962**
**10176922.2 / 2 270 166**
**10178358.7 / 2 270 167**

(73) Proprietor: **Mendel Biotechnology, Inc.**
**Hayward, CA 94545 (US)**

(72) Inventors:
- **JIANG, Cai-Zhong**
  **Fremont, CA 94555 (US)**
- **HEARD, Jacqueline, E.**
  **San Mateo, CA 94402 (US)**
- **RATCLIFFE, Oliver**
  **Oakland, CA 94606 (US)**
- **CREELMAN, Robert, A.**
  **Castro Valley, CA 94546 (US)**
- **ADAM, Luc, J.**
  **Hayward, CA 94545 (US)**
- **REUBER, T., Lynne**
  **San Mateo, CA 94402 (US)**
- **RIECHMANN, Jose, Luis**
  **Pasadena, CA 91101 (US)**
- **HAAKE, Volker**
  **D-10715 Berlin (DE)**
- **DUBELL, Arnold, N.**
  **San Leandro, CA 94578 (US)**
- **KEDDIE, James, S.**
  **San Mateo, CA 94402 (US)**
- **SHERMAN, Bradley, K.**
  **Berkeley, CA 94708 (US)**

(74) Representative: **Brasnett, Adrian Hugh et al**
**Mewburn Ellis LLP**
**33 Gutter Lane**
**London**
**EC2V 8AS (GB)**

(56) References cited:

| | |
|---|---|
| **EP-A- 1 033 405** | **WO-A-2005/001050** |
| **WO-A2-03/013227** | **WO-A2-2008/005210** |
| **US-A- 5 994 622** | **US-A1- 2002 076 775** |

- **DATABASE EMBL [Online] 15 October 1999 (1999-10-15), "Arabidopsis thaliana genomic DNA, chromosome 3, P1 clone: MSA6." XP002449483 retrieved from EBI accession no. EMBL:AP000604 Database accession no. AP000604**
- **DATABASE EMBL [Online] 14 November 2001 (2001-11-14), "Arabidopsis thaliana WRKY transcription factor 51 (WRKY51) mRNA, complete cds." XP002440123 retrieved from EBI accession no. EMBL:AF426252 Database accession no. AF426252**
- **DATABASE EMBL [Online] 13 December 2002 (2002-12-13), "Arabidopsis thaliana At3g21150 mRNA for unknown protein, complete cds, clone: RAFL18-04-G04." XP002449484 retrieved from EBI accession no. EMBL:AK117844 Database accession no. AK117844**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DATABASE GENBANK [Online] 08 November 2001 KUSHNIR ET AL, XP002978983 Retrieved from STIC Database accession no. (AF426252)**
- **DATABASE GENBANK [Online] 07 September 2000 JOFUKU ET AL, XP002978984 Retrieved from STIC Database accession no. (AR091882)**

## Description

## TECHNICAL FIELD

This invention relates to the field of plant biology, and to compositions and methods for modifying the phenotype of a plant.

## BACKGROUND OF THE INVENTION

A plant's traits, such as its biochemical, developmental, or phenotypic characteristics, may be controlled through a number of cellular processes. One important way to manipulate that control is through transcription factors - proteins that influence the expression of a particular gene or sets of genes. Transformed and transgenic plants comprise cells having altered levels of at least one selected transcription factor, and may possess advantageous or desirable traits. Strategies for manipulating traits by altering a plant cell's transcription factor content can therefore result in plants and crops with new and/or improved commercially valuable properties.

Transcription factors can modulate gene expression, either increasing or decreasing (inducing or repressing) the rate of transcription. This modulation results in differential levels of gene expression at various developmental stages, in different tissues and cell types, and in response to different exogenous (e.g., environmental) and endogenous stimuli throughout the life cycle of the organism.

Because transcription factors are key controlling elements of biological pathways, altering the expression levels of one or more transcription factors can change entire biological pathways in an organism. For example, manipulation of the levels of selected transcription factors may result in increased expression of economically useful proteins or biomolecules in plants or improvement in other agriculturally relevant characteristics. Conversely, blocked or reduced expression of a transcription factor may reduce biosynthesis of unwanted compounds or remove an undesirable trait. Therefore, manipulating transcription factor levels in a plant offers tremendous potential in agricultural biotechnology for modifying a plant's traits.

EP 1 033 405 A discloses a sequence which has about 53.6% identity to SEQ ID NO:328 of the present disclosure. EMBL Database accession no. AP000604 is an *Arabidopsis thaliana* genomic DNA, chromosome 3, sequence which corresponds to SEQ ID NO:327 of the present disclosure.

We have identified polynucleotides encoding transcription factors, developed numerous transgenic plants using these polynucleotides, and have analyzed the plants for a variety of important traits. In so doing, we have identified important polynucleotide and polypeptide sequences for producing commercially valuable plants and crops as well as the methods for making them and using them. Other aspects and embodiments of the invention are described below and can be derived from the teachings of this disclosure as a whole.

## SUMMARY OF THE INVENTION

The present invention provides a transgenic plant that over-expresses a recombinant polynucleotide, wherein said transgenic plant has improved yield as compared to a non-transgenic plant or wild-type plant, wherein the recombinant polynucleotide encodes a polypeptide that has at least 95% amino acid sequence identity over the entire length of SEQ ID NO:328, said polypeptide providing for improved yield as compared to a non-transgenic plant that does not over-express the polypeptide.

In one aspect, the polypeptide comprises a conserved domain which has at least 80% sequence identity with the conserved domain of residues 5-50 of the polypeptide of SEQ ID NO:328.

In one aspect, said polypeptide comprises SEQ ID NO:328.

In one embodiment, in the transgenic plant of the invention, the recombinant polynucleotide comprises a constitutive, inducible, or tissue-specific promoter operably linked to said polynucleotide sequence.

The invention further provides a host plant cell comprising an expression cassette that over-expresses a recombinant polynucleotide which encodes a polypeptide, wherein said polypeptide is as defined above.

In another aspect, the transgenic plant of the invention comprises longer hypocotyls and elongated petioles compared to a non-transgenic plant or wild-type plant.

The transgenic plant of the invention may be selected from the group consisting of: soybean, potato, cotton, oilseed rape, canola, sunflower, alfalfa, clover, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, pumpkin, spinach, squash, tobacco, tomato, tomatillo, watermelon, rosaceous fruits, fruit trees, brassicas, barley; wheat, corn, sweet corn, rice, rye; sugarcane, turf; millet; sorghum; currant; avocado; citrus fruits, oranges, lemons, grapefruit, tangerines, artichoke, cherries; walnut, peanut; endive; leek; arrowroot, beet, cassava, turnip, radish, yam, sweet potato; beans, pine, poplar, eucalyptus, and mint.

The invention further provides a plant part of the transgenic plant of the above-defined invention comprising an expression cassette that comprises the recombinant polynucleotide, wherein the plant part is selected from fruit, leaf, root, plant tissue including vascular or ground tissue, or plant cells. The invention further provides a seed of the transgenic plant of the above defined invention, wherein said seed comprises an expression cassette that comprises the recombinant polynucleotide.

The invention further provides a method for producing a transgenic plant of the preceding claims, the method steps comprising: (a) producing an expression cassette comprising a recombinant polynucleotide that encodes a polypeptide that has at least 95% amino acid sequence identity over the entire length of SEQ ID NO:328; (b) introducing the expression cassette into the plant; and (c) identifying and selecting a plant with improved yield as compared to a non-transgenic plant or wild-type plant.

The invention also provides a method for producing a transgenic plant having the altered trait of improved yield as compared to a non-transgenic plant or wild-type plant, the method steps comprising: (a) providing an expression vector comprising: (i) a recombinant polynucleotide that the recombinant polynucleotide encodes a polypeptide that has at least 95% amino acid sequence identity over the entire length of SEQ ID NO:328; and (ii) at least one regulatory element flanking the polynucleotide sequence, said at least one regulatory element being effective in controlling expression of said recombinant polynucleotide in a target plant; (b) introducing the expression vector into a plant cell, thereby producing a transgenic plant cell; (c) growing the transgenic plant cell into a transgenic plant and allowing the transgenic plant to over-express a polypeptide encoded by the recombinant polynucleotide, said polypeptide having the property of improved yield in a plant as compared to a non-transgenic plant that does not over-express the polypeptide; and (d) identifying at least one transgenic plant with said improved yield by comparing said transgenic plant with at least one non-transgenic plant that does not over-express the polypeptide.

The invention further provides the use of a recombinant polynucleotide that the recombinant polynucleotide encodes a polypeptide that has at least 95% amino acid sequence identity over the entire length of SEQ ID NO:328, for producing a transgenic plant having improved yield as compared to a non-transgenic plant or wild-type plant, said polypeptide providing for improved yield as compared to a non-transgenic plant that does not over-express the polypeptide. Optionally, the polynucleotide comprises a constitutive, inducible, or tissue-specific promoter operably linked to said polynucleotide sequence.

## Brief Description of the Sequence Listing and Figures

The Sequence Listing provides exemplary polynucleotide and polypeptide sequences. The traits associated with the use of the sequences are included in the Examples.

Figure 1 shows a conservative estimate of phylogenetic relationships among the orders of flowering plants (modified from Angiosperm Phylogeny Group (1998) Ann. Missouri Bot. Gard. 84: 1-49). Those plants with a single cotyledon (monocots) are a monophyletic clade nested within at least two major lineages of dicots; the eudicots are further divided into rosids and asterids. *Arabidopsis* is a rosid eudicot classified within the order Brassicales; rice is a member of the monocot order Poales. Figure 1 was adapted from Daly et al. ((2001) Plant Physiol. 127: 1328-1333).

Figure 2 shows a phylogenic dendogram depicting phylogenetic relationships of higher plant taxa, including clades containing tomato and *Arabidopsis;* adapted from Ku et al. (2000) Proc. Natl. Acad. Sci. 97: 9121-9126; and Chase et al. (1993) Ann. Missouri Bot. Gard. 80: 528-580.

## DETAILED DESCRIPTION OF EXEMPLARY EMBODIMENTS

In an important aspect, the present invention relates to the use of polynucleotides and polypeptides, for example, for modifying phenotypes of plants. Throughout this disclosure, various information sources are referred to. The information sources include scientific journal articles, patent documents, textbooks, and World Wide Web browser-inactive page addresses, for example. The contents and teachings of each and every one of the information sources can be relied on and used to make and use embodiments of the invention.

It must be noted that as used herein and in the appended claims, the singular forms "a," "an," and "the" include plural reference unless the context clearly dictates otherwise. Thus, for example, a reference to "a plant" includes a plurality of such plants, and a reference to "a stress" is a reference to one or more stresses and equivalents thereof known to those skilled in the art, and so forth.

The polynucleotide sequences described herein encode polypeptides that are members of well-known transcription factor families, including plant transcription factor families, as disclosed in Tables 4 - 9. Generally, the transcription factors encoded by the present sequences are involved in cell differentiation and proliferation and the regulation of growth. Accordingly, one skilled in the art would recognize that by expressing the present sequences in a plant, one may change the expression of autologous genes or induce the expression of introduced genes. By affecting the expression of similar autologous sequences in a plant that have the biological activity of the present sequences, or by introducing the present sequences into a plant, one may alter a plant's phenotype to one with improved traits. The sequences may also be used to transform a plant and introduce desirable traits not found in the wild-type cultivar or strain. Plants may then be selected for those that produce the most desirable degree of over- or under-expression of target genes of interest and coincident trait improvement.

The sequences may be from any species, particularly plant species, in a naturally occurring form or from any source whether natural, synthetic, semi-synthetic or recombinant. The sequences of may also include fragments of the present amino acid sequences. Where "amino acid sequence" is recited to refer to an amino acid sequence of a naturally occurring protein molecule, "amino acid sequence" and like terms are not meant to limit the amino acid sequence to the complete native amino acid sequence associated with the recited protein molecule.

As one of ordinary skill in the art recognizes, transcription factors can be identified by the presence of a region or domain of structural similarity or identity to a specific consensus sequence or the presence of a specific consensus DNA-binding site or DNA-binding site motif (see, for example, Riechmann et al. (2000) Science 290: 2105-2110). Plant transcription factors may belong to one of the following transcription factor families: the AP2 (APETALA2) domain transcription factor family (Riechmann and Meyerowitz (1998) Biol. Chem. 379: 633-646); the MYB transcription factor family (ENBib; Martin and Paz-Ares (1997) Trends Genet. 13: 67-73); the MADS domain transcription factor family (Riechmann and Meyerowitz (1997) Biol. Chem. 378: 1079-1101); the WRKY protein family (Ishiguro and Nakamura (1994) Mol. Gen. Genet. 244: 563-571); the ankyrin-repeat protein family (Zhang et al. (1992) Plant Cell 4: 1575-1588); the zinc finger protein (Z) family (Klug and Schwabe (1995) FASEB J. 9: 597-604); Takatsuji (1998) Cell. Mol. Life Sci. 54:582-596); the homeobox (HB) protein family (Buerglin (1994) in Guidebook to the Homeobox Genes, Duboule (ed.) Oxford University Press); the CAAT-element binding proteins (Forsburg and Guarente (1989) Genes Dev. 3: 1166-1178); the squamosa promoter binding proteins (SPB) (Klein et al. (1996) Mol. Gen. Genet. 1996 250: 7-16); the NAM protein family (Souer et al. (1996) Cell 85: 159-170); the IAA/AUX proteins (Abel et al. (1995) J. Mol. Biol. 251: 533-549); the HLH/MYC protein family (Littlewood et al. (1994) Prot. Profile 1: 639-709); the DNA-binding protein (DBP) family (Tucker et al. (1994) EMBO J. 13: 2994-3002); the bZIP family of transcription factors (Foster et al. (1994) FASEB J. 8: 192-200); the Box P-binding protein (the BPF-1) family (da Costa e Silva et al. (1993) Plant J. 4: 125-135); the high mobility group (HMG) family (Bustin and Reeves (1996) Prog. Nucl. Acids Res. Mol. Biol. 54: 35-100); the scarecrow (SCR) family (Di Laurenzio et al. (1996) Cell 86: 423-433); the GF14 family (Wu et al. (1997) Plant Physiol. 114: 1421-1431); the polycomb (PCOMB) family (Goodrich et al. (1997) Nature 386: 44-51); the teosinte branched (TEO) family (Luo et al. (1996) Nature 383: 794-799); the ABI3 family (Giraudat et al. (1992) Plant Cell 4: 1251-1261); the triple helix (TH) family (Dehesh et al. (1990) Science 250: 1397-1399); the EIL family (Chao et al. (1997) Cell 89: 1133-44); the AT-HOOK family (Reeves and Nissen (1990) J. Biol. Chem. 265: 8573-8582); the S1FA family (Zhou et al. (1995) Nucleic Acids Res. 23: 1165-1169); the bZIPT2 family (Lu and Ferl (1995) Plant Physiol. 109: 723); the YABBY family (Bowman et al. (1999) Development 126: 2387-96); the PAZ family (Bohmert et al. (1998) EMBO J. 17: 170-80); a family of miscellaneous (MISC) transcription factors including the DPBF family (Kim et al. (1997) Plant J. 11: 1237-1251) and the SPF1 family (Ishiguro and Nakamura (1994) Mol. Gen. Genet. 244: 563-571); the GARP family (Hall et al. (1998) Plant Cell 10: 925-936), the TUBBY family (Boggin et al (1999) Science 286 : 2119-2125), the heat shock family (Wu (1995) Annu. Rev. Cell Dev, Biol. 11: 441-469), the ENBP family (Christiansen et al. (1996) Plant Mol. Biol. 32: 809-821), the RING-zinc family (Jensen et al. (1998) FEBS Letters 436: 283-287), the PDBP family (Janik et al. (1989) Virology 168: 320-329), the PCF family (Cubas et al. Plant J. (1999) 18: 215-22), the SRS (SHI-related) family (Fridborg et al. (1999) Plant Cell 11: 1019-1032), the CPP (cysteine-rich polycomb-like) family (Cvitanich et al. (2000) Proc. Natl. Acad. Sci. 97: 8163-8168), the ARF (auxin response factor) family (Ulmasov et al. (1999) Proc. Natl. Acad. Sci. 96: 5844-5849), the SWI/SNF family (Collingwood et al. (1999) J. Mol. Endocrinol. 23: 255-275), the ACBF family (Seguin et al. (1997) Plant Mol. Biol. 35: 281-291), PCGL (CG-1 like) family (da Costa e Silva et al. (1994) Plant Mol. Biol. 25: 921-924) the ARID family (Vazquez et al. (1999) Development 126: 733-742), the Jumonji family (Balciunas et al. (2000), Trends Biochem. Sci. 25: 274-276), the bZIP-NIN family (Schauser et al. (1999) Nature 402: 191-195), the E2F family (Kaelin et al. (1992) Cell 70: 351-364) and the GRF-like family (Knaap et al. (2000) Plant Physiol. 122: 695-704). As indicated by any part of the list above and as known in the art, transcription factors have been sometimes categorized by class, family, and sub-family according to their structural content and consensus DNA-binding site motif, for example. Many of the classes and many of the families and sub-families are listed here. The list provided here is merely an example of the types of transcription factors and the knowledge available concerning the consensus sequences and consensus DNA-binding site motifs that help define them as known to those of skill in the art. A transcription factor may include, but is not limited to, any polypeptide that can activate or repress transcription of a single gene or a number of genes. This polypeptide group includes, but is not limited to, DNA-binding proteins, DNA-binding protein binding proteins, protein kinases, protein phosphatases, protein methyltransferases, GTP-binding proteins, and receptors, and the like.

In addition to methods for modifying a plant phenotype by employing one or more polynucleotides and polypeptides described herein, the polynucleotides and polypeptides have a variety of additional uses. These uses include their use in the recombinant production (i.e., expression) of proteins; as regulators of plant gene expression, as diagnostic probes for the presence of complementary or partially complementary nucleic acids (including for detection of natural coding nucleic acids); as substrates for further reactions, e.g., mutation reactions, PCR reactions, or the like; as substrates for cloning e.g., including digestion or ligation reactions; and for identifying exogenous or endogenous modulators of the transcription factors.

Definitions

"Nucleic acid molecule" refers to a oligonucleotide, polynucleotide or any fragment thereof. It may be DNA or RNA of genomic or synthetic origin, double-stranded or single-stranded, and combined with carbohydrate, lipids, protein, or other materials to perform a particular activity such as transformation or form a useful composition such as a peptide nucleic acid (PNA).

"Polynucleotide" is a nucleic acid molecule comprising a plurality of polymerized nucleotides, e.g., at least about 15 consecutive polymerized nucleotides, optionally at least about 30 consecutive nucleotides, at least about 50 consecutive nucleotides. A polynucleotide may be a nucleic acid, oligonucleotide, nucleotide, or any fragment thereof. In many instances, a polynucleotide comprises a nucleotide sequence encoding a polypeptide (or protein) or a domain or fragment thereof. Additionally, the polynucleotide may comprise a promoter, an intron, an enhancer region, a polyadenylation site, a translation initiation site, 5' or 3' untranslated regions, a reporter gene, a selectable marker, or the like. The polynucleotide can be single stranded or double stranded DNA or RNA. The polynucleotide optionally comprises modified bases or a modified backbone. The polynucleotide can be, e.g., genomic DNA or RNA, a transcript (such as an mRNA), a cDNA, a PCR product, a cloned DNA, a synthetic DNA or RNA, or the like. The polynucleotide can be combined with carbohydrate, lipids, protein, or other materials to perform a particular activity such as transformation or form a useful composition such as a peptide nucleic acid (PNA). The polynucleotide can comprise a sequence in either sense or antisense orientations. "Oligonucleotide" is substantially equivalent to the terms amplimer, primer, oligomer, element, target, and probe and is preferably single stranded.

"Gene" or "gene sequence" refers to the partial or complete coding sequence of a gene, its complement, and its 5' or 3' untranslated regions. A gene is also a functional unit of inheritance, and in physical terms is a particular segment or sequence of nucleotides along a molecule of DNA (or RNA, in the case of RNA viruses) involved in producing a polypeptide chain. The latter may be subjected to subsequent processing such as splicing and folding to obtain a functional protein or polypeptide.. A gene may be isolated, partially isolated, or be found with an organism's genome. By way of example, a transcription factor gene encodes a transcription factor polypeptide, which may be functional or require processing to function as an initiator of transcription.

Operationally, genes may be defined by the cis-trans test, a genetic test that determines whether two mutations occur in the same gene and which may be used to determine the limits of the genetically active unit (Rieger et al. (1976) Glossary of Genetics and Cytogenetics: Classical and Molecular, 4th ed., Springer Verlag. Berlin). A gene generally includes regions preceding ("leaders"; upstream) and following ("trailers"; downstream) of the coding region. A gene may also include intervening, non-coding sequences, referred to as "introns", located between individual coding segments, referred to as "exons". Most genes have an associated promoter region, a regulatory sequence 5' of the transcription initiation codon (there are some genes that do not have an identifiable promoter). The function of a gene may also be regulated by enhancers, operators, and other regulatory elements.

A "recombinant polynucleotide" is a polynucleotide that is not in its native state, e.g., the polynucleotide comprises a nucleotide sequence not found in nature, or the polynucleotide is in a context other than that in which it is naturally found, e.g., separated from nucleotide sequences with which it typically is in proximity in nature, or adjacent (or contiguous with) nucleotide sequences with which it typically is not in proximity. For example, the sequence at issue can be cloned into a vector, or otherwise recombined with one or more additional nucleic acid.

An "isolated polynucleotide" is a polynucleotide whether naturally occurring or recombinant, that is present outside the cell in which it is typically found in nature, whether purified or not. Optionally, an isolated polynucleotide is subject to one or more enrichment or purification procedures, e.g., cell lysis, extraction, centrifugation, precipitation, or the like.

A "polypeptide" is an amino acid sequence comprising a plurality of consecutive polymerized amino acid residues e.g., at least about 15 consecutive polymerized amino acid residues, optionally at least about 30 consecutive polymerized amino acid residues, at least about 50 consecutive polymerized amino acid residues. In many instances, a polypeptide comprises a polymerized amino acid residue sequence that is a transcription factor or a domain or portion or fragment thereof. Additionally, the polypeptide may comprise 1) a localization domain, 2) an activation domain, 3) a repression domain, 4) an oligomerization domain, or 5) a DNA-binding domain, or the like. The polypeptide optionally comprises modified amino acid residues, naturally occurring amino acid residues not encoded by a codon, non-naturally occurring amino acid residues.

"Protein" refers to an amino acid sequence, oligopeptide, peptide, polypeptide or portions thereof whether naturally occurring or synthetic.

"Portion", as used herein, refers to any part of a protein used for any purpose, but especially for the screening of a library of molecules which specifically bind to that portion or for the production of antibodies.

A "recombinant polypeptide" is a polypeptide produced by translation of a recombinant polynucleotide. A "synthetic polypeptide" is a polypeptide created by consecutive polymerization of isolated amino acid residues using methods well known in the art. An "isolated polypeptide," whether a naturally occurring or a recombinant polypeptide, is more enriched in (or out of) a cell than the polypeptide in its natural state in a wild-type cell, e.g., more than about 5% enriched, more than about 10% enriched, or more than about 20%, or more than about 50%, or more, enriched, i.e., alternatively denoted: 105%, 110%, 120%, 150% or more, enriched relative to wild type standardized at 100%. Such an enrichment is not the result of a natural response of a wild-type plant. Alternatively, or additionally, the isolated polypeptide is separated from other cellular components with which it is typically associated, e.g., by any of the various protein purification methods herein.

"Homology" refers to sequence similarity between a reference sequence and at least a fragment of a newly sequenced clone insert or its encoded amino acid sequence.

"Hybridization complex" refers to a complex between two nucleic acid molecules by virtue of the formation of hydrogen bonds between purines and pyrimidines.

"Identity" or "similarity" refers to sequence similarity between two polynucleotide sequences or between two polypeptide sequences, with identity being a more strict comparison. The phrases "percent identity" and "% identity" refer to the percentage of sequence similarity found in a comparison of two or more polynucleotide sequences or two or more polypeptide sequences. "Sequence similarity" refers to the percent similarity in base pair sequence (as determined by any suitable method) between two or more polynucleotide sequences. Two or more sequences can be anywhere from 0-100% similar, or any integer value therebetween. Identity or similarity can be determined by comparing a position in each sequence that may be aligned for purposes of comparison. When a position in the compared sequence is occupied by the same nucleotide base or amino acid, then the molecules are identical at that position. A degree of similarity or identity between polynucleotide sequences is a function of the number of identical or matching nucleotides at positions shared by the polynucleotide sequences. A degree of identity of polypeptide sequences is a function of the number of identical amino acids at positions shared by the polypeptide sequences. A degree of homology or similarity of polypeptide sequences is a function of the number of amino acids at positions shared by the polypeptide sequences.

The term "amino acid consensus motif" refers to the portion or subsequence of a polypeptide sequence that is substantially conserved among the polypeptide transcription factors listed in the Sequence Listing.

"Alignment" refers to a number of DNA or amino acid sequences aligned by lengthwise comparison so that components in common (i.e., nucleotide bases or amino acid residues) may be readily and graphically identified. The number of components in common is related to the homology or identity between the sequences. Alignments may be used to identify "conserved domains" and relatedness within these domains. An alignment may suitably be determined by means of computer programs Known in the art, such as MacVector (1999) (Accelrys, Inc., San Diego, CA).

A "conserved domain" or "conserved region" as used herein refers to a region in heterologous polynucleotide or polypeptide sequences where there is a relatively high degree of sequence identity between the distinct sequences.

With respect to polynucleotides encoding presently disclosed transcription factors, a conserved region is preferably at least 10 base pairs (bp) in length.

A "conserved domain", with respect to presently disclosed polypeptides refers to a domain within a transcription factor family that exhibits a higher degree of sequence homology, such as at least 80% sequence identity, and even more preferably at least 85%, or at least about 86%, or at least about 87%, or at least about 88%, or at least about 90%, or at least about 95%, or at least about 98% amino acid residue sequence identity of a polypeptide of consecutive amino acid residues. A fragment or domain can be referred to as outside a conserved domain, outside a consensus sequence, or outside a consensus DNA-binding site that is known to exist or that exists for a particular transcription factor class, family, or sub-family. In this case, the fragment or domain will not include the exact amino acids of a consensus sequence or consensus DNA-binding site of a transcription factor class, family or sub-family, or the exact amino acids of a particular transcription factor consensus sequence or consensus DNA-binding site. Furthermore, a particular fragment, region, or domain of a polypeptide, or a polynucleotide encoding a polypeptide, can be "outside a conserved domain" if all the amino acids of the fragment, region, or domain fall outside of a defined conserved domain(s) for a polypeptide or protein. Sequences having lesser degrees of identity but comparable biological activity are considered to be equivalents.

As one of ordinary skill in the art recognizes, conserved domains may be identified as regions or domains of identity to a specific consensus sequence (see, for example, Riechmann et al. (2000) *supra).* Thus, by using alignment methods well known in the art, the conserved domains of the plant transcription factors for each of the following may be determined: the AP2 (APETALA2) domain transcription factor family (Riechmann and Meyerowitz (1998) *supra;* the MYB transcription factor family (ENBib; Martin and Paz-Ares (1997) *supra);* the MADS domain transcription factor family (Riechmann and Meyerowitz (1997) *supra;* Immink et al. (2003) *supra);* the WRKY protein family (Ishiguro and Nakamura (1994) *supra);* the ankyrin-repeat protein family (Zhang et al. (1992) *supra);* the zinc finger protein (Z) family (Klug and Schwabe (1995) *supra;* Takatsuji (1998) *supra);* the homeobox (HB) protein family (Buerglin (1994) *supra*); the CAAT-element binding proteins (Forsburg and Guarente (1989) *supra);* the squamosa promoter binding proteins (SPB) (Klein et al. (1996) *supra);* the NAM protein family (Souer et al. (1996) *supra);* the IAA/AUX proteins (Abel et al. (1995) *supra*); the HLH/MYC protein family (Littlewood et al. (1994) *supra);* the DNA-binding protein (DBP) family (Tucker et al. (1994) *supra);* the bZIP family of transcription factors (Foster et al. (1994) *supra*); the Box P-binding protein (the BPF-1) family (da Costa e Silva et al. (1993) *supra*); the high mobility group (HMG) family (Bustin and Reeves (1996) *supra);* the scarecrow (SCR) family (Di Laurenzio et al. (1996) *supra);* the GF14 family (Wu et al. (1997) *supra);* the polycomb (PCOMB) family (Goodrich et al. (1997) *supra*); the teosinte branched (TEO) family (Luo et al. (1996) *supra*); the ABI3 family (Giraudat et al. (1992) *supra*); the triple helix (TH) family (Dehesh et al. (1990) *supra*); the EIL family (Chao et al. (1997) *Cell supra*); the AT-HOOK family (Reeves and Nissen (1990 *supra*); the S1FA family (Zhou et al. (1995) *supra*); the bZIPT2 family (Lu and Ferl (1995) *supra*); the YABBY family (Bowman et al. (1999) *supra*); the PAZ family (Bohmert et al. (1998) *supra*); a family of miscellaneous (MISC) transcription factors including the DPBF family (Kim et al. (1997) *supra*) and the SPF1 family (Ishiguro and Nakamura (1994) *supra*); the GARP family (Hall et al. (1998) *supra*), the TUBBY family (Boggin et al. (1999) *supra*), the heat shock family (Wu (1995 *supra*), the ENBP family (Christiansen et al. (1996) *supra*), the RING-zinc family (Jensen et al. (1998) *supra*), the PDBP family (Janik et al. (1989) *supra*), the PCF family (Cubas et al. (1999) *supra*), the SRS (SHI-related) family (Fridborg et al. (1999) *supra*), the CPP (cysteine-rich polycomb-like) family (Cvitanich et al. (2000) *supra),* the ARF (auxin response factor) family (Ulmasov et al. (1999) *supra),* the SWI/SNF family (Collingwood et al. (1999) *supra*), the ACBF family (Seguin et al. (1997) *supra*), PCGL (CG-1 like) family (da Costa e Silva et al. (1994) *supra*) the ARID family (Vazquez et al. (1999) *supra*), the Jumonji family, (Balciunas et al. (2000) *supra*), the bZIP-NIN family (Schauser et al. (1999) *supra*), the E2F family Kaelin et al. (1992) *supra*) and the GRF-like family (Knaap et al (2000) *supra*).

The conserved domains of SEQ ID NO: 328 is listed in Table 5.

"Complementary" refers to the natural hydrogen bonding by base pairing between purines and pyrimidines. For example, the sequence A-C-G-T (5' -> 3') forms hydrogen bonds with its complements A-C-G-T (5' -> 3') or A-C-G-U (5' -> 3'). Two single-stranded molecules may be considered partially complementary, if only some of the nucleotides bond, or "completely complementary" if all of the nucleotides bond. The degree of complementarity between nucleic acid strands affects the efficiency and strength of the hybridization and amplification reactions. "Fully complementary" refers to the case where bonding occurs between every base pair and its complement in a pair of sequences, and the two sequences have the same number of nucleotides.

The terms "highly stringent" or "highly stringent condition" refer to conditions that permit hybridization of DNA strands whose sequences are highly complementary, wherein these same conditions exclude hybridization of significantly mismatched DNAs. Polynucleotide sequences capable of hybridizing under stringent conditions with the polynucleotides of the present disclosure may be, for example, variants of the disclosed polynucleotide sequences, including allelic or splice variants, or sequences that encode orthologs or paralogs of presently disclosed polypeptides. Nucleic acid hybridization methods are disclosed in detail by Kashima et al. (1985) Nature 313:402-404, and Sambrook et al. (1989) Molecular Cloning: A Laboratory Manual, 2nd Ed., Cold Spring Harbor Laboratory, Cold Spring Harbor, N.Y ("Sambrook"); and by Haymes et al., "Nucleic Acid Hybridization: A Practical Approach", IRL Press, Washington, D.C. (1985), which references are incorporated herein by reference.

In general, stringency is determined by the temperature, ionic strength, and concentration of denaturing agents (e.g., formamide) used in a hybridization and washing procedure (for a more detailed description of establishing and determining stringency, see below). The degree to which two nucleic acids hybridize under various conditions of stringency is correlated with the extent of their similarity. Thus, similar nucleic acid sequences from a variety of sources, such as within a plant's genome (as in the case of paralogs) or from another plant (as in the case of orthologs) that may perform similar functions can be isolated on the basis of their ability to hybridize with known transcription factor sequences. Numerous variations are possible in the conditions and means by which nucleic acid hybridization can be performed to isolate transcription factor sequences having similarity to transcription factor sequences known in the art and are not limited to those explicitly disclosed herein. Such an approach may be used to isolate polynucleotide sequences having various degrees of similarity with disclosed transcription factor sequences, such as, for example, transcription factors having 60% identity, or more preferably greater than about 70% identity, most preferably 72% or greater identity with disclosed transcription factors.

The term "equivalog" describes members of a set of homologous proteins that are conserved with respect to function since their last common ancestor. Related proteins are grouped into equivalog families, and otherwise into protein families with other hierarchically defined homology types. This definition is provided at the Institute for Genomic Research (TIGR) world wide web (www) website, " tigr.org " under the heading "Terms associated with TIGRFAMs".

The term "variant", as used herein, may refer to polynucleotides or polypeptides, that differ from the presently disclosed polynucleotides or polypeptides, respectively, in sequence from each other, and as set forth below.

With regard to polynucleotide variants, differences between presently disclosed polynucleotides and polynucleotide variants are limited so that the nucleotide sequences of the former and the latter are closely similar overall and, in many regions, identical. Due to the degeneracy of the genetic code, differences between the former and latter nucleotide sequences o may be silent (i.e., the amino acids encoded by the polynucleotide are the same, and the variant polynucleotide sequence encodes the same amino acid sequence as the presently disclosed polynucleotide. Variant nucleotide sequences may encode different amino acid sequences, in which case such nucleotide differences will result in amino acid substitutions, additions, deletions, insertions, truncations or fusions with respect to the similar disclosed polynucleotide sequences. These variations result in polynucleotide variants encoding polypeptides that share at least one functional characteristic. The degeneracy of the genetic code also dictates that many different variant polynucleotides can encode identical and/or substantially similar polypeptides in addition to those sequences illustrated in the Sequence Listing.

Also described herein is a variant of a transcription factor nucleic acid listed in the Sequence Listing, that is, one having a sequence that differs from the one of the polynucleotide sequences in the Sequence Listing, or a complementary sequence, that encodes a functionally equivalent polypeptide (i.e., a polypeptide having some degree of equivalent or similar biological activity) but differs in sequence from the sequence in the Sequence Listing, due to degeneracy in the genetic code. Included within this definition are polymorphisms that may or may not be readily detectable using a particular oligonucleotide probe of the polynucleotide encoding polypeptide, and improper or unexpected hybridization to allelic variants, with a locus other than the normal chromosomal locus for the polynucleotide sequence encoding polypeptide.

"Allelic variant" or "polynucleotide allelic variant" refers to any of two or more alternative forms of a gene occupying the same chromosomal locus. Allelic variation arises naturally through mutation, and may result in phenotypic polymorphism within populations. Gene mutations may be "silent" or may encode polypeptides having altered amino acid sequence. "Allelic variant" and "polypeptide allelic variant" may also be used with respect to polypeptides, and in this case the term refer to a polypeptide encoded by an allelic variant of a gene.

"Splice variant" or "polynucleotide splice variant" as used herein refers to alternative forms of RNA transcribed from a gene. Splice variation naturally occurs as a result of alternative sites being spliced within a single transcribed RNA molecule or between separately transcribed RNA molecules, and may result in several different forms of mRNA transcribed from the same gene. This, splice variants may encode polypeptides having different amino acid sequences, which may or may not have similar functions in the organism. "Splice variant" or "polypeptide splice variant" may also refer to a polypeptide encoded by a splice variant of a transcribed mRNA.

As used herein, "polynucleotide variants" may also refer to polynucleotide sequences that encode paralogs and orthologs of the presently disclosed polypeptide sequences. "Polypeptide variants" may refer to polypeptide sequences that are paralogs and orthologs of the presently disclosed polypeptide sequences.

Differences between presently disclosed polypeptides and polypeptide variants are limited so that the sequences of the former and the latter are closely similar overall and, in many regions, identical. Presently disclosed polypeptide sequences and similar polypeptide variants may differ in amino acid sequence by one or more substitutions, additions, deletions, fusions and truncations, which may be present in any combination. These differences may produce silent changes and result in a functionally equivalent transcription factor. Thus, it will be readily appreciated by those of skill in the art, that any of a variety of polynucleotide sequences is capable of encoding the transcription factors and transcription factor homolog polypeptides. A polypeptide sequence variant may have "conservative" changes, wherein a substituted amino acid has similar structural or chemical properties. Deliberate amino acid substitutions may thus be made on the basis of similarity in polarity, charge, solubility, hydrophobicity, hydrophilicity, and/or the amphipathic nature of the residues, as long as the functional or biological activity of the transcription factor is retained. For example, negatively charged amino acids may include aspartic acid and glutamic acid, positively charged amino acids may include lysine and arginine, and amino acids with uncharged polar head groups having similar hydrophilicity values may include leucine, isoleucine, and valine; glycine and alanine; asparagine and glutamine; serine and threonine; and phenylalanine and tyrosine (for more detail on conservative substitutions, see Table 2). More rarely, a variant may have "non-conservative" changes, e.g., replacement of a glycine with a tryptophan. Similar minor variations may also include amino acid deletions or insertions, or both. Related polypeptides may comprise, for example, additions and/or deletions of one or more N-linked or O-linked glycosylation sites, or an addition and/or a deletion of one or more cysteine residues. Guidance in determining which and how many amino acid residues may be substituted, inserted or deleted without abolishing functional or biological activity may be found using computer programs well known in the art, for example, DNASTAR software (see USPN 5,840,544).

"Ligand" refers to any molecule, agent, or compound that will bind specifically to a complementary site on a nucleic acid molecule or protein. Such ligands stabilize or modulate the activity of nucleic acid molecules or proteins and may be composed of at least one of the following: inorganic and organic substances including nucleic acids, proteins, carbohydrates, fats, and lipids.

"Modulates" refers to a change in activity (biological, chemical, or immunological) or lifespan resulting from specific binding between a molecule and either a nucleic acid molecule or a protein.

The term "plant" includes whole plants, shoot vegetative organs/structures (*e.g.*, leaves, stems and tubers), roots, flowers and floral organs/structures (*e.g.*, bracts, sepals, petals, stamens, carpels, anthers and ovules), seed (including embryo, endosperm, and seed coat) and fruit (the mature ovary), plant tissue (*e.g.*, vascular tissue, ground tissue, and the like) and cells (*e.g.*, guard cells, egg cells, and the like), and progeny of same. The class of plants that can be used in the method of the invention is generally as broad as the class of higher and lower plants amenable to transformation techniques, including angiosperms (monocotyledonous and dicotyledonous plants), gymnosperms, ferns, horsetails, psilophytes, lycophytes, bryophytes, and multicellular algae. (See for example, Figure 1, adapted from Daly et al. (2001) Plant Physiol. 127: 1328-1333; Figure 2, adapted from Ku et al. (2000) Proc. Natl. Acad. Sci. 97: 9121-9126; and see also Tudge in The Variety of Life, Oxford University Press, New York, NY (2000) pp. 547-606).

A "transgenic plant" refers to a plant that contains genetic material not found in a wild-type plant of the same species, variety or cultivar. The genetic material may include a transgene, an insertional mutagenesis event (such as by transposon or T-DNA insertional mutagenesis), an activation tagging sequence, a mutated sequence, a homologous recombination event or a sequence modified by chimeraplasty. Typically, the foreign genetic material has been introduced into the plant by human manipulation, but any method can be used as one of skill in the art recognizes.

A transgenic plant may contain an expression vector or cassette. The expression cassette typically comprises a polypeptide-encoding sequence operably linked (i.e., under regulatory control of) to appropriate inducible or constitutive regulatory sequences that allow for the expression of polypeptide. The expression cassette can be introduced into a plant by transformation or by breeding after transformation of a parent plant. A plant refers to a whole plant as well as to a plant part, such as seed, fruit, leaf, or root, plant tissue, plant cells or any other plant material, e.g., a plant explant, as well as to progeny thereof, and to *in vitro* systems that mimic biochemical or cellular components or processes in a cell.

"Control plant" refers to a plant that serves as a standard of comparison for testing the results of a treatment or genetic alteration, or the degree of altered expression of a gene or gene product. Examples of control plants include plants that are untreated, or genetically unaltered (i.e., wild-type).

"Wild type", as used herein, refers to a cell, tissue or plant that has not been genetically modified to knock out or overexpress one or more of the presently disclosed transcription factors. Wild-type cells, tissue or plants may be used as controls to compare levels of expression and the extent and nature of trait modification with cells, tissue or plants in which transcription factor expression is altered or ectopically expressed, e.g., in that it has been knocked out or over-expressed.

"Fragment", with respect to a polynucleotide, refers to a clone or any part of a polynucleotide molecule that retains a usable, functional characteristic. Useful fragments include oligonucleotides and polynucleotides that may be used in hybridization or amplification technologies or in the regulation of replication, transcription or translation. A polynucleotide fragment" refers to any subsequence of a polynucleotide, typically, of at least about 9 consecutive nucleotides, preferably at least about 30 nucleotides, more preferably at least about 50 nucleotides, of any of the sequences provided herein. Exemplary polynucleotide fragments are the first sixty consecutive nucleotides of the transcription factor polynucleotides listed in the Sequence Listing. Exemplary fragments also include fragments that comprise a region that encodes a conserved domain of a transcription factor.

Fragments may also include subsequences of polypeptides and protein molecules, or a subsequence of the polypeptide. Fragments may have uses in that they may have antigenic potential. In some cases, the fragment or domain is a subsequence of the polypeptide which performs at least one biological function of the intact polypeptide in substantially the same manner, or to a similar extent, as does the intact polypeptide. For example, a polypeptide fragment can comprise a recognizable structural motif or functional domain such as a DNA-binding site or domain that binds to a DNA promoter region, an activation domain, or a domain for protein-protein interactions, and may initiate transcription. Fragments can vary in size from as few as 3 amino acids to the full length of the intact polypeptide, but are preferably at least about 30 amino acids in length and more preferably at least about 60 amino acids in length. Exemplary polypeptide fragments are the first twenty consecutive amino acids of a mammalian protein encoded by are the first twenty consecutive amino acids of the transcription factor polypeptides listed in the Sequence Listing. Exemplary fragments also include fragments that comprise a conserved domain of a transcription factor.

The present disclosure also encompasses production of DNA sequences that encode transcription factors and transcription factor derivatives, or fragments thereof, entirely by synthetic chemistry. After production, the synthetic sequence may be inserted into any of the many available expression vectors and cell systems using reagents well known in the art. Moreover, synthetic chemistry may be used to introduce mutations into a sequence encoding transcription factors or any fragment thereof.

"Derivative" refers to the chemical modification of a nucleic acid molecule or amino acid sequence. Chemical modifications can include replacement of hydrogen by an alkyl, acyl, or amino group or glycosylation, pegylation, or any similar process that retains or enhances biological activity or lifespan of the molecule or sequence.

A "trait" refers to a physiological, morphological, biochemical, or physical characteristic of a plant or particular plant material or cell. In some instances, this characteristic is visible to the human eye, such as seed or plant size, or can be measured by biochemical techniques, such as detecting the protein, starch, or oil content of seed or leaves, or by observation of a metabolic or physiological process, e.g, by measuring uptake of carbon dioxide, or by the observation of the expression level of a gene or genes, e.g., by employing Northern analysis, RT-PCR, microarray gene expression assays, or reporter gene expression systems, or by agricultural observations such as stress tolerance, yield, or pathogen tolerance. Any technique can be used to measure the amount of, comparative level of, or difference in any selected chemical compound or macromolecule in the transgenic plants, however.

"Trait modification" refers to a detectable difference in a characteristic in a plant of the present invention ectopically expressing a polynucleotide or polypeptide relative to a plant not doing so, such as a wild-type plant. In some cases, the trait modification can be evaluated quantitatively. For example, the trait modification can entail at least about a 2% increase or decrease in an observed trait (difference), at least a 5% difference, at least about a 10% difference, at least about a 20% difference, at least about a 30%, at least about a 50%, at least about a 70%, or at least about a 100%, or an even greater difference compared with a wild-type plant. It is known that there can be a natural variation in the modified trait. Therefore, the trait modification observed entails a change of the normal distribution of the trait in the plants compared with the distribution observed in wild-type plants.

The term "transcript profile" refers to the expression levels of a set of genes in a cell in a particular state, particularly by comparison with the expression levels of that same set of genes in a cell of the same type in a reference state. For example, the transcript profile of a particular transcription factor in a suspension cell is the expression levels of a set of genes in a cell overexpressing that transcription factor compared with the expression levels of that same set of genes in a suspension cell that has normal levels of that transcription factor. The transcript profile can be presented as a list of those genes whose expression level is significantly different between the two treatments, and the difference ratios.. Differences and similarities between expression levels may also be evaluated and calculated using statistical and clustering methods.

"Ectopic expression or altered expression" in reference to a polynucleotide indicates that the pattern of expression in, e.g., a transgenic plant or plant tissue, is different from the expression pattern in a wild-type plant or a reference plant of the same species. The pattern of expression may also be compared with a reference expression pattern in a wild-type plant of the same species. For example, the polynucleotide or polypeptide is expressed in a cell or tissue type other than a cell or tissue type in which the sequence is expressed in the wild-type plant, or by expression at a time other than at the time the sequence is expressed in the wild-type plant, or by a response to different inducible agents, such as hormones or environmental signals, or at different expression levels (either higher or lower) compared with those found in a wild-type plant. The term also refers to altered expression patterns that are produced by lowering the levels of expression to below the detection level or completely abolishing expression. The resulting expression pattern can be transient or stable, constitutive or inducible. In reference to a polypeptide, the term "ectopic expression or altered expression" further may relate to altered activity levels resulting from the interactions of the polypeptides with exogenous or endogenous modulators or from interactions with factors or as a result of the chemical modification of the polypeptides.

The term "overexpression" as used herein refers to a greater expression level of a gene in a plant, plant cell or plant tissue, compared to expression in a wild-type plant, cell or tissue, at any developmental or temporal stage for the gene. Overexpression can occur when, for example, the genes encoding one or more transcription factors are under the control of a strong expression signal, such as one of the promoters described herein (e.g., the cauliflower mosaic virus 35S transcription initiation region). Overexpression may occur throughout a plant or in specific tissues of the plant, depending on the promoter used, as described below.

Overexpression may take place in plant cells normally lacking expression of polypeptides functionally equivalent or identical to the present transcription factors. Overexpression may also occur in plant cells where endogenous expression of the present transcription factors or functionally equivalent molecules normally occurs, but such normal expression is at a lower level.. Overexpression thus results in a greater than normal production, or "overproduction" of the transcription factor in the plant, cell or tissue.

The term "phase change" refers to a plant's progression from embryo to adult, and, by some definitions, the transition wherein flowering plants gain reproductive competency. It is believed that phase change occurs either after a certain number of cell divisions in the shoot apex of a developing plant, or when the shoot apex achieves a particular distance from the roots. Thus, altering the timing of phase changes may affect a plant's size, which, in turn, may affect yield and biomass.

"Tolerance" results from specific, heritable characteristics of a host plant that allow a pathogen to develop and multiply in the host while the host, either by lacking receptor sites for, or by inactivating or compensating for the irritant secretions of the pathogen, still manages to thrive or, in the case of crop plants, produce a good crop. Tolerant plants are susceptible to the pathogen but are not killed by it and generally show little damage from the pathogen (Agrios (1988) Plant Pathology, 3rd ed. Academic Press, N.Y., p. 129).

"Resistance", also referred to as "true resistance", results when a plant contains one or more genes that make the plant and a potential pathogen more or less incompatible with each other, either because of a lack of chemical recognition between the host and the pathogen, or because the host plant can defend itself against the pathogen by defense mechanisms already present or activated in response to infection (Agrios (1988)) Plant Pathology, 3rd ed. Academic Press, N.Y., p. 125).

A "sample" with respect to a material containing nucleic acid molecules may comprise a bodily fluid; an extract from a cell, chromosome, organelle, or membrane isolated from a cell; genomic DNA, RNA, or cDNA in solution or bound to a substrate; a cell; a tissue; a tissue print; a forensic sample; and the like. In this context "substrate" refers to any rigid or semi-rigid support to which nucleic acid molecules or proteins are bound and includes membranes, filters, chips, slides, wafers, fibers, magnetic or nonmagnetic beads, gels, capillaries or other tubing, plates, polymers, and microparticles with a variety of surface forms including wells, trenches, pins, channels and pores. A substrate may also refer to a reactant in a chemical or biological reaction, or a substance acted upon (e.g., by an enzyme).

"Substantially purified" refers to nucleic acid molecules or proteins that are removed from their natural environment and are isolated or separated, and are at least about 60% free, preferably about 75% free, and most preferably about 90% free, from other components with which they are naturally associated.

Traits That May Be Modified in Overexpressing or Knock-out Plants

Trait modifications of particular interest include those to seed (such as embryo or endosperm), fruit, root, flower, leaf, stem, shoot, seedling or the like, including: enhanced tolerance to environmental conditions including freezing, chilling, heat, drought, water saturation, radiation and ozone; improved tolerance to microbial, fungal or viral diseases; improved tolerance to pest infestations, including insects, nematodes, mollicutes, parasitic higher plants or the like; decreased herbicide sensitivity; improved tolerance of heavy metals or enhanced ability to take up heavy metals; improved growth under poor photoconditions (e.g., low light and/or short day length), or changes in expression levels of genes of interest. Other phenotype that can be modified relate to the production of plant metabolites, such as variations in the production of taxol, tocopherol, tocotrienol, sterols, phytosterols, vitamins, wax monomers, anti-oxidants, amino acids, lignins, cellulose, tannins, prenyllipids (such as chlorophylls and carotenoids), glucosinolates, and terpenoids, enhanced or compositionally altered protein or oil production (especially in seeds), or modified sugar (insoluble or soluble) and/or starch composition. Physical plant characteristics that can be modified include cell development (such as the number of trichomes), fruit and seed size and number, yields of plant parts such as stems, leaves, inflorescences, and roots, the stability of the seeds during storage, characteristics of the seed pod (e.g., susceptibility to shattering), root hair length and quantity, internode distances, or the quality of seed coat. Plant growth characteristics that can be modified include growth rate, germination rate of seeds, vigor of plants and seedlings, leaf and flower senescence, male sterility, apomixis, flowering time, flower abscission, rate of nitrogen uptake, osmotic sensitivity to soluble sugar concentrations, biomass or transpiration characteristics, as well as plant architecture characteristics such as apical dominance, branching patterns, number of organs, organ identity, organ shape or size.

Transcription Factors Modify Expression of Endogenous Genes

Expression of genes that encode transcription factors that modify expression of endogenous genes, polynucleotides, and proteins are well known in the art. In addition, transgenic plants comprising isolated polynucleotides encoding transcription factors may also modify expression of endogenous genes, polynucleotides, and proteins. Examples include Peng et al. (1997, Genes Development 11: 3194-3205) and Peng et al. (1999, Nature, 400: 256-261). In addition, many others have demonstrated that an *Arabidopsis* transcription factor expressed in an exogenous plant species elicits the same or very similar phenotypic response. See, for example, Fu et al. (2001, Plant Cell 13: 1791-1802); Nandi et al. (2000, Curr. Biol. 10: 215-218); Coupland (1995, Nature 377: 482-483); and Weigel and Nilsson (1995, Nature 377: 482-500).

In another example, Mandel et al. (1992, Cell 71-133-143) and Suzuki et al. (2001, Plant J. 28: 409-418) teach that a transcription factor expressed in another plant species elicits the same or very similar phenotypic response of the endogenous sequence, as often predicted in earlier studies of *Arabidopsis* transcription factors in *Arabidopsis* (see Mandel et al. 1992, *supra;* Suzuki et al. 2001, *supra).*

Other examples include Miller et al. (2001, Plant J. 28: 169-179); Kim et al. (2001, Plant J. 25: 247-259); Kyozuka and Shimamoto (2002, Plant Cell Physiol. 43: 130-135); Boss and Thomas (2002, Nature, 416: 847-850); He et al. (2000, Transgenic Res. 9: 223-227); and Robson et al. (2001, Plant J. 28:619-631).

In yet another example, Gilmour et al. (1998, Plant J. 16: 433-442) teach an *Arabidopsis* AP2 transcription factor, CBF1, which, when overexpressed in transgenic plants, increases plant freezing tolerance. Jaglo et al. (2001, Plant Physiol. 127: 910-917) further identified sequences in *Brassica napus* which encode CBF-like genes and that transcripts for these genes accumulated rapidly in response to low temperature. Transcripts encoding CBF-like proteins were also found to accumulate rapidly in response to low temperature in wheat, as well as in tomato. An alignment of the CBF proteins from *Arabidopsis, B. napus,* wheat, rye, and tomato revealed the presence of conserved consecutive amino acid residues, PKK/RPAGRxKFxETRHP and DSAWR, that bracket the AP2/EREBP DNA binding domains of the proteins and distinguish them from other members of the - AP2/EREBP protein family. (See Jaglo et *al. supra.)*

Transcription factors mediate cellular responses and control traits through altered expression of genes containing cis-acting nucleotide sequences that are targets of the introduced transcription factor. It is well appreciated in the Art that the effect of a transcription factor on cellular responses or a cellular trait is determined by the particular genes whose expression is either directly or indirectly (e.g., by a cascade of transcription factor binding events and transcriptional changes) altered by transcription factor binding. In a global analysis of transcription comparing a standard condition with one in which a transcription factor is overexpressed, the resulting transcript profile associated with transcription factor overexpression is related to the trait or cellular process controlled by that transcription factor. For example, the PAP2 gene (and other genes in the MYB family) have been shown to control anthocyanin biosynthesis through regulation of the expression of genes known to be involved in the anthocyanin biosynthetic pathway (Bruce et al. (2000) Plant Cell 12: 65-79; and Borevitz et al. (2000) Plant Cell 12: 2383-2393). Further, global transcript profiles have been used successfully as diagnostic tools for specific cellular states (e.g., cancerous vs. non-cancerous; Bhattacharjee et al. (2001) Proc. Natl. Acad. Sci. USA 98: 13790-13795; and Xu et al. (2001) Proc Natl Acad Sci, USA 98: 15089-15094). Consequently, it is evident to one skilled in the art that similarity of transcript profile upon overexpression of different transcription factors would indicate similarity of transcription factor function.

Polypeptides and Polynucleotides

Described herein among other things, transcription factors (TFs), and transcription factor homolog polypeptides, and isolated or recombinant polynucleotides encoding the polypeptides, or novel sequence variant polypeptides or polynucleotides encoding novel variants of transcription factors derived from the specific sequences provided here. These polypeptides and polynucleotides may be employed to modify a plant's characteristics.

Exemplary polynucleotides encoding the polypeptides were identified in the *Arabidopsis thaliana* GenBank database using publicly available sequence analysis programs and parameters. Sequences initially identified were then further characterized to identify sequences comprising specified sequence strings corresponding to sequence motifs present in families of known transcription factors. In addition, further exemplary polynucleotides encoding the polypeptides were identified in the plant GenBank database using publicly available sequence analysis programs and parameters. Sequences initially identified were then further characterized to identify sequences comprising specified sequence strings corresponding to sequence motifs present in families of known transcription factors. Polynucleotide sequences meeting such criteria were confirmed as transcription factors.

Additional polynucleotides were identified by screening *Arabidopsis thaliana* and/or other plant cDNA libraries with probes corresponding to known transcription factors under low stringency hybridization conditions. Additional sequences, including full length coding sequences were subsequently recovered by the rapid amplification of cDNA ends (RACE) procedure, using a commercially available kit according to the manufacturer's instructions. Where necessary, multiple rounds of RACE are performed to isolate 5' and 3' ends. The full-length cDNA was then recovered by a routine end-to-end polymerase chain reaction (PCR) using primers specific to the isolated 5' and 3' ends. Exemplary sequences are provided in the Sequence Listing.

The polynucleotides can be or were ectopically expressed in overexpressor or knockout plants and the changes in the characteristic(s) or trait(s) of the plants observed. Therefore, the polynucleotides and polypeptides can be employed to improve the characteristics of plants.

The polynucleotides can be or were ectopically expressed in overexpressor plant cells and the changes in the expression levels of a number of genes, polynucleotides, and/or proteins of the plant cells observed. Therefore, the polynucleotides and polypeptides can be employed to change expression levels of a genes, polynucleotides, and/or proteins of plants.

Producing Polypeptides

The polynucleotides include sequences that encode transcription factors and transcription factor homolog polypeptides and sequences complementary thereto, as well as unique fragments of coding sequence, or sequence complementary thereto. Such polynucleotides can be, e.g., DNA or RNA, e.g., mRNA, cRNA, synthetic RNA, genomic DNA, cDNA synthetic DNA, oligonucleotides, etc. The polynucleotides are either double-stranded or single-stranded, and include either, or both sense (i.e., coding) sequences and antisense (i.e., non-coding, complementary) sequences. The polynucleotides include the coding sequence of a transcription factor, or transcription factor homolog polypeptide, in isolation, in combination with additional coding sequences (e.g., a purification tag, a localization signal, as a fusion-protein, as a pre-protein, or the like), in combination with non-coding sequences (e.g., introns or inteins, regulatory elements such as promoters, enhancers, terminators, and the like), and/or in a vector or host environment in which the polynucleotide encoding a transcription factor or transcription factor homolog polypeptide is an endogenous or exogenous gene.

A variety of methods exist for producing the polynucleotides described herein. Procedures for identifying and isolating DNA clones are well known to those of skill in the art, and are described in, e.g., Berger and Kimmel, Guide to Molecular Cloning Techniques, Methods in Enzymology, vol. 152 Academic Press, Inc., San Diego, CA ("Berger");

Sambrook et al. Molecular Cloning - A Laboratory Manual (2nd Ed.), Vol. 1-3, Cold Spring Harbor Laboratory, Cold Spring Harbor, New York, 1989 ("Sambrook") and Current Protocols in Molecular Biology, Ausubel et al. eds., Current Protocols, a joint venture between Greene Publishing Associates, Inc. and John Wiley & Sons, Inc., (supplemented through 2000) ("Ausubel").

Alternatively, polynucleotides can be produced by a variety of in vitro amplification methods adapted to the present disclosure by appropriate selection of specific or degenerate primers. Examples of protocols sufficient to direct persons of skill through in vitro amplification methods, including the polymerase chain reaction (PCR) the ligase chain reaction (LCR), Qbeta-replicase amplification and other RNA polymerase mediated techniques (e.g., NASBA), e.g., for the production of the homologous nucleic acids are found in Berger (supra), Sambrook (supra), and Ausubel (supra), as well as Mullis et al. (1987) PCR Protocols A Guide to Methods and Applications (Innis et al. eds) Academic Press Inc. San Diego, CA (1990) (Innis). Improved methods for cloning in vitro amplified nucleic acids are described in Wallace et al. US Pat. No. 5,426,039. Improved methods for amplifying large nucleic acids by PCR are summarized in Cheng et al. (1994) Nature 369: 684-685 and the references cited therein, in which PCR amplicons of up to 40kb are generated. One of skill will appreciate that essentially any RNA can be converted into a double stranded DNA suitable for restriction digestion, PCR expansion and sequencing using reverse transcriptase and a polymerase. See, e.g., Ausubel, Sambrook and Berger, all *supra.*

Alternatively, polynucleotides and oligonucleotides can be assembled from fragments produced by solid-phase synthesis methods. Typically, fragments of up to approximately 100 bases are individually synthesized and then enzymatically or chemically ligated to produce a desired sequence, e.g., a polynucleotide encoding all or part of a transcription factor. For example, chemical synthesis using the phosphoramidite method is described, e.g., by Beaucage et al. (1981) Tetrahedron Letters 22: 1859-1869; and Matthes et al. (1984) EMBO J. 3: 801-805. According to such methods, oligonucleotides are synthesized, purified, annealed to their complementary strand, ligated and then optionally cloned into suitable vectors. And if so desired, the polynucleotides and polypeptides can be custom ordered from any of a number of commercial suppliers.

Homologous Sequences

Sequences homologous to SEQ ID NO:328, i.e. those that have at least 95% amino acid sequence identity over the entire length of SEQ ID NO:328 derived from *Arabidopsis thaliana* or from other plants of choice, may also be used in the plants, methods and uses of the invention.

Homologous sequences can be derived from any plant including monocots and dicots and in particular agriculturally important plant species, including but not limited to, crops such as soybean, wheat, corn (maize), potato, cotton, rice, rape, oilseed rape (including canola), sunflower, alfalfa, clover, sugarcane, and turf; or fruits and vegetables, such as banana, blackberry, blueberry, strawberry, and raspberry, cantaloupe, carrot, cauliflower, coffee, cucumber, eggplant, grapes, honeydew, lettuce, mango, melon, onion, papaya, peas, peppers, pineapple, pumpkin, spinach, squash, sweet corn, tobacco, tomato, tomatillo, watermelon, rosaceous fruits (such as apple, peach, pear, cherry and plum) and vegetable brassicas (such as broccoli, cabbage, cauliflower, Brussels sprouts, and kohlrabi). Other crops, including fruits and vegetables, whose phenotype can be changed and which comprise homologous sequences include barley; rye; millet; sorghum; currant; avocado; citrus fruits such as oranges, lemons, grapefruit and tangerines, artichoke, cherries; nuts such as the walnut and peanut; endive; leek; roots such as arrowroot, beet, cassava, turnip, radish, yam, and sweet potato; and beans. The homologous sequences may also be derived from woody species, such pine, poplar and eucalyptus, or mint or other labiates. In addition, homologous sequences may be derived from plants that are evolutionarily-related to crop plants, but which may not have yet been used as crop plants. Examples include deadly nightshade *(Atropa belladona),* related to tomato; jimson weed (Datura strommium), related to peyote; and teosinte *(Zea* species), related to corn (maize).

Orthologs and Paralogs

Homologous sequences as described above can comprise orthologous or paralogous sequences. Several different methods are known by those of skill in the art for identifying and defining these functionally homologous sequences. Three general methods for defining orthologs and paralogs are described; an ortholog, paralog or homolog may be identified by one or more of the methods described below.

Orthologs and paralogs are evolutionarily related genes that have similar sequence and similar functions. Orthologs are structurally related genes in different species that are derived by a speciation event. Paralogs are structurally related genes within a single species that are derived by a duplication event.

Within a single plant species, gene duplication may cause two copies of a particular gene, giving rise to two or more genes with similar sequence and often similar function known as paralogs. A paralog is therefore a similar gene formed by duplication within the same species. Paralogs typically cluster together or in the same clade (a group of similar genes) when a gene family phylogeny is analyzed using programs such as CLUSTAL (Thompson et al. (1994) Nucleic Acids Res. 22: 4673-4680; Higgins et al. (1996) Methods Enzymol. 266: 383-402). Groups of similar genes can also be identified with pair-wise BLAST analysis (Feng and Doolittle (1987) J. Mol. Evol. 25: 351-360). For example, a clade of very similar MADS domain transcription factors from *Arabidopsis* all share a common function in flowering time (Ratcliffe et al. (2001) Plant Physiol. 126: 122-132), and a group of very similar AP2 domain transcription factors from *Arabidopsis* are involved in tolerance of plants to freezing (Gilmour et al. (1998) Plant J. 16: 433-442). Analysis of groups of similar genes with similar function that fall within one clade can yield sub-sequences that are particular to the clade. These sub-sequences, known as consensus sequences, can not only be used to define the sequences within each clade, but define the functions of these genes; genes within a clade may contain paralogous sequences, or orthologous sequences that share the same function (see also, for example, Mount (2001), in Bioinformatics: Sequences and Genome Analysis Cold Spring Harbor Laboratory Press, Cold Spring Harbor, New York, page 543.)

Speciation, the production of new species from a parental species, can also give rise to two or more genes with similar sequence and similar function. These genes, termed orthologs, often have an identical function within their host plants and are often interchangeable between species without losing function. Because plants have common ancestors, many genes in any plant species will have a corresponding orthologous gene in another plant species. Once a phylogenic tree for a gene family of one species has been constructed using a program such as CLUSTAL (Thompson et al. (1994) Nucleic Acids Res. 22: 4673-4680; Higgins et al. (1996) *supra)* potential orthologous sequences can be placed into the phylogenetic tree and their relationship to genes from the species of interest can be determined. Orthologous sequences can also be identified by a reciprocal BLAST strategy. Once an orthologous sequence has been identified, the function of the ortholog can be deduced from the identified function of the reference sequence.

Transcription factor gene sequences are conserved across diverse eukaryotic species lines (Goodrich et al. (1993) Cell 75: 519-530; Lin et al. (1991) Nature 353: 569-571; Sadowski et al. (1988) Nature 335: 563-564). Plants are no exception to this observation; diverse plant species possess transcription factors that have similar sequences and functions.

Orthologous genes from different organisms have highly conserved functions, and very often essentially identical functions (Lee et al. (2002) Genome Res. 12: 493-502; Remm et al. (2001) J. Mol. Biol. 314: 1041-1052). Paralogous genes, which have diverged through gene duplication, may retain similar functions of the encoded proteins. In such cases, paralogs can be used interchangeably with respect to certain embodiments of the instant invention (for example, transgenic expression of a coding sequence). An example of such highly related paralogs is the CBF family, with three well-defined members in *Arabidopsis* and at least one ortholog in *Brassica napus* (SEQ ID NOs: 2238, 2240, 2242, and 2244, respectively), all of which control pathways involved in both freezing and drought stress (Gilmour et al. (1998) Plant J. 16: 433-442; Jaglo et al. (1998) Plant Physiol. 127: 910-917).

The following references represent a small sampling of the many studies that demonstrate that conserved transcription factor genes from diverse species are likely to function similarly (i.e., regulate similar target sequences and control the same traits), and that transcription factors may be transformed into diverse species to confer or improve traits.

(1) The *Arabidopsis* NPR1 gene regulates systemic acquired resistance (SAR); over-expression of NPR1 leads to enhanced resistance in *Arabidopsis.* When either *Arabidopsis* NPR1 or the rice NPR1 ortholog was overexpressed in rice (which, as a monocot, is diverse from *Arabidopsis),* challenge with the rice bacterial blight pathogen *Xanthomonas oryzae* pv. Oryzae, the transgenic plants displayed enhanced resistance (Chern et al. (2001) Plant J. 27: 101-113). NPR1 acts through activation of expression of transcription factor genes, such as TGA2 (Fan and Dong (2002) Plant Cell 14: 1377-1389).

(2) E2F genes are involved in transcription of plant genes for proliferating cell nuclear antigen (PCNA). Plant E2Fs share a high degree of similarity in amino acid sequence between monocots and dicots, and are even similar to the conserved domains of the animal E2Fs. Such conservation indicates a functional similarity between plant and animal E2Fs. E2F transcription factors that regulate meristem development act through common cis-elements, and regulate related (PCNA) genes (Kosugi and Ohashi, (2002) Plant J. 29: 45-59).

(3) The ABI5 gene (ABA insensitive 5) encodes a basic leucine zipper factor required for ABA response in the seed and vegetative tissues. Co-transformation experiments with ABI5 cDNA constructs in rice protoplasts resulted in specific transactivation of the ABA-inducible wheat, *Arabidopsis,* bean, and barley promoters. These results demonstrate that sequentially similar ABI5 transcription factors are key targets of a conserved ABA signaling pathway in diverse plants. (Gampala et al. (2001) J. Biol. Chem. 277: 1689-1694).

(4) Sequences of three *Arabidopsis* GAMYB-like genes were obtained on the basis of sequence similarity to GAMYB genes from barley, rice, and *L. temulentum.* These three *Arabadopsis* genes were determined to encode transcription factors (AtMYB33, AtMYB65, and AtMYB101) and could substitute for a barley GAMYB and control alpha-amylase expression (Gocal et al. (2001) Plant Physiol. 127: 1682-1693).

(5) The floral control gene LEAFY from *Arabidopsis* can dramatically accelerate flowering in numerous dictoyledonous plants. Constitutive expression *of Arabidopsis* LEAFY also caused early flowering in transgenic rice (a monocot), with a heading date that was 26-34 days earlier than that of wild-type plants. These observations indicate that floral regulatory genes from *Arabidopsis* are useful tools for heading date improvement in cereal crops (He et al. (2000) Transgenic Res. 9: 223-227).

(6) Bioactive gibberellins (GAs) are essential endogenous regulators of plant growth. GA signaling tends to be conserved across the plant kingdom. GA signaling is mediated via GAI, a nuclear member of the GRAS family of plant transcription factors. *Arabidopsis* GAI has been shown to function in rice to inhibit gibberellin response pathways (Fu et al. (2001) Plant Cell 13: 1791-1802).

(7) The *Arabidopsis* gene SUPERMAN (SUP), encodes a putative transcription factor that maintains the boundary between stamens and carpels. By over-expressing *Arabidopsis* SUP in rice, the effect of the gene's presence on whorl boundaries was shown to be conserved. This demonstrated that SUP is a conserved regulator of floral whorl boundaries and affects cell proliferation (Nandi et al. (2000) Curr. Biol. 10: 215-218).

(8) Maize, petunia and *Arabidopsis* myb transcription factors that regulate flavonoid biosynthesis are very genetically similar and affect the same trait in their native species, therefore sequence and function of these myb transcription factors correlate with each other in these diverse species (Borevitz et al. (2000) Plant Cell 12: 2383-2394).

(9) Wheat reduced height-1 (Rht-B 1/Rht-D1) and maize dwarf-8 (d8) genes are orthologs of the *Arabidopsis* gibberellin insensitive (GAI) gene. Both of these genes have been used to produce dwarf grain varieties that have improved grain yield. These genes encode proteins that resemble nuclear transcription factors and contain an SH2-like domain, indicating that phosphotyrosine may participate in gibberellin signaling. Transgenic rice plants containing a mutant GAI allele from *Arabidopsis* have been shown to produce reduced responses to gibberellin and are dwarfed, indicating that mutant GAI orthologs could be used to increase yield in a wide range of crop species (Peng et al. (1999) Nature 400: 256-261).

At the nucleotide level, the sequences will typically share at least about 40% nucleotide sequence identity, preferably at least about 50%, about 60%, about 70% or about 80% sequence identity, and more preferably about 85%, about 90%, about 95% or about 97% or more sequence identity to one or more of the listed sequences, or to a listed sequence but excluding or outside a known consensus sequence or consensus DNA-binding site, or outside one or all conserved domain. The degeneracy of the genetic code enables major variations in the nucleotide sequence of a polynucleotide while maintaining the amino acid sequence of the encoded protein. Conserved domains within a transcription factor family may exhibit a higher degree of sequence homology, such as at least at least 80% sequence identity, and more preferably at least 85%, or at least about 86%, or at least about 87%, or at least about 88%, or at least about 90%, or at least about 95%, or at least about 98% sequence identity. Transcription factors that are homologous to the listed sequences should share at least 95% amino acid sequence identity over the entire length of the polypeptide or the homolog.

Percent identity can be determined electronically, e.g., by using the MEGALIGN program (DNASTAR, Inc. Madison, Wis.). The MEGALIGN program can create alignments between two or more sequences according to different methods, for example, the clustal method. (See, for example, Higgins and Sharp (1988) Gene 73: 237-244.) The clustal algorithm groups sequences into clusters by examining the distances between all pairs. The clusters are aligned pairwise and then in groups. Other alignment algorithms or programs may be used, including FASTA, BLAST, or ENTREZ, FASTA and BLAST, and which may be used to calculate percent similarity. These are available as a part of the GCG sequence analysis package (University of Wisconsin, Madison, Wis.), and can be used with or without default settings. ENTREZ is available through the National Center for Biotechnology Information. In one embodiment, the percent identity of two sequences can be determined by the GCG program with a gap weight of 1, e.g., each amino acid gap is weighted as if it were a single amino acid or nucleotide mismatch between the two sequences (see USPN 6,262,333).

Other techniques for alignment are described in Methods in Enzymology, vol. 266, Computer Methods for Macromolecular Sequence Analysis (1996), ed. Doolittle, Academic Press, Inc., San Diego, Calif., USA. Preferably, an alignment program that permits gaps in the sequence is utilized to align the sequences. The Smith-Waterman is one type of algorithm that permits gaps in sequence alignments (see Shpaer (1997) Methods Mol. Biol. 70: 173-187). Also, the GAP program using the Needleman and Wunsch alignment method can be utilized to align sequences. An alternative search strategy uses MPSRCH software, which runs on a MASPAR computer. MPSRCH uses a Smith-Waterman algorithm to score sequences on a massively parallel computer. This approach improves ability to pick up distantly related matches, and is especially tolerant of small gaps and nucleotide sequence errors. Nucleic acid-encoded amino acid sequences can be used to search both protein and DNA databases.

The percentage similarity between two polypeptide sequences, e.g., sequence A and sequence B, is calculated by dividing the length of sequence A, minus the number of gap residues in sequence A, minus the number of gap residues in sequence B, into the sum of the residue matches between sequence A and sequence B, times one hundred. Gaps of low or of no similarity between the two amino acid sequences are not included in determining percentage similarity. Percent identity between polynucleotide sequences can also be counted or calculated by other methods known in the art, e.g., the Jotun Hein method. (See, e.g., Hein (1990) Methods Enzymol. 183: 626-645.) Identity between sequences can also be determined by other methods known in the art, e.g., by varying hybridization conditions (see US Patent Application No. 20010010913).

The percent identity between two conserved domains of a transcription factor DNA-binding domain consensus polypeptide sequence can be as low as 16%, as exemplified in the case of GATA1 family of eukaryotic $Cys_2/Cys_2$-type zinc finger transcription factors. The DNA-binding domain consensus polypeptide sequence of the GATA1 family is $CX_2CX_{17}CX_2C$, where X is any amino acid residue. (See, for example, Takatsuji, supra.) Other examples of such conserved consensus polypeptide sequences with low overall percent sequence identity are well known to those of skill in the art.

Thus, described herein are methods for identifying a sequence similar or paralogous or orthologous or homologous to one or more polynucleotides as noted herein, or one or more target polypeptides encoded by the polynucleotides, or otherwise noted herein and may include linking or associating a given plant phenotype or gene function with a sequence. In the methods, a sequence database is provided (locally or across an internet or intranet) and a query is made against the sequence database using the relevant sequences herein and associated plant phenotypes or gene functions.

In addition, one or more polynucleotide sequences or one or more polypeptides encoded by the polynucleotide sequences may be used to search against a BLOCKS (Bairoch et al. (1997) Nucleic Acids Res. 25: 217-221), PFAM, and other databases which contain previously identified and annotated motifs, sequences and gene functions. Methods that search for primary sequence patterns with secondary structure gap penalties (Smith et al. (1992) Protein Engineering 5: 35-51) as well as algorithms such as Basic Local Alignment Search Tool (BLAST; Altschul (1993) J. Mol. Evol. 36: 290-300; Altschul et al. (1990) *supra*), BLOCKS (Henikoff and Henikoff (1991) Nucleic Acids Res. 19: 6565-6572), Hidden Markov Models (HMM; Eddy (1996) Curr. Opin. Str. Biol. 6: 361-365; Sonnhammer et al. (1997) Proteins 28: 405-420), and the like, can be used to manipulate and analyze polynucleotide and polypeptide sequences encoded by polynucleotides. These databases, algorithms and other methods are well known in the art and are described in Ausubel et al. (1997; Short Protocols in Molecular Biology, John Wiley & Sons, New York, NY, unit 7.7) and in Meyers (1995; Molecular Biology and Biotechnology, Wiley VCH, New York, NY, p 856-853).

A further method for identifying or confirming that specific homologous sequences control the same function is by comparison of the transcript profile(s) obtained upon overexpression or knockout of two or more related transcription factors. Since transcript profiles are diagnostic for specific cellular states, one skilled in the art will appreciate that genes that have a highly similar transcript profile (e.g., with greater than 50% regulated transcripts in common, more preferably with greater than 70% regulated transcripts in common, most preferably with greater than 90% regulated transcripts in common) will have highly similar functions. Fowler et al. (2002) Plant Cell 14: 1675-79) have shown that three paralogous AP2 family genes (CBF1, CBF2 and CBF3), each of which is induced upon cold treatment, and each of which can condition improved freezing tolerance, have highly similar transcript profiles. Once a transcription factor has been shown to provide a specific function, its transcript profile becomes a diagnostic tool to determine whether putative paralogs or orthologs have the same function.

Furthermore, methods using manual alignment of sequences similar or homologous to one or more polynucleotide sequences or one or more polypeptides encoded by the polynucleotide sequences may be used to identify regions of similarity and conserved domains. Such manual methods are well-known of those of skill in the art and can include, for example, comparisons of tertiary structure between a polypeptide sequence encoded by a polynucleotide which comprises a known function with a polypeptide sequence encoded by a polynucleotide sequence which has a function not yet determined. Such examples of tertiary structure may comprise predicted alpha helices, beta-sheets, amphipathic helices, leucine zipper motifs, zinc finger motifs, proline-rich regions, cysteine repeat motifs, and the like.

Orthologs and paralogs of presently disclosed transcription factors may be cloned using compositions provided by the present disclosure according to methods well known in the art. cDNAs can be cloned using mRNA from a plant cell or tissue that expresses one of the present transcription factors. Appropriate mRNA sources may be identified by interrogating Northern blots with probes designed from the present transcription factor sequences, after which a library is prepared from the mRNA obtained from a positive cell or tissue. Transcription factor-encoding cDNA is then isolated using, for example, PCR, using primers designed from a presently disclosed transcription factor gene sequence, or by probing with a partial or complete cDNA or with one or more sets of degenerate probes based on the disclosed sequences. The cDNA library may be used to transform plant cells. Expression of the cDNAs of interest is detected using, for example, methods disclosed herein such as microarrays, Northern blots, quantitative PCR, or any other technique for monitoring changes in expression. Genomic clones may be isolated using similar techniques to those.

Identifying_Polynucleotides or Nucleic Acids by Hybridization

Polynucleotides homologous to the sequences illustrated in the Sequence Listing and tables can be identified, e.g., by hybridization to each other under stringent or under highly stringent conditions. Single stranded polynucleotides hybridize when they associate based on a variety of well characterized physical-chemical forces, such as hydrogen bonding, solvent exclusion, base stacking and the like. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number thereof), as described in more detail in the references cited above.

Encompassed by the disclosure are polynucleotide sequences that are capable of hybridizing to the claimed polynucleotide sequences, including any of the transcription factor polynucleotides within the Sequence Listing, and fragments thereof under various conditions of stringency (See, for example, Wahl and Berger (1987) Methods Enzymol. 152: 399-407; and Kimmel (1987) Methods Enzymol. 152: 507-511). In addition to the nucleotide sequences listed in Tables 4-9, full length cDNA, orthologs, and paralogs of the present nucleotide sequences may be identified and isolated using well-known methods. The cDNA libraries, orthologs, and paralogs of the present nucleotide sequences may be screened using hybridization methods to determine their utility as hybridization target or amplification probes.

With regard to hybridization, conditions that are highly stringent, and means for achieving them, are well known in the art. See, for example, Sambrook et al. (1989) "Molecular Cloning: A Laboratory Manual" (2nd ed., Cold Spring Harbor Laboratory); Berger and Kimmel, eds., (1987) "Guide to Molecular Cloning Techniques", In Methods in Enzymology:152: 467-469; and Anderson and Young (1985) "Quantitative Filter Hybridisation." In: Hames and Higgins, ed., Nucleic Acid Hybridisation, A Practical Approach. Oxford, IRL Press, 73-111.

Stability of DNA duplexes is affected by such factors as base composition, length, and degree of base pair mismatch. Hybridization conditions may be adjusted to allow DNAs of different sequence relatedness to hybridize. The melting temperature ($T_m$) is defined as the temperature when 50% of the duplex molecules have dissociated into their constituent single strands. The melting temperature of a perfectly matched duplex, where the hybridization buffer contains formamide as a denaturing agent, may be estimated by the following equations:

(I) DNA-DNA:

$$T_m(^\circ C)=81.5+16.6(\log [Na+])+0.41(\%\ G+C)-0.62(\%\ formamide)-500/L$$

(II) DNA-RNA:

$$T_m(^\circ C)=79.8+18.5(\log [Na+])+0.58(\%\ G+C)+0.12(\%G+C)^2-0.5(\%\ formamide)-820/L$$

(III) RNA-RNA:

$$T_m(^\circ C)=79.8+18.5(\log [Na+])+0.58(\%\ G+C)+0.12(\%G+C)^2-0.35(\%\ formamide)-820/L$$

where L is the length of the duplex formed, [Na+] is the molar concentration of the sodium ion in the hybridization or washing solution, and % G+C is the percentage of (guanine+cytosine) bases in the hybrid. For imperfectly matched hybrids, approximately 1° C is required to reduce the melting temperature for each 1% mismatch.

Hybridization experiments are generally conducted in a buffer of pH between 6.8 to 7.4, although the rate of hybridization is nearly independent of pH at ionic strengths likely to be used in the hybridization buffer (Anderson et al. (1985) *supra*). In addition, one or more of the following may be used to reduce non-specific hybridization: sonicated salmon sperm DNA or another non-complementary DNA, bovine serum albumin, sodium pyrophosphate, sodium dodecylsulfate (SDS), polyvinylpyrrolidone, ficoll and Denhardt's solution. Dextran sulfate and polyethylene glycol 6000 act to exclude DNA from solution, thus raising the effective probe DNA concentration and the hybridization signal within a given unit of time. In some instances, conditions of even greater stringency may be desirable or required to reduce non-specific and/or background hybridization. These conditions may be created with the use of higher temperature, lower ionic strength and higher concentration of a denaturing agent such as formamide.

Stringency conditions can be adjusted to screen for moderately similar fragments such as homologous sequences from distantly related organisms, or to highly similar fragments such as genes that duplicate functional enzymes from closely related organisms. The stringency can be adjusted either during the hybridization step or in the post-hybridization washes. Salt concentration, formamide concentration, hybridization temperature and probe lengths are variables that can be used to alter stringency (as described by the formula above). As a general guidelines high stringency is typically performed at $T_m$-5° C to $T_m$-20° C, moderate stringency at $T_m$-20° C to $T_m$-35° C and low stringency at $T_m$-35° C to $T_m$-50°C for duplex >150 base pairs. Hybridization may be performed at low to moderate stringency (25-50° C below $T_m$), followed by post-hybridization washes at increasing stringencies. Maximum rates of hybridization in solution are determined empirically to occur at $T_m$-25° C for DNA-DNA duplex and $T_m$-15° C for RNA-DNA duplex. Optionally, the degree of dissociation may be assessed after each wash step to determine the need for subsequent, higher stringency wash steps.

High stringency conditions may be used to select for nucleic acid sequences with high degrees of identity to the disclosed sequences. An example of stringent hybridization conditions obtained in a filter-based method such as a Southern or northern blot for hybridization of complementary nucleic acids that have more than 100 complementary residues is about 5°C to 20°C lower than the thermal melting point ($T_m$) for the specific sequence at a defined ionic strength and pH. Conditions used for hybridization may include about 0.02 M to about 0.15 M sodium chloride, about 0.5% to about 5% casein, about 0.02% SDS or about 0.1% N-laurylsarcosine, about 0.001 M to about 0.03 M sodium citrate, at hybridization temperatures between about 50° C and about 70° C. More preferably, high stringency conditions are about 0.02 M sodium chloride, about 0.5% casein, about 0.02% SDS, about 0.001 M sodium citrate, at a temperature of about 50° C. Nucleic acid molecules that hybridize under stringent conditions will typically hybridize to a probe based on either the entire DNA molecule or selected portions, e.g., to a unique subsequence, of the DNA.

Stringent salt concentration will ordinarily be less than about 750 mM NaCl and 75 mM trisodium citrate. Increasingly stringent conditions may be obtained with less than about 500 mM NaCl and 50 mM trisodium citrate, to even greater stringency with less than about 250 mM NaCl and 25 mM trisodium citrate. Low stringency hybridization can be obtained in the absence of organic solvent, e.g., formamide, whereas high stringency hybridization may be obtained in the presence of at least about 35% formamide, and more preferably at least about 50% formamide. Stringent temperature conditions will ordinarily include temperatures of at least about 30° C, more preferably of at least about 37° C, and most preferably of at least about 42° C with formamide present. Varying additional parameters, such as hybridization time, the concentration of detergent, e.g., sodium dodecyl sulfate (SDS) and ionic strength, are well known to those skilled in the art. Various levels of stringency are accomplished by combining these various conditions as needed.

The washing steps that follow hybridization may also vary in stringency; the post-hybridization wash steps primarily determine hybridization specificity, with the most critical factors being temperature and the ionic strength of the final wash solution. Wash stringency can be increased by decreasing salt concentration or by increasing temperature. Stringent salt concentration for the wash steps will preferably be less than about 30 mM NaCl and 3 mM trisodium citrate, and most preferably less than about 15 mM NaCl and 1.5 mM trisodium citrate.

Thus, hybridization and wash conditions that may be used to bind and remove polynucleotides with less than the desired homology to the nucleic acid sequences or their complements that encode the present transcription factors include, for example:

6X SSC at 65° C;
50% formamide, 4X SSC at 42° C; or
0.5X SSC, 0.1% SDS at 65° C;

with, for example, two wash steps of 10 - 30 minutes each.. Useful variations on these conditions will be readily apparent to those skilled in the art.

A person of skill in the art would not expect substantial variation among polynucleotide species encompassed within the scope of the present disclosure because the highly stringent conditions set forth in the above formulae yield structurally similar polynucleotides.

If desired, one may employ wash steps of even greater stringency, including about 0.2x SSC, 0.1% SDS at 65° C and washing twice, each wash step being about 30 min, or about 0.1 x SSC, 0.1% SDS at 65° C and washing twice for 30 min. The temperature for the wash solutions will ordinarily be at least about 25° C, and for greater stringency at least about 42° C. Hybridization stringency may be increased further by using the same conditions as in the hybridization steps, with the wash temperature raised about 3° C to about 5° C, and stringency may be increased even further by using the same conditions except the wash temperature is raised about 6° C to about 9° C. For identification of less closely related homologs, wash steps may be performed at a lower temperature, e.g., 50°C.

An example of a low stringency wash step employs a solution and conditions of at least 25° C in 30 mM NaCl, 3 mM trisodium citrate, and 0.1% SDS over 30 min. Greater stringency may be obtained at 42° C in 15 mM NaCl, with 1.5 mM trisodium citrate, and 0.1 % SDS over 30 min. Even higher stringency wash conditions are obtained at 65° C -68° C in a solution of 15 mM NaCl, 1.5 mM trisodium citrate, and 0.1% SDS. Wash procedures will generally employ at least two final wash steps. Additional variations on these conditions will be readily apparent to those skilled in the art (see, for example, US Patent Application No. 20010010913).

Stringency conditions can be selected such that an oligonucleotide that is perfectly complementary to the coding oligonucleotide hybridizes to the coding oligonucleotide with at least about a 5-10x higher signal to noise ratio than the ratio for hybridization of the perfectly complementary oligonucleotide to a nucleic acid encoding a transcription factor known as of the filing date of the application. It may be desirable to select conditions for a particular assay such that a higher signal to noise ratio, that is, about 15x or more, is obtained. Accordingly, a subject nucleic acid will hybridize to a unique coding oligonucleotide with at least a 2x or greater signal to noise ratio as compared to hybridization of the coding oligonucleotide to a nucleic acid encoding known polypeptide. The particular signal will depend on the label used in the relevant assay, e.g., a fluorescent label, a colorimetric label, a radioactive label, or the like. Labeled hybridization or PCR probes for detecting related polynucleotide sequences may be produced by oligolabeling, nick translation, end-labeling, or PCR amplification using a labeled nucleotide.

Encompassed by the disclosure are polynucleotide sequences encoding polypeptides capable of regulating transcription, said polynucleotide sequences being capable of hybridizing to the claimed polynucleotide sequences, including those listed in the Sequence Listing, or polynucleotides that encode the polypeptides listed in the Sequence Listing, and specifically SEQ ID NOs: 1-2237, and fragments thereof under various conditions of stringency. (See, e.g., Wahl and Berger (1987) Methods Enzymol. 152: 399-407; Kimmel (1987) Methods Enzymol. 152: 507-511.) Estimates of homology are provided by either DNA-DNA or DNA-RNA hybridization under conditions of stringency as is well understood by those skilled in the art (Hames and Higgins, Eds. (1985) Nucleic Acid Hybridisation, IRL Press, Oxford, U.K.). Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions.

Identifying_Polynucleotides or Nucleic Acids with Expression Libraries

In addition to hybridization methods, transcription factor homolog polypeptides can be obtained by screening an expression library using antibodies specific for one or more transcription factors. With the provision herein of the disclosed transcription factor, and transcription factor homolog nucleic acid sequences, the encoded polypeptide(s) can be expressed and purified in a heterologous expression system (e.g., *E. coli)* and used to raise antibodies (monoclonal or polyclonal) specific for the polypeptide(s) in question. Antibodies can also be raised against synthetic peptides derived from transcription factor, or transcription factor homolog, amino acid sequences. Methods of raising antibodies are well known in the art and are described in Harlow and Lane (1988), Antibodies: A Laboratory Manual, Cold Spring Harbor Laboratory, New York. Such antibodies can then be used to screen an expression library produced from the plant from which it is desired to clone additional transcription factor homologs, using the methods described above. The selected cDNAs can be confirmed by sequencing and enzymatic activity.

Sequence Variations

It will readily be appreciated by those of skill in the art, that any of a variety of polynucleotide sequences are capable of encoding the transcription factors and transcription factor homolog polypeptides discussed herein. Due to the degeneracy of the genetic code, many different polynucleotides can encode identical and/or substantially similar polypeptides in addition to those sequences illustrated in the Sequence Listing. Nucleic acids having a sequence that differs from the sequences shown in the Sequence Listing, or complementary sequences, that encode functionally equivalent peptides (i.e., peptides having some degree of equivalent or similar biological activity) but differ in sequence from the sequence shown in the Sequence Listing due to degeneracy in the genetic code, are also contemplated.

Altered polynucleotide sequences encoding polypeptides include those sequences with deletions, insertions, or substitutions of different nucleotides, resulting in a polynucleotide encoding a polypeptide with at least one functional characteristic of the instant polypeptides. Included within this definition are polymorphisms which may or may not be readily detectable using a particular oligonucleotide probe of the polynucleotide encoding the instant polypeptides, and improper or unexpected hybridization to allelic variants, with a locus other than the normal chromosomal locus for the polynucleotide sequence encoding the instant polypeptides.

Allelic variant refers to any of two or more alternative forms of a gene occupying the same chromosomal locus. Allelic variation arises naturally through mutation, and may result in phenotypic polymorphism within populations. Gene mutations can be silent (i.e., no change in the encoded polypeptide) or may encode polypeptides having altered amino acid sequence. The term allelic variant is also used herein to denote a protein encoded by an allelic variant of a gene.

Splice variant refers to alternative forms of RNA transcribed from a gene. Splice variation arises naturally through use of alternative splicing sites within a transcribed RNA molecule, or less commonly between separately transcribed RNA molecules, and may result in several mRNAs transcribed from the same gene. Splice variants may encode polypeptides having altered amino acid sequence. The term splice variant is also used herein to denote a protein encoded by a splice variant of an mRNA transcribed from a gene.

cDNAs generated from alternatively spliced mRNAs, which retain the properties of the transcription factor are included within the scope of the present disclosure, as are polypeptides encoded by such cDNAs and mRNAs. Allelic variants and splice variants of these sequences can be cloned by probing cDNA or genomic libraries from different individual organisms or tissues according to standard procedures known in the art (see USPN 6,388,064).

Related nucleic acid molecules also include nucleotide sequences encoding a polypeptide comprising or consisting essentially of a substitution, modification, addition and/or deletion of one or more amino acid residues compared to the polypeptides as set forth in the Sequence Listing. Such related polypeptides may comprise, for example, additions and/or deletions of one or more N-linked or O-linked glycosylation sites, or an addition and/or a deletion of one or more cysteine residues.

For example, Table 1 illustrates, e.g., that the codons AGC, AGT, TCA, TCC, TCG, and TCT all encode the same amino acid: serine. Accordingly, at each position in the sequence where there is a codon encoding serine, any of the above trinucleotide sequences can be used without altering the encoded polypeptide.

Table 1

| Amino acid | | | Possible Codons | | | | | |
|---|---|---|---|---|---|---|---|---|
| Alanine | Ala | A | GCA | GCC | GCG | GCU | | |
| Cysteine | Cys | C | TGC | TGT | | | | |
| Aspartic acid | Asp | D | GAC | GAT | | | | |
| Glutamic acid | Glu | E | GAA | GAG | | | | |
| Phenylalanine | Phe | F | TTC | TTT | | | | |
| Glycine | Gly | G | GGA | GGC | GGG | GGT | | |
| Histidine | His | H | CAC | CAT | | | | |
| Isoleucine | Ile | I | ATA | ATC | ATT | | | |
| Lysine | Lys | K | AAA | AAG | | | | |
| Leucine | Leu | L | TTA | TTG | CTA | CTC | CTG | CTT |
| Methionine | Met | M | ATG | | | | | |
| Asparagine | Asn | N | AAC | AAT | | | | |
| Proline | Pro | P | CCA | CCC | CCG | CCT | | |
| Glutamine | Gln | Q | CAA | CAG | | | | |
| Arginine | Arg | R | AGA | AGG | CGA | CGC | CGG | CGT |
| Serine | Ser | S | AGC | AGT | TCA | TCC | TCG | TCT |
| Threonine | Thr | T | ACA | ACC | ACG | ACT | | |
| Valine | Val | V | GTA | GTC | GTG | GTT | | |
| Tryptophan | Trp | W | TGG | | | | | |
| Tyrosine | Tyr | Y | TAC | TAT | | | | |

Sequence alterations that do not change the amino acid sequence encoded by the polynucleotide are termed "silent" variations. With the exception of the codons ATG and TGG, encoding methionine and tryptophan, respectively, any of the possible codons for the same amino acid can be substituted by a variety of techniques, e.g., site-directed mutagenesis, available in the art. Accordingly, any and all such variations of a sequence selected from the above table are a feature of the invention.

In addition to silent variations, other conservative variations that alter one, or a few amino acids in the encoded polypeptide, can be made without altering the function of the polypeptide, these conservative variants are, likewise, a feature of the disclosure.

For example, substitutions, deletions and insertions introduced into the sequences provided in the Sequence Listing, are also envisioned. Such sequence modifications can be engineered into a sequence by site-directed mutagenesis (Wu (ed.) Methods Enzymol. (1993) vol. 217, Academic Press) or the other methods noted below. Amino acid substitutions are typically of single residues; insertions usually will be on the order of about from 1 to 10 amino acid residues; and deletions will range about from 1 to 30 residues. In preferred embodiments, deletions or insertions are made in adjacent pairs, e.g., a deletion of two residues or insertion of two residues. Substitutions, deletions, insertions or any combination thereof can be combined to arrive at a sequence. The mutations that are made in the polynucleotide encoding the transcription factor should not place the sequence out of reading frame and should not create complementary regions that could produce secondary mRNA structure. Preferably, the polypeptide encoded by the DNA performs the desired function.

Conservative substitutions are those in which at least one residue in the amino acid sequence has been removed and a different residue inserted in its place. Such substitutions generally are made in accordance with the Table 2 when it is desired to maintain the activity of the protein. Table 2 shows amino acids which can be substituted for an amino acid in a protein and which are typically regarded as conservative substitutions.

**Table 2**

| Residue | Conservative Substitutions |
|---|---|
| Ala | Ser |
| Arg | Lys |
| Asn | Gln; His |
| Asp | Glu |
| Gln | Asn |
| Cys | Ser |
| Glu | Asp |
| Gly | Pro |
| His | Asn; Gln |
| Ile | Leu, Val |
| Leu | Ile; Val |
| Lys | Arg; Gln |
| Met | Leu; Ile |
| Phe | Met; Leu; Tyr |
| Ser | Thr; Gly |
| Thr | Ser; Val |
| Trp | Tyr |
| Tyr | Trp; Phe |
| Val | Ile; Leu |

Similar substitutions are those in which at least one residue in the amino acid sequence has been removed and a different residue inserted in its place. Such substitutions generally are made in accordance with the Table 3 when it is desired to maintain the activity of the protein. Table 3 shows amino acids which can be substituted for an amino acid in a protein and which are typically regarded as structural and functional substitutions. For example, a residue in column 1 of Table 3 may be substituted with a residue in column 2; in addition, a residue in column 2 of Table 3 may be substituted with the residue of column 1.

**Table 3**

| Residue | Similar Substitutions |
|---|---|
| Ala | Ser; Thr; Gly; Val; Leu; Ile |
| Arg | Lys; His; Gly |
| Asn | Gln; His; Gly; Ser; Thr |
| Asp | Glu, Ser; Thr |

(continued)

| Residue | Similar Substitutions |
|---|---|
| Gln | Asn; Ala |
| Cys | Ser; Gly |
| Glu | Asp |
| Gly | Pro; Arg |
| His | Asn; Gln; Tyr; Phe; Lys; Arg |
| Ile | Ala; Leu; Val; Gly; Met |
| Leu | Ala; Ile; Val; Gly; Met |
| Lys | Arg; His; Gln; Gly; Pro |
| Met | Leu; Ile; Phe |
| Phe | Met; Leu; Tyr; Trp; His; Val; Ala |
| Ser | Thr; Gly; Asp; Ala; Val; Ile; His |
| Thr | Ser; Val; Ala; Gly |
| Trp | Tyr; Phe; His |
| Tyr | Trp; Phe; His |
| Val | Ala; Ile; Leu; Gly; Thr; Ser; Glu |

Substitutions that are less conservative than those in Table 2 can be selected by picking residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions which in general are expected to produce the greatest changes in protein properties will be those in which (a) a hydrophilic residue, e.g., seryl or threonyl, is substituted for (or by) a hydrophobic residue, e.g., leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine.

Further Modifying Sequences - Mutation/Forced Evolution

In addition to generating silent or conservative substitutions as noted, above, the present disclosure optionally includes methods of modifying the sequences of the Sequence Listing. In the methods, nucleic acid or protein modification methods are used to alter the given sequences to produce new sequences and/or to chemically or enzymatically modify given sequences to change the properties of the nucleic acids or proteins.

Thus, in one embodiment, given nucleic acid sequences are modified, e.g., according to standard mutagenesis or artificial evolution methods to produce modified sequences. The modified sequences may be created using purified natural polynucleotides isolated from any organism or may be synthesized from purified compositions and chemicals using chemical means well know to those of skill in the art. For example, Ausubel, *supra,* provides additional details on mutagenesis methods. Artificial forced evolution methods are described, for example, by Stemmer (1994) Nature 370: 389-391, Stemmer (1994) Proc. Natl. Acad. Sci. 91: 10747-10751, and US Patents 5,811,238, 5,837,500, and 6,242,568. Methods for engineering synthetic transcription factors and other polypeptides are described, for example, by Zhang et al. (2000) J. Biol. Chem. 275: 33850-33860, Liu et al. (2001) J Biol. Chem. 276: 11323-11334, and Isalan et al. (2001) Nature Biotechnol. 19: 656-660. Many other mutation and evolution methods are also available and expected to be within the skill of the practitioner.

Similarly, chemical or enzymatic alteration of expressed nucleic acids and polypeptides can be performed by standard methods. For example, sequence can be modified by addition of lipids, sugars, peptides, organic or inorganic compounds, by the inclusion of modified nucleotides or amino acids, or the like. For example, protein modification techniques are illustrated in Ausubel, *supra.* Further details on chemical and enzymatic modifications can be found herein. These modification methods can be used to modify any given sequence, or to modify any sequence produced by the various mutation and artificial evolution modification methods noted herein.

Accordingly, the disclosure provides for modification of any given nucleic acid by mutation, evolution, chemical or enzymatic modification, or other available methods, as well as for the products produced by practicing such methods, e.g., using the sequences herein as a starting substrate for the various modification approaches.

For example, optimized coding sequence containing codons preferred by a particular prokaryotic or eukaryotic host can be used e.g., to increase the rate of translation or to produce recombinant RNA transcripts having desirable properties, such as a longer half-life, as compared with transcripts produced using a non-optimized sequence. Translation stop codons can also be modified to reflect host preference. For example, preferred stop codons for *Saccharomyces cerevisiae* and mammals are TAA and TGA, respectively. The preferred stop codon for monocotyledonous plants is TGA, whereas insects and *E. coli* prefer to use TAA as the stop codon.

The polynucleotide sequences can also be engineered in order to alter a coding sequence for a variety of reasons, including but not limited to, alterations which modify the sequence to facilitate cloning, processing and/or expression of the gene product. For example, alterations are optionally introduced using techniques which are well known in the art, e.g., site-directed mutagenesis, to insert new restriction sites, to alter glycosylation patterns, to change codon preference, to introduce splice sites, etc.

Furthermore, a fragment or domain derived from any of the polypeptides can be combined with domains derived from other transcription factors or synthetic domains to modify the biological activity of a transcription factor. For instance, a DNA-binding domain derived from a transcription factor can be combined with the activation domain of another transcription factor or with a synthetic activation domain. A transcription activation domain assists in initiating transcription from a DNA-binding site. Examples include the transcription activation region of VP16 or GAL4 (Moore et al. (1998) Proc. Natl. Acad. Sci. 95: 376-381; Aoyama et al. (1995) Plant Cell 7: 1773-1785), peptides derived from bacterial sequences (Ma and Ptashne (1987) Cell 51: 113-119) and synthetic peptides (Giniger and Ptashne (1987) Nature 330: 670-672).

Expression and Modification of Polypeptides

Typically, polynucleotide sequences are incorporated into recombinant DNA (or RNA) molecules that direct expression of polypeptides in appropriate host cells, transgenic plants, in vitro translation systems, or the like. Due to the inherent degeneracy of the genetic code, nucleic acid sequences which encode substantially the same or a functionally equivalent amino acid sequence can be substituted for any listed sequence to provide for cloning and expressing the relevant homolog.

The transgenic plants of the present invention comprising recombinant polynucleotide sequences are generally derived from parental plants, which may themselves be non-transformed (or non-transgenic) plants. Overexpressing transgenic "progeny" plants will exhibit greater mRNA levels, wherein the mRNA encodes a transcription factor, that is, a DNA-binding protein that is capable of binding to a DNA regulatory sequence and inducing transcription, and preferably, expression of a plant trait gene. Preferably, the mRNA expression level will be at least threefold greater than that of the parental plant, or more preferably at least ten-fold greater mRNA levels compared to said parental plant, and most preferably at least fifty-fold greater compared to said parental plant.

Vectors, Promoters, and Expression Systems

The present disclosure includes recombinant constructs comprising one or more of the nucleic acid sequences herein. The constructs typically comprise a vector, such as a plasmid, a cosmid, a phage, a virus (e.g., a plant virus), a bacterial artificial chromosome (BAC), a yeast artificial chromosome (YAC), or the like, into which a nucleic acid sequence has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available.

General texts that describe molecular biological techniques useful herein, including the use and production of vectors, promoters and many other relevant topics, include Berger, Sambrook, *supra* and Ausubel, *supra.* Any of the identified sequences can be incorporated into a cassette or vector, e.g., for expression in plants. A number of expression vectors suitable for stable transformation of plant cells or for the establishment of transgenic plants have been described including those described in Weissbach and Weissbach (1989) Methods for Plant Molecular Biology, Academic Press, and Gelvin et al. (1990) Plant Molecular Biology Manual Kluwer Academic Publishers. Specific examples include those derived from a Ti plasmid of *Agrobacterium tumefaciens,* as well as those disclosed by Herrera-Estrella et al. (1983) Nature 303: 209, Bevan (1984) Nucleic Acids Res. 12: 8711-8721, Klee (1985) Bio/Technology 3: 637-642, for dicotyledonous plants.

Alternatively, non-Ti vectors can be used to transfer the DNA into monocotyledonous plants and cells by using free DNA delivery techniques. Such methods can involve, for example, the use of liposomes, electroporation, microprojectile bombardment, silicon carbide whiskers, and viruses. By using these methods transgenic plants such as wheat, rice (Christou (1991) Bio/Technology 9: 957-962) and corn (Gordon-Kamm (1990) Plant Cell 2: 603-618) can be produced. An immature embryo can also be a good target tissue for monocots for direct DNA delivery techniques by using the particle gun (Weeks et al. (1993) Plant Physiol. 102: 1077-1084; Vasil (1993) Bio/Technology 10: 667-674; Wan and Lemeaux (1994) Plant Physiol. 104: 37-48, and for *Agrobacterium*-mediated DNA transfer (Ishida et al. (1996) Nature Biotechnol. 14: 745-750).

Typically, plant transformation vectors include one or more cloned plant coding sequence (genomic or cDNA) under the transcriptional control of 5' and 3' regulatory sequences and a dominant selectable marker. Such plant transformation vectors typically also contain a promoter (e.g., a regulatory region controlling inducible or constitutive, environmentally-or developmentally-regulated, or cell- or tissue-specific expression), a transcription initiation start site, an RNA processing signal (such as intron splice sites), a transcription termination site, and/or a polyadenylation signal.

A potential utility for the transcription factor polynucleotides disclosed herein is the isolation of promoter elements from these genes that can be used to program expression in plants of any genes. Each transcription factor gene disclosed herein is expressed in a unique fashion, as determined by promoter elements located upstream of the start of translation, and additionally within an intron of the transcription factor gene or downstream of the termination codon of the gene. As is well known in the art, for a significant portion of genes, the promoter sequences are located entirely in the region directly upstream of the start of translation. In such cases, typically the promoter sequences are located within 2.0 kb of the start of translation, or within 1.5 kb of the start of translation, frequently within 1.0 kb of the start of translation, and sometimes within 0.5 kb of the start of translation.

The promoter sequences can be isolated according to methods known to one skilled in the art.

Examples of constitutive plant promoters which can be useful for expressing the TF sequence include: the cauliflower mosaic virus (CaMV) 35S promoter, which confers constitutive, high-level expression in most plant tissues (*see,* e.g., Odell et al. (1985) Nature 313: 810-812); the nopaline synthase promoter (An et al. (1988) Plant Physiol. 88: 547-552); and the octopine synthase promoter (Fromm et al. (1989) Plant Cell 1: 977-984).

A variety of plant gene promoters that regulate gene expression in response to environmental, hormonal, chemical, developmental signals, and in a tissue-active manner can be used for expression of a TF sequence in plants. Choice of a promoter is based largely on the phenotype of interest and is determined by such factors as tissue (e.g., seed, fruit, root, pollen, vascular tissue, flower, carpel, etc.), inducibility (e.g., in response to wounding, heat, cold, drought, light, pathogens, etc.), timing, developmental stage, and the like. Numerous known promoters have been characterized and can favorably be employed to promote expression of a polynucleotide in a transgenic plant or cell of interest. For example, tissue specific promoters include: seed-specific promoters (such as the napin, phaseolin or DC3 promoter described in US Pat. No. 5,773,697), fruit-specific promoters that are active during fruit ripening (such as the dru 1 promoter (US Pat. No. 5,783,393), or the 2A11 promoter (US Pat. No. 4,943,674) and the tomato polygalacturonase promoter (Bird et al. (1988) Plant Mol. Biol. 11: 651-662), root-specific promoters, such as those disclosed in US Patent Nos. 5,618,988, 5,837,848 and 5,905,186, pollen-active promoters such as PTA29, PTA26 and PTA13 (US Pat. No. 5,792,929), promoters active in vascular tissue (Ringli and Keller (1998) Plant Mol. Biol. 37: 977-988), flower-specific (Kaiser et al. (1995) Plant Mol. Biol. 28: 231-243), pollen (Baerson et al. (1994) Plant Mol. Biol. 26: 1947-1959), carpels (Ohl et al. (1990) Plant Cell 2: 837-848), pollen and ovules (Baerson et al. (1993) Plant Mol. Biol. 22: 255-267), auxin-inducible promoters (such as that described in van der Kop et al. (1999) Plant Mol. Biol. 39: 979-990 or Baumann et al., (1999) Plant Cell 11: 323-334), cytokinin-inducible promoter (Guevara-Garcia (1998) Plant Mol. Biol. 38: 743-753), promoters responsive to gibberellin (Shi et al. (1998) Plant Mol. Biol. 38: 1053-1060, Willmott et al. (1998) Plant Molec. Biol. 38: 817-825) and the like. Additional promoters are those that elicit expression in response to heat (Ainley et al. (1993) Plant Mol. Biol. 22: 13-23), light (e.g., the pea rbcS-3A promoter, Kuhlemeier et al. (1989) Plant Cell 1: 471-478, and the maize rbcS promoter, Schaffner and Sheen (1991) Plant Cell 3: 997-1012); wounding (e.g., *wun*I, Siebertz et al. (1989) Plant Cell 1: 961-968); pathogens (such as the PR-1 promoter described in Buchel et al. (1999) Plant Mol. Biol. 40: 387-396, and the PDF1.2 promoter described in Manners et al. (1998) Plant Mol. Biol. 38: 1071-1080), and chemicals such as methyl jasmonate or salicylic acid (Gatz (1997) Annu. Rev. Plant Physiol. Plant Mol. Biol. 48: 89-108). In addition, the timing of the expression can be controlled by using promoters such as those acting at senescence (Gan and Amasino (1995) Science 270: 1986-1988); or late seed development (Odell et al. (1994) Plant Physiol. 106: 447-458).

Plant expression vectors can also include RNA processing signals that can be positioned within, upstream or downstream of the coding sequence. In addition, the expression vectors can include additional regulatory sequences from the 3'-untranslated region of plant genes, e.g., a 3' terminator region to increase mRNA stability of the mRNA, such as the PI-II terminator region of potato or the octopine or nopaline synthase 3' terminator regions.

Additional Expression Elements

Specific initiation signals can aid in efficient translation of coding sequences. These signals can include, e.g., the ATG initiation codon and adjacent sequences. In cases where a coding sequence, its initiation codon and upstream sequences are inserted into the appropriate expression vector, no additional translational control signals may be needed.

However, in cases where only coding sequence (e.g., a mature protein coding sequence), or a portion thereof, is inserted, exogenous transcriptional control signals including the ATG initiation codon can be separately provided. The initiation codon is provided in the correct reading frame to facilitate transcription. Exogenous transcriptional elements and initiation codons can be of various origins, both natural and synthetic. The efficiency of expression can be enhanced by the inclusion of enhancers appropriate to the cell system in use.

Expression Hosts

The present invention also relates to host cells which are transduced with vectors of the invention, and the production of polypeptides (including fragments thereof) by recombinant techniques. Host cells are genetically engineered (i.e., nucleic acids are introduced, e.g., transduced, transformed or transfected) with the vectors of this disclosure, which may be, for example, a cloning vector or an expression vector comprising the relevant nucleic acids herein. The vector is optionally a plasmid, a viral particle, a phage, a naked nucleic acid, etc. The engineered host cells can be cultured in conventional nutrient media modified as appropriate for activating promoters, selecting transformants, or amplifying the relevant gene. The culture conditions, such as temperature, pH and the like, are those previously used with the host cell selected for expression, and will be apparent to those skilled in the art and in the references cited herein, including, Sambrook, *supra* and Ausubel, *supra.*

The host cell can be a eukaryotic cell, such as a yeast cell, or a plant cell, or the host cell can be a prokaryotic cell, such as a bacterial cell. Plant protoplasts are also suitable for some applications. For example, the DNA fragments are introduced into plant tissues, cultured plant cells or plant protoplasts by standard methods including electroporation (Fromm et al. (1985) Proc. Natl. Acad. Sci. 82: 5824-5828, infection by viral vectors such as cauliflower mosaic virus (CaMV) (Hohn et al. (1982) Molecular Biology of Plant Tumors Academic Press, New York, NY, pp. 549-560; US 4,407,956), high velocity ballistic penetration by small particles with the nucleic acid either within the matrix of small beads or particles, or on the surface (Klein et al. (1987) Nature 327: 70-73), use of pollen as vector (WO 85/01856), or use of *Agrobacterium tumefaciens* or *A. rhizogenes* carrying a T-DNA plasmid in which DNA fragments are cloned. The T-DNA plasmid is transmitted to plant cells upon infection by *Agrobacterium tumefaciens,* and a portion is stably integrated into the plant genome (Horsch et al. (1984) Science 233: 496-498; Fraley et al. (1983) Proc. Natl. Acad. Sci. 80: 4803-4807).

The cell can include a nucleic acid that encodes a polypeptide, wherein the cell expresses a polypeptide. The cell can also include vector sequences, or the like. Furthermore, cells and transgenic plants that include any polypeptide or nucleic acid above or throughout this specification, e.g., produced by transduction of a vector of the disclosure, are an additional feature.

For long-term, high-yield production of recombinant proteins, stable expression can be used. Host cells transformed with a nucleotide sequence encoding a polypeptide are optionally cultured under conditions suitable for the expression and recovery of the encoded protein from cell culture. The protein or fragment thereof produced by a recombinant cell may be secreted, membrane-bound, or contained intracellularly, depending on the sequence and/or the vector used. As will be understood by those of skill in the art, expression vectors containing polynucleotides encoding mature proteins can be designed with signal sequences which direct secretion of the mature polypeptides through a prokaryotic or eukaryotic cell membrane.

Modified Amino Acid Residues

Polypeptides may contain one or more modified amino acid residues. The presence of modified amino acids may be advantageous in, for example, increasing polypeptide half-life, reducing polypeptide antigenicity or toxicity, increasing polypeptide storage stability, or the like. Amino acid residue(s) are modified, for example, co-translationally or post-translationally during recombinant production or modified by synthetic or chemical means.

Non-limiting examples of a modified amino acid residue include incorporation or other use of acetylated amino acids, glycosylated amino acids, sulfated amino acids, prenylated (e.g., famesylated, geranylgeranylated) amino acids, PEG modified (e.g., "PEGylated") amino acids, biotinylated amino acids, carboxylated amino acids, phosphorylated amino acids, etc. References adequate to guide one of skill in the modification of amino acid residues are replete throughout the literature.

The modified amino acid residues may prevent or increase affinity of the polypeptide for another molecule, including, but not limited to, polynucleotide, proteins, carbohydrates, lipids and lipid derivatives, and other organic or synthetic compounds.

Identification of Additional Factors

A transcription factor provided by the present disclosure can also be used to identify additional endogenous or exogenous molecules that can affect a phentoype or trait of interest. On the one hand, such molecules include organic (small or large molecules) and/or inorganic compounds that affect expression of (i.e., regulate) a particular transcription factor. Alternatively, such molecules include endogenous molecules that are acted upon either at a transcriptional level by a transcription factor to modify a phenotype as desired. For example, the transcription factors can be employed to identify one or more downstream genes that are subject to a regulatory effect of the transcription factor. In one approach, a transcription factor or transcription factor homolog is expressed in a host cell, e.g., a transgenic plant cell, tissue or explant, and expression products, either RNA or protein, of likely or random targets are monitored, e.g., by hybridization to a microarray of nucleic acid probes corresponding to genes expressed in a tissue or cell type of interest, by two-dimensional gel electrophoresis of protein products, or by any other method known in the art for assessing expression of gene products at the level of RNA or protein. Alternatively, a transcription factor can be used to identify promoter sequences (such as binding sites on DNA sequences) involved in the regulation of a downstream target. After identifying a promoter sequence, interactions between the transcription factor and the promoter sequence can be modified by changing specific nucleotides in the promoter sequence or specific amino acids in the transcription factor that interact with the promoter sequence to alter a plant trait. Typically, transcription factor DNA-binding sites are identified by gel shift assays. After identifying the promoter regions, the promoter region sequences can be employed in double-stranded DNA arrays to identify molecules that affect the interactions of the transcription factors with their promoters (Bulyk et al. (1999) Nature Biotechnol. 17: 573-577).

The identified transcription factors are also useful to identify proteins that modify the activity of the transcription factor. Such modification can occur by covalent modification, such as by phosphorylation, or by protein-protein (homo or-heteropolymer) interactions. Any method suitable for detecting protein-protein interactions can be employed. Among the methods that can be employed are co-immunoprecipitation, cross-linking and co-purification through gradients or chromatographic columns, and the two-hybrid yeast system.

The two-hybrid system detects protein interactions in vivo and is described in Chien et al. ((1991) Proc. Natl. Acad. Sci. 88: 9578-9582) and is commercially available from Clontech (Palo Alto, Calif.). In such a system, plasmids are constructed that encode two hybrid proteins: one consists of the DNA-binding domain of a transcription activator protein fused to the TF polypeptide and the other consists of the transcription activator protein's activation domain fused to an unknown protein that is encoded by a cDNA that has been recombined into the plasmid as part of a cDNA library. The DNA-binding domain fusion plasmid and the cDNA library are transformed into a strain of the yeast *Saccharomyces cerevisiae* that contains a reporter gene (e.g., lacZ) whose regulatory region contains the transcription activator's binding site. Either hybrid protein alone cannot activate transcription of the reporter gene. Interaction of the two hybrid proteins reconstitutes the functional activator protein and results in expression of the reporter gene, which is detected by an assay for the reporter gene product. Then, the library plasmids responsible for reporter gene expression are isolated and sequenced to identify the proteins encoded by the library plasmids. After identifying proteins that interact with the transcription factors, assays for compounds that interfere with the TF protein-protein interactions can be preformed.

## Identification of Modulators

In addition to the intracellular molecules described above, extracellular molecules that alter activity or expression of a transcription factor, either directly or indirectly, can be identified. For example, the methods can entail first placing a candidate molecule in contact with a plant or plant cell. The molecule can be introduced by topical administration, such as spraying or soaking of a plant, or incubating a plant in a solution containing the molecule, and then the molecule's effect on the expression or activity of the TF polypeptide or the expression of the polynucleotide monitored. Changes in the expression of the TF polypeptide can be monitored by use of polyclonal or monoclonal antibodies, gel electrophoresis or the like. Changes in the expression of the corresponding polynucleotide sequence can be detected by use of microarrays, Northerns, quantitative PCR, or any other technique for monitoring changes in mRNA expression. These techniques are exemplified in Ausubel et al. (eds.) Current Protocols in Molecular Biology, John Wiley & Sons (1998, and supplements through 2001). Changes in the activity of the transcription factor can be monitored, directly or indirectly, by assaying the function of the transcription factor, for example, by measuring the expression of promoters known to be controlled by the transcription factor (using promoter-reporter constructs), measuring the levels of transcripts using microarrays, Northern blots, quantitative PCR, etc. Such changes in the expression, levels can be correlated with modified plant traits and thus identified molecules can be useful for soaking or spraying on fruit, vegetable and grain crops to modify traits in plants.

Essentially any available composition can be tested for modulatory activity of expression or activity of any nucleic acid or polypeptide herein. Thus, available libraries of compounds such as chemicals, polypeptides, nucleic acids and the like can be tested for modulatory activity. Often, potential modulator compounds can be dissolved in aqueous or organic (e.g., DMSO-based) solutions for easy delivery to the cell or plant of interest in which the activity of the modulator is to be tested. Optionally, the assays are designed to screen large modulator composition libraries by automating the assay steps and providing compounds from any convenient source to assays, which are typically run in parallel (e.g., in microtiter formats on micrometer plates in robotic assays).

In one embodiment, high throughput screening methods involve providing a combinatorial library containing a large number of potential compounds (potential modulator compounds). Such "combinatorial chemical libraries" are then screened in one or more assays, as described herein, to identify those library members (particular chemical species or subclasses) that display a desired characteristic activity. The compounds thus identified can serve as target compounds.

A combinatorial chemical library can be, e.g., a collection of diverse chemical compounds generated by chemical synthesis or biological synthesis. For example, a combinatorial chemical library such as a polypeptide library is formed by combining a set of chemical building blocks (e.g., in one example, amino acids) in every possible way for a given compound length (i.e., the number of amino acids in a polypeptide compound of a set length). Exemplary libraries include peptide libraries, nucleic acid libraries, antibody libraries (see, e.g., Vaughn et al. (1996) Nature Biotechnol. 14: 309-314 and PCT/US96/10287), carbohydrate libraries (see, e.g., Liang et al. Science (1996) 274: 1520-1522 and US Patent 5,593,853), peptide nucleic acid libraries (see, e.g., US Patent 5,539,083), and small organic molecule libraries (see, e.g., benzodiazepines, in Baum Chem. & Engineering News Jan 18, 1993, page 33; isoprenoids, US Patent 5,569,588; thiazolidinones and metathiazanones, US Patent 5,549,974; pyrrolidines, US Patents 5,525,735 and 5,519,134; morpholino compounds, US Patent 5,506,337) and the like.

Preparation and screening of combinatorial or other libraries is well known to those of skill in the art. Such combinatorial chemical libraries include, but are not limited to, peptide libraries (see, e.g., US Patent 5,010,175; Furka, (1991) Int. J. Pept. Prot. Res. 37: 487-493; and Houghton et al. (1991) Nature 354: 84-88). Other chemistries for generating chemical diversity libraries can also be used.

In addition, as noted, compound screening equipment for high-throughput screening is generally available, e.g., using any of a number of well known robotic systems that have also been developed for solution phase chemistries useful in assay systems. These systems include automated workstations including an automated synthesis apparatus and robotic systems utilizing robotic arms. Any of the above devices are suitable for use with this aspect of the disclosure, e.g., for high-throughput screening of potential modulators. The nature and implementation of modifications to these devices (if any) so that they can operate as discussed herein will be apparent to persons skilled in the relevant art.

Indeed, entire high-throughput screening systems are commercially available. These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization. Similarly, microfluidic implementations of screening are also commercially available.

The manufacturers of such systems provide detailed protocols the various high throughput. Thus, for example, Zymark Corp. provides technical bulletins describing screening systems for detecting the modulation of gene transcription, ligand binding, and the like. The integrated systems herein, in addition to providing for sequence alignment and, optionally, synthesis of relevant nucleic acids, can include such screening apparatus to identify modulators that have an effect on one or more polynucleotides or polypeptides described herein.

In some assays it is desirable to have positive controls to ensure that the components of the assays are working properly. At least two types of positive controls are appropriate. That is, known transcriptional activators or inhibitors can be incubated with cells or plants, for example, in one sample of the assay, and the resulting increase/decrease in transcription can be detected by measuring the resulting increase in RNA levels and/or protein expression, for example, according to the methods herein. It will be appreciated that modulators can also be combined with transcriptional activators or inhibitors to find modulators that inhibit transcriptional activation or transcriptional repression. Either expression of the nucleic acids and proteins herein or any additional nucleic acids or proteins activated by the nucleic acids or proteins herein, or both, can be monitored.

In an embodiment, the present disclosure sets forth a method for identifying compositions that modulate the activity or expression of a polynucleotide or polypeptide of the disclosure. For example, a test compound, whether a small or large molecule, is placed in contact with a cell, plant (or plant tissue or explant), or composition comprising the polynucleotide or polypeptide of interest and a resulting effect on the cell, plant, (or tissue or explant) or composition is evaluated by monitoring, either directly or indirectly, one or more of: expression level of the polynucleotide or polypeptide, activity (or modulation of the activity) of the polynucleotide or polypeptide. In some cases, an alteration in a plant phenotype can be detected following contact of a plant (or plant cell, or tissue or explant) with the putative modulator, e.g., by modulation of expression or activity of a polynucleotide or polypeptide. Modulation of expression or activity of a polynucleotide or polypeptide may also be caused by molecular elements in a signal transduction second messenger pathway and such modulation can affect similar elements in the same or another signal transduction second messenger pathway.

## Subsequences

Also described herein are uses of polynucleotides, also referred to herein as oligonucleotides, typically having at least 12 bases, preferably at least 15, more preferably at least 20, 30, or 50 bases, which hybridize under at least highly stringent (or ultra-high stringent or ultra-ultra-high stringent conditions) conditions to a polynucleotide sequence described above. The polynucleotides may be used as probes, primers, sense and antisense agents, and the like, according to methods as noted *supra.*

Subsequences of the polynucleotides, including polynucleotide fragments and oligonucleotides are useful as nucleic acid probes and primers. An oligonucleotide suitable for use as a probe or primer is at least about 15 nucleotides in length, more often at least about 18 nucleotides, often at least about 21 nucleotides, frequently at least about 30 nucleotides, or about 40 nucleotides, or more in length. A nucleic acid probe is useful in hybridization protocols, e.g., to identify additional polypeptide homologs including protocols for microarray experiments. Primers can be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, e.g., by the polymerase chain reaction (PCR) or other nucleic-acid amplification methods. See Sambrook, *supra,* and Ausubel, *supra.*

In addition, described herein is an isolated or recombinant polypeptide including a subsequence of at least about 15 contiguous amino acids encoded by the recombinant or isolated polynucleotides. For example, such polypeptides, or domains or fragments thereof, can be used as immunogens, e.g., to produce antibodies specific for the polypeptide sequence, or as probes for detecting a sequence of interest. A subsequence can range in size from about 15 amino acids in length up to and including the full length of the polypeptide.

An expressed polypeptide which comprises such a polypeptide subsequence performs at least one biological function of the intact polypeptide in substantially the same manner, or to a similar extent, as does the intact polypeptide. For example, a polypeptide fragment can comprise a recognizable structural motif or functional domain such as a DNA binding domain that activates transcription, e.g., by binding to a specific DNA promoter region an activation domain, or a domain for protein-protein interactions.

## Production of Transgenic Plants

### Modification of Traits

The polynucleotides of the disclosure are favorably employed to produce transgenic plants with various traits, or characteristics, that have been modified in a desirable manner, e.g., to improve the seed characteristics of a plant. For example, alteration of expression levels or patterns (e.g., spatial or temporal expression patterns) of one or more of the transcription factors (or transcription factor homologs) as compared with the levels of the same protein found in a wild-type plant, can be used to modify a plant's traits. An illustrative example of trait modification, improved characteristics, by altering expression levels of a particular transcription factor is described further in the Examples and the Sequence Listing.

### *Arabidopsis* as a model system

*Arabidopsis thaliana* is the object of rapidly growing attention as a model for genetics and metabolism in plants. *Arabidopsis* has a small genome, and well-documented studies are available. It is easy to grow in large numbers and mutants defining important genetically controlled mechanisms are either available, or can readily be obtained. Various methods to introduce and express isolated homologous genes are available (see Koncz et al., eds., Methods in Arabidopsis Research (1992) World Scientific, New Jersey, NJ, in "Preface"). Because of its small size, short life cycle, obligate autogamy and high fertility, *Arabidopsis* is also a choice organism for the isolation of mutants and studies in morphogenetic and development pathways, and control of these pathways by transcription factors (Koncz *supra,* p. 72). A number of studies introducing transcription factors into *A. thaliana* have demonstrated the utility of this plant for understanding the mechanisms of gene regulation and trait alteration in plants. (See, for example, Koncz *supra,* and US Patent Number 6,417,428).

### Homologous genes introduced into transgenic plants.

Homologous genes that may be derived from any plant, or from any source whether natural, synthetic, semi-synthetic or recombinant, and that share significant sequence identity or similarity to those provided herein, may be introduced into plants, for example, crop plants, to confer desirable or improved traits. Consequently, transgenic plants may be produced that comprise a recombinant expression vector or cassette with a promoter operably linked to one or more sequences homologous to presently disclosed sequences. The promoter may be, for example, a plant or viral promoter.

The invention thus provides for methods for preparing transgenic plants, and for modifying plant traits as defined in the claims. These methods include introducing into a plant a recombinant expression vector or cassette comprising a functional promoter operably linked to one or more sequences homologous to presently disclosed sequences. Plants and kits for producing these plants that result from the application of these methods are also encompassed by the present invention.

Transcription factors of interest for the modification of plant traits

Currently, the existence of a series of maturity groups for different latitudes represents a major barrier to the introduction of new valuable traits. Any trait (e.g. disease resistance) has to be bred into each of the different maturity groups separately, a laborious and costly exercise. The availability of single strain, which could be grown at any latitude, would therefore greatly increase the potential for introducing new traits to crop species such as soybean and cotton.

For many of the specific effects, traits and utilities listed in Table 4 and Table 6 that may be conferred to plants, one or more transcription factor genes may be used to increase or decrease, advance or delay, or improve or prove deleterious to a given trait. More than one transcription factor gene may be introduced into a plant, either by transforming the plant with one or more vectors comprising two or more transcription factors, or by selective breeding of plants to yield hybrid crosses that comprise more than one introduced transcription factor.

A listing of specific effects and utilities that the presently disclosed transcription factor genes have on plants, as determined by direct observation and assay analysis, is provided in Table 4. Table 4 shows the polynucleotides identified by SEQ ID NO; Gene ID No. (GID); and if the polynucleotide was tested in a transgenic assay. The first column shows the polynucleotide SEQ ID NO; the second column shows the GID; the third column shows whether the gene was overexpressed (OE) or knocked out (KO) in plant studies; the fourth column shows the category of modified trait resulting from the knock out or overexpression of the polynucleotide in the transgenic plant; and the fifth column ("Experimental Observations"), includes specific observations made with respect to the polynucleotide of the respective first column.

Table 4. Traits, trait categories, and effects and utilities that transcription factor genes have on plants.

| SEQ ID NO: | GID | OE/K O | Category | Experimental Observations |
|---|---|---|---|---|
| 327 | G198 8 | OE | Nutrient; Tolerance to low N<br>Nutrient; Tolerance to low $PO_4$<br>Flowering time<br>Light response; Long petiole<br>Light response; Long hypocotyl | Better growth on low nitrogen plus glutamine, better growth on low phosphate; long hypocotyl, long petiole, early flowering |

Table 5 shows the polypeptides identified by SEQ ID NO; Gene ID (GID) No; the transcription factor family to which the polypeptide belongs, and conserved domains of the polypeptide. The first column shows the polypeptide SEQ ID NO; the third column shows the transcription factor family to which the polynucleotide belongs; and the fourth column shows the amino acid residue positions of the conserved domain in amino acid (AA) coordinates.

Table 5. Gene families and conserved domains

| Polypeptide SEQ ID NO: | GID No. | Family | Conserved Domains in Amino Acid Coordinates |
|---|---|---|---|
| 328 | G1988 | 5-50 | Z-CO-like |

Examples of some of the utilities that may be desirable in plants, and that may be provided by transforming the plants with the presently disclosed sequences, are listed in Table 6. Many of the transcription factors listed in Table 6 may be operably linked with a specific promoter that causes the transcription factor to be expressed in response to environmental, tissue-specific or temporal signals. For examples of other tissue-specific, temporal-specific or inducible promoters, see the above discussion under the heading "Vectors, Promoters, and Expression Systems".

Table 6. Genes, traits and utilities that affect plant characteristics

| Trait Category | Phenotypic alteration(s) | Transcription factor genes that impact traits | Utility |
|---|---|---|---|
| Abiotic stress | Nitrogen stress<br>Less sensitive to N limitation | G1988 | Improved yield and nutrient stress tolerance, decreased fertilizer usage |
| | Phosphate stress<br>Less sensitive to $PO_4$ limitation | G1988 | Improved yield and nutrient stress tolerance, decreased fertilizer usage |
| | | | |
| Growth regulator | Altered C/N sensing | G1988 | Alteration or control of assimilate partitioning |
| | | | |
| Flowering time | Early flowering | G1988 | Faster generation time; synchrony of flowering; additional harvests within a growing season, shortening of breeding programs |
| | | | |
| Development and morphology | Altered hypocotyl shape, color, development | G1988 | Ornamental applications; altered light response (see "Light Response", below) |
| | | | |
| Light response/shade avoidance | Altered hypocotyl<br>Altered petiole | G1988<br>G1988 | Increased planting densities and yield enhancement |
| Abbreviations: N=nitrogen P=phosphate ABA=abscisic acid C/N=carbon/nitrogen balance | | | |

Detailed description of genes, traits and utilities that affect plant characteristics

The following descriptions of traits and utilities associated with the present transcription factors offer a more comprehensive description than that provided in Table 6.

Abiotic stress, general considerations

Plant transcription factors can modulate gene expression, and, in turn, be modulated by the environmental experience of a plant. Significant alterations in a plant's environment invariably result in a change in the plant's transcription factor gene expression pattern. Altered transcription factor expression patterns generally result in phenotypic changes in the plant. Transcription factor gene product(s) in transgenic plants then differ(s) in amounts or proportions from that found in wild-type or non-transformed plants, and those transcription factors likely represent polypeptides that are used to alter the response to the environmental change. By way of example, it is well accepted in the art that analytical methods based on altered expression patterns may be used to screen for phenotypic changes in a plant far more effectively than can be achieved using traditional methods.

Nutrient uptake and utilization: nitrogen and phosphorus. Presently disclosed transcription factor genes introduced into plants provide a means to improve uptake of essential nutrients, including nitrogenous compounds, phosphates, potassium, and trace minerals. The enhanced performance of, for example, G1988, and other overexpressing lines under low nitrogen conditions or G1988, under low phosphorus conditions indicate that these genes and their homologs could be used to engineer crops that could thrive under conditions of reduced nutrient availability. Phosphorus, in particular, tends to be a limiting nutrient in soils and is generally added as a component in fertilizers. Young plants have a rapid intake of phosphate and sufficient phosphate is important for yield of root crops such as carrot, potato and parsnip.

The effect of these modifications is to increase the seedling germination and range of ornamental and crop plants. The utilities of presently disclosed transcription factor genes conferring tolerance to conditions of low nutrients also include cost savings to the grower by reducing the amounts of fertilizer needed, environmental benefits of reduced fertilizer runoff into watersheds; and improved yield and stress tolerance. In addition, by providing improved nitrogen uptake capability, these genes can be used to alter seed protein amounts and/or composition in such a way that could impact yield as well as the nutritional value and production of various food products.

Growth regulator: carbon and nitrogen balance. A number of the transcription factor-overexpressing lines, including, G1988, may be used to produce plants with altered C/N sensing. These plants may, for example, make less anthocyanin on high sucrose plus glutamine, indicating that these genes can be used to modify carbon and nitrogen status, and hence assimilate partitioning (assimilate partitioning refers to the manner in which an essential element, such as nitrogen, is distributed among different pools inside a plant, generally in a reduced form, for the purpose of transport to various tissues).

Flowering time: early and late flowering. Presently disclosed transcription factor genes that accelerate flowering, which include could have valuable applications in such programs, since they allow much faster generation times. In a number of species, for example, broccoli, cauliflower, where the reproductive parts of the plants constitute the crop and the vegetative tissues are discarded, it would be advantageous to accelerate time to flowering. Accelerating flowering could shorten crop and tree breeding programs. Additionally, in some instances, a faster generation time would allow additional harvests of a crop to be made within a given growing season. A number of *Arabidopsis* genes have already been shown to accelerate flowering when constitutively expressed. These include LEAFY, APETALA1 and CONSTANS (Mandel et al. (1995) Nature 377: 522-524; Weigel and Nilsson (1995) Nature 377: 495-500; Simon et al. (1996) Nature 384: 59-62).

By regulating the expression of potential flowering using inducible promoters, flowering could be triggered by application of an inducer chemical. This would allow flowering to be synchronized across a crop and facilitate more efficient harvesting. Such inducible systems could also be used to tune the - flowering of crop varieties to different latitudes. At present, species such as soybean and cotton are available as a series of maturity groups that are suitable for different latitudes on the basis of their flowering time (which is governed by day-length). A system in which flowering could be chemically controlled would allow a single high-yielding northern maturity group to be grown at any latitude. In southern regions such plants could be grown for longer periods before flowering was induced, thereby increasing yields. In more northern areas, the induction would be used to ensure that the crop flowers prior to the first winter frosts.

Presently disclosed transcription factors that extend flowering time have utility in engineering plants with longer-lasting flowers for the horticulture industry, and for extending the time in which the plant is fertile.

Development and morphology: cotyledon, hypocotyl. The morphological phenotypes shown by plants overexpressing several of the transcription factor genes in Table 6 indicate that these genes, including those that produce altered hypocotyls (G1988), can be used to manipulate light responses such as shade avoidance. As these genes also alter plant architecture, they may find use in the ornamental horticulture industry.

Light response/shade avoidance: altered cotyledon, hypocotyl, petiole development, altered leaf orientation, constitutive photomorphogenesis, photomorphogenesis in low light. Presently disclosed transcription factor genes, including G1988, and its equivalogs that can modify a plant's response to light may be useful for modifying plant growth or development, for example, photomorphogenesis in poor light, or accelerating flowering time in response to various light intensities, quality or duration to which a non-transformed plant would not similarly respond. Examples of such responses that have been demonstrated include leaf number and arrangement, and early flower bud appearances. Elimination of shading responses may lead to increased planting densities with subsequent yield enhancement. As these genes may also alter plant architecture, they may find use in the ornamental horticulture industry.

Antisense and Co-suppression

In addition to expression of the nucleic acids of the invention as gene replacement or plant phenotype modification nucleic acids, the nucleic acids are also useful for sense and anti-sense suppression of expression, e.g. to down-regulate expression of a nucleic acid of the disclosure, e.g. as a further mechanism for modulating plant phenotype. That is, the nucleic acids of the invention, or subsequences or anti-sense sequences thereof, can be used to block expression of naturally occurring homologous nucleic acids. A variety of sense and anti-sense technologies are known in the art, e.g. as set forth in Lichtenstein and Nellen (1997) Antisense Technology: A Practical Approach IRL Press at Oxford University Press, Oxford, U.K. Antisense regulation is also described in Crowley et al. (1985) Cell 43: 633-641; Rosenberg et al. (1985) Nature 313: 703-706; Preiss et al. (1985) Nature 313: 27-32; Melton (1985) Proc. Natl. Acad.

Sci. 82: 144-148; Izant and Weintraub (1985) Science 229: 345-352; and Kim and Wold (1985) Cell 42: 129-138. Additional methods for antisense regulation are known in the art. Antisense regulation has been used to reduce or inhibit expression of plant genes in, for example in European Patent Publication No. 271988. Antisense RNA may be used to reduce gene expression to produce a visible or biochemical phenotypic change in a plant (Smith et al. (1988) Nature, 334: 724-726; Smith et al. (1990) Plant Mol. Biol. 14: 369-379). In general, sense or anti-sense sequences are introduced into a cell, where they are optionally amplified, e.g. by transcription. Such sequences include both simple oligonucleotide sequences and catalytic sequences such as ribozymes.

For example, a reduction or elimination of expression (i.e., a "knock-out") of a transcription factor or transcription factor homolog polypeptide in a transgenic plant, e.g., to modify a plant trait, can be obtained by introducing an antisense construct corresponding to the polypeptide of interest as a cDNA. For antisense suppression, the transcription factor or homolog cDNA is arranged in reverse orientation (with respect to the coding sequence) relative to the promoter sequence in the expression vector. The introduced sequence need not be the full length cDNA or gene, and need not be identical to the cDNA or gene found in the plant type to be transformed. Typically, the antisense sequence need only be capable of hybridizing to the target gene or RNA of interest. Thus, where the introduced sequence is of shorter length, a higher degree of homology to the endogenous transcription factor sequence will be needed for effective antisense suppression. While antisense sequences of various lengths can be utilized, preferably, the introduced antisense sequence in the vector will be at least 30 nucleotides in length, and improved antisense suppression will typically be observed as the length of the antisense sequence increases. Preferably, the length of the antisense sequence in the vector will be greater than 100 nucleotides. Transcription of an antisense construct as described results in the production of RNA molecules that are the reverse complement of mRNA molecules transcribed from the endogenous transcription factor gene in the plant cell.

Suppression of endogenous transcription factor gene expression can also be achieved using RNA interference , or RNAi. RNAi is a post-transcriptional, targeted gene-silencing technique that uses double-stranded RNA (dsRNA) to incite degradation of messenger RNA (mRNA) containing the same sequence as the dsRNA (Constans, (2002) The Scientist 16:36). Small interfering RNAs, or siRNAs are produced in at least two steps: an endogenous ribonuclease cleaves longer dsRNA into shorter, 21-23 nucleotide-long RNAs. The siRNA segments then mediate the degradation of the target mRNA (Zamore, (2001) Nature Struct. Biol., 8:746-50). RNAi has been used for gene function determination in a manner similar to antisense oligonucleotides (Constans, (2002) The Scientist 16:36). Expression vectors that continually express siRNAs in transiently and stably transfected have been engineered to express small hairpin RNAs (shRNAs), which get processed in vivo into siRNAs-like molecules capable of carrying out gene-specific silencing (Brummelkamp et al., (2002) Science 296:550-553, and Paddison, et al. (2002) Genes & Dev. 16:948-958). Post-transcriptional gene silencing by double-stranded RNA is discussed in further detail by Hammond et al. (2001) Nature Rev Gen 2: 110-119, Fire et al. (1998) Nature 391: 806-811 and Timmons and Fire (1998) Nature 395: 854. Vectors in which RNA encoded by a transcription factor or transcription factor homolog cDNA is over-expressed can also be used to obtain co-suppression of a corresponding endogenous gene, e.g., in the manner described in US Patent No. 5,231,020 to Jorgensen. Such co-suppression (also termed sense suppression) does not require that the entire transcription factor cDNA be introduced into the plant cells, nor does it require that the introduced sequence be exactly identical to the endogenous transcription factor gene of interest. However, as with antisense suppression, the suppressive efficiency will be enhanced as specificity of hybridization is increased, e.g., as the introduced sequence is lengthened, and/or as the sequence similarity between the introduced sequence and the endogenous transcription factor gene is increased.

Vectors expressing an untranslatable form of the transcription factor mRNA, e.g., sequences comprising one or more stop codon, or nonsense mutation) can also be used to suppress expression of an endogenous transcription factor, thereby reducing or eliminating its activity and modifying one or more traits. Methods for producing such constructs are described in US Patent No. 5,583,021. Preferably, such constructs are made by introducing a premature stop codon into the transcription factor gene. Alternatively, a plant trait can be modified by gene silencing using double-strand RNA (Sharp (1999) Genes and Development 13: 139-141). Another method for abolishing the expression of a gene is by insertion mutagenesis using the T-DNA of *Agrobacterium tumefaciens.* After generating the insertion mutants, the mutants can be screened to identify those containing the insertion in a transcription factor or transcription factor homolog gene. Plants containing a single transgene insertion event at the desired gene can be crossed to generate homozygous plants for the mutation. Such methods are well known to those of skill in the art (See for example Koncz et al. (1992) Methods in Arabidopsis Research, World Scientific Publishing Co. Pte. Ltd., River Edge, NJ).

Alternatively, a plant phenotype can be altered by eliminating an endogenous gene, such as a transcription factor or transcription factor homolog, e.g., by homologous recombination (Kempin et al. (1997) Nature 389: 802-803).

A plant trait can also be modified by using the Cre-lox system (for example, as described in US Pat. No. 5,658,772). A plant genome can be modified to include first and second lox sites that are then contacted with a Cre recombinase. If the lox sites are in the same orientation, the intervening DNA sequence between the two sites is excised. If the lox sites are in the opposite orientation, the intervening sequence is inverted.

The polynucleotides and polypeptides of this disclosure can also be expressed in a plant in the absence of an expression cassette by manipulating the activity or expression level of the endogenous gene by other means, such as, for example, by ectopically expressing a gene by T-DNA activation tagging (Ichikawa et al. (1997) Nature 390 698-701; Kakimoto et al. (1996) Science 274: 982-985). This method entails transforming a plant with a gene tag containing multiple transcriptional enhancers and once the tag has inserted into the genome, expression of a flanking gene coding sequence becomes deregulated. In another example, the transcriptional machinery in a plant can be modified so as to increase transcription levels of a polynucleotide (*See,* e.g., PCT Publications WO 96/06166 and WO 98/53057 which describe the modification of the DNA-binding specificity of zinc finger proteins by changing particular amino acids in the DNA-binding motif).

The transgenic plant can also include the machinery necessary for expressing or altering the activity of a polypeptide encoded by an endogenous gene, for example, by altering the phosphorylation state of the polypeptide to maintain it in an activated state.

Transgenic plants (or plant cells, or plant explants, or plant tissues) incorporating the polynucleotides of the disclosure and/or expressing the polypeptides of the disclosure can be produced by a variety of well established techniques as described above. Following construction of a vector, most typically an expression cassette, including a polynucleotide, e.g., encoding a transcription factor or transcription factor homolog, standard techniques can be used to introduce the polynucleotide into a plant, a plant cell, a plant explant or a plant tissue of interest. Optionally, the plant cell, explant or tissue can be regenerated to produce a transgenic plant.

The plant can be any higher plant, including gymnosperms, monocotyledonous and dicotyledenous plants. Suitable protocols are available for *Leguminosae* (alfalfa, soybean, clover, etc.), *Umbelliferae* (carrot, celery, parsnip), *Cruciferae* (cabbage, radish, rapeseed, broccoli, etc.), *Curcurbitaceae* (melons and cucumber), *Gramineae* (wheat, corn, rice, barley, millet, etc.), *Solanaceae* (potato, tomato, tobacco, peppers, etc.), and various other crops. See protocols described in Ammirato et al., eds., (1984) Handbook of Plant Cell Culture -Crop Species, Macmillan Publ. Co., New York, NY; Shimamoto et al. (1989) Nature 338: 274-276; Fromm et al. (1990) BiolTechnol. 8: 833-839; and Vasil et al. (1990) Bio/Technol. 8: 429-434.

Transformation and regeneration of both monocotyledonous and dicotyledonous plant cells is now routine, and the selection of the most appropriate transformation technique will be determined by the practitioner. The choice of method will vary with the type of plant to be transformed; those skilled in the art will recognize the suitability of particular methods for given plant types. Suitable methods can include, but are not limited to: electroporation of plant protoplasts; liposome-mediated transformation; polyethylene glycol (PEG) mediated transformation; transformation using viruses; micro-injection of plant cells; micro-projectile bombardment of plant cells; vacuum infiltration; and *Agrobacterium tumefaciens* mediated transformation. Transformation means introducing a nucleotide sequence into a plant in a manner to cause stable or transient expression of the sequence.

Successful examples of the modification of plant characteristics by transformation with cloned sequences which serve to illustrate the current knowledge in this field of technology, and which are herein incorporated by reference, include: US Patent Nos. 5,571,706; 5,677,175; 5,510,471; 5,750,386; 5,597,945; 5,589,615; 5,750,871; 5,268,526; 5,780,708; 5,538,880; 5,773,269; 5,736,369 and 5,610,042.

Following transformation, plants are preferably selected using a dominant selectable marker incorporated into the transformation vector. Typically, such a marker will confer antibiotic or herbicide resistance on the transformed plants, and selection of transformants can be accomplished by exposing the plants to appropriate concentrations of the antibiotic or herbicide.

After transformed plants are selected and grown to maturity, those plants showing a modified trait are identified. The modified trait can be any of those traits described above. Additionally, to confirm that the modified trait is due to changes in expression levels or activity of the polypeptide or polynucleotide can be determined by analyzing mRNA expression using Northern blots, RT-PCR or microarrays, or protein expression using immunoblots or Western blots or gel shift assays.

Integrated Systems - Sequence Identity

Additionally, described herein is an integrated system, i.e. a computer or computer readable medium that comprises an instruction set for determining the identity of one or more sequences in a database. In addition, the instruction set can be used to generate or identify sequences that meet any specified criteria. Furthermore, the instruction set may be used to associate or link certain functional benefits, such improved characteristics, with one or more identified sequence.

For example, the instruction set can include, e.g., a sequence comparison or other alignment program, e.g., an available program such as, for example, the Wisconsin Package Version 10.0, such as BLAST, FASTA, PILEUP, FINDPATTERNS or the like (GCG, Madison, WI). Public sequence databases such as GenBank, EMBL, Swiss-Prot and PIR or private sequence databases such as PHYTOSEQ sequence database (Incyte Genomics, Palo Alto, CA) can be searched.

Alignment of sequences for comparison can be conducted by the local homology algorithm of Smith and Waterman (1981) Adv. Appl. Math. 2: 482-489, by the homology alignment algorithm of Needleman and Wunsch (1970) J. Mol. Biol. 48: 443-453, by the search for similarity method of Pearson and Lipman (1988) Proc. Natl. Acad. Sci. 85: 2444-2448, by computerized implementations of these algorithms. After alignment, sequence comparisons between two (or more) polynucleotides or polypeptides are typically performed by comparing sequences of the two sequences over a comparison window to identify and compare local regions of sequence similarity. The comparison window can be a segment of at least about 20 contiguous positions, usually about 50 to about 200, more usually about 100 to about 150 contiguous positions. A description of the method is provided in Ausubel et al. *supra.*

A variety of methods for determining sequence relationships can be used, including manual alignment and computer assisted sequence alignment and analysis. This later approach is a preferred approach, due to the increased throughput afforded by computer assisted methods. As noted above, a variety of computer programs for performing sequence alignment are available, or can be produced by one of skill.

One example algorithm that is suitable for determining percent sequence identity and sequence similarity is the BLAST algorithm, which is described in Altschul et al. (1990) J. Mol. Biol. 215: 403-410. Software for performing BLAST analyses is publicly available, e.g., through the National Library of Medicine's National Center for Biotechnology Information (ncbi.nlm.nih; see at world wide web (www) National Institutes of Health US government (gov) website). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al. *supra*). These initial neighborhood word hits act as seeds for initiating searches to fmd longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (see Henikoff and Henikoff (1992) Proc. Natl. Acad. Sci. 89: 10915-10919). Unless otherwise indicated, "sequence identity" here refers to the % sequence identity generated from a tblastx using the NCBI version of the algorithm at the default settings using gapped alignments with the filter "off" (see, for example, NIH NLM NCBI website at ncbi.nlm.nih, *supra*).

In addition to calculating percent sequence identity, the BLAST algorithm also performs a statistical analysis of the similarity between two sequences (see, e.g. Karlin and Altschul (1993) Proc. Natl. Acad. Sci. 90: 5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence (and, therefore, in this context, homologous) if the smallest sum probability in a comparison of the test nucleic acid to the reference nucleic acid is less than about 0.1, or less than about 0.01, and or even less than about 0.001. An additional example of a useful sequence alignment algorithm is PILEUP. PILEUP creates a multiple sequence alignment from a group of related sequences using progressive, pairwise alignments. The program can align, e.g., up to 300 sequences of a maximum length of 5,000 letters.

The integrated system, or computer typically includes a user input interface allowing a user to selectively view one or more sequence records corresponding to the one or more character strings, as well as an instruction set which aligns the one or more character strings with each other or with an additional character string to identify one or more region of sequence similarity. The system may include a link of one or more character strings with a particular phenotype or gene function. Typically, the system includes a user readable output element that displays an alignment produced by the alignment instruction set.

The above methods can be implemented in a localized or distributed computing environment. In a distributed environment, the methods may implemented on a single computer comprising multiple processors or on a multiplicity of computers. The computers can be linked, e.g. through a common bus, but more preferably the computer(s) are nodes on a network. The network can be a generalized or a dedicated local or wide-area network and, in certain preferred embodiments, the computers may be components of an intra-net or an internet.

Thus, the disclosure provides methods for identifying a sequence similar or homologous to one or more polynucleotides as noted herein, or one or more target polypeptides encoded by the polynucleotides, or otherwise noted herein and may include linking or associating a given plant phenotype or gene function with a sequence. In the methods, a sequence database is provided (locally or across an inter or intra net) and a query is made against the sequence database using the relevant sequences herein and associated plant phenotypes or gene functions.

Any sequence herein can be entered into the database, before or after querying the database. This provides for both expansion of the database and, if done before the querying step, for insertion of control sequences into the database. The control sequences can be detected by the query to ensure the general integrity of both the database and the query. As noted, the query can be performed using a web browser based interface. For example, the database can be a centralized public database such as those noted herein, and the querying can be done from a remote terminal or computer across an internet or intranet.

Any sequence herein can be used to identify a similar, homologous, paralogous, or orthologous sequence in another plant. This provides means for identifying endogenous sequences in other plants that may be useful to alter a trait of progeny plants, which results from crossing two plants of different strain. For example, sequences that encode an ortholog of any of the sequences herein that naturally occur in a plant with a desired trait can be identified using the sequences disclosed herein. The plant is then crossed with a second plant of the same species but which does not have the desired trait to produce progeny which can then be used in further crossing experiments to produce the desired trait in the second plant. Therefore the resulting progeny plant contains no transgenes; expression of the endogenous sequence may also be regulated by treatment with a particular chemical or other means, such as EMR. Some examples of such compounds well known in the art include: ethylene; cytokinins; phenolic compounds, which stimulate the transcription of the genes needed for infection; specific monosaccharides and acidic environments which potentiate vir gene induction; acidic polysaccharides which induce one or more chromosomal genes; and opines; other mechanisms include light or dark treatment (for a review of examples of such treatments, see, Winans (1992) Microbiol. Rev. 56: 12-31; Eyal et al. (1992) Plant Mol. Biol. 19: 589-599; Chrispeels et al. (2000) Plant Mol. Biol. 42: 279-290; Piazza et al. (2002) Plant Physiol. 128: 1077-1086).

Table 7 lists sequences discovered to be orthologous to a number of representative transcription factors of the present disclosure. The column headings include the transcription factors listed by (a) the SEQ ID NO: of the *Arabidopsis* sequence that was used to discover the non-*Arabidopsis* orthologous sequence; (b) the GID sequence identifier of the *Arabidopsis* sequence; (c) the Sequence Identifier or GenBank Accession Number of the orthologous sequence;(d) the species from which the orthologous sequence is derived; (e) the SEQ ID NO: of the non-*Arabidopsis* orthologous sequence, and (e) the smallest sum probability pairwise comparison of each orthologous sequence to the similar *Arabidopsis* sequence determined by BLAST analysis.

Table 7. Orthologs of Representative *Arabidopsis* Transcription Factor Genes

| SEQ ID NO: of *Arabidopsis* Sequence Used to Discover Ortholog | GID No. | Species from Which Ortholog is Derived | Sequence Identifier or Accession Number | SEQ ID NO: of Orthologous Sequence | Smallest Sum Probability to Ortholog, When Known |
|---|---|---|---|---|---|
| 327 | G1988 | *Glycine max* | GLYMA-28NOV01-CLUSTER75453_1 | 1098 | |
| 327 | G1988 | *Glycine max* | GLYMA-28NOV01-CLUSTER75453 2 | 1099 | |
| 327 | G1988 | *Oryza sativa* | ORYSA-22JAN02-CLUSTER153439 2 | 1100 | |
| 327 | G1988 | *Zea mays* | ZEAMA-08NOV01-CLUSTER10890_1 | 1101 | |
| 327 | G1988 | *Zea mays* | ZEAMA-08NOV01-CLUSTER10890_3 | 1102 | |
| 327 | G1988 | *Zea mays* | ZEAMA-08NOV01-CLUSTER201962_1 | 1103 | |
| 327 | G1988 | *Zea mays* | ZEAMA-08NOV01-CLUSTER3040 3 | 1104 | |
| 327 | G1988 | *Oryza sativa* | Os_S91481 | 1601 | |
| 327 | G1988 | *Lycopersicon esculentum* | SGN-UNIGENE-SINGLET-5090 | 2045 | |
| 328 | G1988 | *Brassica oleracea* | BH478747 | | 5.00E-23 |

(continued)

| SEQ ID NO: of *Arabidopsis* Sequence Used to Discover Ortholog | GID No. | Species from Which Ortholog is Derived | Sequence Identifier or Accession Number | SEQ ID NO: of Orthologous Sequence | Smallest Sum Probability to Ortholog, When Known |
|---|---|---|---|---|---|
| 328 | G1988 | *Populus balsamifera* subsp. *trichocarpa* | BU873581 | | 7.00E-22 |
| 328 | G1988 | *Citrus unshiu* | C95300 | | 2.00E-18 |
| 328 | G1988 | *Lycopersicon esculentum* | AW034552 | | 2.00E-18 |
| 328 | G1988 | *Oryza sativa* (*indica* cultivar-group) | AAAA01000340 | | 1.00E-17 |
| 328 | G1988 | *Beta vulgaris* | BQ594583 | | 1.00E-16 |
| 328 | G 1988 | *Zea mays* | CC655765 | | 2.00E-15 |
| 328 | G1988 | *Glycine max* | BI469275 | | 8.00E-15 |
| 328 | G1988 | *Prunus persica* | BU046688 | | 7.00E-14 |
| 328 | G1988 | *Vitis vinifera* | CD719941 | | 2.00E-13 |
| 328 | G1988 | *Malus x domestica* | gi4091806 | | 2.60E-07 |
| 328 | G1988 | *Brassica napus* | gi30984027 | | 1.10E-06 |
| 328 | G1988 | *Brassica nigra* | gi22854920 | | 1.10E-06 |
| 328 | G1988 | *Raphanus sativus* | gi3341723 | | 2.70E-06 |
| 328 | G1988 | *Oryza sativa (japonica* cultivar-group) | gi32488104 | | 4.80E-06 |
| 328 | G1198 | *Ipomoea nil* | gi10946337 | | 5.10E-06 |
| 328 | G1988 | *Oryza sativa* | gi11094211 | | 2.20E-05 |
| 328 | G1988 | *Hordeum vulgare* | gi21667475 | | 4.50E-05 |
| 328 | G1988 | *Hordeum vulgare* subsp. *vulgare* | gi21655168 | | 0.00018 |
| 328 | G1988 | *Pinus radiata* | gi4557093 | | 0.0016 |

Table 9 lists the gene identification number (GID) and relationships for homologous (found using analyses according to Example IX) and variant sequences for the sequences of the Sequence Listing.

Table 9. Similarity relationships found within the Sequence Listing

| SEQ ID NO: | GID | DNA or Protein (PRT) | Species from which Sequence is Derived | Relationship |
|---|---|---|---|---|
| 1098 | | DNA | *Glycine max* | Predicted polypeptide sequence is orthologous to G1988 |

(continued)

| SEQ ID NO: | GID | DNA or Protein (PRT) | Species from which Sequence is Derived | Relationship |
|---|---|---|---|---|
| 1099 | | DNA | *Glycine max* | Predicted polypeptide sequence is orthologous to G1988 |
| 1100 | | DNA | *Oryza sativa* | Predicted polypeptide sequence is orthologous to G1988 |
| 1101 | | DNA | *Zea mays* | Predicted polypeptide sequence is orthologous to G1988 |
| 1102 | | DNA | *Zea mays* | Predicted polypeptide sequence is orthologous to G1988 |
| 1103 | | DNA | *Zea mays* | Predicted polypeptide sequence is orthologous to G1988 |
| 1104 | | DNA | *Zea mays* | Predicted polypeptide sequence is orthologous to G1988 |
| 1601 | | DNA | *Oryza sativa* | Predicted polypeptide sequence is orthologous to G1988 |
| 2045 | | DNA | *Lycopersicon esculentum* | Predicted polypeptide sequence is orthologous to G1988 |

**EXAMPLES**

The invention, now being generally described, will be more readily understood by reference to the following examples, which are included merely for purposes of illustration of certain aspects and embodiments of the present invention and are not intended to limit the invention. It will be recognized by one of skill in the art that a transcription factor that is associated with a particular first trait may also be associated with at least one other, unrelated and inherent second trait which was not predicted by the first trait.

The complete descriptions of the traits associated with each polynucleotide of the invention are fully disclosed in Table 4 and Table 6. The complete description of the transcription factor gene family and identified conserved domains of the polypeptide encoded by the polynucleotide is fully disclosed in Table 5.

**Example I: Full Length Gene Identification and Cloning**

Putative transcription factor sequences (genomic or ESTs) related to known transcription factors were identified in the *Arabidopsis thaliana* GenBank database using the tblastn sequence analysis program using default parameters and a P-value cutoff threshold of -4 or -5 or lower, depending on the length of the query sequence. Putative transcription factor sequence hits were then screened to identify those containing particular sequence strings. If the sequence hits contained such sequence strings, the sequences were confirmed as transcription factors.

Alternatively, *Arabidopsis thaliana* cDNA libraries derived from different tissues or treatments, or genomic libraries were screened to identify novel members of a transcription family using a low stringency hybridization approach. Probes were synthesized using gene specific primers in a standard PCR reaction (annealing temperature 60°C) and labeled with $^{32}P$ dCTP using the High Prime DNA Labeling Kit (Boehringer Mannheim Corp. (now Roche Diagnostics Corp., Indianapolis, IN). Purified radiolabelled probes were added to filters immersed in Church hybridization medium (0.5 M $NaPO_4$ pH 7.0, 7% SDS, 1% w/v bovine serum albumin) and hybridized overnight at 60°C with shaking. Filters were washed two times for 45 to 60 minutes with 1xSCC, 1% SDS at 60° C.

To identify additional sequence 5' or 3' of a partial cDNA sequence in a cDNA library, 5' and 3' rapid amplification of cDNA ends (RACE) was performed using the MARATHON cDNA amplification kit (Clontech, Palo Alto, CA). Generally, the method entailed first isolating poly(A) mRNA, performing first and second strand cDNA synthesis to generate double stranded cDNA, blunting cDNA ends, followed by ligation of the MARATHON Adaptor to the cDNA to form a library of adaptor-ligated ds cDNA.

Gene-specific primers were designed to be used along with adaptor specific primers for both 5' and 3' RACE reactions. Nested primers, rather than single primers, were used to increase PCR specificity. Using 5' and 3' RACE reactions, 5' and 3' RACE fragments were obtained, sequenced and cloned. The process can be repeated until 5' and 3' ends of the full-length gene were identified. Then the full-length cDNA was generated by PCR using primers specific to 5' and 3' ends of the gene by end-to-end PCR.

**Example II: Construction of Expression Vectors**

The sequence was amplified from a genomic or cDNA library using primers specific to sequences upstream and downstream of the coding region. The expression vector was pMEN20 or pMEN65, which are both derived from pMON316 (Sanders et al. (1987) Nucleic Acids Res. 15:1543-1558) and contain the CaMV 35S promoter to express transgenes. To clone the sequence into the vector, both pMEN20 and the amplified DNA fragment were digested separately with SAII and NotI restriction enzymes at 37° C for 2 hours. The digestion products were subject to electrophoresis in a 0.8% agarose gel and visualized by ethidium bromide staining. The DNA fragments containing the sequence and the linearized plasmid were excised and purified by using a QIAQUICK gel extraction kit (Qiagen, Valencia CA). The fragments of interest were ligated at a ratio of 3:1 (vector to insert). Ligation reactions using T4 DNA ligase (New England Biolabs, Beverly MA) were carried out at 16° C for 16 hours. The ligated DNAs were transformed into competent cells of the *E. coli* strain DH5alpha by using the heat shock method. The transformations were plated on LB plates containing 50 mg/l kanamycin (Sigma Chemical Co. St. Louis MO). Individual colonies were grown overnight in five milliliters of LB broth containing 50 mg/l kanamycin at 37° C. Plasmid DNA was purified by using Qiaquick Mini Prep kits (Qiagen).

**Example III: Transformation of *Agrobacterium* with the Expression Vector**

After the plasmid vector containing the gene was constructed, the vector was used to transform *Agrobacterium tumefaciens* cells expressing the gene products. The stock of *Agrobacterium tumefaciens* cells for transformation were made as described by Nagel et al. (1990) FEMSMicrobiol Letts. 67: 325-328. *Agrobacterium* strain ABI was grown in 250 ml LB medium (Sigma) overnight at 28°C with shaking until an absorbance over 1 cm at 600 nm ($A_{600}$) of 0.5 -1.0 was reached. Cells were harvested by centrifugation at 4,000 x g for 15 min at 4° C. Cells were then resuspended in 250 μl chilled buffer (1 mM HEPES, pH adjusted to 7.0 with KOH). Cells were centrifuged again as described above and resuspended in 125 μl chilled buffer. Cells were then centrifuged and resuspended two more times in the same HEPES buffer as described above at a volume of 100 μl and 750 μl, respectively. Resuspended cells were then distributed into 40 μl aliquots, quickly frozen in liquid nitrogen, and stored at -80° C.

*Agrobacterium* cells were transformed with plasmids prepared as described above following the protocol described by Nagel et al. (*supra*). For each DNA construct to be transformed, 50 - 100 ng DNA (generally resuspended in 10 mM Tris-HCl, 1 mM EDTA, pH 8.0) was mixed with 40 μl of *Agrobacterium* cells. The DNA/cell mixture was then transferred to a chilled cuvette with a 2mm electrode gap and subject to a 2.5 kV charge dissipated at 25 μF and 200 μF using a Gene Pulser II apparatus (Bio-Rad, Hercules, CA). After electroporation, cells were immediately resuspended in 1.0 ml LB and allowed to recover without antibiotic selection for 2 - 4 hours at 28° C in a shaking incubator. After recovery, cells were plated onto selective medium of LB broth containing 100 μg/ml spectinomycin (Sigma) and incubated for 24-48 hours at 28° C. Single colonies were then picked and inoculated in fresh medium. The presence of the plasmid construct was verified by PCR amplification and sequence analysis.

**Example IV: Transformation of *Arabidopsis* Plants with *Agrobacterium tumefaciens* with Expression Vector**

After transformation of *Agrobacterium tumefaciens* with plasmid vectors containing the gene, single *Agrobacterium* colonies were identified, propagated, and used to transform *Arabidopsis* plants. Briefly, 500 ml cultures of LB medium containing 50 mg/l kanamycin were inoculated with the colonies and grown at 28° C with shaking for 2 days until an optical absorbance at 600 nm wavelength over 1 cm ($A_{600}$) of > 2.0 is reached. Cells were then harvested by centrifugation at 4,000 x g for 10 min, and resuspended in infiltration medium (1/2 X Murashige and Skoog salts (Sigma), 1 X Gamborg's B-5 vitamins (Sigma), 5.0% (w/v) sucrose (Sigma), 0.044 μM benzylamino purine (Sigma), 200 μl/l Silwet L-77 (Lehle Seeds) until an $A_{600}$ of 0.8 was reached.

Prior to transformation, *Arabidopsis thaliana* seeds (ecotype Columbia) were sown at a density of ~10 plants per 4" pot onto Pro-Mix BX potting medium (Hummert International) covered with fiberglass mesh (18 mm X 16 mm). Plants were grown under continuous illumination (50-75 μE/$m^2$/sec) at 22-23° C with 65-70% relative humidity. After about 4 weeks, primary inflorescence stems (bolts) are cut off to encourage growth of multiple secondary bolts. After flowering of the mature secondary bolts, plants were prepared for transformation by removal of all siliques and opened flowers.

The pots were then immersed upside down in the mixture of *Agrobacterium* infiltration medium as described above for 30 sec, and placed on their sides to allow draining into a 1' x 2' flat surface covered with plastic wrap. After 24 h, the plastic wrap was removed and pots are turned upright. The immersion procedure was repeated one week later, for a total of two immersions per pot. Seeds were then collected from each transformation pot and analyzed following the protocol described below.

### Example V: Identification of *Arabidopsis* Primary Transformants

Seeds collected from the transformation pots were sterilized essentially as follows. Seeds were dispersed into in a solution containing 0.1% (v/v) Triton X-100 (Sigma) and sterile water and washed by shaking the suspension for 20 min. The wash solution was then drained and replaced with fresh wash solution to wash the seeds for 20 min with shaking. After removal of the ethanol/detergent solution, a solution containing 0.1% (v/v) Triton X-100 and 30% (v/v) bleach (CLOROX; Clorox Corp. Oakland CA) was added to the seeds, and the suspension was shaken for 10 min. After removal of the bleach/detergent solution, seeds were then washed five times in sterile distilled water. The seeds were stored in the last wash water at 4° C for 2 days in the dark before being plated onto antibiotic selection medium (1 X Murashige and Skoog salts (pH adjusted to 5.7 with 1M KOH), 1 X Gamborg's B-5 vitamins, 0.9% phytagar (Life Technologies), and 50 mg/l kanamycin). Seeds were germinated under continuous illumination (50-75 μE/$m^2$/sec) at 22-23° C. After 7-10 days of growth under these conditions, kanamycin resistant primary transformants (T1 generation) were visible and obtained. These seedlings were transferred first to fresh selection plates where the seedlings continued to grow for 3-5 more days, and then to soil (Pro-Mix BX potting medium).

Primary transformants were crossed and progeny seeds ($T_2$) collected; kanamycin resistant seedlings were selected and analyzed. The expression levels of the recombinant polynucleotides in the transformants varies from about a 5% expression level increase to a least a 100% expression level increase. Similar observations are made with respect to polypeptide level expression.

### Example VI: Identification *of Arabidopsis* Plants with Transcription Factor Gene Knockouts

The screening of insertion mutagenized *Arabidopsis* collections for null mutants in a known target gene was essentially as described in Krysan et al. (1999) Plant Cell 11: 2283-2290. Briefly, gene-specific primers, nested by 5-250 base pairs to each other, were designed from the 5' and 3' regions of a known target gene. Similarly, nested sets of primers were also created specific to each of the T-DNA or transposon ends (the "right" and "left" borders). All possible combinations of gene specific and T-DNA/transposon primers were used to detect by PCR an insertion event within or close to the target gene. The amplified DNA fragments were then sequenced which allows the precise determination of the T-DNA/transposon insertion point relative to the target gene. Insertion events within the coding or intervening sequence of the genes were deconvoluted from a pool comprising a plurality of insertion events to a single unique mutant plant for functional characterization. The method is described in more detail in Yu and Adam, US Application Serial No. 09/177,733 filed October 23, 1998.

### Example VII: Identification of Modified Phenotypes in Overexpression or Gene Knockout Plants.

Experiments were performed to identify those transformants or knockouts that exhibited modified biochemical characteristics. Among the biochemicals that were assayed were insoluble sugars, such as arabinose, fucose, galactose, mannose, rhamnose or xylose or the like; prenyl lipids, such as lutein, beta-carotene, xanthophyll-1, xanthophyll-2, chlorophylls A or B, or alpha-, delta- or gamma-tocopherol or the like; fatty acids, such as 16:0 (palmitic acid), 16:1 (palmitoleic acid), 18:0 (stearic acid), 18:1 (oleic acid), 18:2 (linoleic acid), 20:0 , 18:3 (linolenic acid), 20:1 (eicosenoic acid), 20:2, 22:1 (erucic acid) or the like; waxes, such as by altering the levels of C29, C31, or C33 alkanes; sterols, such as brassicasterol, campesterol, stigmasterol, sitosterol or stigmastanol or the like, glucosinolates, protein or oil levels.

Fatty acids were measured using two methods depending on whether the tissue was from leaves or seeds. For leaves, lipids were extracted and esterified with hot methanolic $H_2SO_4$ and partitioned into hexane from methanolic brine. For seed fatty acids, seeds were pulverized and extracted in methanol:heptane:toluene:2,2-dimethoxypropane: $H_2SO_4$ (39:34:20:5:2) for 90 minutes at 80°C. After cooling to room temperature the upper phase, containing the seed fatty acid esters, was subjected to GC analysis. Fatty acid esters from both seed and leaf tissues were analyzed with a SUPELCO SP-2330 column (Supelco, Bellefonte, PA).

Glucosinolates were purified from seeds or leaves by first heating the tissue at 95°C for 10 minutes. Preheated ethanol:water (50:50) is added and after heating at 95°C for a further 10 minutes, the extraction solvent is applied to a DEAE SEPHADEX column (Pharmacia) which had been previously equilibrated with 0.5 M pyridine acetate. Desulfoglucosinolates were eluted with 300 ul water and analyzed by reverse phase HPLC monitoring at 226 nm.

For wax alkanes, samples were extracted using an identical method as fatty acids and extracts were analyzed on a HP 5890 GC coupled with a 5973 MSD. Samples were chromatographically isolated on a J&W DB35 mass spectrometer (J&W Scientific Agilent Technologies, Folsom, CA).

To measure prenyl lipid levels, seeds or leaves were pulverized with 1 to 2% pyrogallol as an antioxidant. For seeds, extracted samples were filtered and a portion removed for tocopherol and carotenoid/chlorophyll analysis by HPLC. The remaining material was saponified for sterol determination. For leaves, an aliquot was removed and diluted with methanol and chlorophyll A, chlorophyll B, and total carotenoids measured by spectrophotometry by determining optical absorbance at 665.2 nm, 652.5 nm, and 470 nm. An aliquot was removed for tocopherol and carotenoid/chlorophyll composition by HPLC using a Waters μBondapak C18 column (4.6 mm x 150 mm). The remaining methanolic solution was saponified with 10% KOH at 80° C for one hour. The samples were cooled and diluted with a mixture of methanol and water. A solution of 2% methylene chloride in hexane was mixed in and the samples were centrifuged. The aqueous methanol phase was again re-extracted 2% methylene chloride in hexane and, after centrifugation, the two upper phases were combined and evaporated. 2% methylene chloride in hexane was added to the tubes and the samples were then extracted with one ml of water. The upper phase was removed, dried, and resuspended in 400 ul of 2% methylene chloride in hexane and analyzed by gas chromatography using a 50 m DB-5ms (0.25 mm ID, 0.25 um phase, J&W Scientific).

Insoluble sugar levels were measured by the method essentially described by Reiter et al. (1999), Plant J. 12: 335-345. This method analyzes the neutral sugar composition of cell wall polymers found in *Arabidopsis* leaves. Soluble sugars were separated from sugar polymers by extracting leaves with hot 70% ethanol. The remaining residue containing the insoluble polysaccharides was then acid hydrolyzed with allose added as an internal standard. Sugar monomers generated by the hydrolysis were then reduced to the corresponding alditols by treatment with NaBH4, then were acetylated to generate the volatile alditol acetates which were then analyzed by GC-FID. Identity of the peaks was determined by comparing the retention times of known sugars converted to the corresponding alditol acetates with the retention times of peaks from wild-type plant extracts. Alditol acetates were analyzed on a Supelco SP-2330 capillary column (30 m x 250 μm x 0.2 μm) using a temperature program beginning at 180° C for 2 minutes followed by an increase to 220° C in 4 minutes. After holding at 220° C for 10 minutes, the oven temperature is increased to 240° C in 2 minutes and held at this temperature for 10 minutes and brought back to room temperature.

To identify plants with alterations in total seed oil or protein content, 150mg of seeds from T2 progeny plants were subjected to analysis by Near Infrared Reflectance Spectroscopy (NIRS) using a Foss NirSystems Model 6500 with a spinning cup transport system. NIRS is a non-destructive analytical method used to determine seed oil and protein composition. Infrared is the region of the electromagnetic spectrum located after the visible region in the direction of longer wavelengths. 'Near infrared' owns its name for being the infrared region near to the visible region of the electromagnetic spectrum. For practical purposes, near infrared comprises wavelengths between 800 and 2500 nm. NIRS is applied to organic compounds rich in O-H bonds (such as moisture, carbohydrates, and fats), C-H bonds (such as organic compounds and petroleum derivatives), and N-H bonds (such as proteins and amino acids). The NIRS analytical instruments operate by statistically correlating NIRS signals at several wavelengths with the characteristic or property intended to be measured. All biological substances contain thousands of C-H, O-H, and N-H bonds. Therefore, the exposure to near infrared radiation of a biological sample, such as a seed, results in a complex spectrum which contains qualitative and quantitative information about the physical and chemical composition of that sample.

The numerical value of a specific analyte in the sample, such as protein content or oil content, is mediated by a calibration approach known as chemometrics. Chemometrics applies statistical methods such as multiple linear regression (MLR), partial least squares (PLS), and principle component analysis (PCA) to the spectral data and correlates them with a physical property or other factor, that property or factor is directly determined rather than the analyte concentration itself. The method first provides "wet chemistry" data of the samples required to develop the calibration.

Calibration of NIRS response was performed using data obtained by wet chemical analysis of a population of *Arabidopsis* ecotypes that were expected to represent diversity of oil and protein levels.

The exact oil composition of each ecotype used in the calibration experiment was performed using gravimetric analysis of oils extracted from seed samples (0.5 g or 1.0 g) by the accelerated solvent extraction method (ASE; Dionex Corp, Sunnyvale, CA). The extraction method was validated against certified canola samples (Community Bureau of Reference, Belgium). Seed samples from each ecotype (0.5 g or 1g) were subjected to accelerated solvent extraction and the resulting extracted oil weights compared to the weight of oil recovered from canola seed that has been certified for oil content (Community Bureau of Reference). The oil calibration equation was based on 57 samples with a range of oil contents from 27.0 % to 50.8 %. To check the validity of the calibration curve, an additional set of samples was extracted by ASE and predicted using the oil calibration equation. This validation set counted 46 samples, ranging from 27.9 % to 47.5 % oil, and had a predicted standard error of performance of 0.63 %. The wet chemical method for protein was elemental analysis (%N X 6.0) using the average of 3 representative samples of 5 mg each validated against certified ground corn (NIST). The instrumentation was an Elementar Vario-EL III elemental analyzer operated in CNS operating mode (Elementar Analysensysteme GmbH, Hanau, Germany).

The protein calibration equation was based on a library of 63 samples with a range of protein contents from 17.4 % to 31.2 %. An additional set of samples was analyzed for protein by elemental analysis (n = 57) and scanned by NIRS in order to validate the protein prediction equation. The protein range of the validation set was from 16.8 % to 31.2 % and the standard error of prediction was 0.468 %.

NIRS analysis of *Arabidopsis* seed was carried out on between 40-300 mg experimental sample. The oil and protein contents were predicted using the respective calibration equations.

Data obtained from NIRS analysis was analyzed statistically using a nearest-neighbor (N-N) analysis. The N-N analysis allows removal of within-block spatial variability in a fairly flexible fashion, which does not require prior knowledge of the pattern of variability in the chamber. Ideally, all hybrids are grown under identical experimental conditions within a block (rep). In reality, even in many block designs, significant within-block variability exists. Nearest-neighbor procedures are based on assumption that environmental effect of a plot is closely related to that of its neighbors. Nearest-neighbor methods use information from adjacent plots to adjust for within-block heterogeneity and so provide more precise estimates of treatment means and differences. If there is within-plot heterogeneity on a spatial scale that is larger than a single plot and smaller than the entire block, then yields from adjacent plots will be positively correlated. Information from neighboring plots can be used to reduce or remove the unwanted effect of the spatial heterogeneity, and hence improve the estimate of the treatment effect. Data from neighboring plots can also be used to reduce the influence of competition between adjacent plots. The Papadakis N-N analysis can be used with designs to remove within-block variability that would not be removed with the standard split plot analysis ((Papadakis (1973) Inst. d'Amelior. Plantes Thessaloniki (Greece) Bull. Scientif. No. 23; Papadakis (1984) Proc. Acad. Athens 59: 326-342).

Experiments were performed to identify those transformants or knockouts that exhibited modified sugar sensing. For such studies, seeds from transformants were germinated on media containing 5% glucose or 9.4% sucrose which normally partially restrict hypocotyl elongation. Plants with altered sugar sensing may have either longer or shorter hypocotyls than normal plants when grown on this media. Additionally, other plant traits may be varied such as root mass.

Experiments may be performed to identify those transformants or knockouts that exhibited an improved pathogen tolerance. For such studies, the transformants are exposed to biotropic fungal pathogens, such as *Erysiphe orontii,* and necrotropic fungal pathogens, such as *Fusarium oxysporum. Fusarium oxysporum* isolates cause vascular wilts and damping off of various annual vegetables, perennials and weeds (Mauch-Mani and Slusarenko (1994) Molec Plant-Microbe Interact. 7: 378-383). For *Fusarium oxysporum* experiments, plants are grown on Petri dishes and sprayed with a fresh spore suspension of *F. oxysporum.* The spore suspension is prepared as follows: A plug of fungal hyphae from a plate culture is placed on a fresh potato dextrose agar plate and allowed to spread for one week. Five ml sterile water is then added to the plate, swirled, and pipetted into 50 ml Armstrong *Fusarium* medium. Spores are grown overnight in *Fusarium* medium and then sprayed onto plants using a Preval paint sprayer. Plant tissue is harvested and frozen in liquid nitrogen 48 hours post-infection.

*Erysiphe orontii* is a causal agent of powdery mildew. For *Erysiphe orontii* experiments, plants are grown approximately 4 weeks in a greenhouse under 12 hour light (20°C, ~30% relative humidity (rh)). Individual leaves are infected with *E. orontii* spores from infected plants using a camel's hair brush, and the plants are transferred to a Percival growth chamber (20°C, 80% rh.). Plant tissue is harvested and frozen in liquid nitrogen 7 days post-infection.

*Botrytis cinerea* is a necrotrophic pathogen. *Botrytis cinerea* is grown on potato dextrose agar under 12 hour light (20°C, ~30% relative humidity (rh)). A spore culture is made by spreading 10 ml of sterile water on the fungus plate, swirling and transferring spores to 10 ml of sterile water. The spore inoculum (approx. 105 spores/ml) is then used to spray 10 day-old seedlings grown under sterile conditions on MS (minus sucrose) media. Symptoms are evaluated every day up to approximately 1 week.

*Sclerotinia sclerotiorum* hyphal cultures are grown in potato dextrose broth. One gram of hyphae is ground, filtered, spun down and resuspended in sterile water. A 1:10 dilution is used to spray 10 day-old seedlings grown aseptically under a 12 hour light/dark regime on MS (minus sucrose) media. Symptoms are evaluated every day up to approximately 1 week.

*Pseudomonas syringae* pv maculicola (Psm) strain 4326 and pv maculicola strain 4326 was inoculated by hand at two doses. Two inoculation doses allows the differentiation between plants with enhanced susceptibility and plants with enhanced resistance to the pathogen. Plants are grown for 3 weeks in the greenhouse, then transferred to the growth chamber for the remainder of their growth. Psm ES4326 may be hand inoculated with 1 ml syringe on 3 fully-expanded leaves per plant (4 1/2 wk old), using at least 9 plants per overexpressing line at two inoculation doses, OD=0.005 and OD=0.0005. Disease scoring is performed at day 3 post-inoculation with pictures of the plants and leaves taken in parallel.

In some instances, expression patterns of the pathogen-induced genes (such as defense genes) may be monitored by microarray experiments. In these experiments, cDNAs are generated by PCR and resuspended at a final concentration of ~ 100 ng/ul in 3X SSC or 150mM Na-phosphate (Eisen and Brown (1999) Methods Enzymol. 303: 179-205). The cDNAs are spotted on microscope glass slides coated with polylysine. The prepared cDNAs are aliquoted into 384 well plates and spotted on the slides using, for example, an x-y-z gantry (OmniGrid) which may be purchased from GeneMachines (Menlo Park, CA) outfitted with quill type pins which may be purchased from Telechem International (Sunnyvale, CA). After spotting, the arrays are cured for a minimum of one week at room temperature, rehydrated and blocked following the protocol recommended by Eisen and Brown (1999; supra).

Sample total RNA (10 μg) samples are labeled using fluorescent Cy3 and Cy5 dyes. Labeled samples are resuspended in 4X SSC/0.03% SDS/4 μg salmon sperm DNA/2 μg tRNA/ 50mM Napyrophosphate, heated for 95°C for 2.5 minutes, spun down and placed on the array. The array is then covered with a glass coverslip and placed in a sealed chamber. The chamber is then kept in a water bath at 62°C overnight. The arrays are washed as described in Eisen and Brown (1999, *supra*) and scanned on a General Scanning 3000 laser scanner. The resulting files are subsequently quantified using IMAGENE, software (BioDiscovery, Los Angeles CA).

RT-PCR experiments may be performed to identify those genes induced after exposure to biotropic fungal pathogens, such as *Erysiphe orontii,* necrotropic fungal pathogens, such as *Fusarium oxysporum,* bacteria, viruses and salicylic acid, the latter being involved in a nonspecific resistance response in *Arabidopsis thaliana.* Generally, the gene expression patterns from ground plant leaf tissue is examined.

Reverse transcriptase PCR was conducted using gene specific primers within the coding region for each sequence identified. The primers were designed near the 3' region of each DNA binding sequence initially identified.

Total RNA from these ground leaf tissues was isolated using the CTAB extraction protocol. Once extracted total RNA was normalized in concentration across all the tissue types to ensure that the PCR reaction for each tissue received the same amount of cDNA template using the 28S band as reference. Poly(A+) RNA was purified using a modified protocol from the Qiagen OLIGOTEX purification kit batch protocol. cDNA was synthesized using standard protocols. After the first strand cDNA synthesis, primers for Actin 2 were used to normalize the concentration of cDNA across the tissue types. Actin 2 is found to be constitutively expressed in fairly equal levels across the tissue types we are investigating.

For RT PCR, cDNA template was mixed with corresponding primers and Taq DNA polymerase. Each reaction consisted of 0.2 μl cDNA template, 2 μl 10X Tricine buffer, 2 μl 10X Tricine buffer and 16.8 μl water, 0.05 μl Primer 1, 0.05 μl, Primer 2, 0.3 μl Taq DNA polymerase and 8.6 μl water.

The 96 well plate is covered with microfilm and set in the thermocycler to start the reaction cycle. By way of illustration, the reaction cycle may comprise the following steps:

Step 1: 93° C for 3 min;
Step 2: 93° C for 30 sec;
Step 3: 65° C for 1 min;
Step 4: 72° C for 2 min;
Steps 2, 3 and 4 are repeated for 28 cycles;
Step 5: 72° C for 5 min; and
STEP 6 4° C.

To amplify more products, for example, to identify genes that have very low expression, additional steps may be performed: The following method illustrates a method that may be used in this regard. The PCR plate is placed back in the thermocycler for 8 more cycles of steps 2-4.

Step 2 93° C for 30 sec;
Step 3 65° C for 1 min;
Step 4 72° C for 2 min, repeated for 8 cycles; and
Step 5 4° C.

Eight microliters of PCR product and 1.5 μl of loading dye are loaded on a 1.2% agarose gel for analysis after 28 cycles and 36 cycles. Expression levels of specific transcripts are considered low if they were only detectable after 36 cycles of PCR. Expression levels are considered medium or high depending on the levels of transcript compared with observed transcript levels for an internal control such as actin2. Transcript levels are determined in repeat experiments and compared to transcript levels in control (e.g., non-transformed) plants.

Experiments were performed to identify those transformants or knockouts that exhibited an improved environmental stress tolerance. For such studies, the transformants were exposed to a variety of environmental stresses. Plants were exposed to chilling stress (6 hour exposure to 4-8° C), heat stress (6 hour exposure to 32-37° C), high salt stress (6 hour exposure to 200 mM NaCl), drought stress (168 hours after removing water from trays), osmotic stress (6 hour exposure to 3 M mannitol), or nutrient limitation (nitrogen, phosphate, and potassium) (nitrogen: all components of MS medium remained constant except N was reduced to 20 mg/l of $NH_4NO_3$; phosphate: all components of MS medium except $KH2PO_4$, which was replaced by $K_2SO_4$; potassium: all components of MS medium except removal of $KNO_3$ and $KH_2PO_4$, which were replaced by $NaH_4PO_4$).

Experiments were performed to identify those transformants or knockouts that exhibited a modified structure and development characteristics. For such studies, the transformants were observed by eye to identify novel structural or developmental characteristics associated with the ectopic expression of the polynucleotides or polypeptides of the invention.

Flowering time was measured by the number of rosette leaves present when a visible inflorescence of approximately 3 cm is apparent. Rosette and total leaf number on the progeny stem are tightly correlated with the timing of flowering (Koornneef et al. (1991) Mol. Gen. Genet. 229: 57-66). The vernalization response was also measured. For vernalization treatments, seeds were sown to MS agar plates, sealed with micropore tape, and placed in a 4°C cold room with low light levels for 6-8 weeks. The plates were then transferred to the growth rooms alongside plates containing freshly sown non-vernalized controls. Rosette leaves were counted when a visible inflorescence of approximately 3 cm was apparent.

Modified phenotypes observed for particular overexpressor or knockout plants are provided in Table 4. For a particular overexpressor that shows a less beneficial characteristic, it may be more useful to select a plant with a decreased expression of the particular transcription factor. For a particular knockout that shows a less beneficial characteristic, it may be more useful to select a plant with an increased expression of the particular transcription factor.

The sequences of the Sequence Listing or those in Tables 4 - 9, or those disclosed here, can be used to prepare transgenic plants and plants with altered traits. The specific transgenic plants listed below are produced from the sequences of the Sequence Listing, as noted. Tables 4 and 6 provide exemplary polynucleotide and polypeptide sequences of the invention.

**Example VIII: Examples of Genes that Confer Significant Improvements to Plants**

Examples of genes and homologs that confer significant improvements to knockout or overexpressing plants are noted below. Experimental observations made by us with regard to specific genes whose expression has been modified in overexpressing or knock-out plants, and potential applications based on these observations, are also presented.

This example provides experimental evidence for increased biomass and abiotic stress tolerance controlled by the transcription factor polypeptides and polypeptides of the invention.

Salt stress assays are intended to find genes that confer better germination, seedling vigor or growth in high salt. Evaporation from the soil surface causes upward water movement and salt accumulation in the upper soil layer where the seeds are placed. Thus, germination normally takes place at a salt concentration much higher than the mean salt concentration of in the whole soil profile. Plants differ in their tolerance to NaCl depending on their stage of development, therefore seed germination, seedling vigor, and plant growth responses are evaluated.

Osmotic stress assays (including NaCl and mannitol assays) are intended to determine if an osmotic stress phenotype is NaCl-specific or if it is a general osmotic stress related phenotype. Plants tolerant to osmotic stress could also have more tolerance to drought and/or freezing.

Drought assays are intended to find genes that mediate better plant survival after short-term, severe water deprivation. Ion leakage will be measured if needed. Osmotic stress tolerance would also support a drought tolerant phenotype.

Temperature stress assays are intended to find genes that confer better germination, seedling vigor or plant growth under temperature stress (cold, freezing and heat).

Sugar sensing assays are intended to find genes involved in sugar sensing by germinating seeds on high concentrations of sucrose and glucose and looking for degrees of hypocotyl elongation. The germination assay on mannitol controls for responses related to osmotic stress. Sugars are key regulatory molecules that affect diverse processes in higher plants including germination, growth, flowering, senescence, sugar metabolism and photosynthesis. Sucrose is the major transport form of photosynthate and its flux through cells has been shown to affect gene expression and alter storage compound accumulation in seeds (source-sink relationships). Glucose-specific hexose-sensing has also been described in plants and is implicated in cell division and repression of "famine" genes (photosynthetic or glyoxylate cycles).

Germination assays followed modifications of the same basic protocol. Sterile seeds were sown on the conditional media listed below. Plates were incubated at 22° C under 24-hour light (120-130 $\mu Ein/m^2/s$) in a growth chamber. Evaluation of germination and seedling vigor was conducted 3 to 15 days after planting. The basal media was 80% Murashige-Skoog medium (MS) + vitamins.

For salt and osmotic stress germination experiments, the medium was supplemented with 150 mM NaCl or 300 mM mannitol. Growth regulator sensitivity assays were performed in MS media, vitamins, and either 0.3 μM ABA, 9.4% sucrose, or 5% glucose.

Temperature stress cold germination experiments were carried out at 8 °C. Heat stress germination experiments were conducted at 32 °C to 37° C for 6 hours of exposure.

For stress experiments conducted with more mature plants, seeds were germinated and grown for seven days on MS + vitamins + 1% sucrose at 22 °C and then transferred to chilling and heat stress conditions. The plants were either exposed to chilling stress (6 hour exposure to 4-8° C), or heat stress (32° C was applied for five days, after which the plants were transferred back 22 °C for recovery and evaluated after 5 days relative to controls not exposed to the depressed or elevated temperature).

Published Information

G1988 (At3g21150) is in P1 clone MSA6 (GenBank accession number AP000604) and was identified based on its sequence similarity within the conserved domain to other CONSTANS-like related proteins in *Arabidopsis.* There is no published or public information about the function of G1988.

Experimental Observations.

The function of G1988 was studied using transgenic plants in which the gene was expressed under the control of the 35S promoter. Evidence from physiological and morphological assays indicates that G1988 may play a role in developmental processes regulated by light; 35S::G1988 seedlings displayed longer hypocotyls, elongated petioles, and a number of lines flowered early.

When grown on limited phosphate, all lines appeared larger and had more root growth than controls. Seedlings germinated on plates that contained limited nitrogen (supplemented with glutamine) appeared less stressed than controls.

Utilities

Based on the results from physiological assays, G1988 might be used to engineer plants that show enhanced growth and survivability in low nutrient environments.

G1988 could also have a role in modulating developmental processes regulated by light, such as shade avoidance. Eliminating shading responses could lead to increased planting densities with subsequent yield enhancement. The gene might also be useful in manipulating flowering time.

**Example IX: Identification of Homologous Sequences**

This example describes identification of genes that are orthologous to *Arabidopsis thaliana* transcription factors from a computer homology search.

Homologous sequences, including those of paralogs and orthologs from *Arabidopsis* and other plant species, were identified using database sequence search tools, such as the Basic Local Alignment Search Tool (BLAST) (Altschul et al. (1990) J. Mol. Biol. 215: 403-410; and Altschul et al. (1997) Nucleic Acid Res. 25: 3389-3402). The tblastx sequence analysis programs were employed using the BLOSUM-62 scoring matrix (Henikoff and Henikoff (1992) Proc. Natl. Acad. Sci. 89: 10915-10919). The entire NCBI GenBank database was filtered for sequences from all plants except *Arabidopsis thaliana* by selecting all entries in the NCBI GenBank database associated with NCBI taxonomic ID 33090 (Viridiplantae; all plants) and excluding entries associated with taxonomic ID 3701 (*Arabidopsis thaliana*).

These sequences are compared to sequences representing genes of the Sequence Listing, using the Washington University TBLASTX algorithm (version 2.0a19MP) at the default settings using gapped alignments with the filter "off". For each of these gene sequences, individual comparisons were ordered by probability score (P-value), where the score reflects the probability that a particular alignment occurred by chance. For example, a score of 3.6e-40 is 3.6 x 10-40. In addition to P-values, comparisons were also scored by percentage identity. Percentage identity reflects the degree to which two segments of DNA or protein are identical over a particular length. Examples of sequences so identified are presented in Tables 7, and 9. Paralogous or orthologous sequences were readily identified from proprietary databases and in GenBank. The percent sequence identity among these sequences can be as low as 47%, or even lower sequence identity.

Candidate paralogous sequences were identified among *Arabidopsis* transcription factors through alignment, identity, and phylogenic relationships. Candidate orthologous sequences were identified from proprietary unigene sets of plant gene sequences in *Zea mays, Glycine max* and *Oryza sativa* based on significant homology to *Arabidopsis* transcription factors. These candidates were reciprocally compared to the set of *Arabidopsis* transcription factors. If the candidate showed maximal similarity in the protein domain to the eliciting transcription factor or to a paralog of the eliciting transcription factor, then it was considered to be an ortholog. Identified non-*Arabidopsis* sequences that were shown in this manner to be orthologous to the *Arabidopsis* sequences are provided in Tables 7 and 9.

**Example X: Screen of Plant cDNA library for Sequence Encoding a Transcription Factor DNA Binding Domain That Binds To a Transcription Factor Binding Promoter Element and Demonstration of Protein Transcription Regulation Activity.**

The "one-hybrid" strategy (Li and Herskowitz (1993) Science 262: 1870-1874) is used to screen for plant cDNA clones encoding a polypeptide comprising a transcription factor DNA binding domain, a conserved domain. In brief, yeast strains are constructed that contain a lacZ reporter gene with either wild-type or mutant transcription factor binding promoter element sequences in place of the normal UAS (upstream activator sequence) of the GALL promoter. Yeast reporter strains are constructed that carry transcription factor binding promoter element sequences as UAS elements are operably linked upstream (5') of a lacZ reporter gene with a minimal GAL1 promoter. The strains are transformed with a plant expression library that contains random cDNA inserts fused to the GAL4 activation domain (GAL4-ACT) and screened for blue colony formation on X-gal-treated filters (X-gal: 5-bromo-4-chloro-3-indolyl-β-D-galactoside; Invitrogen Corporation, Carlsbad CA). Alternatively, the strains are transformed with a cDNA polynucleotide encoding a known transcription factor DNA binding domain polypeptide sequence.

Yeast strains carrying these reporter constructs produce low levels of beta-galactosidase and form white colonies on filters containing X-gal. The reporter strains carrying wild-type transcription factor binding promoter element sequences are transformed with a polynucleotide that encodes a polypeptide comprising a plant transcription factor DNA binding domain operably linked to the acidic activator domain of the yeast GAL4 transcription factor, "GAL4-ACT". The clones that contain a polynucleotide encoding a transcription factor DNA binding domain operably linked to GLA4-ACT can bind upstream of the lacZ reporter genes carrying the wild-type transcription factor binding promoter element sequence, activate transcription of the lacZ gene and result in yeast forming blue colonies on X-gal-treated filters.

Upon screening about 2 x $10^6$ yeast transformants, positive cDNA clones are isolated; i.e., clones that cause yeast strains carrying lacZ reporters operably linked to wild-type transcription factor binding promoter elements to form blue colonies on X-gal-treated filters. The cDNA clones do not cause a yeast strain carrying a mutant type transcription factor binding promoter elements fused to LacZ to turn blue. Thus, a polynucleotide encoding transcription factor DNA binding domain, a conserved domain, is shown to activate transcription of a gene.

**Example XI: Gel Shift Assays.**

The presence of a transcription factor comprising a DNA binding domain which binds to a DNA transcription factor binding element is evaluated using the following gel shift assay. The transcription factor is recombinantly expressed and isolated from *E. coli* or isolated from plant material. Total soluble protein, including transcription factor, (40 ng) is incubated at room temperature in 10 μl of 1 x binding buffer (15 mM HEPES (pH 7.9), 1 mM EDTA, 30 mM KCl, 5% glycerol, 5% bovine serum albumin, 1 mM DTT) plus 50 ng poly(dl-dC):poly(dl-dC) (Pharmacia, Piscataway NJ) with or without 100 ng competitor DNA. After 10 minutes incubation, probe DNA comprising a DNA transcription factor binding element (1 ng) that has been $^{32}$P-labeled by end-filling (Sambrook et al. (1989) *supra)* is added and the mixture incubated for an additional 10 minutes. Samples are loaded onto polyacrylamide gels (4% w/v) and fractionated by electrophoresis at 150V for 2h (Sambrook et al. *supra).* The degree of transcription factor-probe DNA binding is visualized using autoradiography. Probes and competitor DNAs are prepared from oligonucleotide inserts ligated into the BamHI site of pUC118 (Vieira et al. (1987) Methods Enzymol. 153: 3-11). Orientation and concatenation number of the inserts are determined by dideoxy DNA sequence analysis (Sambrook et al. *supra).* Inserts are recovered after restriction digestion with EcoRI and HindIII and fractionation on polyacrylamide gels (12% w/v) (Sambrook et al. *supra).*

**Example XII. Introduction of Polynucleotides into Dicotyledonous Plants**

Any of the transcription factor sequences of the invention listed in the Sequence Listing, and paralogous, and orthologous sequences, may be recombined into pMEN20 or pMEN65 expression vectors and then are transformed into a plant for the purpose of modifying plant traits. The cloning vector may be introduced into a variety of cereal plants by means well known in the art such as, for example, direct DNA transfer or *Agrobacteriurn tumefaciens*-mediated transformation. It is now routine to produce transgenic plants using most dicot plants (see Weissbach and Weissbach, (1989) *supra;* Gelvin et al. (1990) *supra;* Herrera-Estrella et al. (1983) *supra;* Bevan (1984) *supra;* and Klee (1985) *supra).* Methods for analysis of traits are routine in the art and examples are disclosed above.

## Example XIII: Transformation of Cereal Plants with an Expression Vector

Cereal plants such as, but not limited to, corn, wheat, rice, sorghum, or barley, may also be transformed with the present polynucleotide sequences in pMEN20 or pMEN65 expression vectors for the purpose of modifying plant traits. For example, pMEN020 may be modified to replace the NptII coding region with the BAR gene of *Streptomyces hygroscopicus* that confers resistance to phosphinothricin. The KpnI and BglII sites of the Bar gene are removed by site-directed mutagenesis with silent codon changes.

The cloning vector may be introduced into a variety of cereal plants by means well known in the art such as, for example, direct DNA transfer or *Agrobacterium tumefaciens*-mediated transformation. It is now routine to produce transgenic plants of most cereal crops (Vasil (1994) Plant Mol. Biol. 25: 925-937) such as corn, wheat, rice, sorghum (Cassas et al. (1993) Proc. Natl. Acad. Sci. 90: 11212-11216, and barley (Wan and Lemeaux (1994) Plant Physiol. 104: 37-48. DNA transfer methods such as the microprojectile can be used for corn (Fromm et al. (1990) Bio/Technol. 8: 833-839); Gordon-Kamm et al. (1990) Plant Cell 2: 603-618; Ishida (1990) Nature Biotechnol. 14:745-750), wheat (Vasil et al. (1992) Bio/Technol. 10:667-674; Vasil et al. (1993) Bio/Technol. 11:1553-1558; Weeks et al. (1993) Plant Physiol. 102:1077-1084), rice (Christou (1991) Bio/Technol. 9:957-962; Hiei et al. (1994) Plant J. 6:271-282; Aldemita and Hodges (1996) Planta 199:612-617; and Hiei et al. (1997) Plant Mol. Biol. 35:205-218). For most cereal plants, embryogenic cells derived from immature scutellum tissues are the preferred cellular targets for transformation (Hiei et al. (1997) Plant Mol. Biol. 35:205-218; Vasil (1994) Plant Mol. Biol. 25: 925-937).

Vectors according to the present invention may be transformed into corn embryogenic cells derived from immature scutellar tissue by using microprojectile bombardment, with the A188XB73 genotype as the preferred genotype (Fromm et al. (1990) Bio/Technol. 8: 833-839; Gordon-Kamm et al. (1990) Plant Cell 2: 603-618). After microprojectile bombardment the tissues are selected on phosphinothricin to identify the transgenic embryogenic cells (Gordon-Kamm et al. (1990) Plant Cell 2: 603-618). Transgenic plants are regenerated by standard corn regeneration techniques (Fromm et al. (1990) Bio/Technol. 8: 833-839; Gordon-Kamm et al. (1990) Plant Cell 2: 603-618).

The plasmids prepared as described above can also be used to produce transgenic wheat and rice plants (Christou (1991) Bio/Technol. 9:957-962; Hiei et al. (1994) Plant J. 6:271-282; Aldemita and Hodges (1996) Planta 199: 612-617; and Hiei et al. (1997) Plant Mol. Biol. 35:205-218) that coordinately express genes of interest by following standard transformation protocols known to those skilled in the art for rice and wheat (Vasil et al. (1992) Bio/Technol. 10:667-674; Vasil et al. (1993) Bio/Technol. 11:1553-1558; and Weeks et al. (1993) Plant Physiol. 102:1077-1084), where the bar gene is used as the selectable marker.

## Reference Example XIV: Identification of Orthologous and Paralogous Sequences

Orthologs to *Arabidopsis* genes may identified by several methods, including hybridization, amplification, or bioinformatically. This example describes how one may identify homologs to the *Arabidopsis* AP2 family transcription factor CBF1 (polynucleotide SEQ ID NO: 2238, encoded polypeptide SEQ ID NO: 2239), which confers tolerance to abiotic stresses (Thomashow et al. (2002) US Patent No. 6,417,428), and an example to confirm the function of homologous sequences. In this example, orthologs to CBF1 were found in canola (*Brassica napus*) using polymerase chain reaction (PCR).

Degenerate primers were designed for regions of AP2 binding domain and outside of the AP2 (carboxyl terminal domain):

Mol 368 (reverse) 5'- CAY CCN ATH TAY MGN GGN GT -3' (SEQ ID NO: 2246)

Mol 378 (forward) 5'- GGN ARN ARC ATN CCY TCN GCC -3' (SEQ ID NO: 2247)

(Y: C/T, N: A/C/G/T, H: A/C/T, M: A/C, R:A/G)

Primer Mol 368 is in the AP2 binding domain of CBF1 (amino acid sequence: His-Pro-Ile-Tyr-Arg-Gly-Val) while primer Mol 378 is outside the AP2 domain (carboxyl terminal domain) (amino acid sequence: Met-Ala-Glu-Gly-Met-Leu-Leu-Pro).

The genomic DNA isolated from *B. napus* was PCR-amplified by using these primers following these conditions: an initial denaturation step of 2 min at 93° C; 35 cycles of 93° C for 1 min, 55°C for 1 min, and 72° C for 1 min ; and a final incubation of 7 min at 72° C at the end of cycling.

The PCR products were separated by electrophoresis on a 1.2% agarose gel and transferred to nylon membrane and hybridized with the AT CBF1 probe prepared from *Arabidopsis* genomic DNA by PCR amplification. The hybridized products were visualized by colorimetric detection system (Boehringer Mannheim) and the corresponding bands from a similar agarose gel were isolated using the Qiagen Extraction Kit (Qiagen). The DNA fragments were ligated into the TA clone vector from TOPO TA Cloning Kit (Invitrogen) and transformed into *E. coli* strain TOP10 (Invitrogen).

Seven colonies were picked and the inserts were sequenced on an ABI 377 machine from both strands of sense and antisense after plasmid DNA isolation. The DNA sequence was edited by sequencer and aligned with the AtCBF1 by GCG software and NCBI blast searching.

The nucleic acid sequence and amino acid sequence of one canola ortholog found in this manner (bnCBF1; polynucleotide SEQ ID NO: 2244 and polypeptide SEQ ID NO: 2245) identified by this process is shown in the Sequence Listing.

The aligned amino acid sequences show that the bnCBF1 gene has 88% identity with the *Arabidopsis* sequence in the AP2 domain region and 85% identity with the *Arabidopsis* sequence outside the AP2 domain when aligned for two insertion sequences that are outside the AP2 domain.

Similarly, paralogous sequences to *Arabidopsis* genes, such as *CBF1,* may also be identified.

Two paralogs of CBF1 from *Arabidopsis thaliana: CBF2* and *CBF3*. *CBF2* and *CBF3* have been cloned and sequenced as described below. The sequences of the DNA SEQ ID NO: 2240 and 2242 and encoded proteins SEQ ID NO: 2241 and 2243 are set forth in the Sequence Listing.

A lambda cDNA library prepared from RNA isolated from *Arabidopsis thaliana* ecotype Columbia (Lin and Thomashow (1992) Plant Physiol. 99: 519-525) was screened for recombinant clones that carried inserts related to the *CBF1* gene (Stockinger et al. (1997) Proc. Natl. Acad. Sci. 94:1035-1040). CBF1 was $^{32}$P-radiolabeled by random priming (Sambrook et al. *supra)* and used to screen the library by the plaque-lift technique using standard stringent hybridization and wash conditions (Hajela et al. (1990) Plant Physiol. 93:1246-1252; Sambrook et al. *supra)* 6 X SSPE buffer, 60° C for hybridization and 0.1 X SSPE buffer and 60° C for washes). Twelve positively hybridizing clones were obtained and the DNA sequences of the cDNA inserts were determined. The results indicated that the clones fell into three classes. One class carried inserts corresponding to *CBF1.* The two other classes carried sequences corresponding to two different homologs of *CBF1,* designated *CBF2* and *CBF3.* The nucleic acid sequences and predicted protein coding sequences for *Arabidopsis CBF1, CBF2* and *CBF3* are listed in the Sequence Listing (SEQ ID NOs:2238, 2240, 2242 and SEQ ID NOs: 2239, 2241, and 2243, respectively). The nucleic acid sequences and predicted protein coding sequence for *Brassica napus CBF* ortholog is listed in the Sequence Listing (SEQ ID NOs: 2244 and 2245, respectively).

A comparison of the nucleic acid sequences of *Arabidopsis CBF1, CBF2* and *CBF3* indicate that they are 83 to 85% identical as shown in Table 11.

**TABLE 11**

| | Percent identity[a] | |
|---|---|---|
| | DNA[b] | Polypeptide |
| cbf1/cbf2 | 85 | 86 |
| cbf1/cbf3 | 83 | 84 |
| cbf2/cbf3 | 84 | 85 |

[a] Percent identity was determined using the *Clustal* algorithm from the Megalign program (DNASTAR, Inc.).
[b] Comparisons of the nucleic acid sequences of the open reading frames are shown.

Similarly, the amino acid sequences of the three CBF polypeptides range from 84 to 86% identity. An alignment of the three amino acidic sequences reveals that most of the differences in amino acid sequence occur in the acidic C-terminal half of the polypeptide. This region of CBF1 serves as an activation domain in both yeast and *Arabidopsis* (not shown).

Residues 47 to 106 of CBF1 correspond to the AP2 domain of the protein, a DNA binding motif that to date, has only been found in plant proteins. A comparison of the AP2 domains of CBF1, CBF2 and CBF3 indicates that there are a few differences in amino acid sequence. These differences in amino acid sequence might have an effect on DNA binding specificity.

**Reference Example XV: Transformation of Canola with a Plasmid Containing CBF1, CBF2, or CBF3**

After identifying homologous genes to CBF1, canola was transformed with a plasmid containing the *Arabidopsis* CBF1, CBF2, or CBF3 genes cloned into the vector pGA643 (An (1987) *Methods Enzymol.* 253: 292). In these constructs the CBF genes were expressed constitutively under the CaMV 35S promoter. In addition, the CBF1 gene was cloned under the control of the *Arabidopsis* COR15 promoter in the same vector pGA643. Each construct was transformed into *Agrobacterium* strain GV3101. Transformed Agrobacteria were grown for 2 days in minimal AB medium containing appropriate antibiotics.

Spring canola (*B. napus* cv. Westar) was transformed using the protocol of Moloney et al. ((1989) Plant Cell Reports 8: 238) with some modifications as described. Briefly, seeds were sterilized and plated on half strength MS medium, containing 1% sucrose. Plates were incubated at 24° C under 60-80 $\mu E/m^2 s$ light using a16 hour light/ 8 hour dark photoperiod. Cotyledons from 4-5 day old seedlings were collected, the petioles cut and dipped into the *Agrobacterium* solution. The dipped cotyledons were placed on co-cultivation medium at a density of 20 cotyledons/plate and incubated as described above for 3 days. Explants were transferred to the same media, but containing 300 mg/l timentin (SmithKline Beecham, PA) and thinned to 10 cotyledons/plate. After 7 days explants were transferred to Selection/ Regeneration medium. Transfers were continued every 2-3 weeks (2 or 3 times) until shoots had developed. Shoots were transferred to Shoot-Elongation medium every 2-3 weeks. Healthy looking shoots were transferred to rooting medium. Once good roots had developed, the plants were placed into moist potting soil.

The transformed plants were then analyzed for the presence of the NPTII gene/ kanamycin resistance by ELISA, using the ELISA NPTII kit from 5Prime-3Prime Inc. (Boulder, CO). Approximately 70% of the screened plants were NPTII positive. Only those plants were further analyzed.

From Northern blot analysis of the plants that were transformed with the constitutively expressing constructs, showed expression of the CBF genes and all CBF genes were capable of inducing the *Brassica napus* cold-regulated gene BN115 (homolog of the *Arabidopsis* COR15 gene). Most of the transgenic plants appear to exhibit a normal growth phenotype. As expected, the transgenic plants are more freezing tolerant than the wild-type plants. Using the electrolyte leakage of leaves test, the control showed a 50% leakage at -2 to -3° C. Spring canola transformed with either CBF1 or CBF2 showed a 50% leakage at -6 to -7° C. Spring canola transformed with CBF3 shows a 50% leakage at about -10 to -15° C. Winter canola transformed with CBF3 may show a 50% leakage at about -16 to -20° C. Furthermore, if the spring or winter canola are cold acclimated the transformed plants may exhibit a further increase in freezing tolerance of at least-2° C.

To test salinity tolerance of the transformed plants, plants were watered with 150 mM NaCl, Plants overexpressing CBF1, CBF2 or CBF3 grew better compared with plants that had not been transformed with CBF1, CBF2 or CBF3.

These results demonstrate that homologs of *Arabidopsis* transcription factors can be identified and shown to confer similar functions in non-*Arabidopsis* plant species.

**Reference Example XVI: Cloning of transcription factor promoters**

Promoters are isolated from transcription factor genes that have gene expression patterns useful for a range of applications, as determined by methods well known in the art (including transcript profile analysis with cDNA or oligonucleotide microarrays, Northern blot analysis, semi-quantitative or quantitative RT-PCR). Interesting gene expression profiles are revealed by determining transcript abundance for a selected transcription factor gene after exposure of plants to a range of different experimental conditions, and in a range of different tissue or organ types, or developmental stages. Experimental conditions to which plants are exposed for this purpose includes cold, heat, drought, osmotic challenge, varied hormone concentrations (ABA, GA, auxin, cytokinin, salicylic acid, brassinosteroid), pathogen and pest challenge. The tissue types and developmental stages include stem, root, flower, rosette leaves, cauline leaves, siliques, germinating seed, and meristematic tissue. The set of expression levels provides a pattern that is determined by the regulatory elements of the gene promoter.

Transcription factor promoters for the genes disclosed herein are obtained by cloning 1.5 kb to 2.0 kb of genomic sequence immediately upstream of the translation start codon for the coding sequence of the encoded transcription factor protein. This region includes the 5'-UTR of the transcription factor gene, which can comprise regulatory elements. The 1.5 kb to 2.0 kb region is cloned through PCR methods, using primers that include one in the 3' direction located at the translation start codon (including appropriate adaptor sequence), and one in the 5' direction located from 1.5 kb to 2.0 kb upstream of the translation start codon (including appropriate adaptor sequence). The desired fragments are PCR-amplified from *Arabidopsis* Col-0 genomic DNA using high-fidelity Taq DNA polymerase to minimize the incorporation of point mutation(s). The cloning primers incorporate two rare restriction sites, such as Not1 and Sfi1, found at low frequency throughout the *Arabidopsis* genome. Additional restriction sites are used in the instances where a Not1 or Sfi1 restriction site is present within the promoter.

The 1.5-2.0 kb fragment upstream from the translation start codon, including the 5'-untranslated region of the transcription factor, is cloned in a binary transformation vector immediately upstream of a suitable reporter gene, or a transactivator gene that is capable of programming expression of a reporter gene in a second gene construct. Reporter genes used include green fluorescent protein (and related fluorescent protein color variants), beta-glucuronidase, and luciferase. Suitable transactivator genes include LexA-GAL4, along with a transactivatable reporter in a second binary plasmid (as disclosed in US patent application 09/958,131, incorporated herein by reference). The binary plasmid(s) is transferred into *Agrobacterium* and the structure of the plasmid confirmed by PCR. These strains are introduced into *Arabidopsis* plants as described in other examples, and gene expression patterns determined according to standard methods know to one skilled in the art for monitoring GFP fluorescence, beta-glucuronidase activity, or luminescence.

The promoter region for G1753 is obtained from *Arabidopsis* chromosome 2 clone F1011 (AC006919), gene At2g36450, from position 43906-45410 of the genomic clone. The complement of this sequence is the promoter oriented in the 5'-3' direction, with the translation start codon for G1753 the complement of positions 43903-43905.

The present invention is not limited by the specific embodiments described herein.

## Claims

1. A transgenic plant that over-expresses a recombinant polynucleotide, wherein said transgenic plant has improved yield as compared to a non-transgenic plant or wild-type plant,
wherein the recombinant polynucleotide encodes a polypeptide that has at least 95% amino acid sequence identity over the entire length of SEQ ID NO:328,
said polypeptide providing for improved yield as compared to a non-transgenic plant that does not over-express the polypeptide.

2. A transgenic plant according to claim 1 wherein said polypeptide comprises a conserved domain that has at least 80% sequence identity with the conserved domain of residues 5-50 of the polypeptide of SEQ ID NO:328.

3. A transgenic plant according to claim 1 wherein said polypeptide comprises SEQ ID NO:328.

4. The transgenic plant of claim 1, 2 or 3, wherein the recombinant polynucleotide comprises a constitutive, inducible, or tissue-specific promoter operably linked to said polynucleotide sequence.

5. A cultured host plant cell comprising an expression cassette that over-expresses a recombinant polynucleotide which encodes a polypeptide, wherein said polypeptide is as defined in any one of claims 1-3.

6. The transgenic plant of claim 1, 2 or 3, wherein said plant comprises longer hypocotyls and elongated petioles compared to a non-transgenic plant or wild-type plant.

7. The transgenic plant of any one of the preceding claims, wherein the transgenic plant is selected from the group consisting of: soybean, potato, cotton, oilseed rape, canola, sunflower, alfalfa, clover, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, pumpkin, spinach, squash, tobacco, tomato, tomatillo, watermelon, rosaceous fruits, fruit trees, brassicas, barley; wheat, corn, sweet corn, rice, rye; sugarcane, turf; millet; sorghum; currant; avocado; citrus fruits, oranges, lemons, grapefruit, tangerines, artichoke, cherries; walnut, peanut; endive; leek; arrowroot, beet, cassava, turnip, radish, yam, sweet potato; beans, pine, poplar, eucalyptus, and mint.

8. A plant part of the transgenic plant of any one of claims 1-4, 6 and 7 comprising an expression cassette that comprises the recombinant polynucleotide, wherein the plant part is selected from fruit, leaf, root, plant tissue including vascular or ground tissue, or plant cells.

9. A seed of the transgenic plant of any one of claims 1-4, 6 and 7, wherein said seed comprises an expression cassette that comprises the recombinant polylnlucleotide.

10. A method for producing a transgenic plant of the preceding claims, the method steps comprising:

(a) producing an expression cassette comprising a recombinant polynucleotide that encodes a polypeptide that has at least 95% amino acid sequence identity over the entire length of SEQ ID NO:328;
(b) introducing the expression cassette into the plant; and
(c) identifying and selecting a plant with improved yield as compared to a non-transgenic plant or wild-type plant.

11. A method for producing a transgenic plant having the altered trait of improved yield as compared to a non-transgenic plant or wild-type plant, the method steps comprising:

(a) providing an expression vector comprising:

(i) a recombinant polynucleotide that the recombinant polynucleotide encodes a polypeptide that has at least 95% amino acid sequence identity over the entire length of SEQ ID NO:328; and
(ii) at least one regulatory element flanking the polynucleotide sequence, said at least one regulatory element being effective in controlling expression of said recombinant polynucleotide in a target plant;

(b) introducing the expression vector into a plant cell, thereby producing a transgenic plant cell;
(c) growing the transgenic plant cell into a transgenic plant and allowing the transgenic plant to over-express a polypeptide encoded by the recombinant polynucleotide, said polypeptide having the property of improved yield in a plant as compared to a non-transgenic plant that does not over-express the polypeptide; and
(d) identifying at least one transgenic plant with said improved yield by comparing said transgenic plant with at least one non-transgenic plant that does not over-express the polypeptide.

12. The method according to claim 10 or 11 wherein said polypeptide comprises a conserved domain that has at least 80% sequence identity with the conserved domain of residues 5-50 of the polypeptide of SEQ ID NO:328.

13. The method according to claim 10 or 11 wherein said polypeptide comprises SEQ ID NO:328.

14. Use of a recombinant polynucleotide that the recombinant polynucleotide encodes a polypeptide that has at least 95% amino acid sequence identity over the entire length of SEQ ID NO:328, for producing a transgenic plant having improved yield as compared to a non-transgenic plant or wild-type plant, said polypeptide providing for improved yield as compared to a non-transgenic plant that does not over-express the polypeptide.

15. Use according to claim 14 wherein said polypeptide comprises a conserved domain that has at least 80% sequence identity with the conserved domain of residues 5-50 of the polypeptide of SEQ ID NO:328.

16. Use according to claim 15 wherein said polypeptide comprises SEQ ID NO:328.

17. Use according to claim 14 or 15, wherein the polynucleotide comprises a constitutive, inducible, or tissue-specific promoter operably linked to said polynucleotide sequence.

**Patentansprüche**

1. Transgene Pflanze, die ein rekombinantes Polynucleotid überexprimiert, wobei die transgene Pflanze verbesserten Ertrag im Vergleich zu einer nichttransgenen Pflanze oder Wildtyp-Pflanze aufweist,
wobei das rekombinante Polynucleotid für ein Polypeptid kodiert, das zumindest 95 % Aminosäuresequenzidentität mit der gesamten Länge der Seq.-ID Nr. 328 aufweist,
wobei das Polypeptid verbesserten Ertrag im Vergleich zu einer nichttransgenen Pflanze bereitstellt, die das Polypeptid nicht überexprimiert.

2. Transgene Pflanze nach Anspruch 1, wobei das Polypeptid eine konservierte Domäne umfasst, die zumindest 80 % Sequenzidentität mit der konservierten Domäne der Reste 5-50 des Polypeptids der Seq.-ID Nr. 328 aufweist.

3. Transgene Pflanze nach Anspruch 1, wobei das Polypeptid Seq.-ID Nr. 328 umfasst.

4. Transgene Pflanze nach Anspruch 1, 2 oder 3, wobei das rekombinante Polynucleotid einen konstitutiven, induzierbaren oder gewebespezifischen Promotor umfasst, der operabel an die Polynucleotidsequenz gebunden ist.

5. Kultivierte Wirtspflanzenzelle, die eine Expressionskassette umfasst, die ein rekombinantes Polynucleotid überexprimiert, das für ein Polypeptid kodiert, wobei das Polypeptid wie nach einem der Ansprüche 1 bis 3 definiert ist.

6. Transgene Pflanze nach Anspruch 1, 2 oder 3, wobei die Pflanze längere Hypokotyle und verlängerte Petioli im Vergleich zu einer nichttransgenen Pflanze oder Wildtyp-Pflanze umfasst.

7. Transgene Pflanze nach einem der vorangegangenen Ansprüche, wobei die transgene Pflanze aus folgender Gruppe ausgewählt ist: Sojabohne, Kartoffel, Baumwolle, Raps, Canola, Sonnenblume, Luzerne, Klee, Banane, Brombeere, Heidelbeere, Erdbeere, Himbeere, Kantalupe, Karotte, Karfiol, Kaffee, Gurke, Melanzani, Weintraube, Honigmelone, Kopfsalat, Mango, Melone, Zwiebel, Papaya, Erbsen, Paprika, Ananas, Kürbis, Spinat, Kürbispflanze, Tabak, Tomaten, Tomatillo, Wassermelone, Obst-Rosengewächse, Obstbäume, Kohl, Gerste; Weizen, Mais, Zukkermais, Reis, Roggen; Zuckerrohr, Rasen; Hirse; Sorghum; Johannisbeere; Avocado; Zitrusfrüchte, Orangen, Zitronen, Grapefruit, Mandarinen, Artischocke, Kirschen; Walnuss, Erdnuss; Endivie; Porree; Pfeilwurz, Bete, Kassava, Rüben, Rettich, Süßkartoffel, Batate; Bohnen, Kiefern, Pappeln, Eukalyptus und Minze.

8. Pflanzenteil einer transgenen Pflanze nach einem der Ansprüche 1-4, 6 und 7, umfassend eine Expressionskassette, die das rekombinante Polynucleotid umfasst, wobei der Pflanzenteil aus Frucht, Blatt, Wurzel, Pflanzengewebe, einschließlich Leitgewebe und Grundgewebe, oder Pflanzenzelle ausgewählt ist.

9. Samen einer transgenen Pflanzen nach einem der Ansprüche 1-4, 6 und 7, wobei der Samen eine Expressionskassette umfasst, die das rekombinante Polynucleotid umfasst.

10. Verfahren zur Herstellung einer transgenen Pflanze nach einem der vorangegangenen Ansprüche, wobei die Verfahrensschritte Folgendes umfassen:

    (a) das Produzieren einer Expressionskassette, die ein rekombinantes Polynucleotid umfasst, das für ein Polypeptid kodiert, das zumindest 95 % Aminosäuresequenzidentität mit der gesamten Länge der Seq.-ID Nr. 328 aufweist;
    (b) das Einführen der Expressionskassette in die Pflanze; und
    (c) das Identifizieren und Auswählen einer Pflanze mit verbessertem Ertrag im Vergleich zu einer nichttransgenen Pflanze oder Wildtyp-Pflanze.

11. Verfahren zur Herstellung einer transgenen Pflanze mit dem veränderten Merkmal eines verbesserten Ertrags im Vergleich zu einer nichttransgenen Pflanze oder Wildtyp-Pflanze, wobei die Verfahrensschritte Folgendes umfassen:

    (a) das Bereitstellen eines Expressionsvektors, der Folgendes umfasst:

        (i) ein rekombinantes Polynucleotid, wobei das rekombinante Polynucleotid für ein Polypeptid kodiert, das zumindest 95 % Aminosäuresequenzidentität mit der gesamten Länge der Seq.-ID Nr. 328 aufweist; und
(ii) zumindest ein regulatorisches Element, das die Polynucleotidsequenz flankiert, wobei das zumindest eine regulatorische Element wirksam zur Steuerung der Expression des rekombinanten Polynucleotids in der Zielpflanze ist;

(b) das Einführen des Expressionsvektors in eine Pflanzenzelle, wodurch eine transgene Pflanzenzelle hergestellt wird;
(c) das Züchten der transgenen Pflanzenzelle zu einer transgenen Pflanze und Ermöglichen, dass die transgene Pflanze ein Polypeptid überexprimiert, für welches das rekombinante Polynucleotid kodiert, wobei das Polypeptid die Eigenschaft eines verbesserten Ertrags der Pflanze im Vergleich zu einer nichttransgenen Pflanze, die das Polypeptid nicht überexprimiert, aufweist; und
(d) das Identifizieren zumindest einer transgenen Pflanze mit dem verbesserten Ertrag durch Vergleichen der transgenen Pflanze mit zumindest einer nichttransgenen Pflanze, die das Polypeptid nicht überexprimiert.

**12.** Verfahren nach Anspruch 10 oder 11, wobei das Polypeptid eine konservierte Domäne umfasst, die zumindest 80 % Sequenzidentität mit der konservierten Domäne aus den Resten 5-50 des Polypeptids der Seq.-ID Nr. 328 aufweist.

**13.** Verfahren nach Anspruch 10 oder 11, wobei das Polypeptid die Seq.-ID Nr. 328 umfasst.

**14.** Verwendung eines rekombinanten Polynucleotids, wobei das rekombinante Polynucleotid für ein Polypeptid kodiert, das zumindest 95 % Aminosäuresequenzidentität mit der gesamten Länge der Seq.-ID Nr. 328 aufweist, zur Herstellung einer transgenen Pflanze mit verbessertem Ertrag im Vergleich zu einer nichttransgenen Pflanze oder Wildtyp-Pflanze, wobei das Polypeptid verbesserten Ertrag im Vergleich zu einer nichttransgenen Pflanze bereitstellt, die das Polypeptid nicht überexprimiert.

**15.** Verwendung nach Anspruch 14, wobei das Polypeptid eine konservierte Domäne umfasst, die zumindest 80 % Sequenzidentität mit der konservierten Domäne aus den Resten 5-50 des Polypeptids der Seq.-ID Nr. 328 aufweist.

**16.** Verwendung nach Anspruch 15, wobei das Polypeptid Seq.-ID Nr. 328 umfasst.

**17.** Verwendung nach Anspruch 14 oder 15, wobei das Polynucleotid einen konstitutiven, induzierbaren oder gewebespezifischen Promotor operabel an die Polynucleotidsequenz gebunden umfasst.

## Revendications

**1.** Plante transgénique qui surexprime un polynucléotide recombinant, où ladite plante transgénique présente un rendement amélioré par comparaison à une plante non transgénique ou à une plante de type sauvage,
où le polynucléotide recombinant code pour un polypeptide qui présente au moins 95 % d'identité de séquence d'acides aminés sur toute la longueur de SEQ ID NO: 328,
ledit polypeptide permettant d'obtenir un rendement amélioré par comparaison à une plante non transgénique qui ne surexprime pas le polypeptide.

**2.** Plante transgénique selon la revendication 1, dans laquelle ledit polypeptide comprend un domaine conservé qui présente au moins 80 % d'identité de séquence avec le domaine conservé des résidus 5-50 du polypeptide de SEQ ID NO: 328.

**3.** Plante transgénique selon la revendication 1, dans laquelle ledit polypeptide comprend SEQ ID NO: 328.

**4.** Plante transgénique selon la revendication 1, 2 ou 3, dans laquelle le polynucléotide recombinant comprend un promoteur constitutif, inductible, ou spécifique à un tissu, en liaison fonctionnelle avec ladite séquence polynucléotidique.

**5.** Cellule végétale hôte cultivée comprenant une cassette d'expression qui surexprime un polynucléotide recombinant qui code pour un polypeptide, où ledit polypeptide est tel que défini dans l'une quelconque des revendications 1 à 3.

**6.** Plante transgénique selon la revendication 1, 2 ou 3, où ladite plante comprend des hypocotyles plus longs et des pétioles allongés par comparaison à une plante non transgénique ou à une plante de type sauvage.

7. Plante transgénique selon l'une quelconque des revendications précédentes, où la plante transgénique est sélectionnée dans le groupe consistant en: le soja, la pomme de terre, le coton, le colza, le canola, le tournesol, la luzerne, le trèfle, la banane, la mûre, la myrtille, la fraise, la framboise, le cantaloup, la carotte, le chou-fleur, le café, le concombre, l'aubergine, le raisin, le melon miel, la laitue, la mangue, le melon, l'oignon, la papaye, le pois, le poivre, l'ananas, la citrouille, l'épinard, la courge, le tabac, la tomate, la tomatille, la pastèque, des fruits rouges, des arbres fruitiers, le chou, l'orge; le blé, le maïs, le maïs doux, le riz, le seigle; la canne à sucre, le gazon; le millet; le sorgho; le raisin de Corinthe; l'avocat; des agrumes, l'orange, le citron, le pamplemousse, la mandarine, l'artichaut, la cerise; la noix, la cacahuète; l'endive; le poireau; l'herbe aux flèches, la betterave, le manioc, le navet, le radis, l'igname, la patate douce; le haricot, le pin, le peuplier, l'eucalyptus, et la menthe.

8. Partie végétale de la plante transgénique selon l'une quelconque des revendications 1 à 4, 6 et 7, comprenant une cassette d'expression qui comprend le polynucléotide recombinant, où la partie végétale est sélectionnée parmi un fruit, une feuille, une racine, un tissu végétal comprenant un tissu vasculaire ou un tissu de soutien, ou des cellules végétales.

9. Graine de la plante transgénique selon l'une quelconque des revendications 1 à 4, 6 et 7, où ladite graine comprend une cassette d'expression qui comprend le polynucléotide recombinant.

10. Procédé pour produire une plante transgénique selon les revendications précédentes, les étapes du procédé consistant à:

(a) produire une cassette d'expression comprenant un polynucléotide recombinant qui code pour un polypeptide qui présente au moins 95 % d'identité de séquence d'acides aminés sur toute la longueur de SEQ ID NO: 328;
(b) introduire la cassette d'expression dans la plante; et
(c) identifier et sélectionner une plante présentant un rendement amélioré par comparaison à une plante non transgénique ou à une plante de type sauvage.

11. Procédé pour produire une plante transgénique présentant la caractéristique altérée d'un rendement amélioré par comparaison à une plante non transgénique ou à une plante de type sauvage, les étapes du procédé consistant à:

(a) mettre à disposition un vecteur d'expression comprenant:

(i) un polynucléotide recombinant, où le polynucléotide recombinant code pour un polypeptide qui présente au moins 95 % d'identité de séquence d'acides aminés sur toute la longueur de SEQ ID NO: 328; et
(ii) au moins un élément régulateur flanquant la séquence polynucléotidique, ledit au moins un élément régulateur étant efficace pour contrôler l'expression dudit polynucléotide recombinant chez une plante cible;

(b) introduire le vecteur d'expression dans une cellule végétale, ce qui permet ainsi de produire une cellule végétale transgénique;
(c) cultiver la cellule végétale transgénique en une plante transgénique et permettre à la plante transgénique de surexprimer un polypeptide codé par le polynucléotide recombinant, ledit polypeptide présentant la propriété d'un rendement amélioré chez une plante par comparaison à une plante non transgénique qui ne surexprime pas le polypeptide; et
(d) identifier au moins une plante transgénique présentant ledit rendement amélioré en comparant ladite plante transgénique à au moins une plante non transgénique qui ne surexprime pas le polypeptide.

12. Procédé selon la revendication 10 ou 11, dans lequel ledit polypeptide comprend un domaine conservé qui présente au moins 80 % d'identité de séquence avec le domaine conservé des résidus 5-50 du polypeptide de SEQ ID NO: 328.

13. Procédé selon la revendication 10 ou 11, dans lequel ledit polypeptide comprend SEQ ID NO: 328.

14. Utilisation d'un polynucléotide recombinant, où le polynucléotide recombinant code pour un polypeptide qui présente au moins 95 % d'identité de séquence d'acides aminés sur toute la longueur de SEQ ID NO: 328, afin de produire une plante transgénique présentant un rendement amélioré par comparaison à une plante non transgénique ou à une plante de type sauvage, ledit polypeptide permettant d'obtenir un rendement amélioré par comparaison à une plante non transgénique qui ne surexprime pas le polypeptide.

15. Utilisation selon la revendication 14, dans laquelle ledit polypeptide comprend un domaine conservé qui présente au moins 80 % d'identité de séquence avec le domaine conservé des résidus 5-50 du polypeptide de SEQ ID NO: 328.

**16.** Utilisation selon la revendication 15, dans laquelle ledit polypeptide comprend SEQ ID NO: 328.

**17.** Utilisation selon la revendication 14 ou 15, dans laquelle le polynucléotide comprend un promoteur constitutif, inductible, ou spécifique à un tissu, en liaison fonctionnelle avec ladite séquence polynucléotidique.

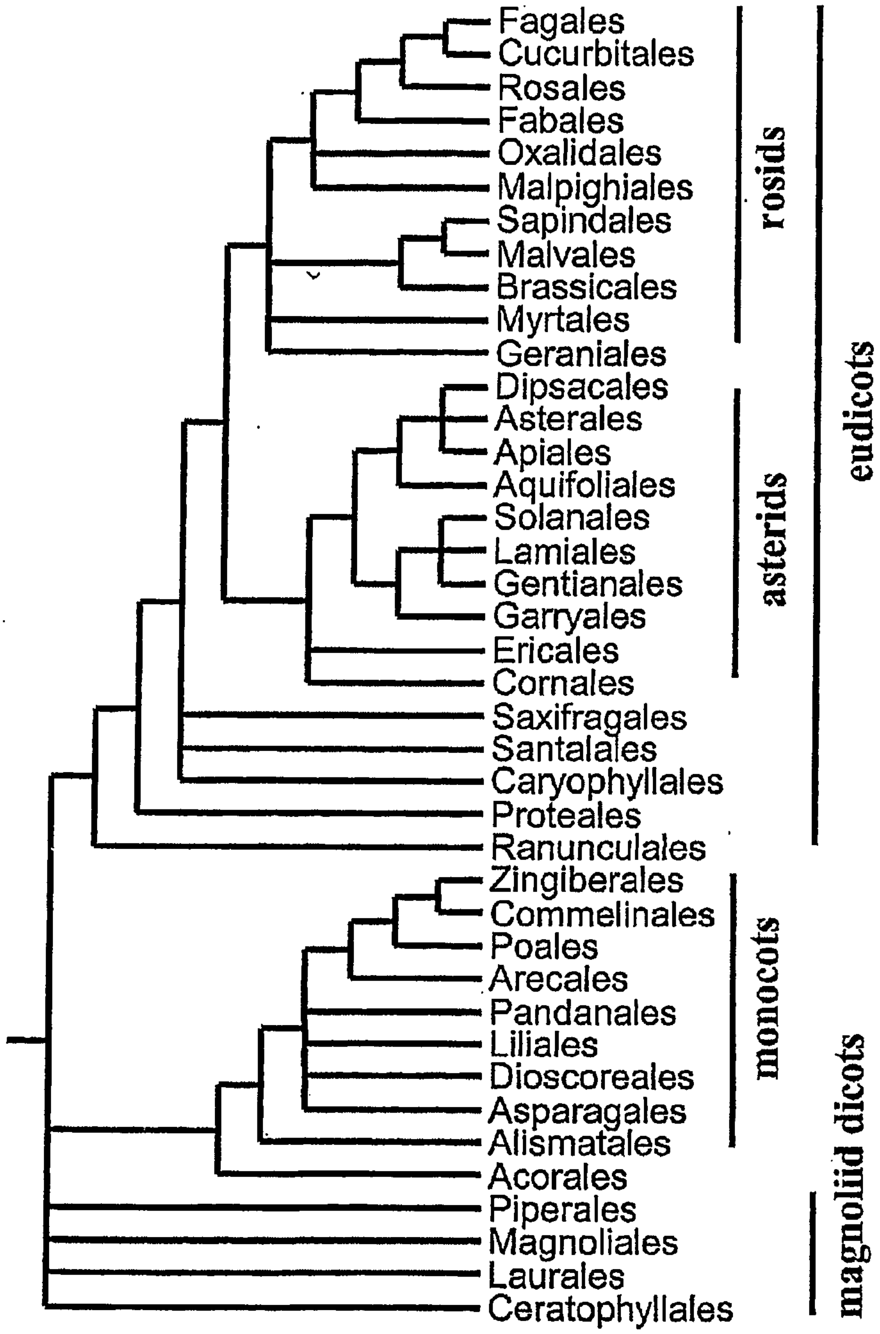
Fagales
Cucurbitales
Rosales
Fabales
Oxalidales
Malpighiales
Sapindales
Malvales
Brassicales
Myrtales
Geraniales
Dipsacales
Asterales
Apiales
Aquifoliales
Solanales
Lamiales
Gentianales
Garryales
Ericales
Cornales
Saxifragales
Santalales
Caryophyllales
Proteales
Ranunculales
Zingiberales
Commelinales
Poales
Arecales
Pandanales
Liliales
Dioscoreales
Asparagales
Alismatales
Acorales
Piperales
Magnoliales
Laurales
Ceratophyllales
rosids
asterids
eudicots
monocots
magnoliid dicots

FIGURE 1

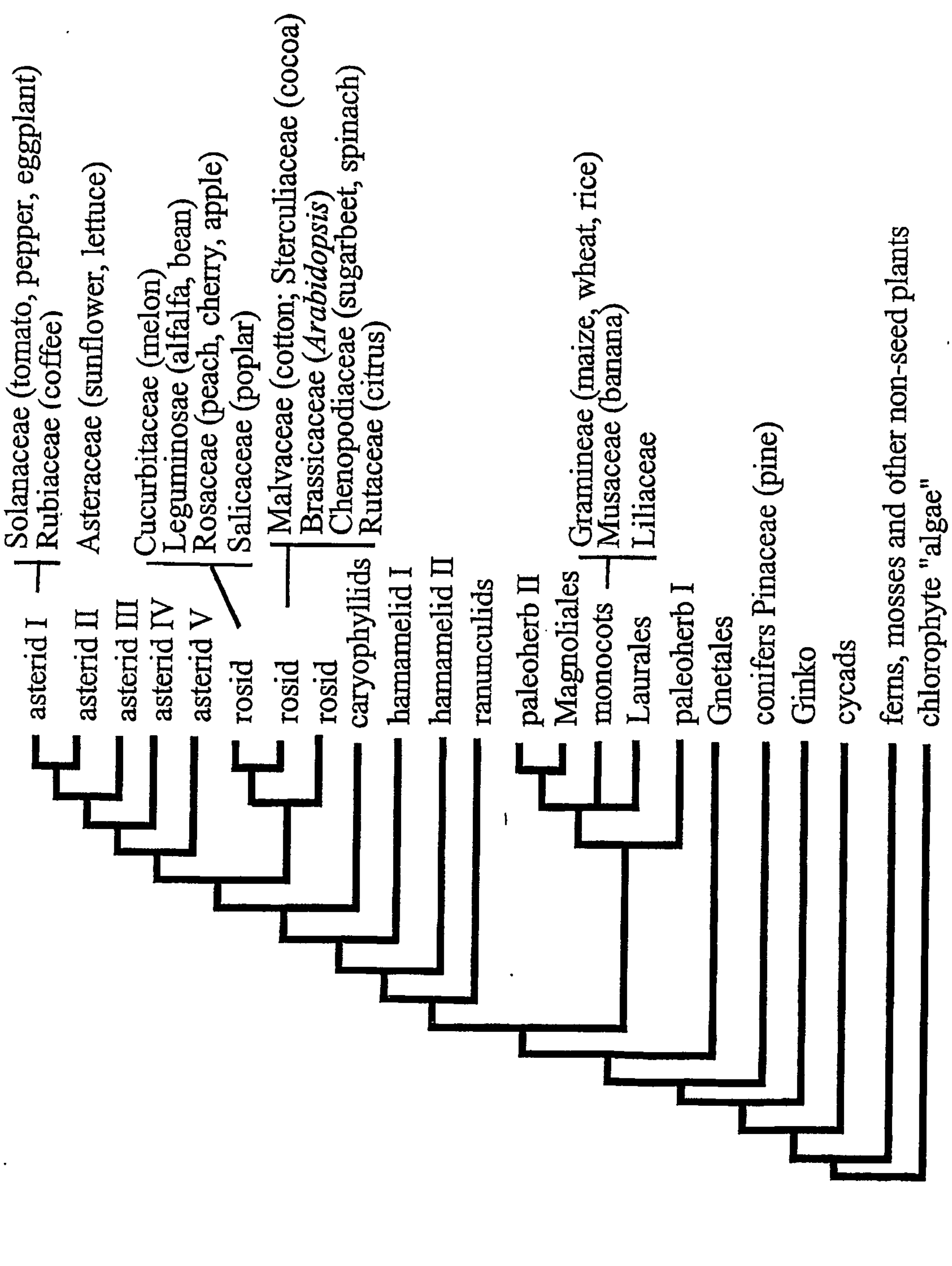

asterid I
Solanaceae (tomato, pepper, eggplant)
Rubiaceae (coffee)
asterid II
Asteraceae (sunflower, lettuce)
asterid III
asterid IV
asterid V
Cucurbitaceae (melon)
Leguminosae (alfalfa, bean)
Rosaceae (peach, cherry, apple)
Salicaceae (poplar)
rosid
rosid
Malvaceae (cotton; Sterculiaceae (cocoa)
Brassicaceae (*Arabidopsis*)
rosid
Chenopodiaceae (sugarbeet, spinach)
caryophyllids
Rutaceae (citrus)
hamamelid I
hamamelid II
ranunculids
paleoherb II
Magnoliales
Gramineae (maize, wheat, rice)
monocots
Musaceae (banana)
Laurales
Liliaceae
paleoherb I
Gnetales
conifers Pinaceae (pine)
Ginko
cycads
ferns, mosses and other non-seed plants
chlorophyte "algae"


FIGURE 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1033405 A
- US 5426039 A, Wallace
- US PN6262333 A
- US 20010010913 A
- US 5811238 A
- US 5837500 A
- US 6242568 B
- US 5773697 A
- US 5783393 A
- US 4943674 A
- US 5618988 A
- US 5837848 A
- US 5905186 A
- US 5792929 A
- US 4407956 A
- WO 8501856 A
- US 9610287 W
- US 5593853 A
- US 5539083 A
- US 5569588 A
- US 5549974 A
- US 5525735 A
- US 5519134 A
- US 5506337 A
- US 5010175 A
- US 6417428 B
- US 271988 A
- US 5231020 A
- US 5583021 A
- US 5658772 A
- WO 9606166 A
- WO 9853057 A
- US 5571706 A
- US 5677175 A
- US 5510471 A
- US 5750386 A
- US 5597945 A
- US 5589615 A
- US 5750871 A
- US 5268526 A
- US 5780708 A
- US 5538880 A
- US 5773269 A
- US 5736369 A
- US 5610042 A
- US 17773398 A
- US 09958131 B
- EP 10178358 A
- EP 03779082 A
- US 2003030292 W
- US 60411837 B
- US 60434166 B
- US 60465809 B


### Non-patent literature cited in the description


- *Ann. Missouri Bot. Gard.,* 1998, vol. 84, 1-49
- **DALY et al.** *Plant Physiol.,* 2001, vol. 127, 1328-1333
- **KU et al.** *Proc. Natl. Acad. Sci.,* 2000, vol. 97, 9121-9126
- **CHASE et al.** *Ann. Missouri Bot. Gard.,* 1993, vol. 80, 528-580
- **RIECHMANN et al.** *Science,* 2000, vol. 290, 2105-2110
- **RIECHMANN ; MEYEROWITZ.** *Biol. Chem.,* 1998, vol. 379, 633-646
- **MARTIN ; PAZ-ARES.** *Trends Genet.,* 1997, vol. 13, 67-73
- **RIECHMANN ; MEYEROWITZ.** *Biol. Chem.,* 1997, vol. 378, 1079-1101
- **ISHIGURO ; NAKAMURA.** *Mol. Gen. Genet.,* 1994, vol. 244, 563-571
- **ZHANG et al.** *Plant Cell,* 1992, vol. 4, 1575-1588
- **KLUG ; SCHWABE.** *FASEB J.,* 1995, vol. 9, 597-604
- **TAKATSUJI.** *Cell. Mol. Life Sci.,* 1998, vol. 54, 582-596
- **BUERGLIN.** Guidebook to the Homeobox Genes. Oxford University Press, 1994
- **FORSBURG ; GUARENTE.** *Genes Dev.,* 1989, vol. 3, 1166-1178
- **KLEIN et al.** *Mol. Gen. Genet.,* 1996, vol. 250, 7-16
- **SOUER et al.** *Cell,* 1996, vol. 85, 159-170
- **ABEL et al.** *J. Mol. Biol.,* 1995, vol. 251, 533-549
- **LITTLEWOOD et al.** *Prot. Profile,* 1994, vol. 1, 639-709

- **TUCKER et al.** *EMBO J.,* 1994, vol. 13, 2994-3002
- **FOSTER et al.** *FASEB J.,* 1994, vol. 8, 192-200
- **DA COSTA E SILVA et al.** *Plant J.,* 1993, vol. 4, 125-135
- **BUSTIN ; REEVES.** *Prog. Nucl. Acids Res. Mol. Biol.,* 1996, vol. 54, 35-100
- **DI LAURENZIO et al.** *Cell,* 1996, vol. 86, 423-433
- **WU et al.** *Plant Physiol.,* 1997, vol. 114, 1421-1431
- **GOODRICH et al.** *Nature,* 1997, vol. 386, 44-51
- **LUO et al.** *Nature,* 1996, vol. 383, 794-799
- **GIRAUDAT et al.** *Plant Cell,* 1992, vol. 4, 1251-1261
- **DEHESH et al.** *Science,* 1990, vol. 250, 1397-1399
- **CHAO et al.** *Cell,* 1997, vol. 89, 1133-44
- **REEVES ; NISSEN.** *J. Biol. Chem.,* 1990, vol. 265, 8573-8582
- **ZHOU et al.** *Nucleic Acids Res.,* 1995, vol. 23, 1165-1169
- **LU ; FERL.** *Plant Physiol.,* 1995, vol. 109, 723
- **BOWMAN et al.** *Development,* 1999, vol. 126, 2387-96
- **BOHMERT et al.** *EMBO J.,* 1998, vol. 17, 170-80
- **KIM et al.** *Plant J.,* 1997, vol. 11, 1237-1251
- **HALL et al.** *Plant Cell,* 1998, vol. 10, 925-936
- **BOGGIN et al.** *Science,* 1999, vol. 286, 2119-2125
- **WU.** *Annu. Rev. Cell Dev, Biol.,* 1995, vol. 11, 441-469
- **CHRISTIANSEN et al.** *Plant Mol. Biol.,* 1996, vol. 32, 809-821
- **JENSEN et al.** *FEBS Letters,* 1998, vol. 436, 283-287
- **JANIK et al.** *Virology,* 1989, vol. 168, 320-329
- **CUBAS et al.** *Plant J.,* 1999, vol. 18, 215-22
- **FRIDBORG et al.** *Plant Cell,* 1999, vol. 11, 1019-1032
- **CVITANICH et al.** *Proc. Natl. Acad. Sci.,* 2000, vol. 97, 8163-8168
- **ULMASOV et al.** *Proc. Natl. Acad. Sci.,* 1999, vol. 96, 5844-5849
- **COLLINGWOOD et al.** *J. Mol. Endocrinol.,* 1999, vol. 23, 255-275
- **SEGUIN et al.** *Plant Mol. Biol.,* 1997, vol. 35, 281-291
- **DA COSTA E SILVA et al.** *Plant Mol. Biol.,* 1994, vol. 25, 921-924
- **VAZQUEZ et al.** *Development,* 1999, vol. 126, 733-742
- **BALCIUNAS et al.** *Trends Biochem. Sci.,* 2000, vol. 25, 274-276
- **SCHAUSER et al.** *Nature,* 1999, vol. 402, 191-195
- **KAELIN et al.** *Cell,* 1992, vol. 70, 351-364
- **KNAAP et al.** *Plant Physiol.,* 2000, vol. 122, 695-704
- **RIEGER et al.** Glossary of Genetics and Cytogenetics: Classical and Molecular. Springer Verlag, 1976
- **KASHIMA et al.** *Nature,* 1985, vol. 313, 402-404
- **SAMBROOK et al.** Molecular Cloning: A Laboratory Manual. Cold Spring Harbor Laboratory, 1989
- **HAYMES et al.** Nucleic Acid Hybridization: A Practical Approach. IRL Press, 1985
- **TUDGE.** The Variety of Life. Oxford University Press, 2000, 547-606
- **AGRIOS.** Plant Pathology. Academic Press, 1988, 129
- **AGRIOS.** Plant Pathology. Academic Press, 1988, 125
- **PENG et al.** *Genes Development,* 1997, vol. 11, 3194-3205
- **PENG et al.** *Nature,* 1999, vol. 400, 256-261
- **FU et al.** *Plant Cell,* 2001, vol. 13, 1791-1802
- **NANDI et al.** *Curr. Biol.,* 2000, vol. 10, 215-218
- **COUPLAND.** *Nature,* 1995, vol. 377, 482-483
- **WEIGEL ; NILSSON.** *Nature,* 1995, vol. 377, 482-500
- **MANDEL et al.** *Cell,* 1992, vol. 71, 133-143
- **SUZUKI et al.** *Plant J.,* 2001, vol. 28, 409-418
- **MILLER et al.** *Plant J.,* 2001, vol. 28, 169-179
- **KIM et al.** *Plant J.,* 2001, vol. 25, 247-259
- **KYOZUKA ; SHIMAMOTO.** *Plant Cell Physiol.,* 2002, vol. 43, 130-135
- **BOSS ; THOMAS.** *Nature,* 2002, vol. 416, 847-850
- **HE et al.** *Transgenic Res.,* 2000, vol. 9, 223-227
- **ROBSON et al.** *Plant J.,* 2001, vol. 28, 619-631
- **GILMOUR et al.** *Plant J.,* 1998, vol. 16, 433-442
- **JAGLO et al.** *Plant Physiol.,* 2001, vol. 127, 910-917
- **BRUCE et al.** *Plant Cell,* 2000, vol. 12, 65-79
- **BOREVITZ et al.** *Plant Cell,* 2000, vol. 12, 2383-2393
- **BHATTACHARJEE et al.** *Proc. Natl. Acad. Sci. USA,* 2001, vol. 98, 13790-13795
- **XU et al.** *Proc Natl Acad Sci, USA,* 2001, vol. 98, 15089-15094
- Methods in Enzymology. **BERGER ; KIMMEL.** Guide to Molecular Cloning Techniques. Academic Press, Inc, vol. 152

- **SAMBROOK et al.** Molecular Cloning - A Laboratory Manual. Cold Spring Harbor Laboratory, 1989, vol. 1-3
- Current Protocols. Current Protocols in Molecular Biology. Greene Publishing Associates, Inc. and John Wiley & Sons, Inc, 2000
- **MULLIS et al.** PCR Protocols A Guide to Methods and Applications. Academic Press Inc, 1987
- **CHENG et al.** *Nature,* 1994, vol. 369, 684-685
- **BEAUCAGE et al.** *Tetrahedron Letters,* 1981, vol. 22, 1859-1869
- **MATTHES et al.** *EMBO J.,* 1984, vol. 3, 801-805
- **THOMPSON et al.** *Nucleic Acids Res.,* 1994, vol. 22, 4673-4680
- **HIGGINS et al.** *Methods Enzymol.,* 1996, vol. 266, 383-402
- **FENG ; DOOLITTLE.** *J. Mol. Evol.,* 1987, vol. 25, 351-360
- **RATCLIFFE et al.** *Plant Physiol.,* 2001, vol. 126, 122-132
- **MOUNT.** Bioinformatics: Sequences and Genome Analysis. Cold Spring Harbor Laboratory Press, 2001, 543
- **GOODRICH et al.** *Cell,* 1993, vol. 75, 519-530
- **LIN et al.** *Nature,* 1997, vol. 353, 569-571
- **SADOWSKI et al.** *Nature,* 1988, vol. 335, 563-564
- **LEE et al.** *Genome Res.,* 2002, vol. 12, 493-502
- **REMM et al.** *J. Mol. Biol.,* 2001, vol. 314, 1041-1052
- **JAGLO et al.** *Plant Physiol.,* 1998, vol. 127, 910-917
- **CHERN et al.** *Plant J.,* 2001, vol. 27, 101-113
- **FAN ; DONG.** *Plant Cell,* 2002, vol. 14, 1377-1389
- **KOSUGI ; OHASHI.** *Plant J.,* 2002, vol. 29, 45-59
- **GAMPALA et al.** *J. Biol. Chem.,* 2001, vol. 277, 1689-1694
- **GOCAL et al.** *Plant Physiol.,* 2001, vol. 127, 1682-1693
- **BOREVITZ et al.** *Plant Cell,* 2000, vol. 12, 2383-2394
- **HIGGINS ; SHARP.** *Gene,* 1988, vol. 73, 237-244
- Computer Methods for Macromolecular Sequence Analysis. Methods in Enzymology. Academic Press, Inc, 1996, vol. 266
- **SHPAER.** *Methods Mol. Biol.,* 1997, vol. 70, 173-187
- **HEIN.** *Methods Enzymol.,* 1990, vol. 183, 626-645
- **BAIROCH et al.** *Nucleic Acids Res.,* 1997, vol. 25, 217-221
- **SMITH et al.** *Protein Engineering,* 1992, vol. 5, 35-51
- **ALTSCHUL.** *J. Mol. Evol.,* 1993, vol. 36, 290-300
- **HENIKOFF ; HENIKOFF.** *Nucleic Acids Res.,* 1991, vol. 19, 6565-6572
- **EDDY.** *Curr. Opin. Str. Biol.,* 1996, vol. 6, 361-365
- **SONNHAMMER et al.** *Proteins,* 1997, vol. 28, 405-420
- **AUSUBEL et al.** Short Protocols in Molecular Biology. John Wiley & Sons, 1997
- **MEYERS.** Molecular Biology and Biotechnology. Wiley VCH, 1995, 856-853
- **FOWLER et al.** *Plant Cell,* 2002, vol. 14, 1675-79
- **WAHL ; BERGER.** *Methods Enzymol.,* 1987, vol. 152, 399-407
- **KIMMEL.** *Methods Enzymol.,* 1987, vol. 152, 507-511
- Guide to Molecular Cloning Techniques. Methods in Enzymology. 1987, vol. 152, 467-469
- Quantitative Filter Hybridisation. **ANDERSON ; YOUNG.** Nucleic Acid Hybridisation, A Practical Approach. IRL Press, 1985, 73-111
- Nucleic Acid Hybridisation. IRL Press, 1985
- **HARLOW ; LANE.** Antibodies: A Laboratory Manual. Cold Spring Harbor Laboratory, 1988
- Methods Enzymol. Academic Press, 1993, vol. 217
- **STEMMER.** *Nature,* 1994, vol. 370, 389-391
- **STEMMER.** *Proc. Natl. Acad. Sci.,* 1994, vol. 91, 10747-10751
- **ZHANG et al.** *J. Biol. Chem.,* 2000, vol. 275, 33850-33860
- **LIU et al.** *J Biol. Chem.,* 2001, vol. 276, 11323-11334
- **ISALAN et al.** *Nature Biotechnol.,* 2001, vol. 19, 656-660
- **MOORE et al.** *Proc. Natl. Acad. Sci.,* 1998, vol. 95, 376-381
- **AOYAMA et al.** *Plant Cell,* 1995, vol. 7, 1773-1785
- **MA ; PTASHNE.** *Cell,* 1987, vol. 51, 113-119
- **GINIGER ; PTASHNE.** *Nature,* 1987, vol. 330, 670-672
- **WEISSBACH ; WEISSBACH.** Methods for Plant Molecular Biology. Academic Press, 1989
- **GELVIN et al.** Plant Molecular Biology Manual. Kluwer Academic Publishers, 1990
- **HERRERA-ESTRELLA et al.** *Nature,* 1983, vol. 303, 209
- **BEVAN.** *Nucleic Acids Res.,* 1984, vol. 12, 8711-8721
- **KLEE.** *Bio/Technology,* 1985, vol. 3, 637-642
- **CHRISTOU.** *Bio/Technology,* 1991, vol. 9, 957-962
- **GORDON-KAMM.** *Plant Cell,* 1990, vol. 2, 603-618

- **WEEKS et al.** *Plant Physiol.,* 1993, vol. 102, 1077-1084
- **VASIL.** *Bio/Technology,* 1993, vol. 10, 667-674
- **WAN ; LEMEAUX.** *Plant Physiol.,* 1994, vol. 104, 37-48
- **ISHIDA et al.** *Nature Biotechnol.,* 1996, vol. 14, 745-750
- **ODELL et al.** *Nature,* 1985, vol. 313, 810-812
- **AN et al.** *Plant Physiol.,* 1988, vol. 88, 547-552
- **FROMM et al.** *Plant Cell,* 1989, vol. 1, 977-984
- **BIRD et al.** *Plant Mol. Biol.,* 1988, vol. 11, 651-662
- **RINGLI ; KELLER.** *Plant Mol. Biol.,* 1998, vol. 37, 977-988
- **KAISER et al.** *Plant Mol. Biol.,* 1995, vol. 28, 231-243
- **BAERSON et al.** *Plant Mol. Biol.,* 1994, vol. 26, 1947-1959
- **OHL et al.** *Plant Cell,* 1990, vol. 2, 837-848
- **BAERSON et al.** *Plant Mol. Biol.,* 1993, vol. 22, 255-267
- **VAN DER KOP et al.** *Plant Mol. Biol.,* vol. 39, 979-990
- **BAUMANN et al.** *Plant Cell,* 1999, vol. 11, 323-334
- **GUEVARA-GARCIA.** *Plant Mol. Biol.,* 1998, vol. 38, 743-753
- **SHI et al.** *Plant Mol. Biol.,* 1998, vol. 38, 1053-1060
- **WILLMOTT et al.** *Plant Molec. Biol.,* 1998, vol. 38, 817-825
- **AINLEY et al.** *Plant Mol. Biol.,* 1993, vol. 22, 13-23
- **KUHLEMEIER et al.** *Plant Cell,* 1989, vol. 1, 471-478
- **SCHAFFNER ; SHEEN.** *Plant Cell,* 1991, vol. 3, 997-1012
- **SIEBERTZ et al.** *Plant Cell,* 1989, vol. 1, 961-968
- **BUCHEL et al.** *Plant Mol. Biol.,* 1999, vol. 40, 387-396
- **MANNERS et al.** *Plant Mol. Biol.,* 1998, vol. 38, 1071-1080
- **GATZ.** *Annu. Rev. Plant Physiol. Plant Mol. Biol.,* 1997, vol. 48, 89-108
- **GAN ; AMASINO.** *Science,* 1995, vol. 270, 1986-1988
- **ODELL et al.** *Plant Physiol.,* 1994, vol. 106, 447-458
- **FROMM et al.** *Proc. Natl. Acad. Sci.,* 1985, vol. 82, 5824-5828
- **HOHN et al.** Molecular Biology of Plant Tumors. Academic Press, 1982, 549-560
- **KLEIN et al.** *Nature,* 1987, vol. 327, 70-73
- **HORSCH et al.** *Science,* 1984, vol. 233, 496-498
- **FRALEY et al.** *Proc. Natl. Acad. Sci.,* 1983, vol. 80, 4803-4807
- **BULYK et al.** *Nature Biotechnol.,* 1999, vol. 17, 573-577
- **CHIEN et al.** *Proc. Natl. Acad. Sci.,* 1991, vol. 88, 9578-9582
- Current Protocols in Molecular Biology. John Wiley & Sons, 1998
- **VAUGHN et al.** *Nature Biotechnol.,* 1996, vol. 14, 309-314
- **LIANG et al.** *Science,* 1996, vol. 274, 1520-1522
- **BAUM.** *Chem. & Engineering News,* 18 January 1993, 33
- **FURKA.** *Int. J. Pept. Prot. Res.,* 1991, vol. 37, 487-493
- **HOUGHTON et al.** *Nature,* 1991, vol. 354, 84-88
- Methods in Arabidopsis Research. World Scientific, 1992
- **MANDEL et al.** *Nature,* 1995, vol. 377, 522-524
- **WEIGEL ; NILSSON.** *Nature,* 1995, vol. 377, 495-500
- **SIMON et al.** *Nature,* 1996, vol. 384, 59-62
- **LICHTENSTEIN ; NELLEN.** Antisense Technology: A Practical Approach. IRL Press at Oxford University Press, 1997
- **CROWLEY et al.** *Cell,* 1985, vol. 43, 633-641
- **ROSENBERG et al.** *Nature,* 1985, vol. 313, 703-706
- **PREISS et al.** *Nature,* 1985, vol. 313, 27-32
- **MELTON.** *Proc. Natl. Acad. Sci.,* 1985, vol. 82, 144-148
- **IZANT ; WEINTRAUB.** *Science,* 1985, vol. 229, 345-352
- **KIM ; WOLD.** *Cell,* 1985, vol. 42, 129-138
- **SMITH et al.** *Nature,* 1988, vol. 334, 724-726
- **SMITH et al.** *Plant Mol. Biol.,* 1990, vol. 14, 369-379
- **CONSTANS.** *The Scientist,* 2002, vol. 16, 36
- **ZAMORE.** *Nature Struct. Biol.,* 2001, vol. 8, 746-50
- **BRUMMELKAMP et al.** *Science,* 2002, vol. 296, 550-553
- **PADDISON et al.** *Genes & Dev.,* 2002, vol. 16, 948-958
- **HAMMOND et al.** *Nature Rev Gen,* 2001, vol. 2, 110-119
- **FIRE et al.** *Nature,* 1998, vol. 391, 806-811
- **TIMMONS ; FIRE.** *Nature,* 1998, vol. 395, 854
- **SHARP.** *Genes and Development,* 1999, vol. 13, 139-141
- **KONCZ et al.** Methods in Arabidopsis Research. World Scientific Publishing Co. Pte. Ltd, 1992

- **KEMPIN et al.** *Nature,* 1997, vol. 389, 802-803
- **ICHIKAWA et al.** *Nature,* 1997, vol. 390, 698-701
- **KAKIMOTO et al.** *Science,* 1996, vol. 274, 982-985
- Handbook of Plant Cell Culture -Crop Species. Macmillan Publ. Co, 1984
- **SHIMAMOTO et al.** *Nature,* 1989, vol. 338, 274-276
- **FROMM et al.** *BiolTechnol.,* 1990, vol. 8, 833-839
- **VASIL et al.** *Bio/Technol.,* 1990, vol. 8, 429-434
- **SMITH ; WATERMAN.** *Adv. Appl. Math.,* 1981, vol. 2, 482-489
- **NEEDLEMAN ; WUNSCH.** *J. Mol. Biol.,* 1970, vol. 48, 443-453
- **PEARSON ; LIPMAN.** *Proc. Natl. Acad. Sci.,* 1988, vol. 85, 2444-2448
- **ALTSCHUL et al.** *J. Mol. Biol.,* 1990, vol. 215, 403-410
- **HENIKOFF ; HENIKOFF.** *Proc. Natl. Acad. Sci.,* 1992, vol. 89, 10915-10919
- **KARLIN ; ALTSCHUL.** *Proc. Natl. Acad. Sci.,* 1993, vol. 90, 5873-5787
- **WINANS.** *Microbiol. Rev.,* 1992, vol. 56, 12-31
- **EYAL et al.** *Plant Mol. Biol.,* 1992, vol. 19, 589-599
- **CHRISPEELS et al.** *Plant Mol. Biol.,* 2000, vol. 42, 279-290
- **PIAZZA et al.** *Plant Physiol.,* 2002, vol. 128, 1077-1086
- **SANDERS et al.** *Nucleic Acids Res.,* 1987, vol. 15, 1543-1558
- **NAGEL et al.** *FEMSMicrobiol Letts.,* 1990, vol. 67, 325-328
- **KRYSAN et al.** *Plant Cell,* 1999, vol. 11, 2283-2290
- **REITER et al.** *Plant J.,* 1999, vol. 12, 335-345
- **PAPADAKIS.** *Inst. d'Amelior. Plantes Thessaloniki (Greece) Bull. Scientif.,* 1973, (23
- **PAPADAKIS.** *Proc. Acad. Athens,* 1984, vol. 59, 326-342
- **MAUCH-MANI ; SLUSARENKO.** *Molec Plant-Microbe Interact.,* 1994, vol. 7, 378-383
- **EISEN ; BROWN.** *Methods Enzymol.,* 1999, vol. 303, 179-205
- **KOORNNEEF et al.** *Mol. Gen. Genet.,* 1991, vol. 229, 57-66
- **ALTSCHUL et al.** *Nucleic Acid Res.,* 1997, vol. 25, 3389-3402
- **LI ; HERSKOWITZ.** *Science,* 1993, vol. 262, 1870-1874
- **VIEIRA et al.** *Methods Enzymol.,* 1987, vol. 153, 3-11
- **VASIL.** *Plant Mol. Biol.,* 1994, vol. 25, 925-937
- **CASSAS et al.** *Proc. Natl. Acad. Sci.,* 1993, vol. 90, 11212-11216
- **FROMM et al.** *Bio/Technol.,* 1990, vol. 8, 833-839
- **GORDON-KAMM et al.** *Plant Cell,* 1990, vol. 2, 603-618
- **ISHIDA.** *Nature Biotechnol.,* 1990, vol. 14, 745-750
- **VASIL et al.** *Bio/Technol.,* 1992, vol. 10, 667-674
- **VASIL et al.** *Bio/Technol.,* 1993, vol. 11, 1553-1558
- **CHRISTOU.** *Bio/Technol.,* 1991, vol. 9, 957-962
- **HIEI et al.** *Plant J.,* 1994, vol. 6, 271-282
- **ALDEMITA ; HODGES.** *Planta,* 1996, vol. 199, 612-617
- **HIEI et al.** *Plant Mol. Biol.,* 1997, vol. 35, 205-218
- **LIN ; THOMASHOW.** *Plant Physiol.,* 1992, vol. 99, 519-525
- **STOCKINGER et al.** *Proc. Natl. Acad. Sci.,* 1997, vol. 94, 1035-1040
- **HAJELA et al.** *Plant Physiol.,* 1990, vol. 93, 1246-1252
- **MOLONEY et al.** *Plant Cell Reports,* 1989, vol. 8, 238

SEQUENCE LISTING

<110> Mendel Biotechnology, Inc.

<120> Polynucleotides and Polypeptides in Plants

<130> AHB/FP6293542

<140> EP 10178358.7
<141> 2003-09-18

<150> EP 03779082.1
<151> 2003-09-18

<150> PCT/US2003/030292
<151> 2003-09-18

<150> US 60/411,837
<151> 2002-09-18

<150> US 60/434,166
<151> 2002-12-17

<150> US 60/465,809
<151> 2003-04-24

<160> 2247

<170> PatentIn version 3.2

<210> 1
<211> 0
<212> DNA
<213> Arabidopsis thaliana

<400> 1
000

<210> 2
<211> 0
<212> PRT
<213> Arabidopsis thaliana

<400> 2
000

<210> 3
<211> 0
<212> DNA
<213> Arabidopsis thaliana

<400> 3
000

<210> 4
<211> 0
<212> PRT
<213> Arabidopsis thaliana

<400> 4
000

<210> 5
<211> 0
<212> DNA
<213> Arabidopsis thaliana

<400> 5
000

<210> 6
<211> 0
<212> PRT
<213> Arabidopsis thaliana

<400> 6
000

<210> 7
<211> 0
<212> DNA
<213> Arabidopsis thaliana

<400> 7
000

<210> 8
<211> 0
<212> PRT
<213> Arabidopsis thaliana

<400> 8
000

<210> 9
<211> 0
<212> DNA
<213> Arabidopsis thaliana

<400> 9
000

<210> 10
<211> 0
<212> PRT
<213> Arabidopsis thaliana

<400> 10
000

<210> 11
<211> 0
<212> DNA
<213> Arabidopsis thaliana

<400> 11
000

<210> 12
<211> 0
<212> PRT
<213> Arabidopsis thaliana

<400> 12
000

<210> 13
<211> 0
<212> DNA
<213> Arabidopsis thaliana

<400> 13
000

<210> 14
<211> 0
<212> PRT
<213> Arabidopsis thaliana

<400> 14
000

<210> 15
<211> 0
<212> DNA
<213> Arabidopsis thaliana

<400> 15
000

<210> 16
<211> 0
<212> PRT
<213> Arabidopsis thaliana

<400> 16
000

<210> 17
<211> 0

<212> DNA
<213> Arabidopsis thaliana

<400> 17
000

<210> 18
<211> 0
<212> PRT
<213> Arabidopsis thaliana

<400> 18
000

<210> 19
<211> 0
<212> DNA
<213> Arabidopsis thaliana

<400> 19
000

<210> 20
<211> 0
<212> PRT
<213> Arabidopsis thaliana

<400> 20
000

<210> 21
<211> 0
<212> DNA
<213> Arabidopsis thaliana

<400> 21
000

<210> 22
<211> 0
<212> PRT
<213> Arabidopsis thaliana

<400> 22
000

<210> 23
<211> 0
<212> DNA
<213> Arabidopsis thaliana

<400> 23
000

<210> 24
<211> 0
<212> PRT
<213> Arabidopsis thaliana

<400> 24
000

<210> 25
<211> 0
<212> DNA
<213> Arabidopsis thaliana

<400> 25
000

<210> 26
<211> 0
<212> PRT
<213> Arabidopsis thaliana

<400> 26
000

<210> 27
<211> 0
<212> DNA
<213> Arabidopsis thaliana

<400> 27
000

<210> 28
<211> 0
<212> PRT
<213> Arabidopsis thaliana

<400> 28
000

<210> 29
<211> 0
<212> DNA
<213> Arabidopsis thaliana

<400> 29
000

<210> 30
<211> 0
<212> PRT
<213> Arabidopsis thaliana

<400> 30
000

<210> 31
<211> 0
<212> DNA
<213> Arabidopsis thaliana

<400> 31
000

<210> 32
<211> 0
<212> PRT
<213> Arabidopsis thaliana

<400> 32
000

<210> 33
<211> 0
<212> DNA
<213> Arabidopsis thaliana

<400> 33
000

<210> 34
<211> 0
<212> PRT
<213> Arabidopsis thaliana

<400> 34
000

<210> 35
<211> 0
<212> DNA
<213> Arabidopsis thaliana

<400> 35
000

<210> 36
<211> 0
<212> PRT
<213> Arabidopsis thaliana

<400> 36
000

<210> 37
<211> 0
<212> DNA
<213> Arabidopsis thaliana

<400> 37
000

<210> 38
<211> 0
<212> PRT
<213> Arabidopsis thaliana

<400> 38
000

<210> 39
<211> 0

<212> DNA
<213> Arabidopsis thaliana

<400> 39
000

<210> 40
<211> 0
<212> PRT
<213> Arabidopsis thaliana

<400> 40
000

<210> 41
<211> 0
<212> DNA
<213> Arabidopsis thaliana

<400> 41
000

<210> 42
<211> 0
<212> PRT
<213> Arabidopsis thaliana

<400> 42
000

<210> 43
<211> 0
<212> DNA
<213> Arabidopsis thaliana

<400> 43
000

<210> 44
<211> 0
<212> PRT
<213> Arabidopsis thaliana

<400> 44
000

<210> 45
<211> 0
<212> DNA
<213> Arabidopsis thaliana

<400> 45
000

<210> 46
<211> 0
<212> PRT
<213> Arabidopsis thaliana

<400> 46
000

<210> 47
<211> 0
<212> DNA
<213> Arabidopsis thaliana

<400> 47
000

<210> 48
<211> 0
<212> PRT
<213> Arabidopsis thaliana

<400> 48
000

<210> 49
<211> 0
<212> DNA
<213> Arabidopsis thaliana

<400> 49
000

<210> 50
<211> 0
<212> PRT
<213> Arabidopsis thaliana

<400> 50
000

<210> 51
<211> 0
<212> DNA
<213> Arabidopsis thaliana

<400> 51
000

<210> 52
<211> 0
<212> PRT
<213> Arabidopsis thaliana

<400> 52
000

<210> 53
<211> 0
<212> DNA
<213> Arabidopsis thaliana

<400> 53
000

<210> 54
<211> 0
<212> PRT
<213> Arabidopsis thaliana

<400> 54
000

<210> 55
<211> 0
<212> DNA
<213> Arabidopsis thaliana

<400> 55
000

<210> 56
<211> 0
<212> PRT
<213> Arabidopsis thaliana

<400> 56
000

<210> 57
<211> 0
<212> DNA
<213> Arabidopsis thaliana

<400> 57
000

<210> 58
<211> 0
<212> PRT
<213> Arabidopsis thaliana

<400> 58
000

<210> 59
<211> 0
<212> DNA
<213> Arabidopsis thaliana

<400> 59
000

<210> 60
<211> 0
<212> PRT
<213> Arabidopsis thaliana

<400> 60
000

<210> 61
<211> 0

<212> DNA
<213> Arabidopsis thaliana

<400> 61
000

<210> 62
<211> 0
<212> PRT
<213> Arabidopsis thaliana

<400> 62
000

<210> 63
<211> 0
<212> DNA
<213> Arabidopsis thaliana

<400> 63
000

<210> 64
<211> 0
<212> PRT
<213> Arabidopsis thaliana

<400> 64
000

<210> 65
<211> 0
<212> DNA
<213> Arabidopsis thaliana

<400> 65
000

<210> 66
<211> 0
<212> PRT
<213> Arabidopsis thaliana

<400> 66
000

<210> 67
<211> 0
<212> DNA
<213> Arabidopsis thaliana

<400> 67
000

<210> 68
<211> 0
<212> PRT
<213> Arabidopsis thaliana

<400> 68
000

<210> 69
<211> 0
<212> DNA
<213> Arabidopsis thaliana

<400> 69
000

<210> 70
<211> 0
<212> PRT
<213> Arabidopsis thaliana

<400> 70
000

<210> 71
<211> 0
<212> DNA
<213> Arabidopsis thaliana

<400> 71
000

<210> 72
<211> 0
<212> PRT
<213> Arabidopsis thaliana

<400> 72
000

<210> 73
<211> 0
<212> DNA
<213> Arabidopsis thaliana

<400> 73
000

<210> 74
<211> 0
<212> PRT
<213> Arabidopsis thaliana

<400> 74
000

<210> 75
<211> 0
<212> DNA
<213> Arabidopsis thaliana

<400> 75
000

<210> 76
<211> 0
<212> PRT
<213> Arabidopsis thaliana

<400> 76
000

<210> 77
<211> 0
<212> DNA
<213> Arabidopsis thaliana

<400> 77
000

<210> 78
<211> 0
<212> PRT
<213> Arabidopsis thaliana

<400> 78
000

<210> 79
<211> 0
<212> DNA
<213> Arabidopsis thaliana

<400> 79
000

<210> 80
<211> 0
<212> PRT
<213> Arabidopsis thaliana

<400> 80
000

<210> 81
<211>
<212> DNA
<213> Arabidopsis thaliana

<400> 81
000

<210> 82
<211> 0
<212> PRT
<213> Arabidopsis thaliana

<400> 82
000

<210> 83
<211> 0

<212> DNA
<213> Arabidopsis thaliana

<400> 83
000

<210> 84
<211> 0
<212> PRT
<213> Arabidopsis thaliana

<400> 84
000

<210> 85
<211> 0
<212> DNA
<213> Arabidopsis thaliana

<400> 85
000

<210> 86
<211> 0
<212> PRT
<213> Arabidopsis thaliana

<400> 86
000

<210> 87
<211> 0
<212> DNA
<213> Arabidopsis thaliana

<400> 87
000

<210> 88
<211> 0
<212> PRT
<213> Arabidopsis thaliana

<400> 88
000

<210> 89
<211> 0
<212> DNA
<213> Arabidopsis thaliana

<400> 89
000

<210> 90
<211> 0
<212> PRT
<213> Arabidopsis thaliana

<400> 90
000

<210> 91
<211> 0
<212> DNA
<213> Arabidopsis thaliana

<400> 91
000

<210> 92
<211> 0
<212> PRT
<213> Arabidopsis thaliana

<400> 92
000

<210> 93
<211> 0
<212> DNA
<213> Arabidopsis thaliana

<400> 93
000

<210> 94
<211> 0
<212> PRT
<213> Arabidopsis thaliana

<400> 94
000

<210> 95
<211> 0
<212> DNA
<213> Arabidopsis thaliana

<400> 95
000

<210> 96
<211> 0
<212> PRT
<213> Arabidopsis thaliana

<400> 96
000

<210> 97
<211> 0
<212> DNA
<213> Arabidopsis thaliana

<400> 97
000

<210> 98
<211> 0
<212> PRT
<213> Arabidopsis thaliana

<400> 98
000

<210> 99
<211> 0
<212> DNA
<213> Arabidopsis thaliana

<400> 99
000

<210> 100
<211> 0
<212> PRT
<213> Arabidopsis thaliana

<400> 100
000

<210> 101
<211> 0
<212> DNA
<213> Arabidopsis thaliana

<400> 101
000

<210> 102
<211> 0
<212> PRT
<213> Arabidopsis thaliana

<400> 102
000

<210> 103
<211> 0
<212> DNA
<213> Arabidopsis thaliana

<400> 103
000

<210> 104
<211> 0
<212> PRT
<213> Arabidopsis thaliana

<400> 104
000

<210> 105
<211> 0

<212> DNA
<213> Arabidopsis thaliana

<400> 105
000

<210> 106
<211> 0
<212> PRT
<213> Arabidopsis thaliana

<400> 106
000

<210> 107
<211> 0
<212> DNA
<213> Arabidopsis thaliana

<400> 107
000

<210> 108
<211> 0
<212> PRT
<213> Arabidopsis thaliana

<400> 108
000

<210> 109
<211> 0
<212> DNA
<213> Arabidopsis thaliana

<400> 109
000

<210> 110
<211> 0
<212> PRT
<213> Arabidopsis thaliana

<400> 110
000

<210> 111
<211> 0
<212> DNA
<213> Arabidopsis thaliana

<400> 111
000

<210> 112
<211> 0
<212> PRT
<213> Arabidopsis thaliana

<400> 112
000

<210> 113
<211> 0
<212> DNA
<213> Arabidopsis thaliana

<400> 113
000

<210> 114
<211> 0
<212> PRT
<213> Arabidopsis thaliana

<400> 114
000

<210> 115
<211> 0
<212> DNA
<213> Arabidopsis thaliana

<400> 115
000

<210> 116
<211> 0
<212> PRT
<213> Arabidopsis thaliana

<400> 116
000

<210> 117
<211> 0
<212> DNA
<213> Arabidopsis thaliana

<400> 117
000

<210> 118
<211> 0
<212> PRT
<213> Arabidopsis thaliana

<400> 118
000

<210> 119
<211> 0
<212> DNA
<213> Arabidopsis thaliana

<400> 119
000

<210> 120
<211> 0
<212> PRT
<213> Arabidopsis thaliana

<400> 120
000

<210> 121
<211> 0
<212> DNA
<213> Arabidopsis thaliana

<400> 121
000

<210> 122
<211> 0
<212> PRT
<213> Arabidopsis thaliana

<400> 122
000

<210> 123
<211> 0
<212> DNA
<213> Arabidopsis thaliana

<400> 123
000

<210> 124
<211> 0
<212> PRT
<213> Arabidopsis thaliana

<400> 124
000

<210> 125
<211> 0
<212> DNA
<213> Arabidopsis thaliana

<400> 125
000

<210> 126
<211> 0
<212> PRT
<213> Arabidopsis thaliana

<400> 126
000

<210> 127
<211> 0

<212> DNA
<213> Arabidopsis thaliana

<400> 127
000

<210> 128
<211> 0
<212> PRT
<213> Arabidopsis thaliana

<400> 128
000

<210> 129
<211> 0
<212> DNA
<213> Arabidopsis thaliana

<400> 129
000

<210> 130
<211> 0
<212> PRT
<213> Arabidopsis thaliana

<400> 130
000

<210> 131
<211> 0
<212> DNA
<213> Arabidopsis thaliana

<400> 131
000

<210> 132
<211> 0
<212> PRT
<213> Arabidopsis thaliana

<400> 132
000

<210> 133
<211> 0
<212> DNA
<213> Arabidopsis thaliana

<400> 133
000

<210> 134
<211> 0
<212> PRT
<213> Arabidopsis thaliana

<400> 134
000

<210> 135
<211> 0
<212> DNA
<213> Arabidopsis thaliana

<400> 135
000

<210> 136
<211> 0
<212> PRT
<213> Arabidopsis thaliana

<400> 136
000

<210> 137
<211> 0
<212> DNA
<213> Arabidopsis thaliana

<400> 137
000

<210> 138
<211> 0
<212> PRT
<213> Arabidopsis thaliana

<400> 138
000

<210> 139
<211> 0
<212> DNA
<213> Arabidopsis thaliana

<400> 139
000

<210> 140
<211> 0
<212> PRT
<213> Arabidopsis thaliana

<400> 140
000

<210> 141
<211> 0
<212> DNA
<213> Arabidopsis thaliana

<400> 141
000

<210> 142
<211> 0
<212> PRT
<213> Arabidopsis thaliana

<400> 142
000

<210> 143
<211> 0
<212> DNA
<213> Arabidopsis thaliana

<400> 143
000

<210> 144
<211> 0
<212> PRT
<213> Arabidopsis thaliana

<400> 144
000

<210> 145
<211> 0
<212> DNA
<213> Arabidopsis thaliana

<400> 145
000

<210> 146
<211> 0
<212> PRT
<213> Arabidopsis thaliana

<400> 146
000

<210> 147
<211> 0
<212> DNA
<213> Arabidopsis thaliana

<400> 147
000

<210> 148
<211> 0
<212> PRT
<213> Arabidopsis thaliana

<400> 148
000

<210> 149
<211> 0

<212> DNA
<213> Arabidopsis thaliana

<400> 149
000

<210> 150
<211> 0
<212> PRT
<213> Arabidopsis thaliana

<400> 150
000

<210> 151
<211> 0
<212> DNA
<213> Arabidopsis thaliana

<400> 151
000

<210> 152
<211> 0
<212> PRT
<213> Arabidopsis thaliana

<400> 152
000

<210> 153
<211> 0
<212> DNA
<213> Arabidopsis thaliana

<400> 153
000

<210> 154
<211> 0
<212> PRT
<213> Arabidopsis thaliana

<400> 154
000

<210> 155
<211> 0
<212> DNA
<213> Arabidopsis thaliana

<400> 155
000

<210> 156
<211> 0
<212> PRT
<213> Arabidopsis thaliana

<400> 156
000

<210> 157
<211> 0
<212> DNA
<213> Arabidopsis thaliana

<400> 157
000

<210> 158
<211> 0
<212> PRT
<213> Arabidopsis thaliana

<400> 158
000

<210> 159
<211> 0
<212> DNA
<213> Arabidopsis thaliana

<400> 159
000

<210> 160
<211> 0
<212> PRT
<213> Arabidopsis thaliana

<400> 160
000

<210> 161
<211> 0
<212> DNA
<213> Arabidopsis thaliana

<400> 161
000

<210> 162
<211> 0
<212> PRT
<213> Arabidopsis thaliana

<400> 162
000

<210> 163
<211> 0
<212> DNA
<213> Arabidopsis thaliana

<400> 163
000

<210> 164
<211> 0
<212> PRT
<213> Arabidopsis thaliana

<400> 164
000

<210> 165
<211> 0
<212> DNA
<213> Arabidopsis thaliana

<400> 165
000

<210> 166
<211> 0
<212> PRT
<213> Arabidopsis thaliana

<400> 166
000

<210> 167
<211> 0
<212> DNA
<213> Arabidopsis thaliana

<400> 167
000

<210> 168
<211> 0
<212> PRT
<213> Arabidopsis thaliana

<400> 168
000

<210> 169
<211> 0
<212> DNA
<213> Arabidopsis thaliana

<400> 169
000

<210> 170
<211> 0
<212> PRT
<213> Arabidopsis thaliana

<400> 170
000

<210> 171
<211> 0

<212> DNA
<213> Arabidopsis thaliana

<400> 171
000

<210> 172
<211> 0
<212> PRT
<213> Arabidopsis thaliana

<400> 172
000

<210> 173
<211> 0
<212> DNA
<213> Arabidopsis thaliana

<400> 173
000

<210> 174
<211> 0
<212> PRT
<213> Arabidopsis thaliana

<400> 174
000

<210> 175
<211> 0
<212> DNA
<213> Arabidopsis thaliana

<400> 175
000

<210> 176
<211> 0
<212> PRT
<213> Arabidopsis thaliana

<400> 176
000

<210> 177
<211> 0
<212> DNA
<213> Arabidopsis thaliana

<400> 177
000

<210> 178
<211> 0
<212> PRT
<213> Arabidopsis thaliana

<400> 178
000

<210> 179
<211> 0
<212> DNA
<213> Arabidopsis thaliana

<400> 179
000

<210> 180
<211> 0
<212> PRT
<213> Arabidopsis thaliana

<400> 180
000

<210> 181
<211> 0
<212> DNA
<213> Arabidopsis thaliana

<400> 181
000

<210> 182
<211> 0
<212> PRT
<213> Arabidopsis thaliana

<400> 182
000

<210> 183
<211> 0
<212> DNA
<213> Arabidopsis thaliana

<400> 183
000

<210> 184
<211> 0
<212> PRT
<213> Arabidopsis thaliana

<400> 184
000

<210> 185
<211> 0
<212> DNA
<213> Arabidopsis thaliana

<400> 185
000

<210> 186
<211> 0
<212> PRT
<213> Arabidopsis thaliana

<400> 186
000

<210> 187
<211> 0
<212> DNA
<213> Arabidopsis thaliana

<400> 187
000

<210> 188
<211> 0
<212> PRT
<213> Arabidopsis thaliana

<400> 188
000

<210> 189
<211> 0
<212> DNA
<213> Arabidopsis thaliana

<400> 189
000

<210> 190
<211> 0
<212> PRT
<213> Arabidopsis thaliana

<400> 190
000

<210> 191
<211> 0
<212> DNA
<213> Arabidopsis thaliana

<400> 191
000

<210> 192
<211> 0
<212> PRT
<213> Arabidopsis thaliana

<400> 192
000

<210> 193
<211> 0

<212> DNA
<213> Arabidopsis thaliana

<400> 193
000

<210> 194
<211> 0
<212> PRT
<213> Arabidopsis thaliana

<400> 194
000

<210> 195
<211> 0
<212> DNA
<213> Arabidopsis thaliana

<400> 195
000

<210> 196
<211> 0
<212> PRT
<213> Arabidopsis thaliana

<400> 196
000

<210> 197
<211> 0
<212> DNA
<213> Arabidopsis thaliana

<400> 197
000

<210> 198
<211> 0
<212> PRT
<213> Arabidopsis thaliana

<400> 198
000

<210> 199
<211> 0
<212> DNA
<213> Arabidopsis thaliana

<400> 199
000

<210> 200
<211> 0
<212> PRT
<213> Arabidopsis thaliana

<400> 200
000

<210> 201
<211> 0
<212> DNA
<213> Arabidopsis thaliana

<400> 201
000

<210> 202
<211> 0
<212> PRT
<213> Arabidopsis thaliana

<400> 202
000

<210> 203
<211> 0
<212> DNA
<213> Arabidopsis thaliana

<400> 203
000

<210> 204
<211> 0
<212> PRT
<213> Arabidopsis thaliana

<400> 204
000

<210> 205
<211> 0
<212> DNA
<213> Arabidopsis thaliana

<400> 205
000

<210> 206
<211> 0
<212> PRT
<213> Arabidopsis thaliana

<400> 206
000

<210> 207
<211> 0
<212> DNA
<213> Arabidopsis thaliana

<400> 207
000

<210> 208
<211> 0
<212> PRT
<213> Arabidopsis thaliana

<400> 208
000

<210> 209
<211> 0
<212> DNA
<213> Arabidopsis thaliana

<400> 209
000

<210> 210
<211> 0
<212> PRT
<213> Arabidopsis thaliana

<400> 210
000

<210> 211
<211> 0
<212> DNA
<213> Arabidopsis thaliana

<400> 211
000

<210> 212
<211> 0
<212> PRT
<213> Arabidopsis thaliana

<400> 212
000

<210> 213
<211> 0
<212> DNA
<213> Arabidopsis thaliana

<400> 213
000

<210> 214
<211> 0
<212> PRT
<213> Arabidopsis thaliana

<400> 214
000

<210> 215
<211> 0

<212> DNA
<213> Arabidopsis thaliana

<400> 215
000

<210> 216
<211> 0
<212> PRT
<213> Arabidopsis thaliana

<400> 216
000

<210> 217
<211> 0
<212> DNA
<213> Arabidopsis thaliana

<400> 217
000

<210> 218
<211> 0
<212> PRT
<213> Arabidopsis thaliana

<400> 218
000

<210> 219
<211> 0
<212> DNA
<213> Arabidopsis thaliana

<400> 219
000

<210> 220
<211> 0
<212> PRT
<213> Arabidopsis thaliana

<400> 220
000

<210> 221
<211> 0
<212> DNA
<213> Arabidopsis thaliana

<400> 221
000

<210> 222
<211> 0
<212> PRT
<213> Arabidopsis thaliana

<400> 222
000

<210> 223
<211> 0
<212> DNA
<213> Arabidopsis thaliana

<400> 223
000

<210> 224
<211> 0
<212> PRT
<213> Arabidopsis thaliana

<400> 224
000

<210> 225
<211> 0
<212> DNA
<213> Arabidopsis thaliana

<400> 225
000

<210> 226
<211> 0
<212> PRT
<213> Arabidopsis thaliana

<400> 226
000

<210> 227
<211> 0
<212> DNA
<213> Arabidopsis thaliana

<400> 227
000

<210> 228
<211> 0
<212> PRT
<213> Arabidopsis thaliana

<400> 228
000

<210> 229
<211> 0
<212> DNA
<213> Arabidopsis thaliana

<400> 229
000

<210> 230
<211> 0
<212> PRT
<213> Arabidopsis thaliana

<400> 230
000

<210> 231
<211> 0
<212> DNA
<213> Arabidopsis thaliana

<400> 231
000

<210> 232
<211> 0
<212> PRT
<213> Arabidopsis thaliana

<400> 232
000

<210> 233
<211> 0
<212> DNA
<213> Arabidopsis thaliana

<400> 233
000

<210> 234
<211> 0
<212> PRT
<213> Arabidopsis thaliana

<400> 234
000

<210> 235
<211> 0
<212> DNA
<213> Arabidopsis thaliana

<400> 235
000

<210> 236
<211> 0
<212> PRT
<213> Arabidopsis thaliana

<400> 236
000

<210> 237
<211> 0

<212> DNA
<213> Arabidopsis thaliana

<400> 237
000

<210> 238
<211> 0
<212> PRT
<213> Arabidopsis thaliana

<400> 238
000

<210> 239
<211> 0
<212> DNA
<213> Arabidopsis thaliana

<400> 239
000

<210> 240
<211> 0
<212> PRT
<213> Arabidopsis thaliana

<400> 240
000

<210> 241
<211> 0
<212> DNA
<213> Arabidopsis thaliana

<400> 241
000

<210> 242
<211> 0
<212> PRT
<213> Arabidopsis thaliana

<400> 242
000

<210> 243
<211> 0
<212> DNA
<213> Arabidopsis thaliana

<400> 243
000

<210> 244
<211> 0
<212> PRT
<213> Arabidopsis thaliana

<400> 244
000

<210> 245
<211> 0
<212> DNA
<213> Arabidopsis thaliana

<400> 245
000

<210> 246
<211> 0
<212> PRT
<213> Arabidopsis thaliana

<400> 246
000

<210> 247
<211> 0
<212> DNA
<213> Arabidopsis thaliana

<400> 247
000

<210> 248
<211> 0
<212> PRT
<213> Arabidopsis thaliana

<400> 248
000

<210> 249
<211> 0
<212> DNA
<213> Arabidopsis thaliana

<400> 249
000

<210> 250
<211> 0
<212> PRT
<213> Arabidopsis thaliana

<400> 250
000

<210> 251
<211> 0
<212> DNA
<213> Arabidopsis thaliana

<400> 251
000

<210> 252
<211> 0
<212> PRT
<213> Arabidopsis thaliana

<400> 252
000

<210> 253
<211> 0
<212> DNA
<213> Arabidopsis thaliana

<400> 253
000

<210> 254
<211> 0
<212> PRT
<213> Arabidopsis thaliana

<400> 254
000

<210> 255
<211> 0
<212> DNA
<213> Arabidopsis thaliana

<400> 255
000

<210> 256
<211> 0
<212> PRT
<213> Arabidopsis thaliana

<400> 256
000

<210> 257
<211> 0
<212> DNA
<213> Arabidopsis thaliana

<400> 257
000

<210> 258
<211> 0
<212> PRT
<213> Arabidopsis thaliana

<400> 258
000

<210> 259
<211> 0

<212> DNA
<213> Arabidopsis thaliana

<400> 259
000

<210> 260
<211> 0
<212> PRT
<213> Arabidopsis thaliana

<400> 260
000

<210> 261
<211> 0
<212> DNA
<213> Arabidopsis thaliana

<400> 261
000

<210> 262
<211> 0
<212> PRT
<213> Arabidopsis thaliana

<400> 262
000

<210> 263
<211> 0
<212> DNA
<213> Arabidopsis thaliana

<400> 263
000

<210> 264
<211> 0
<212> PRT
<213> Arabidopsis thaliana

<400> 264
000

<210> 265
<211> 0
<212> DNA
<213> Arabidopsis thaliana

<400> 265
000

<210> 266
<211> 0
<212> PRT
<213> Arabidopsis thaliana

<400> 266
000

<210> 267
<211> 0
<212> DNA
<213> Arabidopsis thaliana

<400> 267
000

<210> 268
<211> 0
<212> PRT
<213> Arabidopsis thaliana

<400> 268
000

<210> 269
<211> 0
<212> DNA
<213> Arabidopsis thaliana

<400> 269
000

<210> 270
<211> 0
<212> PRT
<213> Arabidopsis thaliana

<400> 270
000

<210> 271
<211> 0
<212> DNA
<213> Arabidopsis thaliana

<400> 271
000

<210> 272
<211> 0
<212> PRT
<213> Arabidopsis thaliana

<400> 272
000

<210> 273
<211> 0
<212> DNA
<213> Arabidopsis thaliana

<400> 273
000

<210> 274
<211> 0
<212> PRT
<213> Arabidopsis thaliana

<400> 274
000

<210> 275
<211> 0
<212> DNA
<213> Arabidopsis thaliana

<400> 275
000

<210> 276
<211> 0
<212> PRT
<213> Arabidopsis thaliana

<400> 276
000

<210> 277
<211> 0
<212> DNA
<213> Arabidopsis thaliana

<400> 277
000

<210> 278
<211> 0
<212> PRT
<213> Arabidopsis thaliana

<400> 278
000

<210> 279
<211> 0
<212> DNA
<213> Arabidopsis thaliana

<400> 279
000

<210> 280
<211> 0
<212> PRT
<213> Arabidopsis thaliana

<400> 280
000

<210> 281
<211> 0

<212> DNA
<213> Arabidopsis thaliana

<400> 281
000

<210> 282
<211> 0
<212> PRT
<213> Arabidopsis thaliana

<400> 282
000

<210> 283
<211> 0
<212> DNA
<213> Arabidopsis thaliana

<400> 283
000

<210> 284
<211> 0
<212> PRT
<213> Arabidopsis thaliana

<400> 284
000

<210> 285
<211> 0
<212> DNA
<213> Arabidopsis thaliana

<400> 285
000

<210> 286
<211> 0
<212> PRT
<213> Arabidopsis thaliana

<400> 286
000

<210> 287
<211> 0
<212> DNA
<213> Arabidopsis thaliana

<400> 287
000

<210> 288
<211> 0
<212> PRT
<213> Arabidopsis thaliana

<400> 288
000

<210> 289
<211> 0
<212> DNA
<213> Arabidopsis thaliana

<400> 289
000

<210> 290
<211> 0
<212> PRT
<213> Arabidopsis thaliana

<400> 290
000

<210> 291
<211> 0
<212> DNA
<213> Arabidopsis thaliana

<400> 291
000

<210> 292
<211> 0
<212> PRT
<213> Arabidopsis thaliana

<400> 292
000

<210> 293
<211> 0
<212> DNA
<213> Arabidopsis thaliana

<400> 293
000

<210> 294
<211> 0
<212> PRT
<213> Arabidopsis thaliana

<400> 294
000

<210> 295
<211> 0
<212> DNA
<213> Arabidopsis thaliana

<400> 295
000

<210> 296
<211> 0
<212> PRT
<213> Arabidopsis thaliana

<400> 296
000

<210> 297
<211> 0
<212> DNA
<213> Arabidopsis thaliana

<400> 297
000

<210> 298
<211> 0
<212> PRT
<213> Arabidopsis thaliana

<400> 298
000

<210> 299
<211> 0
<212> DNA
<213> Arabidopsis thaliana

<400> 299
000

<210> 300
<211> 0
<212> PRT
<213> Arabidopsis thaliana

<400> 300
000

<210> 301
<211> 0
<212> DNA
<213> Arabidopsis thaliana

<400> 301
000

<210> 302
<211> 0
<212> PRT
<213> Arabidopsis thaliana

<400> 302
000

<210> 303
<211> 0

<212> DNA
<213> Arabidopsis thaliana

<400> 303
000

<210> 304
<211> 0
<212> PRT
<213> Arabidopsis thaliana

<400> 304
000

<210> 305
<211> 0
<212> DNA
<213> Arabidopsis thaliana

<400> 305
000

<210> 306
<211> 0
<212> PRT
<213> Arabidopsis thaliana

<400> 306
000

<210> 307
<211> 0
<212> DNA
<213> Arabidopsis thaliana

<400> 307
000

<210> 308
<211> 0
<212> PRT
<213> Arabidopsis thaliana

<400> 308
000

<210> 309
<211> 0
<212> DNA
<213> Arabidopsis thaliana

<400> 309
000

<210> 310
<211> 0
<212> PRT
<213> Arabidopsis thaliana

<400> 310
000

<210> 311
<211> 0
<212> DNA
<213> Arabidopsis thaliana

<400> 311
000

<210> 312
<211> 0
<212> PRT
<213> Arabidopsis thaliana

<400> 312
000

<210> 313
<211> 0
<212> DNA
<213> Arabidopsis thaliana

<400> 313
000

<210> 314
<211> 0
<212> PRT
<213> Arabidopsis thaliana

<400> 314
000

<210> 315
<211> 0
<212> DNA
<213> Arabidopsis thaliana

<400> 315
000

<210> 316
<211> 0
<212> PRT
<213> Arabidopsis thaliana

<400> 316
000

<210> 317
<211> 0
<212> DNA
<213> Arabidopsis thaliana

<400> 317
000

<210> 318
<211> 0
<212> PRT
<213> Arabidopsis thaliana

<400> 318
000

<210> 319
<211> 0
<212> DNA
<213> Arabidopsis thaliana

<400> 319
000

<210> 320
<211> 0
<212> PRT
<213> Arabidopsis thaliana

<400> 320
000

<210> 321
<211> 0
<212> DNA
<213> Arabidopsis thaliana

<400> 321
000

<210> 322
<211> 0
<212> PRT
<213> Arabidopsis thaliana

<400> 322
000

<210> 323
<211> 0
<212> DNA
<213> Arabidopsis thaliana

<400> 323
000

<210> 324
<211> 0
<212> PRT
<213> Arabidopsis thaliana

<400> 324
000

<210> 325
<211> 0

<212> DNA
<213> Arabidopsis thaliana

<400> 325
000

<210> 326
<211> 0
<212> PRT
<213> Arabidopsis thaliana

<400> 326
000

<210> 327
<211> 678
<212> DNA
<213> Arabidopsis thaliana

<400> 327

```
atggtgagct tttgcgagct ttgtggtgcc gaagctgatc tccattgtgc cgcggactct      60
gccttcctct gccgttcttg tgacgctaag ttccatgcct caaattttct cttcgctcgt     120
catttccggc gtgtcatctg cccaaattgc aaatctctta ctcaaaattt cgtttctggt     180
cctcttcttc cttggcctcc acgaacaaca tgttgttcag aatcgtcgtc ttcttcttgc     240
tgctcgtctc ttgactgtgt ctcaagctcc gagctatcgt caacgacgcg tgacgtaaac     300
agagcgcgag ggagggaaaa cagagtgaat gccaaggccg ttgcggttac ggtggcggat     360
ggcatttttg taaattggtg tggtaagtta ggactaaaca gggatttaac aaacgctgtc     420
gtttcatatg cgtctttggc tttggctgtg gagacgaggc caagagcgac gaagagagtg     480
ttcttagcgg cggcgttttg gttcggcgtt aagaacacga cgacgtggca gaatttaaag     540
aaagtagaag atgtgactgg agtttcagct gggatgattc gagcggttga aagcaaattg     600
gcgcgtgcaa tgacgcagca gcttagacgg tggcgcgtgg attcggagga aggatgggct     660

gaaaacgaca acgtttga                                                   678
```

<210> 328
<211> 225
<212> PRT
<213> Arabidopsis thaliana

<400> 328

Met Val Ser Phe Cys Glu Leu Cys Gly Ala Glu Ala Asp Leu His Cys
1               5                   10                  15

Ala Ala Asp Ser Ala Phe Leu Cys Arg Ser Cys Asp Ala Lys Phe His
            20                  25                  30

Ala Ser Asn Phe Leu Phe Ala Arg His Phe Arg Arg Val Ile Cys Pro
        35                  40                  45

Asn Cys Lys Ser Leu Thr Gln Asn Phe Val Ser Gly Pro Leu Leu Pro
    50                  55                  60

Trp Pro Pro Arg Thr Thr Cys Cys Ser Glu Ser Ser Ser Ser Ser Cys
65                  70                  75                  80

Cys Ser Ser Leu Asp Cys Val Ser Ser Ser Glu Leu Ser Ser Thr Thr
                85                  90                  95

Arg Asp Val Asn Arg Ala Arg Gly Arg Glu Asn Arg Val Asn Ala Lys
            100                 105                 110

Ala Val Ala Val Thr Val Ala Asp Gly Ile Phe Val Asn Trp Cys Gly
        115                 120                 125

Lys Leu Gly Leu Asn Arg Asp Leu Thr Asn Ala Val Val Ser Tyr Ala
    130                 135                 140

Ser Leu Ala Leu Ala Val Glu Thr Arg Pro Arg Ala Thr Lys Arg Val
145                 150                 155                 160

Phe Leu Ala Ala Ala Phe Trp Phe Gly Val Lys Asn Thr Thr Thr Trp
                165                 170                 175

Gln Asn Leu Lys Lys Val Glu Asp Val Thr Gly Val Ser Ala Gly Met
            180                 185                 190

Ile Arg Ala Val Glu Ser Lys Leu Ala Arg Ala Met Thr Gln Gln Leu
        195                 200                 205

Arg Arg Trp Arg Val Asp Ser Glu Glu Gly Trp Ala Glu Asn Asp Asn
    210                 215                 220

Val

225

<210> 329
<211> 0
<212> DNA
<213> Arabidopsis thaliana

<400> 329
000

<210> 330
<211> 0
<212> PRT
<213> Arabidopsis thaliana

<400> 330
000

<210> 331
<211> 0
<212> DNA
<213> Arabidopsis thaliana

<400> 331
000

<210> 332
<211> 0
<212> PRT
<213> Arabidopsis thaliana

<400> 332
000

<210> 333
<211> 0
<212> DNA
<213> Arabidopsis thaliana

<400> 333
000

<210> 334
<211> 0
<212> PRT
<213> Arabidopsis thaliana

<400> 334
000

<210> 335
<211> 0
<212> DNA
<213> Arabidopsis thaliana

<400> 335
000

<210> 336
<211> 0
<212> PRT
<213> Arabidopsis thaliana

<400> 336
000

<210> 337
<211> 0
<212> DNA
<213> Arabidopsis thaliana

<400> 337
000

<210> 338
<211> 0
<212> PRT
<213> Arabidopsis thaliana

<400> 338
000

<210> 339
<211> 0
<212> DNA
<213> Arabidopsis thaliana

<400> 339
000

<210> 340
<211> 0
<212> PRT
<213> Arabidopsis thaliana

<400> 340
000

<210> 341
<211> 0
<212> DNA
<213> Arabidopsis thaliana

<400> 341
000

<210> 342
<211> 0
<212> PRT
<213> Arabidopsis thaliana

<400> 342
000

<210> 343
<211> 0
<212> DNA
<213> Arabidopsis thaliana

<400> 343
000

<210> 344
<211> 0

<212> PRT
<213> Arabidopsis thaliana

<400> 344
000

<210> 345
<211> 0
<212> DNA
<213> Arabidopsis thaliana

<400> 345
000

<210> 346
<211> 0
<212> PRT
<213> Arabidopsis thaliana

<400> 346
000

<210> 347
<211> 0
<212> DNA
<213> Arabidopsis thaliana

<400> 347
000

<210> 348
<211> 0
<212> PRT
<213> Arabidopsis thaliana

<400> 348
000

<210> 349
<211> 0
<212> DNA
<213> Arabidopsis thaliana

<400> 349
000

<210> 350
<211> 0
<212> PRT
<213> Arabidopsis thaliana

<400> 350
000

<210> 351
<211> 0
<212> DNA
<213> Arabidopsis thaliana

<400> 351
000

<210> 352
<211> 0
<212> PRT
<213> Arabidopsis thaliana

<400> 352
000

<210> 353
<211> 0
<212> DNA
<213> Arabidopsis thaliana

<400> 353
000

<210> 354
<211> 0
<212> PRT
<213> Arabidopsis thaliana

<400> 354
000

<210> 355
<211> 0
<212> DNA
<213> Arabidopsis thaliana

<400> 355
000

<210> 356
<211> 0
<212> PRT
<213> Arabidopsis thaliana

<400> 356
000

<210> 357
<211> 0
<212> DNA
<213> Arabidopsis thaliana

<400> 357
000

<210> 358
<211> 0
<212> PRT
<213> Arabidopsis thaliana

<400> 358
000

<210> 359
<211> 0
<212> DNA
<213> Arabidopsis thaliana

<400> 359
000

<210> 360
<211> 0
<212> PRT
<213> Arabidopsis thaliana

<400> 360
000

<210> 361
<211> 0
<212> DNA
<213> Arabidopsis thaliana

<400> 361
000

<210> 362
<211> 0
<212> PRT
<213> Arabidopsis thaliana

<400> 362
000

<210> 363
<211> 0
<212> DNA
<213> Arabidopsis thaliana

<400> 363
000

<210> 364
<211> 0
<212> PRT
<213> Arabidopsis thaliana

<400> 364
000

<210> 365
<211> 0
<212> DNA
<213> Arabidopsis thaliana

<400> 365
000

<210> 366
<211> 0

<212> PRT
<213> Arabidopsis thaliana

<400> 366
000

<210> 367
<211> 0
<212> DNA
<213> Arabidopsis thaliana

<400> 367
000

<210> 368
<211> 0
<212> PRT
<213> Arabidopsis thaliana

<400> 368
000

<210> 369
<211> 0
<212> DNA
<213> Arabidopsis thaliana

<400> 369
000

<210> 370
<211> 0
<212> PRT
<213> Arabidopsis thaliana

<400> 370
000

<210> 371
<211> 0
<212> DNA
<213> Arabidopsis thaliana

<400> 371
000

<210> 372
<211> 0
<212> PRT
<213> Arabidopsis thaliana

<400> 372
000

<210> 373
<211> 0
<212> DNA
<213> Arabidopsis thaliana

<400> 373
000

<210> 374
<211> 0
<212> PRT
<213> Arabidopsis thaliana

<400> 374
000

<210> 375
<211> 0
<212> DNA
<213> Arabidopsis thaliana

<400> 375
000

<210> 376
<211> 0
<212> PRT
<213> Arabidopsis thaliana

<400> 376
000

<210> 377
<211> 0
<212> DNA
<213> Arabidopsis thaliana

<400> 377
000

<210> 378
<211> 0
<212> PRT
<213> Arabidopsis thaliana

<400> 378
000

<210> 379
<211> 0
<212> DNA
<213> Arabidopsis thaliana

<400> 379
000

<210> 380
<211> 0
<212> PRT
<213> Arabidopsis thaliana

<400> 380
000

<210> 381
<211> 0
<212> DNA
<213> Arabidopsis thaliana

<400> 381
000

<210> 382
<211> 0
<212> PRT
<213> Arabidopsis thaliana

<400> 382
000

<210> 383
<211> 0
<212> DNA
<213> Arabidopsis thaliana

<400> 383
000

<210> 384
<211> 0
<212> PRT
<213> Arabidopsis thaliana

<400> 384
000

<210> 385
<211> 0
<212> DNA
<213> Arabidopsis thaliana

<400> 385
000

<210> 386
<211> 0
<212> PRT
<213> Arabidopsis thaliana

<400> 386
000

<210> 387
<211> 0
<212> DNA
<213> Arabidopsis thaliana

<400> 387
000

<210> 388
<211> 0

<212> PRT
<213> Arabidopsis thaliana

<400> 388
000

<210> 389
<211> 0
<212> DNA
<213> Arabidopsis thaliana

<400> 389
000

<210> 390
<211> 0
<212> PRT
<213> Arabidopsis thaliana

<400> 390
000

<210> 391
<211> 0
<212> DNA
<213> Arabidopsis thaliana

<400> 391
000

<210> 392
<211> 0
<212> PRT
<213> Arabidopsis thaliana

<400> 392
000

<210> 393
<211> 0
<212> DNA
<213> Arabidopsis thaliana

<400> 393
000

<210> 394
<211> 0
<212> PRT
<213> Arabidopsis thaliana

<400> 394
000

<210> 395
<211> 0
<212> DNA
<213> Arabidopsis thaliana

<400> 395
000

<210> 396
<211> 0
<212> PRT
<213> Arabidopsis thaliana

<400> 396
000

<210> 397
<211> 0
<212> DNA
<213> Arabidopsis thaliana

<400> 397
000

<210> 398
<211> 0
<212> PRT
<213> Arabidopsis thaliana

<400> 398
000

<210> 399
<211> 0
<212> DNA
<213> Arabidopsis thaliana

<400> 399
000

<210> 400
<211> 0
<212> PRT
<213> Arabidopsis thaliana

<400> 400
000

<210> 401
<211> 0
<212> DNA
<213> Arabidopsis thaliana

<400> 401
000

<210> 402
<211> 0
<212> PRT
<213> Arabidopsis thaliana

<400> 402
000

<210> 403
<211> 0
<212> DNA
<213> Arabidopsis thaliana

<400> 403
000

<210> 404
<211> 0
<212> PRT
<213> Arabidopsis thaliana

<400> 404
000

<210> 405
<211> 0
<212> DNA
<213> Arabidopsis thaliana

<400> 405
000

<210> 406
<211> 0
<212> PRT
<213> Arabidopsis thaliana

<400> 406
000

<210> 407
<211> 0
<212> DNA
<213> Arabidopsis thaliana

<400> 407
000

<210> 408
<211> 0
<212> PRT
<213> Arabidopsis thaliana

<400> 408
000

<210> 409
<211> 0
<212> DNA
<213> Arabidopsis thaliana

<400> 409
000

<210> 410
<211> 0

<212> PRT
<213> Arabidopsis thaliana

<400> 410
000

<210> 411
<211> 0
<212> DNA
<213> Arabidopsis thaliana

<400> 411
000

<210> 412
<211> 0
<212> PRT
<213> Arabidopsis thaliana

<400> 412
000

<210> 413
<211> 0
<212> DNA
<213> Arabidopsis thaliana

<400> 413
000

<210> 414
<211> 0
<212> PRT
<213> Arabidopsis thaliana

<400> 414
000

<210> 415
<211> 0
<212> DNA
<213> Arabidopsis thaliana

<400> 415
000

<210> 416
<211> 0
<212> PRT
<213> Arabidopsis thaliana

<400> 416
000

<210> 417
<211> 0
<212> DNA
<213> Arabidopsis thaliana

<400> 417
000

<210> 418
<211> 0
<212> PRT
<213> Arabidopsis thaliana

<400> 418
000

<210> 419
<211> 0
<212> DNA
<213> Arabidopsis thaliana

<400> 419
000

<210> 420
<211> 0
<212> PRT
<213> Arabidopsis thaliana

<400> 420
000

<210> 421
<211> 0
<212> DNA
<213> Arabidopsis thaliana

<400> 421
000

<210> 422
<211> 0
<212> PRT
<213> Arabidopsis thaliana

<400> 422
000

<210> 423
<211> 0
<212> DNA
<213> Arabidopsis thaliana

<400> 423
000

<210> 424
<211> 0
<212> PRT
<213> Arabidopsis thaliana

<400> 424
000

<210> 425
<211> 0
<212> DNA
<213> Arabidopsis thaliana

<400> 425
000

<210> 426
<211> 0
<212> PRT
<213> Arabidopsis thaliana

<400> 426
000

<210> 427
<211> 0
<212> DNA
<213> Arabidopsis thaliana

<400> 427
000

<210> 428
<211> 0
<212> PRT
<213> Arabidopsis thaliana

<400> 428
000

<210> 429
<211> 0
<212> DNA
<213> Arabidopsis thaliana

<400> 429
000

<210> 430
<211> 0
<212> PRT
<213> Arabidopsis thaliana

<400> 430
000

<210> 431
<211> 0
<212> DNA
<213> Arabidopsis thaliana

<400> 431
000

<210> 432
<211> 0

<212> PRT
<213> Arabidopsis thaliana

<400> 432
000

<210> 433
<211> 0
<212> DNA
<213> Arabidopsis thaliana

<400> 433
000

<210> 434
<211> 0
<212> PRT
<213> Arabidopsis thaliana

<400> 434
000

<210> 435
<211> 0
<212> DNA
<213> Arabidopsis thaliana

<400> 435
000

<210> 436
<211> 0
<212> PRT
<213> Arabidopsis thaliana

<400> 436
000

<210> 437
<211> 0
<212> DNA
<213> Arabidopsis thaliana

<400> 437
000

<210> 438
<211> 0
<212> PRT
<213> Arabidopsis thaliana

<400> 438
000

<210> 439
<211> 0
<212> DNA
<213> Arabidopsis thaliana

<400> 439
000

<210> 440
<211> 0
<212> PRT
<213> Arabidopsis thaliana

<400> 440
000

<210> 441
<211> 0
<212> DNA
<213> Arabidopsis thaliana

<400> 441
000

<210> 442
<211> 0
<212> PRT
<213> Arabidopsis thaliana

<400> 442
000

<210> 443
<211> 0
<212> DNA
<213> Arabidopsis thaliana

<400> 443
000

<210> 444
<211> 0
<212> PRT
<213> Arabidopsis thaliana

<400> 444
000

<210> 445
<211> 0
<212> DNA
<213> Arabidopsis thaliana

<400> 445
000

<210> 446
<211> 0
<212> PRT
<213> Arabidopsis thaliana

<400> 446
000

<210> 447
<211> 0
<212> DNA
<213> Arabidopsis thaliana

<400> 447
000

<210> 448
<211> 0
<212> PRT
<213> Arabidopsis thaliana

<400> 448
000

<210> 449
<211> 0
<212> DNA
<213> Arabidopsis thaliana

<400> 449
000

<210> 450
<211> 0
<212> PRT
<213> Arabidopsis thaliana

<400> 450
000

<210> 451
<211> 0
<212> DNA
<213> Arabidopsis thaliana

<400> 451
000

<210> 452
<211> 0
<212> PRT
<213> Arabidopsis thaliana

<400> 452
000

<210> 453
<211> 0
<212> DNA
<213> Arabidopsis thaliana

<400> 453
000

<210> 454
<211> 0

<212> PRT
<213> Arabidopsis thaliana

<400> 454
000

<210> 455
<211> 0
<212> DNA
<213> Arabidopsis thaliana

<400> 455
000

<210> 456
<211> 0
<212> PRT
<213> Arabidopsis thaliana

<400> 456
000

<210> 457
<211> 0
<212> DNA
<213> Arabidopsis thaliana

<400> 457
000

<210> 458
<211> 0
<212> PRT
<213> Arabidopsis thaliana

<400> 458
000

<210> 459
<211> 0
<212> DNA
<213> Arabidopsis thaliana

<400> 459
000

<210> 460
<211> 0
<212> PRT
<213> Arabidopsis thaliana

<400> 460
000

<210> 461
<211> 0
<212> DNA
<213> Arabidopsis thaliana

<400> 461
000

<210> 462
<211> 0
<212> PRT
<213> Arabidopsis thaliana

<400> 462
000

<210> 463
<211> 0
<212> DNA
<213> Arabidopsis thaliana

<400> 463
000

<210> 464
<211> 0
<212> PRT
<213> Arabidopsis thaliana

<400> 464
000

<210> 465
<211> 0
<212> DNA
<213> Arabidopsis thaliana

<400> 465
000

<210> 466
<211> 0
<212> PRT
<213> Arabidopsis thaliana

<400> 466
000

<210> 467
<211> 0
<212> DNA
<213> Arabidopsis thaliana

<400> 467
000

<210> 468
<211> 0
<212> PRT
<213> Arabidopsis thaliana

<400> 468
000

<210> 469
<211> 0
<212> DNA
<213> Arabidopsis thaliana

<400> 469
000

<210> 470
<211> 0
<212> PRT
<213> Arabidopsis thaliana

<400> 470
000

<210> 471
<211> 0
<212> DNA
<213> Arabidopsis thaliana

<400> 471
000

<210> 472
<211> 0
<212> PRT
<213> Arabidopsis thaliana

<400> 472
000

<210> 473
<211> 0
<212> DNA
<213> Arabidopsis thaliana

<400> 473
000

<210> 474
<211> 0
<212> PRT
<213> Arabidopsis thaliana

<400> 474
000

<210> 475
<211> 0
<212> DNA
<213> Arabidopsis thaliana

<400> 475
000

<210> 476
<211> 0

<212> PRT
<213> Arabidopsis thaliana

<400> 476
000

<210> 477
<211> 0
<212> DNA
<213> Arabidopsis thaliana

<400> 477
000

<210> 478
<211> 0
<212> PRT
<213> Arabidopsis thaliana

<400> 478
000

<210> 479
<211> 0
<212> DNA
<213> Arabidopsis thaliana

<400> 479
000

<210> 480
<211> 0
<212> PRT
<213> Arabidopsis thaliana

<400> 480
000

<210> 481
<211> 0
<212> DNA
<213> Arabidopsis thaliana

<400> 481
000

<210> 482
<211> 0
<212> PRT
<213> Arabidopsis thaliana

<400> 482
000

<210> 483
<211> 0
<212> DNA
<213> Arabidopsis thaliana

<400> 483
000

<210> 484
<211> 0
<212> PRT
<213> Arabidopsis thaliana

<400> 484
000

<210> 485
<211> 0
<212> DNA
<213> Arabidopsis thaliana

<400> 485
000

<210> 486
<211> 0
<212> PRT
<213> Arabidopsis thaliana

<400> 486
000

<210> 487
<211> 0
<212> DNA
<213> Arabidopsis thaliana

<400> 487
000

<210> 488
<211> 0
<212> PRT
<213> Arabidopsis thaliana

<400> 488
000

<210> 489
<211> 0
<212> DNA
<213> Arabidopsis thaliana

<400> 489
000

<210> 490
<211> 0
<212> PRT
<213> Arabidopsis thaliana

<400> 490
000

<210> 491
<211> 0
<212> DNA
<213> Arabidopsis thaliana

<400> 491
000

<210> 492
<211> 0
<212> PRT
<213> Arabidopsis thaliana

<400> 492
000

<210> 493
<211> 0
<212> DNA
<213> Arabidopsis thaliana

<400> 493
000

<210> 494
<211> 0
<212> PRT
<213> Arabidopsis thaliana

<400> 494
000

<210> 495
<211> 0
<212> DNA
<213> Arabidopsis thaliana

<400> 495
000

<210> 496
<211> 0
<212> PRT
<213> Arabidopsis thaliana

<400> 496
000

<210> 497
<211> 0
<212> DNA
<213> Arabidopsis thaliana

<400> 497
000

<210> 498
<211> 0

<212> PRT
<213> Arabidopsis thaliana

<400> 498
000

<210> 499
<211> 0
<212> DNA
<213> Arabidopsis thaliana

<400> 499
000

<210> 500
<211> 0
<212> PRT
<213> Arabidopsis thaliana

<400> 500
000

<210> 501
<211> 0
<212> DNA
<213> Arabidopsis thaliana

<400> 501
000

<210> 502
<211> 0
<212> PRT
<213> Arabidopsis thaliana

<400> 502
000

<210> 503
<211> 0
<212> DNA
<213> Arabidopsis thaliana

<400> 503
000

<210> 504
<211> 0
<212> PRT
<213> Arabidopsis thaliana

<400> 504
000

<210> 505
<211> 0
<212> DNA
<213> Arabidopsis thaliana

<400> 505
000

<210> 506
<211> 0
<212> PRT
<213> Arabidopsis thaliana

<400> 506
000

<210> 507
<211> 0
<212> DNA
<213> Arabidopsis thaliana

<400> 507
000

<210> 508
<211> 0
<212> PRT
<213> Arabidopsis thaliana

<400> 508
000

<210> 509
<211> 0
<212> DNA
<213> Arabidopsis thaliana

<400> 509
000

<210> 510
<211> 0
<212> PRT
<213> Arabidopsis thaliana

<400> 510
000

<210> 511
<211> 0
<212> DNA
<213> Arabidopsis thaliana

<400> 511
000

<210> 512
<211> 0
<212> PRT
<213> Arabidopsis thaliana

<400> 512
000

<210> 513
<211> 0
<212> DNA
<213> Arabidopsis thaliana

<400> 513
000

<210> 514
<211> 0
<212> PRT
<213> Arabidopsis thaliana

<400> 514
000

<210> 515
<211> 0
<212> DNA
<213> Arabidopsis thaliana

<400> 515
000

<210> 516
<211> 0
<212> PRT
<213> Arabidopsis thaliana

<400> 516
000

<210> 517
<211> 0
<212> DNA
<213> Arabidopsis thaliana

<400> 517
000

<210> 518
<211> 0
<212> PRT
<213> Arabidopsis thaliana

<400> 518
000

<210> 519
<211> 0
<212> DNA
<213> Arabidopsis thaliana

<400> 519
000

<210> 520
<211> 0

<212> PRT
<213> Arabidopsis thaliana

<400> 520
000

<210> 521
<211> 0
<212> DNA
<213> Arabidopsis thaliana

<400> 521
000

<210> 522
<211> 0
<212> PRT
<213> Arabidopsis thaliana

<400> 522
000

<210> 523
<211> 0
<212> DNA
<213> Arabidopsis thaliana

<400> 523
000

<210> 524
<211> 0
<212> PRT
<213> Arabidopsis thaliana

<400> 524
000

<210> 525
<211> 0
<212> DNA
<213> Arabidopsis thaliana

<400> 525
000

<210> 526
<211> 0
<212> PRT
<213> Arabidopsis thaliana

<400> 526
000

<210> 527
<211> 0
<212> DNA
<213> Arabidopsis thaliana

<400> 527
000

<210> 528
<211> 0
<212> PRT
<213> Arabidopsis thaliana

<400> 528
000

<210> 529
<211> 0
<212> DNA
<213> Arabidopsis thaliana

<400> 529
000

<210> 530
<211> 0
<212> PRT
<213> Arabidopsis thaliana

<400> 530
000

<210> 531
<211> 0
<212> DNA
<213> Arabidopsis thaliana

<400> 531
000

<210> 532
<211> 0
<212> PRT
<213> Arabidopsis thaliana

<400> 532
000

<210> 533
<211> 0
<212> DNA
<213> Arabidopsis thaliana

<400> 533
000

<210> 534
<211> 0
<212> PRT
<213> Arabidopsis thaliana

<400> 534
000

<210> 535
<211> 0
<212> DNA
<213> Arabidopsis thaliana

<400> 535
000

<210> 536
<211> 0
<212> PRT
<213> Arabidopsis thaliana

<400> 536
000

<210> 537
<211> 0
<212> DNA
<213> Arabidopsis thaliana

<400> 537
000

<210> 538
<211> 0
<212> PRT
<213> Arabidopsis thaliana

<400> 538
000

<210> 539
<211> 0
<212> DNA
<213> Arabidopsis thaliana

<400> 539
000

<210> 540
<211> 0
<212> PRT
<213> Arabidopsis thaliana

<400> 540
000

<210> 541
<211> 0
<212> DNA
<213> Arabidopsis thaliana

<400> 541
000

<210> 542
<211> 0

<212> PRT
<213> Arabidopsis thaliana

<400> 542
000

<210> 543
<211> 0
<212> DNA
<213> Arabidopsis thaliana

<400> 543
000

<210> 544
<211> 0
<212> PRT
<213> Arabidopsis thaliana

<400> 544
000

<210> 545
<211> 0
<212> DNA
<213> Arabidopsis thaliana

<400> 545
000

<210> 546
<211> 0
<212> PRT
<213> Arabidopsis thaliana

<400> 546
000

<210> 547
<211> 0
<212> DNA
<213> Arabidopsis thaliana

<400> 547
000

<210> 548
<211> 0
<212> PRT
<213> Arabidopsis thaliana

<400> 548
000

<210> 549
<211> 0
<212> DNA
<213> Arabidopsis thaliana

<400> 549
000

<210> 550
<211> 0
<212> PRT
<213> Arabidopsis thaliana

<400> 550
000

<210> 551
<211> 0
<212> DNA
<213> Arabidopsis thaliana

<400> 551
000

<210> 552
<211> 0
<212> PRT
<213> Arabidopsis thaliana

<400> 552
000

<210> 553
<211> 0
<212> DNA
<213> Arabidopsis thaliana

<400> 553
000

<210> 554
<211> 0
<212> PRT
<213> Arabidopsis thaliana

<400> 554
000

<210> 555
<211> 0
<212> DNA
<213> Arabidopsis thaliana

<400> 555
000

<210> 556
<211> 0
<212> PRT
<213> Arabidopsis thaliana

<400> 556
000

<210> 557
<211> 0
<212> DNA
<213> Arabidopsis thaliana

<400> 557
000

<210> 558
<211> 0
<212> PRT
<213> Arabidopsis thaliana

<400> 558
000

<210> 559
<211> 0
<212> DNA
<213> Arabidopsis thaliana

<400> 559
000

<210> 560
<211> 0
<212> PRT
<213> Arabidopsis thaliana

<400> 560
000

<210> 561
<211> 0
<212> DNA
<213> Arabidopsis thaliana

<400> 561
000

<210> 562
<211> 0
<212> PRT
<213> Arabidopsis thaliana

<400> 562
000

<210> 563
<211> 0
<212> DNA
<213> Arabidopsis thaliana

<400> 563
000

<210> 564
<211> 0

<212> PRT
<213> Arabidopsis thaliana

<400> 564
000

<210> 565
<211> 0
<212> DNA
<213> Arabidopsis thaliana

<400> 565
000

<210> 566
<211> 0
<212> PRT
<213> Arabidopsis thaliana

<400> 566
000

<210> 567
<211> 0
<212> DNA
<213> Arabidopsis thaliana

<400> 567
000

<210> 568
<211> 0
<212> PRT
<213> Arabidopsis thaliana

<400> 568
000

<210> 569
<211> 0
<212> DNA
<213> Arabidopsis thaliana

<400> 569
000

<210> 570
<211> 0
<212> PRT
<213> Arabidopsis thaliana

<400> 570
000

<210> 571
<211> 0
<212> DNA
<213> Arabidopsis thaliana

<400> 571
000

<210> 572
<211> 0
<212> PRT
<213> Arabidopsis thaliana

<400> 572
000

<210> 573
<211> 0
<212> DNA
<213> Arabidopsis thaliana

<400> 573
000

<210> 574
<211> 0
<212> PRT
<213> Arabidopsis thaliana

<400> 574
000

<210> 575
<211> 0
<212> DNA
<213> Arabidopsis thaliana

<400> 575
000

<210> 576
<211> 0
<212> PRT
<213> Arabidopsis thaliana

<400> 576
000

<210> 577
<211> 0
<212> DNA
<213> Arabidopsis thaliana

<400> 577
000

<210> 578
<211> 0
<212> PRT
<213> Arabidopsis thaliana

<400> 578
000

<210> 579
<211> 0
<212> DNA
<213> Arabidopsis thaliana

<400> 579
000

<210> 580
<211> 0
<212> PRT
<213> Arabidopsis thaliana

<400> 580
000

<210> 581
<211> 0
<212> DNA
<213> Arabidopsis thaliana

<400> 581
000

<210> 582
<211> 0
<212> PRT
<213> Arabidopsis thaliana

<400> 582
000

<210> 583
<211> 0
<212> DNA
<213> Arabidopsis thaliana

<400> 583
000

<210> 584
<211> 0
<212> PRT
<213> Arabidopsis thaliana

<400> 584
000

<210> 585
<211> 0
<212> DNA
<213> Arabidopsis thaliana

<400> 585
000

<210> 586

<211> 0
<212> PRT
<213> Arabidopsis thaliana

<400> 586
000

<210> 587
<211> 0
<212> DNA
<213> Arabidopsis thaliana

<400> 587
000

<210> 588
<211> 0
<212> PRT
<213> Arabidopsis thaliana

<400> 588
000

<210> 589
<211> 0
<212> DNA
<213> Arabidopsis thaliana

<400> 589
000

<210> 590
<211> 0
<212> PRT
<213> Arabidopsis thaliana

<400> 590
000

<210> 591
<211> 0
<212> DNA
<213> Arabidopsis thaliana

<400> 591
000

<210> 592
<211> 0
<212> PRT
<213> Arabidopsis thaliana

<400> 592
000

<210> 593
<211> 0
<212> DNA

<213> Arabidopsis thaliana

<400> 593
000

<210> 594
<211> 0
<212> PRT
<213> Arabidopsis thaliana

<400> 594
000

<210> 595
<211> 0
<212> DNA
<213> Arabidopsis thaliana

<400> 595
000

<210> 596
<211> 0
<212> PRT
<213> Arabidopsis thaliana

<400> 596
000

<210> 597
<211> 0
<212> DNA
<213> Arabidopsis thaliana

<400> 597
000

<210> 598
<211> 0
<212> PRT
<213> Arabidopsis thaliana

<400> 598
000

<210> 599
<211> 0
<212> DNA
<213> Arabidopsis thaliana

<400> 599
000

<210> 600
<211> 0
<212> PRT
<213> Arabidopsis thaliana

<400> 600
000

<210> 601
<211> 0
<212> DNA
<213> Arabidopsis thaliana

<400> 601
000

<210> 602
<211> 0
<212> PRT
<213> Arabidopsis thaliana

<400> 602
000

<210> 603
<211> 0
<212> DNA
<213> Arabidopsis thaliana

<400> 603
000

<210> 604
<211> 0
<212> PRT
<213> Arabidopsis thaliana

<400> 604
000

<210> 605
<211> 0
<212> DNA
<213> Arabidopsis thaliana

<400> 605
000

<210> 606
<211> 0
<212> PRT
<213> Arabidopsis thaliana

<400> 606
000

<210> 607
<211> 0
<212> DNA
<213> Arabidopsis thaliana

<400> 607
000

<210> 608
<211> 0
<212> PRT
<213> Arabidopsis thaliana

<400> 608
000

<210> 609
<211> 0
<212> DNA
<213> Arabidopsis thaliana

<400> 609
000

<210> 610
<211> 0
<212> PRT
<213> Arabidopsis thaliana

<400> 610
000

<210> 611
<211> 0
<212> DNA
<213> Arabidopsis thaliana

<400> 611
000

<210> 612
<211> 0
<212> PRT
<213> Arabidopsis thaliana

<400> 612
000

<210> 613
<211> 0
<212> DNA
<213> Arabidopsis thaliana

<400> 613
000

<210> 614
<211> 0
<212> PRT
<213> Arabidopsis thaliana

<400> 614
000

<210> 615
<211> 0

<212> DNA
<213> Arabidopsis thaliana

<400> 615
000

<210> 616
<211> 0
<212> PRT
<213> Arabidopsis thaliana

<400> 616
000

<210> 617
<211> 0
<212> DNA
<213> Arabidopsis thaliana

<400> 617
000

<210> 618
<211> 0
<212> PRT
<213> Arabidopsis thaliana

<400> 618
000

<210> 619
<211> 0
<212> DNA
<213> Arabidopsis thaliana

<400> 619
000

<210> 620
<211> 0
<212> PRT
<213> Arabidopsis thaliana

<400> 620
000

<210> 621
<211> 0
<212> DNA
<213> Arabidopsis thaliana

<400> 621
000

<210> 622
<211> 0
<212> PRT
<213> Arabidopsis thaliana

<400> 622
000

<210> 623
<211> 0
<212> DNA
<213> Arabidopsis thaliana

<400> 623
000

<210> 624
<211> 0
<212> PRT
<213> Arabidopsis thaliana

<400> 624
000

<210> 625
<211> 0
<212> DNA
<213> Arabidopsis thaliana

<400> 625
000

<210> 626
<211> 0
<212> PRT
<213> Arabidopsis thaliana

<400> 626
000

<210> 627
<211> 0
<212> DNA
<213> Arabidopsis thaliana

<400> 627
000

<210> 628
<211> 0
<212> PRT
<213> Arabidopsis thaliana

<400> 628
000

<210> 629
<211> 0
<212> DNA
<213> Arabidopsis thaliana

<400> 629
000

<210> 630
<211> 0
<212> PRT
<213> Arabidopsis thaliana

<400> 630
000

<210> 631
<211> 0
<212> DNA
<213> Arabidopsis thaliana

<400> 631
000

<210> 632
<211> 0
<212> PRT
<213> Arabidopsis thaliana

<400> 632
000

<210> 633
<211> 0
<212> DNA
<213> Arabidopsis thaliana

<400> 633
000

<210> 634
<211> 0
<212> PRT
<213> Arabidopsis thaliana

<400> 634
000

<210> 635
<211> 0
<212> DNA
<213> Arabidopsis thaliana

<400> 635
000

<210> 636
<211> 0
<212> PRT
<213> Arabidopsis thaliana

<400> 636
000

<210> 637
<211> 0

<212> DNA
<213> Arabidopsis thaliana

<400> 637
000

<210> 638
<211> 0
<212> PRT
<213> Arabidopsis thaliana

<400> 638
000

<210> 639
<211> 0
<212> DNA
<213> Arabidopsis thaliana

<400> 639
000

<210> 640
<211> 0
<212> PRT
<213> Arabidopsis thaliana

<400> 640
000

<210> 641
<211> 0
<212> DNA
<213> Arabidopsis thaliana

<400> 641
000

<210> 642
<211> 0
<212> PRT
<213> Arabidopsis thaliana

<400> 642
000

<210> 643
<211> 0
<212> DNA
<213> Arabidopsis thaliana

<400> 643
000

<210> 644
<211> 0
<212> PRT
<213> Arabidopsis thaliana

<400> 644
000

<210> 645
<211> 0
<212> DNA
<213> Arabidopsis thaliana

<400> 645
000

<210> 646
<211> 0
<212> PRT
<213> Arabidopsis thaliana

<400> 646
000

<210> 647
<211> 0
<212> DNA
<213> Arabidopsis thaliana

<400> 647
000

<210> 648
<211> 0
<212> PRT
<213> Arabidopsis thaliana

<400> 648
000

<210> 649
<211> 0
<212> DNA
<213> Arabidopsis thaliana

<400> 649
000

<210> 650
<211> 0
<212> PRT
<213> Arabidopsis thaliana

<400> 650
000

<210> 651
<211> 0
<212> DNA
<213> Arabidopsis thaliana

<400> 651
000

<210> 652
<211> 0
<212> PRT
<213> Arabidopsis thaliana

<400> 652
000

<210> 653
<211> 0
<212> DNA
<213> Arabidopsis thaliana

<400> 653
000

<210> 654
<211> 0
<212> PRT
<213> Arabidopsis thaliana

<400> 654
000

<210> 655
<211> 0
<212> DNA
<213> Arabidopsis thaliana

<400> 655
000

<210> 656
<211> 0
<212> PRT
<213> Arabidopsis thaliana

<400> 656
000

<210> 657
<211> 0
<212> DNA
<213> Arabidopsis thaliana

<400> 657
000

<210> 658
<211> 0
<212> PRT
<213> Arabidopsis thaliana

<400> 658
000

<210> 659
<211> 0

<212> DNA
<213> Arabidopsis thaliana

<400> 659
000

<210> 660
<211> 0
<212> PRT
<213> Arabidopsis thaliana

<400> 660
000

<210> 661
<211> 0
<212> DNA
<213> Arabidopsis thaliana

<400> 661
000

<210> 662
<211> 0
<212> PRT
<213> Arabidopsis thaliana

<400> 662
000

<210> 663
<211> 0
<212> DNA
<213> Arabidopsis thaliana

<400> 663
000

<210> 664
<211> 0
<212> PRT
<213> Arabidopsis thaliana

<400> 664
000

<210> 665
<211> 0
<212> DNA
<213> Arabidopsis thaliana

<400> 665
000

<210> 666
<211> 0
<212> PRT
<213> Arabidopsis thaliana

<400> 666
000

<210> 667
<211> 0
<212> DNA
<213> Arabidopsis thaliana

<400> 667
000

<210> 668
<211> 0
<212> PRT
<213> Arabidopsis thaliana

<400> 668
000
<210> 669
<211> 0
<212> DNA
<213> Arabidopsis thaliana

<400> 669
000

<210> 670
<211> 0
<212> PRT
<213> Arabidopsis thaliana

<400> 670
000

<210> 671
<211> 0
<212> DNA
<213> Glycine max

<400> 671
000
<210> 672
<211> 0
<212> DNA
<213> Glycine max
<400> 672
000

<210> 673
<211> 0
<212> DNA
<213> Glycine max

<400> 673
000

<210> 674
<211> 0

<212> DNA
<213> Glycine max

<400> 674
000

<210> 675
<211> 0
<212> DNA
<213> Glycine max

<400> 675
000

<210> 676
<211> 0

<212> DNA
<213> Glycine max

<400> 676
000

<210> 677
<211> 0
<212> DNA
<213> Glycine max

<400> 677
000

<210> 678
<211> 0
<212> DNA
<213> Oryza sativa

<400> 678
000

<210> 679
<211> 0
<212> DNA
<213> Oryza sativa

<400> 679
000

<210> 680
<211> 0
<212> DNA
<213> Oryza sativa

<400> 680
000

<210> 681
<211> 0
<212> PRT

<213> Oryza sativa

<400> 681
000

<210> 682
<211> 0
<212> PRT
<213> Oryza sativa

<400> 682
000

<210> 683
<211> 0
<212> PRT
<213> Oryza sativa

<400> 683
000
<210> 684
<211> 0
<212> DNA
<213> Zea mays

<400> 684
000

<210> 685
<211> 0
<212> DNA
<213> Glycine max

<400> 685
000

<210> 686
<211> 0
<212> DNA
<213> Glycine max

<400> 686
000

<210> 687
<211> 0
<212> DNA
<213> Glycine max

<400> 687
000

<210> 688
<211> 0
<212> DNA
<213> Glycine max

<400> 688

000

<210> 689
<211> 0
<212> DNA
<213> Glycine max

<400> 689
000

<210> 690
<211> 0
<212> PRT
<213> Oryza sativa

<400> 690
000

<210> 691
<211> 0
<212> DNA
<213> Zea mays

<400> 691
000

<210> 692
<211> 0
<212> DNA
<213> Glycine max

<400> 692
000

<210> 693
<211> 0
<212> DNA
<213> Glycine max

<400> 693
000

<210> 694
<211> 0
<212> DNA
<213> Glycine max

<400> 694
000

<210> 695
<211> 0
<212> DNA
<213> Glycine max

<400> 695
000

<210> 696
<211> 0
<212> DNA
<213> Glycine max

<400> 696
000

<210> 697
<211> 0
<212> DNA
<213> Glycine max

<400> 697
000

<210> 698
<211> 0
<212> DNA
<213> Glycine max

<400> 698
000

<210> 699
<211> 0
<212> DNA
<213> Glycine max

<400> 699
000

<210> 700
<211> 0
<212> PRT
<213> Oryza sativa

<400> 700
000

<210> 701
<211> 0
<212> DNA
<213> Zea mays

<400> 701
000

<210> 702
<211> 0
<212> DNA
<213> Glycine max

<400> 702
000

<210> 703
<211> 0

<212> PRT
<213> Oryza sativa

<400> 703
000

<210> 704
<211> 0
<212> DNA
<213> Glycine max

<400> 704
000

<210> 705
<211> 0
<212> DNA
<213> Glycine max

<400> 705
000

<210> 706
<211> 0
<212> DNA
<213> Oryza sativa

<400> 706
000

<210> 707
<211> 0
<212> PRT
<213> Oryza sativa

<400> 707
000

<210> 708
<211> 0
<212> DNA
<213> Zea mays

<400> 708
000

<210> 709
<211> 0
<212> DNA
<213> Zea mays

<400> 709
000

<210> 710
<211> 0
<212> DNA
<213> Zea mays

<400> 710
000

<210> 711
<211> 0
<212> DNA
<213> Zea mays

<400> 711
000

<210> 712
<211> 0
<212> DNA
<213> Zea mays

<400> 712
000

<210> 713
<211> 0
<212> DNA
<213> Glycine max

<400> 713
000

<210> 714
<211> 0
<212> DNA
<213> Glycine max

<400> 714
000

<210> 715
<211> 0
<212> PRT
<213> Oryza sativa

<400> 715
000

<210> 716
<211> 0
<212> DNA
<213> Zea mays

<400> 716
000

<210> 717
<211> 0
<212> DNA
<213> Zea mays

<400> 717
000

<210> 718
<211> 0
<212> DNA
<213> Zea mays

<400> 718
000

<210> 719
<211> 0
<212> DNA
<213> Glycine max

<400> 719
000

<210> 720
<211> 0
<212> DNA
<213> Glycine max

<400> 720
000

<210> 721
<211> 0
<212> DNA
<213> Glycine max

<400> 721
000

<210> 722
<211> 0
<212> DNA
<213> Glycine max

<400> 722
000

<210> 723
<211> 0
<212> DNA
<213> Glycine max

<400> 723
000

<210> 724
<211> 0
<212> DNA
<213> Glycine max

<400> 724
000

<210> 725
<211> 0

<212> DNA
<213> Glycine max

<400> 725
000

<210> 726
<211> 0
<212> DNA
<213> Glycine max

<400> 726
000

<210> 727
<211> 0
<212> DNA
<213> Oryza sativa

<400> 727
000

<210> 728
<211> 0
<212> DNA
<213> Oryza sativa

<400> 728
000

<210> 729
<211> 0
<212> PRT
<213> Oryza sativa

<400> 729
000

<210> 730
<211> 0
<212> PRT
<213> Oryza sativa

<400> 730
000

<210> 731
<211> 0
<212> PRT
<213> Oryza sativa

<400> 731
000

<210> 732
<211> 0
<212> DNA
<213> Zea mays

<400> 732
000

<210> 733
<211> 0
<212> DNA
<213> Zea mays

<400> 733
000

<210> 734
<211> 0
<212> DNA
<213> Zea mays

<400> 734
000

<210> 735
<211> 0
<212> DNA
<213> Zea mays

<400> 735
000

<210> 736
<211> 0
<212> DNA
<213> Zea mays

<400> 736
000

<210> 737
<211> 0
<212> DNA
<213> Zea mays

<400> 737
000

<210> 738
<211> 0
<212> DNA
<213> Zea mays

<400> 738
000

<210> 739
<211> 0
<212> DNA
<213> Zea mays

<400> 739
000

<210> 740
<211> 0
<212> DNA
<213> Zea mays

<400> 740
000

<210> 741
<211> 0
<212> DNA
<213> Glycine max

<400> 741
000

<210> 742
<211> 0
<212> DNA
<213> Oryza sativa

<400> 742
000

<210> 743
<211> 0
<212> PRT
<213> Oryza sativa

<400> 743
000

<210> 744
<211> 0
<212> DNA
<213> Oryza sativa

<400> 744
000

<210> 745
<211> 0
<212> DNA
<213> Zea mays

<400> 745
000

<210> 746
<211> 0
<212> DNA
<213> Zea mays

<400> 746
000

<210> 747
<211> 0

<212> DNA
<213> Glycine max

<400> 747
000

<210> 748
<211> 0
<212> DNA
<213> Glycine max

<400> 748
000

<210> 749
<211> 0
<212> DNA
<213> Glycine max

<400> 749
000

<210> 750
<211> 0
<212> DNA
<213> Glycine max

<400> 750
000

<210> 751
<211> 0
<212> DNA
<213> Glycine max

<400> 751
000

<210> 752
<211> 0
<212> DNA
<213> Glycine max

<400> 752
000

<210> 753
<211> 0
<212> DNA
<213> Glycine max

<400> 753
000

<210> 754
<211> 0
<212> DNA
<213> Glycine max

<400> 754
000

<210> 755
<211> 0
<212> DNA
<213> Glycine max

<400> 755
000

<210> 756
<211> 0
<212> DNA
<213> Glycine max

<400> 756
000

<210> 757
<211> 0
<212> DNA
<213> Glycine max

<400> 757
000

<210> 758
<211> 0
<212> DNA
<213> Oryza sativa

<400> 758
000

<210> 759
<211> 0
<212> DNA
<213> Oryza sativa

<400> 759
000

<210> 760
<211> 0
<212> PRT
<213> Oryza sativa

<400> 760
000

<210> 761
<211> 0
<212> PRT
<213> Oryza sativa

<400> 761
000

<210> 762
<211> 0
<212> PRT
<213> Oryza sativa

<400> 762
000

<210> 763
<211> 0
<212> PRT
<213> Oryza sativa

<400> 763
000

<210> 764
<211> 0
<212> DNA
<213> Zea mays

<400> 764
000

<210> 765
<211> 0
<212> DNA
<213> Zea mays

<400> 765
000

<210> 766
<211> 0
<212> PRT
<213> Oryza sativa

<400> 766
000

<210> 767
<211> 0
<212> PRT
<213> Oryza sativa

<400> 767
000

<210> 768
<211> 0
<212> DNA
<213> Glycine max

<400> 768
000

<210> 769
<211> 0

<212> DNA
<213> Glycine max

<400> 769
000

<210> 770
<211> 0
<212> DNA
<213> Glycine max

<400> 770
000

<210> 771
<211> 0
<212> DNA
<213> Glycine max

<400> 771
000

<210> 772
<211> 0
<212> DNA
<213> Glycine max

<400> 772
000

<210> 773
<211> 0
<212> DNA
<213> Glycine max

<400> 773
000

<210> 774
<211> 0
<212> DNA
<213> Glycine max

<400> 774
000

<210> 775
<211> 0
<212> DNA
<213> Glycine max

<400> 775
000

<210> 776
<211> 0
<212> DNA
<213> Glycine max

<400> 776
000

<210> 777
<211> 0
<212> DNA
<213> Glycine max

<400> 777
000

<210> 778
<211> 0
<212> DNA
<213> Oryza sativa

<400> 778
000

<210> 779
<211> 0
<212> DNA
<213> Oryza sativa

<400> 779
000

<210> 780
<211> 0
<212> DNA
<213> Oryza sativa

<400> 780
000

<210> 781
<211> 0
<212> DNA
<213> Oryza sativa

<400> 781
000

<210> 782
<211> 0
<212> PRT
<213> Oryza sativa

<400> 782
000

<210> 783
<211> 0
<212> PRT
<213> Oryza sativa

<400> 783
000

<210> 784
<211> 0
<212> PRT
<213> Oryza sativa

<400> 784
000

<210> 785
<211> 0
<212> PRT
<213> Oryza sativa

<400> 785
000

<210> 786
<211> 0
<212> DNA
<213> Oryza sativa

<400> 786
000

<210> 787
<211> 0
<212> DNA
<213> Oryza sativa

<400> 787
000

<210> 788
<211> 0
<212> DNA
<213> Zea mays

<400> 788
000

<210> 789
<211> 0
<212> DNA
<213> Zea mays

<400> 789
000

<210> 790
<211> 0
<212> DNA
<213> Zea mays

<400> 790
000

<210> 791
<211> 0

<212> DNA
<213> Zea mays

<400> 791
000

<210> 792
<211> 0
<212> DNA
<213> Zea mays

<400> 792
000

<210> 793
<211> 0
<212> DNA
<213> Zea mays

<400> 793
000

<210> 794
<211> 0
<212> DNA
<213> Zea mays

<400> 794
000

<210> 795
<211> 0
<212> DNA
<213> Zea mays

<400> 795
000

<210> 796
<211> 0
<212> DNA
<213> Zea mays

<400> 796
000

<210> 797
<211> 0
<212> DNA
<213> Zea mays

<400> 797
000

<210> 798
<211> 0
<212> DNA
<213> Glycine max

<400> 798
000

<210> 799
<211> 0
<212> DNA
<213> Glycine max

<400> 799
000

<210> 800
<211> 0
<212> DNA
<213> Glycine max

<400> 800
000

<210> 801
<211> 0
<212> DNA
<213> Glycine max

<400> 801
000

<210> 802
<211> 0
<212> DNA
<213> Glycine max

<400> 802
000

<210> 803
<211> 0
<212> DNA
<213> Glycine max

<400> 803
000

<210> 804
<211> 0
<212> DNA
<213> Glycine max

<400> 804
000

<210> 805
<211> 0
<212> DNA
<213> Glycine max

<400> 805
000

<210> 806
<211> 0
<212> DNA
<213> Glycine max

<400> 806
000

<210> 807
<211> 0
<212> PRT
<213> Oryza sativa

<400> 807
000

<210> 808
<211> 0
<212> PRT
<213> Oryza sativa

<400> 808
000

<210> 809
<211> 0
<212> PRT
<213> Oryza sativa

<400> 809
000

<210> 810
<211> 0
<212> PRT
<213> Oryza sativa

<400> 810
000

<210> 811
<211> 0
<212> PRT
<213> Oryza sativa

<400> 811
000

<210> 812
<211> 0
<212> DNA
<213> Oryza sativa

<400> 812
000

<210> 813
<211> 0

<212> DNA
<213> Zea mays

<400> 813
000

<210> 814
<211> 0
<212> DNA
<213> Zea mays

<400> 814
000

<210> 815
<211> 0
<212> DNA
<213> Zea mays

<400> 815
000

<210> 816
<211> 0
<212> DNA
<213> Zea mays

<400> 816
000

<210> 817
<211> 0
<212> DNA
<213> Zea mays

<400> 817
000

<210> 818
<211> 0
<212> DNA
<213> Zea mays

<400> 818
000

<210> 819
<211> 0
<212> DNA
<213> Zea mays

<400> 819
000

<210> 820
<211> 0
<212> DNA
<213> Zea mays

<400> 820
000

<210> 821
<211> 0
<212> DNA
<213> Zea mays

<400> 821
000

<210> 822
<211> 0
<212> DNA
<213> Glycine max

<400> 822
000

<210> 823
<211> 0
<212> DNA
<213> Glycine max

<400> 823
000

<210> 824
<211> 0
<212> DNA
<213> Oryza sativa

<400> 824
000

<210> 825
<211> 0
<212> DNA
<213> Glycine max

<400> 825
000

<210> 826
<211> 0
<212> DNA
<213> Glycine max

<400> 826
000

<210> 827
<211> 0
<212> DNA
<213> Glycine max

<400> 827
000

<210> 828
<211> 0
<212> DNA
<213> Glycine max

<400> 828
000

<210> 829
<211> 0
<212> DNA
<213> Oryza sativa

<400> 829
000

<210> 830
<211> 0
<212> PRT
<213> Oryza sativa

<400> 830
000

<210> 831
<211> 0
<212> PRT
<213> Oryza sativa

<400> 831
000

<210> 832
<211> 0
<212> PRT
<213> Oryza sativa

<400> 832
000

<210> 833
<211> 0
<212> PRT
<213> Oryza sativa

<400> 833
000

<210> 834
<211> 0
<212> DNA
<213> Zea mays

<400> 834
000

<210> 835
<211> 0

<212> DNA
<213> Glycine max

<400> 835
000

<210> 836
<211> 0
<212> DNA
<213> Glycine max

<400> 836
000

<210> 837
<211> 0
<212> DNA
<213> Glycine max

<400> 837
000

<210> 838
<211> 0
<212> DNA
<213> Glycine max

<400> 838
000

<210> 839
<211> 0
<212> DNA
<213> Glycine max

<400> 839
000

<210> 840
<211> 0
<212> DNA
<213> Glycine max

<400> 840
000

<210> 841
<211> 0
<212> DNA
<213> Glycine max

<400> 841
000

<210> 842
<211> 0
<212> DNA
<213> Glycine max

<400> 842
000

<210> 843
<211> 0
<212> DNA
<213> Oryza sativa

<400> 843
000

<210> 844
<211> 0
<212> DNA
<213> Oryza sativa

<400> 844
000

<210> 845
<211> 0
<212> PRT
<213> Oryza sativa

<400> 845
000

<210> 846
<211> 0
<212> PRT
<213> Oryza sativa

<400> 846
000

<210> 847
<211> 0
<212> PRT
<213> Oryza sativa

<400> 847
000

<210> 848
<211> 0
<212> PRT
<213> Oryza sativa

<400> 848
000

<210> 849
<211> 0
<212> PRT
<213> Oryza sativa

<400> 849
000

<210> 850
<211> 0
<212> PRT
<213> Oryza sativa

<400> 850
000

<210> 851
<211> 0
<212> PRT
<213> Oryza sativa

<400> 851
000

<210> 852
<211> 0
<212> PRT
<213> Oryza sativa

<400> 852
000

<210> 853
<211> 0
<212> DNA
<213> Oryza sativa

<400> 853
000

<210> 854
<211> 0
<212> DNA
<213> Zea mays

<400> 854
000

<210> 855
<211> 0
<212> DNA
<213> Zea mays

<400> 855
000

<210> 856
<211> 0
<212> DNA
<213> Zea mays

<400> 856
000

<210> 857
<211> 0

<212> DNA
<213> Zea mays

<400> 857
000

<210> 858
<211> 0
<212> DNA
<213> Zea mays

<400> 858
000

<210> 859
<211> 0
<212> DNA
<213> Zea mays

<400> 859
000

<210> 860
<211> 0
<212> DNA
<213> Zea mays

<400> 860
000

<210> 861
<211> 0
<212> DNA
<213> Glycine max

<400> 861
000

<210> 862
<211> 0
<212> DNA
<213> Glycine max

<400> 862
000

<210> 863
<211> 0
<212> DNA
<213> Glycine max

<400> 863
000

<210> 864
<211> 0
<212> DNA
<213> Oryza sativa

<400> 864
000

<210> 865
<211> 0
<212> PRT
<213> Oryza sativa

<400> 865
000

<210> 866
<211> 0
<212> DNA
<213> Zea mays

<400> 866
000

<210> 867
<211> 0
<212> DNA
<213> Zea mays

<400> 867
000

<210> 868
<211> 0
<212> DNA
<213> Glycine max

<400> 868
000

<210> 869
<211> 0
<212> DNA
<213> Glycine max

<400> 869
000

<210> 870
<211> 0
<212> DNA
<213> Glycine max

<400> 870
000

<210> 871
<211> 0
<212> DNA
<213> Oryza sativa

<400> 871
000

<210> 872
<211> 0
<212> PRT
<213> Oryza sativa

<400> 872
000

<210> 873
<211> 0
<212> PRT
<213> Oryza sativa

<400> 873
000

<210> 874
<211> 0
<212> DNA
<213> Oryza sativa

<400> 874
000

<210> 875
<211> 0
<212> DNA
<213> Zea mays

<400> 875
000

<210> 876
<211> 0
<212> DNA
<213> Zea mays

<400> 876
000

<210> 877
<211> 0
<212> DNA
<213> Glycine max

<400> 877
000

<210> 878
<211> 0
<212> DNA
<213> Glycine max

<400> 878
000

<210> 879
<211> 0

<212> DNA
<213> Glycine max

<400> 879
000

<210> 880
<211> 0
<212> DNA
<213> Glycine max

<400> 880
000

<210> 881
<211> 0
<212> DNA
<213> Glycine max

<400> 881
000

<210> 882
<211> 0
<212> DNA
<213> Glycine max

<400> 882
000

<210> 883
<211> 0
<212> DNA
<213> Glycine max

<400> 883
000

<210> 884
<211> 0
<212> DNA
<213> Glycine max

<400> 884
000

<210> 885
<211> 0
<212> PRT
<213> Oryza sativa

<400> 885
000

<210> 886
<211> 0
<212> PRT
<213> Oryza sativa

<400> 886
000

<210> 887
<211> 0
<212> PRT
<213> Oryza sativa

<400> 887
000

<210> 888
<211> 0
<212> DNA
<213> Oryza sativa

<400> 888
000

<210> 889
<211> 0
<212> DNA
<213> Glycine max

<400> 889
000

<210> 890
<211> 0
<212> DNA
<213> Glycine max

<400> 890
000

<210> 891
<211> 0
<212> DNA
<213> Oryza sativa

<400> 891
000

<210> 892
<211> 0
<212> DNA
<213> Oryza sativa

<400> 892
000

<210> 893
<211> 0
<212> DNA
<213> Oryza sativa

<400> 893
000

<210> 894
<211> 0
<212> DNA
<213> Zea mays

<400> 894
000

<210> 895
<211> 0
<212> DNA
<213> Glycine max

<400> 895
000

<210> 896
<211> 0
<212> DNA
<213> Glycine max

<400> 896
000

<210> 897
<211> 0
<212> DNA
<213> Oryza sativa

<400> 897
000

<210> 898
<211> 0
<212> PRT
<213> Oryza sativa

<400> 898
000

<210> 899
<211> 0
<212> DNA
<213> Zea mays

<400> 899
000

<210> 900
<211> 0
<212> DNA
<213> Zea mays

<400> 900
000

<210> 901
<211> 0

<212> DNA
<213> Zea mays

<400> 901
000

<210> 902
<211> 0
<212> DNA
<213> Glycine max

<400> 902
000

<210> 903
<211> 0
<212> DNA
<213> Glycine max

<400> 903
000

<210> 904
<211> 0
<212> DNA
<213> Glycine max

<400> 904
000

<210> 905
<211> 0
<212> DNA
<213> Glycine max

<400> 905
000

<210> 906
<211> 0
<212> DNA
<213> Glycine max

<400> 906
000

<210> 907
<211> 0
<212> DNA
<213> Oryza sativa

<400> 907
000

<210> 908
<211> 0
<212> PRT
<213> Oryza sativa

<400> 908
000

<210> 909
<211> 0
<212> DNA
<213> Oryza sativa

<400> 909
000

<210> 910
<211> 0
<212> DNA
<213> Zea mays

<400> 910
000

<210> 911
<211> 0
<212> DNA
<213> Zea mays

<400> 911
000

<210> 912
<211> 0
<212> DNA
<213> Glycine max

<400> 912
000

<210> 913
<211> 0
<212> DNA
<213> Glycine max

<400> 913
000

<210> 914
<211> 0
<212> DNA
<213> Glycine max

<400> 914
000

<210> 915
<211> 0
<212> DNA
<213> Glycine max

<400> 915
000

<210> 916
<211> 0
<212> DNA
<213> Glycine max

<400> 916
000

<210> 917
<211> 0
<212> DNA
<213> Glycine max

<400> 917
000

<210> 918
<211> 0
<212> DNA
<213> Oryza sativa

<400> 918
000

<210> 919
<211> 0
<212> PRT
<213> Oryza sativa

<400> 919
000

<210> 920
<211> 0
<212> DNA
<213> Zea mays

<400> 920
000

<210> 921
<211> 0
<212> DNA
<213> Zea mays

<400> 921
000

<210> 922
<211> 0
<212> DNA
<213> Zea mays

<400> 922
000

<210> 923
<211> 0

<212> DNA
<213> Zea mays

<400> 923
000

<210> 924
<211> 0
<212> DNA
<213> Zea mays

<400> 924
000

<210> 925
<211> 0
<212> PRT
<213> Oryza sativa

<400> 925
000

<210> 926
<211> 0
<212> DNA
<213> Glycine max

<400> 926
000

<210> 927
<211> 0
<212> DNA
<213> Glycine max

<400> 927
000

<210> 928
<211> 0
<212> DNA
<213> Glycine max

<400> 928
000

<210> 929
<211> 0
<212> DNA

<213> Glycine max

<400> 929
000

<210> 930
<211> 0
<212> DNA

<213> Oryza sativa

<400> 930
000

<210> 931
<211> 0
<212> DNA
<213> Oryza sativa

<400> 931
000

<210> 932
<211> 0
<212> PRT
<213> Oryza sativa

<400> 932
000

<210> 933
<211> 0
<212> PRT
<213> Oryza sativa

<400> 933
000

<210> 934
<211> 0
<212> PRT
<213> Oryza sativa

<400> 934
000

<210> 935
<211> 0
<212> PRT
<213> Oryza sativa

<400> 935
000

<210> 936
<211> 0
<212> PRT
<213> Oryza sativa

<400> 936
000

<210> 937
<211> 0
<212> PRT
<213> Oryza sativa

<400> 937
000

<210> 938
<211> 0
<212> DNA
<213> Oryza sativa

<400> 938
000

<210> 939
<211> 0
<212> DNA
<213> Zea mays

<400> 939
000

<210> 940
<211> 0
<212> DNA
<213> Zea mays

<400> 940
000

<210> 941
<211> 0
<212> DNA
<213> Zea mays

<400> 941
000

<210> 942
<211> 0
<212> DNA
<213> Glycine max

<400> 942
000

<210> 943
<211> 0
<212> PRT
<213> Oryza sativa

<400> 943
000

<210> 944
<211> 0
<212> DNA
<213> Glycine max

<400> 944
000

<210> 945
<211> 0
<212> DNA
<213> Glycine max

<400> 945
000

<210> 946
<211> 0
<212> DNA
<213> Oryza sativa

<400> 946
000

<210> 947
<211> 0
<212> DNA
<213> Zea mays

<400> 947
000

<210> 948
<211> 0
<212> DNA
<213> Glycine max

<400> 948
000

<210> 949
<211> 0
<212> DNA
<213> Glycine max

<400> 949
000

<210> 950
<211> 0
<212> DNA
<213> Glycine max

<400> 950
000

<210> 951
<211> 0
<212> DNA
<213> Glycine max

<400> 951
000

<210> 952
<211> 0

<212> DNA
<213> Glycine max

<400> 952
000

<210> 953
<211> 0
<212> DNA
<213> Oryza sativa

<400> 953
000

<210> 954
<211> 0
<212> DNA
<213> Oryza sativa

<400> 954
000

<210> 955
<211> 0
<212> DNA
<213> Oryza sativa

<400> 955
000

<210> 956
<211> 0
<212> PRT
<213> Oryza sativa

<400> 956
000

<210> 957
<211> 0
<212> PRT
<213> Oryza sativa

<400> 957
000

<210> 958
<211> 0
<212> PRT
<213> Oryza sativa

<400> 958
000

<210> 959
<211> 0

<212> PRT

<213> Oryza sativa

<400> 959
000

<210> 960
<211> 0
<212> DNA
<213> Oryza sativa

<400> 960
000

<210> 961
<211> 0
<212> DNA
<213> Zea mays

<400> 961
000

<210> 962
<211> 0
<212> DNA
<213> Zea mays

<400> 962
000

<210> 963
<211> 0
<212> DNA
<213> Zea mays

<400> 963
000

<210> 964
<211> 0
<212> DNA
<213> Zea mays

<400> 964
000

<210> 965
<211> 0
<212> DNA
<213> Zea mays

<400> 965
000

<210> 966
<211> 0
<212> DNA
<213> Zea mays

<400> 966
000

<210> 967
<211> 0
<212> DNA
<213> Zea mays

<400> 967
000

<210> 968
<211> 0
<212> DNA
<213> Glycine max

<400> 968
000

<210> 969
<211> 0
<212> DNA
<213> Glycine max

<400> 969
000

<210> 970
<211> 0
<212> PRT
<213> Oryza sativa

<400> 970
000

<210> 971
<211> 0
<212> PRT
<213> Oryza sativa

<400> 971
000

<210> 972
<211> 0
<212> DNA
<213> Zea mays

<400> 972
000

<210> 973
<211> 0
<212> DNA
<213> Zea mays

<400> 973
000

<210> 974
<211> 0
<212> DNA
<213> Zea mays

<400> 974
000

<210> 975
<211> 0
<212> DNA
<213> Zea mays

<400> 975
000

<210> 976
<211> 0
<212> DNA
<213> Glycine max

<400> 976
000

<210> 977
<211> 0
<212> DNA
<213> Oryza sativa

<400> 977
000

<210> 978
<211> 0
<212> PRT
<213> Oryza sativa

<400> 978
000

<210> 979
<211> 0
<212> PRT
<213> Oryza sativa

<400> 979
000

<210> 980
<211> 0
<212> DNA
<213> Zea mays

<400> 980
000

<210> 981
<211> 0

<212> DNA
<213> Zea mays

<400> 981
000

<210> 982
<211> 0
<212> DNA
<213> Glycine max

<400> 982
000

<210> 983
<211> 0
<212> DNA
<213> Glycine max

<400> 983
000

<210> 984
<211> 0
<212> DNA
<213> Glycine max

<400> 984
000

<210> 985
<211> 0
<212> DNA
<213> Glycine max

<400> 985
000

<210> 986
<211> 0
<212> DNA
<213> Glycine max

<400> 986
000

<210> 987
<211> 0
<212> DNA
<213> Glycine max

<400> 987
000

<210> 988
<211> 0
<212> DNA
<213> Glycine max

<400> 988
000

<210> 989
<211> 0
<212> DNA
<213> Glycine max

<400> 989
000

<210> 990
<211> 0
<212> DNA
<213> Glycine max

<400> 990
000

<210> 991
<211> 0
<212> DNA
<213> Zea mays

<400> 991
000

<210> 992
<211> 0
<212> DNA
<213> Glycine max

<400> 992
000

<210> 993
<211> 0
<212> DNA
<213> Glycine max

<400> 993
000

<210> 994
<211> 0
<212> DNA
<213> Zea mays

<400> 994
000

<210> 995
<211> 0
<212> DNA
<213> Zea mays

<400> 995
000

<210> 996
<211> 0
<212> DNA
<213> Glycine max

<400> 996
000

<210> 997
<211> 0
<212> DNA
<213> Glycine max

<400> 997
000

<210> 998
<211> 0
<212> DNA
<213> Oryza sativa

<400> 998
000

<210> 999
<211> 0
<212> DNA
<213> Oryza sativa

<400> 999
000

<210> 1000
<211> 0
<212> DNA
<213> Oryza sativa

<400> 1000
000

<210> 1001
<211> 0
<212> PRT
<213> Oryza sativa

<400> 1001
000

<210> 1002
<211> 0
<212> PRT
<213> Oryza sativa

<400> 1002
000

<210> 1003
<211> 0

<212> PRT
<213> Oryza sativa

<400> 1003
000

<210> 1004
<211> 0
<212> PRT
<213> Oryza sativa

<400> 1004
000

<210> 1005
<211> 0
<212> DNA
<213> Zea mays

<400> 1005
000

<210> 1006
<211> 0
<212> DNA
<213> Zea mays

<400> 1006
000

<210> 1007
<211> 0
<212> DNA
<213> Zea mays

<400> 1007
000

<210> 1008
<211> 0
<212> DNA
<213> Zea mays

<400> 1008
000

<210> 1009
<211> 0
<212> DNA
<213> Glycine max

<400> 1009
000

<210> 1010
<211> 0
<212> PRT
<213> Oryza sativa

<400> 1010
000

<210> 1011
<211> 0
<212> PRT
<213> Oryza sativa

<400> 1011
000

<210> 1012
<211> 0
<212> PRT
<213> Oryza sativa

<400> 1012
000

<210> 1013
<211> 0
<212> PRT
<213> Oryza sativa

<400> 1013
000

<210> 1014
<211> 0
<212> DNA
<213> Glycine max

<400> 1014
000

<210> 1015
<211> 0
<212> DNA
<213> Glycine max

<400> 1015
000

<210> 1016
<211> 0
<212> DNA
<213> Glycine max

<400> 1016
000

<210> 1017
<211> 0
<212> DNA
<213> Glycine max

<400> 1017
000

<210> 1018
<211> 0
<212> DNA
<213> Glycine max

<400> 1018
000

<210> 1019
<211> 0
<212> DNA
<213> Oryza sativa

<400> 1019
000

<210> 1020
<211> 0
<212> DNA
<213> Oryza sativa

<400> 1020
000

<210> 1021
<211> 0
<212> DNA
<213> Oryza sativa

<400> 1021
000

<210> 1022
<211> 0
<212> DNA
<213> Oryza sativa

<400> 1022
000

<210> 1023
<211> 0
<212> PRT
<213> Oryza sativa

<400> 1023
000

<210> 1024
<211> 0
<212> PRT
<213> Oryza sativa

<400> 1024
000

<210> 1025
<211> 0

<212> DNA
<213> Zea mays

<400> 1025
000

<210> 1026
<211> 0
<212> DNA
<213> Zea mays

<400> 1026
000

<210> 1027
<211> 0
<212> DNA
<213> Zea mays

<400> 1027
000

<210> 1028
<211> 0
<212> DNA
<213> Zea mays

<400> 1028
000

<210> 1029
<211> 0
<212> DNA
<213> Zea mays

<400> 1029
000

<210> 1030
<211> 0
<212> DNA
<213> Zea mays

<400> 1030
000

<210> 1031
<211> 0
<212> DNA
<213> Oryza sativa

<400> 1031
000

<210> 1032
<211> 0
<212> PRT
<213> Oryza sativa

<400> 1032
000

<210> 1033
<211> 0
<212> DNA
<213> Glycine max

<400> 1033
000

<210> 1034
<211> 0
<212> DNA
<213> Glycine max

<400> 1034
000

<210> 1035
<211> 0
<212> DNA
<213> Zea mays

<400> 1035
000

<210> 1036
<211> 0
<212> DNA
<213> Glycine max

<400> 1036
000

<210> 1037
<211> 0
<212> DNA
<213> Glycine max

<400> 1037
000

<210> 1038
<211> 0
<212> DNA
<213> Oryza sativa

<400> 1038
000

<210> 1039
<211> 0
<212> DNA
<213> Oryza sativa

<400> 1039
000

<210> 1040
<211> 0
<212> PRT
<213> Oryza sativa

<400> 1040
000

<210> 1041
<211> 0
<212> PRT
<213> Oryza sativa

<400> 1041
000

<210> 1042
<211> 0
<212> PRT
<213> Oryza sativa

<400> 1042
000

<210> 1043
<211> 0
<212> DNA
<213> Zea mays

<400> 1043
000

<210> 1044
<211> 0
<212> DNA
<213> Zea mays

<400> 1044
000

<210> 1045
<211> 0
<212> DNA
<213> Zea mays

<400> 1045
000

<210> 1046
<211> 0
<212> DNA
<213> Zea mays

<400> 1046
000

<210> 1047
<211> 0

<212> DNA
<213> Zea mays

<400> 1047
000

<210> 1048
<211> 0
<212> DNA
<213> Zea mays

<400> 1048
000

<210> 1049
<211> 0
<212> DNA
<213> Glycine max

<400> 1049
000

<210> 1050
<211> 0
<212> PRT
<213> Oryza sativa

<400> 1050
000

<210> 1051
<211> 0
<212> DNA
<213> Glycine max

<400> 1051
000

<210> 1052
<211> 0
<212> DNA
<213> Glycine max

<400> 1052
000

<210> 1053
<211> 0
<212> DNA
<213> Glycine max

<400> 1053
000

<210> 1054
<211> 0
<212> PRT
<213> Oryza sativa

<400> 1054
000

<210> 1055
<211> 0
<212> DNA
<213> Zea mays

<400> 1055
000

<210> 1056
<211> 0
<212> DNA
<213> Glycine max

<400> 1056
000

<210> 1057
<211> 0
<212> DNA
<213> Glycine max

<400> 1057
000

<210> 1058
<211> 0
<212> DNA
<213> Glycine max

<400> 1058
000

<210> 1059
<211> 0
<212> DNA
<213> Glycine max

<400> 1059
000

<210> 1060
<211> 0
<212> DNA
<213> Glycine max

<400> 1060
000

<210> 1061
<211> 0
<212> DNA
<213> Glycine max

<400> 1061
000

<210> 1062
<211> 0
<212> DNA
<213> Oryza sativa

<400> 1062
000

<210> 1063
<211> 0
<212> DNA
<213> Oryza sativa

<400> 1063
000

<210> 1064
<211> 0
<212> PRT
<213> Oryza sativa

<400> 1064
000

<210> 1065
<211> 0
<212> PRT
<213> Oryza sativa

<400> 1065
000

<210> 1066
<211> 0
<212> DNA
<213> Zea mays

<400> 1066
000

<210> 1067
<211> 0
<212> DNA
<213> zea mays

<400> 1067
000

<210> 1068
<211> 0
<212> DNA
<213> Glycine max

<400> 1068
000

<210> 1069
<211> 0

<212> DNA
<213> Glycine max

<400> 1069
000

<210> 1070
<211> 0
<212> DNA
<213> Glycine max

<400> 1070
000

<210> 1071
<211> 0
<212> DNA
<213> Glycine max

<400> 1071
000

<210> 1072
<211> 0
<212> DNA
<213> Glycine max

<400> 1072
000

<210> 1073
<211> 0
<212> DNA
<213> Oryza sativa

<400> 1073
000

<210> 1074
<211> 0
<212> PRT
<213> Oryza sativa

<400> 1074
000

<210> 1075
<211> 0
<212> PRT
<213> Oryza sativa

<400> 1075
000

<210> 1076
<211> 0
<212> PRT
<213> Oryza sativa

<400> 1076
000

<210> 1077
<211> 0
<212> PRT
<213> Oryza sativa

<400> 1077
000

<210> 1078
<211> 0
<212> PRT
<213> Oryza sativa

<400> 1078
000

<210> 1079
<211> 0
<212> PRT
<213> Oryza sativa

<400> 1079
000

<210> 1080
<211> 0
<212> DNA
<213> Zea mays

<400> 1080
000

<210> 1081
<211> 0
<212> DNA
<213> Zea mays

<400> 1081
000

<210> 1082
<211> 0
<212> DNA
<213> Glycine max

<400> 1082
000

<210> 1083
<211> 0
<212> DNA
<213> Glycine max

<400> 1083
000

<210> 1084
<211> 0

<212> DNA
<213> Glycine max

<400> 1084
000

<210> 1085
<211> 0
<212> DNA
<213> Oryza sativa

<400> 1085
000

<210> 1086
<211> 0
<212> PRT
<213> Oryza sativa

<400> 1086
000

<210> 1087
<211> 0
<212> PRT
<213> Oryza sativa

<400> 1087
000

<210> 1088
<211> 0
<212> DNA
<213> Zea mays

<400> 1088
000

<210> 1089
<211> 0
<212> DNA
<213> Zea mays

<400> 1089
000

<210> 1090
<211> 0
<212> DNA
<213> Glycine max

<400> 1090
000

<210> 1091

<211> 0
<212> DNA
<213> Glycine max

<400> 1091
000

<210> 1092
<211> 0
<212> PRT
<213> Oryza sativa

<400> 1092
000

<210> 1093
<211> 0
<212> PRT
<213> Oryza sativa

<400> 1093
000

<210> 1094
<211> 0
<212> DNA
<213> Glycine max

<400> 1094
000

<210> 1095
<211> 0
<212> DNA
<213> Glycine max

<400> 1095
000

<210> 1096
<211> 0
<212> DNA
<213> Oryza sativa

<400> 1096
000

<210> 1097
<211> 0
<212> DNA
<213> Glycine max

<400> 1097
000

<210> 1098
<211> 1258
<212> DNA

<213> Glycine max

<400> 1098

```
tggcgtttgt ttagaaaagg ctgatccacg aaccaatgga atcacatcag ctgtgtcttc      60
aattcatatt gaataagctc atttccatca attaatagtt ccgtggaatg tggcttcact     120
agctattagt ttttgctttt tttcaatttc gggggaaaat gagtgtaaga ttgaaaataa     180
gctttaattg atatagtttt ttttattttt attttaaaaa aaaaaaaaaa aaactcgagt     240
tttttttttt tttttttta ctactaaaac cttgtctatt aagcacattt agaatgagtt     300
gcttggctgg ctagtttttc atttatattc gaatttgttg atccaattgc tacacattaa     360
tcaacaacca ttcgtagata caaaaaagaa aagaaaagca aaggaaacta aactcaaatt     420
tgaaatccac acatttggat tacacataca ttacaatgca ttggtatagc tatcatcagc     480
taggactcgc cccatccttc ctgcaattcg cggcggtgcg tgaagacacg tgcgaggttg     540
gcatgtgcgc ccagaatcag ctttgctggc actccagata ttgcctccaa cctcgccaga     600
ttctgacacg tggcgaggcc tctgtcccca caaaatctca gccccaacca aaacgacgtc     660
gcagcagcca ccctgaacgg aagcgacctc cactttccga ggcacacact cagagcgttc     720
gacgccacgc gatttccgtt caccccctaac cctaacccta tctctctgct ccatttctca     780
aacacctccg ccaccgatcc gccatttctc cgccgcttct tcgccgccgg agatgcgtcg     840
tcggtcacgg aggaactcga gaaactcctc ctcctcctcc tcctcttctt ctccgcctta     900
atctgattcg tagagcaaga ctcggaactg gagacgcagg tagaagagga agggagagaa     960
tcggagtcgg cggaaggaat ctccagggag caagaggtgc aggtggagga gaggtggcgg    1020
gatatagcac cggagatgtg aattgcggcg aaacggttgc atttggagca gaggaggcgg    1080
cggaggtggc gagctacgag gaagttggcg gcgtggacgg cggcgtcgca gtggaagcag    1140
agaaatgcgg aatcggaggg acaatagaga gaagctagtt gatgacaaag ctcgcaagtc    1200
ttgggcttca tcttaataag tctctgaggt agagagagtg gagtatgctg tgaccaaa      1258
```

<210> 1099
<211> 1044
<212> DNA
<213> Glycine max

<400> 1099

```
tttttattcg aatttgttga tccaattggt aaacacatta atgaacaagg attggtagaa      60
gcgaaaagca aaggaaacaa aagtaaaatt tgaaatccag gcatttggtt tggctaatga     120
gacgattaca cattggagct agctaggact cgccccatcc ttcctgcaat tcgcggcggt     180
gcgtgaagac acgtgccagg tcgccatgtg cggccagaat cagcttaact ggcactccgg     240
atattgcctc caacctcgcc agattctgac acgaggccag ccctctgtcc ccacaaaatc     300
tcagccccaa ccaaaacgac gtcgcagcag ccaccctgaa cggaagccac ctccactttc     360
ccaggcacac actcagagcg ttcgacgcca cgcgatttcc gtttacccct aaccctaacc     420
ctaaccctat ctctctgctc catttctcaa acacctcctc cgatcctcca ctcctctgcc     480
gcttcttcgc cgccggagat gcgtcgtcgg tcacggagga acccgaccaa ctcctcttct     540
tctccgcctt aatctgcttc gtggagcaag actcggaact ggagacgcag gtagaagagg     600
aagggagaga atcggagtag tcagcggaag gattctccgg ggagcaagag ctgcaggtgg     660
acgagaggtg gcgggatata gcgccggagg agatgtgaaa tccggcgaaa cggttgcatt     720
tggagcagag gaggcgacgg aggtgacgag ctacgagaaa gttggcggcg tgcacggcgg     780
cgtcgcagtc ggagcagaga aatgcggaat cggagggaca atagagagaa gcttgttgat     840
cacaaagctc gcaagtctta cccttcatct tcgcctgaac gttaatttca aaaattaaaa     900
aaagaaaatg attcttctga tgttttgact tgctaaaaat tgagggagta tgacaaaaac     960
tccaccctct tctgtctggg atcagaggta gagtgagtgc gttgtgttgt attgcgttat    1020
tcttcccttg acctcgagcc gaat                                           1044
```

<210> 1100
<211> 1096
<212> DNA
<213> Oryza sativa

<220>
<221> misc_feature
<222> (775)..(775)
<223> n is a, c, g, or t

<220>
<221> misc_feature
<222> (857)..(857)
<223> n is a, c, g, or t

<400> 1100

gcacgaggcc tcgtgccgaa ttcgggacgg cgccagcgtc tcgctcccaa gccagacctc 60
ccccctcgcc gtccgcgcgc gcgcccgcgg tttcccccgc tcgccgccgg tttcccccgc 120
tcgccgccgg tttccccgaa gcgcgccgcg cccgcgcctg cgcccgccgg tcgccatcgc 180
catctcgccc tcgcgcggag actggtgtcc ctgttttgct ctgtagtata aagccacgca 240
aacccccgcc aggtgttcga ccgagtgaca caagagtcca gcctcttgca acctgtaatg 300
gaggtcggca acggcaagtg cggcggtggt ggcgccgggt gcgagctgtg cgggggcgtg 360
gccgcggtgc actgcgccgc tgactccgcg tttctttgct tggtatgtga cgacaaggtg 420
cacggcgcca acttcctcgc gtccaggcac cgccgccgcc ggttgggggt tgaggtggtg 480
gatgaggagg atgacgcccg gtccacggcg tcgagctcgt gcgtgtcgac ggcggactcc 540
gcgtcgtcca cgccgccgcc ggccaccgca cgacgaatct tccggccgcc tgagatagaa 600
agtactaaaa atgcgaaact tgtgggcaat gatagtatgt atgcttcctc cctaattaat 660
taaattaatc tcaaattctt gatcaccatc aaggacccca aaatcttgtg gtttaggaag 720
gcctctcttg tggttaacat ccaatcacaa gtctaaatcc aatggatggg actcnaattt 780
ttctgtgtag tattagtgag acatgatgat agtacaattt gatttgttat taattggtta 840
ttaattaaag gtgattngga tcactgagac tttatgtggt caagaatgtc tccctgtatt 900
gtatgagtga ccactaccac tcgatatttt tttccttcca tcttggctga gtcctgtctt 960
gtgtttgttt attggtatct caatgtactg ggcttaccac ttgtatggac agtattgtta 1020
cactaacaca gtgtgtaccc cccagtcgtg ttagcttgaa tgggaagacc atgatcaaaa 1080
aaaaaaaaaa aaaaaa 1096

<210> 1101
<211> 1397
<212> DNA
<213> Zea mays

<400> 1101

cccacgcgtc cgcccacgcg tccggaccag cccacggtca gtagccatcg gccactacca 60
gcctcgggcg ctggctgtgg cctgtgggct gggcggggcc cctttaaatg ccggccatac 120
gcgcggccta aagcctccag agccacgctg cttcccggcc gaagcctccg tccgcgccca 180
cggtttccat tccatccccc aggcttgcgc cgactcgcca cttgcaccgc cggccggttt 240
cgccgaacgc ctcgcgccca cccgcgcgcg gagacctgca tataagccgg ccgcgtgtcg 300
tccggcgctc ccgggacagg cgatccgttc gtcatccgct gcaaaccacc aggcaccagc 360
tccgcgcgca gaggccggag gactacgacc gacgagggcg acgaacacca aggctccgaa 420
gtccgaggag gcaggaagcg ggagacgtcg ggaatgggcg ctgctcgtga ctccacggcg 480
gcgggccaga agcgcggcac cggcacgcgg tgcgagctct gcgggggcgc ggcggccgtg 540
cactgcgccg cggactcggc gttcctctgc ctgcgctgcg acgccaaggt gcacggcgcc 600
aacttcctgg cgtccaggca cgtgaggcgg cgcctggtgc cgcgccgggc cgccgacccc 660
gaggcgtcgt cggccgcgtc cagcggctcc tcctgcgtgt ccacggccga ctccgcggag 720
tcggccgcca cggcaccggc tccgtgccct tcgaggacgg cggggaggag ggctccggct 780
cgggcgcggc ggccgcgcgc ggaggcggtc ctggaggggt gggccaagcg gatggggttc 840
gcggcggggc cggcgcgccg gcgcgccgcg gcggcggccg ccgcgctccg ggcgctcggc 900
cggggcgtgg ccgctgcccg cgtgccgctc cgcgtcggga tggccggcgc gctctggtcg 960
gaggtccccg ccgggtgccg aggcaatgga ggggaggagg cctcgctgct ccagcggctg 1020
gaggcccccg cgcacgtgcc ggcgcggctg gtgctgaccc ccgcgtcgtg gatggcgcgc 1080
cggccggacg cccggcagga ggaccccgag gagggatggg ccgagtgctc ctgagttcct 1140
gatccagacg ggcccacgtc cgttgctgcc gtgccgccgc tgcacctgca ctgtgtaccg 1200
agccggacct ccgagataga ctcgggagaa aaatgtaaca acgtgtgggg ctgtgggcca 1260
cgtttgtttg tttgttcttt ttcttcttaa tcatataaat atatcgtagg cgtacgagcg 1320
agggtccaag ggattgatcg tgtggctggt tggctgcctc tcgtcaagcc gattgtggtt 1380
tgacttgttt ccccccc 1397

<210> 1102
<211> 856
<212> DNA
<213> Zea mays

<400> 1102

tccgcgcccc ggtttccttc cacccccagc cccagcttgc gccgactcgc cacttgtacc 60
gccggccggt ttcgccgaag gacgaaacgc ctcgcgcgcg gagacctgca tataagccgg 120
ccgcgtgtcg tccggcgctc ccggtacagg cgatccgttc atcatccgct gcaaaccacc 180
aggcaccagc tccgcgcgca gaggccggag gactacgacc gacgagggcg acgaacacca 240

aggctccgaa gtccgaggag gcaggaagcg ggaggacgtc gggaatgggc gctgctcgtg 300
actccgcggc ggcgggccag aagcacggca ccggcacgcg gtgcgagctc tgcgggggcg 360
cggcggccgt gcactgcgcc gcggactcgg cgttcctctg cctgcgctgc gacgccaagg 420
tgcacggcgc caacttcctg gcgtccaggc acgtgaggcg gcgcctggtg ccgcgccggg 480
ccgccgaccc cgaggcgtcg tcggccgcgt ccagcggctc ctcctgcgtg tccacggccg 540
actccgcgga gtcggccgcc acggcaccgg ctccgtgccc ttcgaggacg gcggggagga 600
gggctccggc tcgtgcgcgg cggccgcgcg cggaggcggt cctggagggg tgggccaagc 660
ggatggggtt cgcggcgggg ccggcgcgcc ggcgcgcacg tgccggcgcg gctggtgctg 720
accgccgcgt cgtggatggc gcgccggccg gacgcccggc aggaggacca ctaggaggga 780
tgggccgagt gctcctgagt tcctgatcca gacgggccca cgtccgttgc tgccgtgccg 840
ccgctgcacc tgcact 856

<210> 1103
<211> 698
<212> DNA
<213> Zea mays

<220>
<221> misc_feature
<222> (588)..(588)
<223> n is a, c, g, or t

<400> 1103

gccgggttcc gacgcttaat cgcttcgctt ccgccctcca tcgatcacct ccaaagatcg 60
agagcggctc gctcgtcaag cgctccatcg agccggcggt gcaggagcca aatcatatat 120
atataggcgg acaggaggaa gacccgccga tgggtcacca ccgcggccgc tgccgctgcg 180
agcgctgcgg cgcgcccgcc gccgtgcact gcgcggcgga cgcggccttc ctctgcgccg 240
cctgcgacgc caaggtgcac ggcgccaact tcctcgcgtc gcgccaccgc cgcacgcgcc 300
tcctcctcgc ggcgccggac gagtgcgggt acgagtccgg ggcgagctcc tgcgtgtcca 360
cggcggtcga ctcggcggcg cgggcgctcc gggcccacgc ccacggcctc gcctccgcgc 420
gcgtcgccat ggccgccgcg ctgtggcggg aggtggcggt gcgcggcggg accggcggcg 480
gcggccacgg tcacggcgag gcgctgcggc ggctggaggc gtgcgcgcac gtgccggcgg 540
ggctcgtggt ggcggtggcc aagtccatgg cgcgcgcgcg cgggcggngg gacgaggccg 600
acacggacgc cgcggcggag ggctgggacg agtgcgcctg ggccggtccc aagtccagcc 660
cgccacgtcc ttgatctttt ctcgtgctcg ctgatgaa 698

<210> 1104
<211> 552
<212> DNA
<213> Zea mays

<400> 1104

gccggccggg tcgggaacag cttccaccat cgaccatcca tcacctcacc tccaagctcc 60
aaggaatcta ccctcgttct tctcacaaag cgctgcagcg gacagcagcg cagcacgaga 120

gagccgagcg cgaggtgcag gaccgcaaaa cagaggcgaa gcgaaggacc taacctacgt 180
agcaacgctc cgctccgcgg cgatgggcca cgaggggtcg tgccgctgcg agctctgcgg 240
cgcgcccgcg gccgtgcact gcgcggcgga cgaggctttc ctctgcgccg cctgcgacgc 300
caaggtgcac ggcgccaact tcctcgcgtc gcggcaccgc cgcacgcgcc tccgcctcgc 360
ggccgccgcc cccccgccgc cgcacgacga ggccgggtac gggtccgccg cgagctcctg 420
cgtgtccacg gccgactccg cgccgcggcc gaggcgtggg gcccgcgcgc cggcccggag 480
cgtgcgcccc gagggcgtgc tcgagggctg gggccagcgg atggggctcg gggcgggggg 540
gcgcccgcgc gt 552

<210> 1105
<211> 0
<212> DNA
<213> Glycine max

<400> 1105
000

<210> 1106
<211> 0
<212> DNA
<213> Glycine max

<400> 1106
000

<210> 1107
<211> 0
<212> DNA
<213> Oryza sativa

<400> 1107
000

<210> 1108
<211> 0
<212> DNA
<213> Glycine max

<400> 1108
000

<210> 1109
<211> 0
<212> DNA
<213> Glycine max

<400> 1109
000

<210> 1110
<211> 0
<212> DNA
<213> Glycine max

<400> 1110
000

<210> 1111
<211> 0
<212> DNA
<213> Glycine max

<400> 1111
000

<210> 1112
<211> 0
<212> PRT
<213> Oryza sativa

<400> 1112
000

<210> 1113
<211> 0
<212> DNA
<213> Zea mays

<400> 1113
000

<210> 1114
<211> 0
<212> DNA
<213> Oryza sativa

<400> 1114
000

<210> 1115
<211> 0
<212> DNA
<213> Zea mays

<400> 1115
000

<210> 1116
<211> 0
<212> DNA
<213> Glycine max

<400> 1116
000

<210> 1117
<211> 0
<212> DNA
<213> Glycine max

<400> 1117
000

<210> 1118
<211> 0
<212> DNA
<213> Glycine max

<400> 1118
000

<210> 1119
<211> 0
<212> DNA
<213> Glycine max

<400> 1119
000

<210> 1120
<211> 0
<212> DNA
<213> Glycine max

<400> 1120
000

<210> 1121
<211> 0
<212> DNA
<213> Glycine max

<400> 1121
000

<210> 1122
<211> 0
<212> DNA
<213> Oryza sativa

<400> 1122
000

<210> 1123
<211> 0
<212> DNA
<213> Oryza sativa

<400> 1123
000

<210> 1124
<211> 0

<212> DNA
<213> Zea mays

<400> 1124
000

<210> 1125
<211> 0
<212> DNA
<213> Glycine max

<400> 1125
000

<210> 1126
<211> 0
<212> DNA
<213> Glycine max

<400> 1126
000

<210> 1127
<211> 0
<212> DNA
<213> Glycine max

<400> 1127
000

<210> 1128
<211> 0
<212> DNA
<213> Glycine max

<400> 1128
000

<210> 1129
<211> 0
<212> DNA
<213> Glycine max

<400> 1129
000

<210> 1130
<211> 0
<212> DNA
<213> Oryza sativa

<400> 1130
000

<210> 1131
<211> 0
<212> PRT
<213> Oryza sativa

<400> 1131
000

<210> 1132
<211> 0
<212> PRT
<213> Oryza sativa

<400> 1132
000

<210> 1133
<211> 0
<212> PRT
<213> Oryza sativa

<400> 1133
000

<210> 1134
<211> 0
<212> PRT
<213> Oryza sativa

<400> 1134
000

<210> 1135
<211> 0
<212> DNA
<213> Zea mays

<400> 1135
000

<210> 1136
<211> 0
<212> DNA
<213> Zea mays

<400> 1136
000

<210> 1137
<211> 0
<212> DNA
<213> Glycine max

<400> 1137
000

<210> 1138
<211> 0
<212> DNA
<213> Glycine max

<400> 1138
000

<210> 1139
<211> 0
<212> DNA
<213> Glycine max

<400> 1139
000

<210> 1140
<211> 0
<212> DNA
<213> Glycine max

<400> 1140
000

<210> 1141
<211> 0
<212> DNA
<213> Glycine max

<400> 1141
000

<210> 1142
<211> 0
<212> DNA
<213> Glycine max

<400> 1142
000

<210> 1143
<211> 0
<212> DNA
<213> Glycine max

<400> 1143
000

<210> 1144
<211> 0
<212> DNA
<213> Glycine max

<400> 1144
000

<210> 1145
<211> 0
<212> PRT
<213> Oryza sativa

<400> 1145
000

<210> 1146

<211> 0
<212> DNA
<213> Glycine max

<400> 1146
000

<210> 1147
<211> 0
<212> DNA
<213> Glycine max

<400> 1147
000

<210> 1148
<211> 0
<212> DNA
<213> Oryza sativa

<400> 1148
000

<210> 1149
<211> 0
<212> DNA
<213> Oryza sativa

<400> 1149
000

<210> 1150
<211> 0
<212> PRT
<213> Oryza sativa

<400> 1150
000

<210> 1151
<211> 0
<212> PRT
<213> Oryza sativa

<400> 1151
000

<210> 1152
<211> 0
<212> PRT
<213> Oryza sativa

<400> 1152
000

<210> 1153
<211> 0
<212> DNA

<213> Zea mays

<400> 1153
000

<210> 1154
<211> 0
<212> DNA
<213> Zea mays

<400> 1154
000

<210> 1155
<211> 0
<212> DNA
<213> Zea mays

<400> 1155
000

<210> 1156
<211> 0
<212> DNA
<213> Zea mays

<400> 1156
000

<210> 1157
<211> 0
<212> DNA
<213> Zea mays

<400> 1157
000

<210> 1158
<211> 0
<212> DNA
<213> Oryza sativa

<400> 1158
000

<210> 1159
<211> 0
<212> DNA
<213> Oryza sativa

<400> 1159
000

<210> 1160
<211> 0
<212> DNA
<213> Zea mays

<400> 1160
000

<210> 1161
<211> 0
<212> DNA
<213> Glycine max

<400> 1161
000

<210> 1162
<211> 0
<212> DNA
<213> Zea mays

<400> 1162
000

<210> 1163
<211> 0
<212> DNA
<213> Glycine max

<400> 1163
000

<210> 1164
<211> 0
<212> DNA
<213> Glycine max

<400> 1164
000

<210> 1165
<211> 0
<212> DNA
<213> Glycine max

<400> 1165
000

<210> 1166
<211> 0
<212> DNA
<213> Glycine max

<400> 1166
000

<210> 1167
<211> 0
<212> DNA
<213> Glycine max

<400> 1167
000

<210> 1168
<211> 0
<212> DNA
<213> Oryza sativa

<400> 1168
000

<210> 1169
<211> 0
<212> DNA
<213> Oryza sativa

<400> 1169
000

<210> 1170
<211> 0
<212> PRT
<213> Oryza sativa

<400> 1170
000

<210> 1171
<211> 0
<212> PRT
<213> Oryza sativa

<400> 1171
000

<210> 1172
<211> 0
<212> DNA
<213> Zea mays

<400> 1172
000

<210> 1173
<211> 0
<212> DNA
<213> Glycine max

<400> 1173
000

<210> 1174
<211> 0
<212> DNA
<213> Glycine max

<400> 1174
000

<210> 1175
<211> 0

<212> DNA
<213> Oryza sativa

<400> 1175
000

<210> 1176
<211> 0
<212> DNA
<213> Glycine max

<400> 1176
000

<210> 1177
<211> 0
<212> DNA
<213> Glycine max

<400> 1177
000

<210> 1178
<211> 0
<212> DNA
<213> Glycine max

<400> 1178
000

<210> 1179
<211> 0
<212> DNA
<213> Glycine max

<400> 1179
000

<210> 1180
<211> 0
<212> DNA
<213> Glycine max

<400> 1180
000

<210> 1181
<211> 0
<212> DNA
<213> Glycine max

<400> 1181
000

<210> 1182
<211> 0
<212> DNA
<213> Oryza sativa

<400> 1182
000

<210> 1183
<211> 0
<212> DNA
<213> Oryza sativa

<400> 1183
000

<210> 1184
<211> 0
<212> PRT
<213> Oryza sativa

<400> 1184
000

<210> 1185
<211> 0
<212> PRT
<213> Oryza sativa

<400> 1185
000

<210> 1186
<211> 0
<212> PRT
<213> Oryza sativa

<400> 1186
000

<210> 1187
<211> 0
<212> DNA
<213> Zea mays

<400> 1187
000

<210> 1188
<211> 0
<212> DNA
<213> Zea mays

<400> 1188
000

<210> 1189
<211> 0
<212> DNA
<213> Zea mays

<400> 1189
000

<210> 1190
<211> 0
<212> DNA
<213> Zea mays

<400> 1190
000

<210> 1191
<211> 0
<212> DNA
<213> Zea mays

<400> 1191
000

<210> 1192
<211> 0
<212> DNA
<213> Glycine max

<400> 1192
000

<210> 1193
<211> 0
<212> DNA
<213> Glycine max

<400> 1193
000

<210> 1194
<211> 0
<212> PRT
<213> Oryza sativa

<400> 1194
000

<210> 1195
<211> 0
<212> PRT
<213> Oryza sativa

<400> 1195
000

<210> 1196
<211> 0
<212> DNA
<213> Zea mays

<400> 1196
000

<210> 1197
<211> 0

<212> PRT
<213> Oryza sativa

<400> 1197
000

<210> 1198
<211> 0
<212> PRT
<213> Oryza sativa

<400> 1198
000

<210> 1199
<211> 0
<212> DNA
<213> Zea mays

<400> 1199
000

<210> 1200
<211> 0
<212> DNA
<213> Oryza sativa

<400> 1200
000

<210> 1201
<211> 0
<212> DNA
<213> Glycine max

<400> 1201
000

<210> 1202
<211> 0
<212> PRT
<213> Oryza sativa

<400> 1202
000

<210> 1203
<211> 0
<212> DNA
<213> Oryza sativa

<400> 1203
000

<210> 1204
<211> 0
<212> DNA
<213> Zea mays

<400> 1204
000

<210> 1205
<211> 0
<212> DNA
<213> Zea mays

<400> 1205
000

<210> 1206
<211> 0
<212> DNA
<213> Zea mays

<400> 1206
000

<210> 1207
<211> 0
<212> DNA
<213> Zea mays

<400> 1207
000

<210> 1208
<211> 0
<212> PRT
<213> Oryza sativa

<400> 1208
000

<210> 1209
<211> 0
<212> DNA
<213> Glycine max

<400> 1209
000

<210> 1210
<211> 0
<212> DNA
<213> Glycine max

<400> 1210
000

<210> 1211
<211> 0
<212> DNA
<213> Glycine max

<400> 1211
000

<210> 1212
<211> 0
<212> DNA
<213> Glycine max

<400> 1212
000

<210> 1213
<211> 0
<212> DNA
<213> Glycine max

<400> 1213
000

<210> 1214
<211> 0
<212> DNA
<213> Glycine max

<400> 1214
000

<210> 1215
<211> 0
<212> DNA
<213> Glycine max

<400> 1215
000

<210> 1216
<211> 0
<212> DNA
<213> Oryza sativa

<400> 1216
000

<210> 1217
<211> 0
<212> DNA
<213> Oryza sativa

<400> 1217
000

<210> 1218
<211> 0
<212> DNA
<213> Oryza sativa

<400> 1218
000

<210> 1219
<211> 0

<212> PRT
<213> Oryza sativa

<400> 1219
000

<210> 1220
<211> 0
<212> PRT
<213> Oryza sativa

<400> 1220
000

<210> 1221
<211> 0
<212> PRT
<213> Oryza sativa

<400> 1221
000

<210> 1222
<211> 0
<212> DNA
<213> Zea mays

<400> 1222
000

<210> 1223
<211> 0
<212> DNA
<213> Zea mays

<400> 1223
000

<210> 1224
<211> 0
<212> DNA
<213> Zea mays

<400> 1224
000

<210> 1225
<211> 0
<212> DNA
<213> Zea mays

<400> 1225
000

<210> 1226
<211> 0
<212> DNA
<213> Zea mays

<400> 1226
000

<210> 1227
<211> 0
<212> DNA
<213> Zea mays

<400> 1227
000

<210> 1228
<211> 0
<212> DNA
<213> Oryza sativa

<400> 1228
000

<210> 1229
<211> 0
<212> DNA
<213> Glycine max

<400> 1229
000

<210> 1230
<211> 0
<212> DNA
<213> Glycine max

<400> 1230
000

<210> 1231
<211> 0
<212> DNA
<213> Glycine max

<400> 1231
000

<210> 1232
<211> 0
<212> PRT
<213> Oryza sativa

<400> 1232
000

<210> 1233
<211> 0
<212> PRT
<213> Oryza sativa

<400> 1233
000

<210> 1234
<211> 0
<212> DNA
<213> Oryza sativa

<400> 1234
000

<210> 1235
<211> 0
<212> DNA
<213> Oryza sativa

<400> 1235
000

<210> 1236
<211> 0
<212> DNA
<213> Zea mays

<400> 1236
000

<210> 1237
<211> 0
<212> DNA
<213> Glycine max

<400> 1237
000

<210> 1238
<211> 0
<212> DNA
<213> Glycine max

<400> 1238
000

<210> 1239
<211> 0
<212> DNA
<213> Oryza sativa

<400> 1239
000

<210> 1240
<211> 0
<212> DNA
<213> Oryza sativa

<400> 1240
000

<210> 1241
<211> 0

<212> PRT
<213> Oryza sativa

<400> 1241
000

<210> 1242
<211> 0
<212> DNA
<213> Zea mays

<400> 1242
000

<210> 1243
<211> 0
<212> DNA
<213> Zea mays

<400> 1243
000

<210> 1244
<211> 0
<212> DNA
<213> Glycine max

<400> 1244
000

<210> 1245
<211> 0
<212> DNA
<213> Glycine max

<400> 1245
000

<210> 1246
<211> 0
<212> DNA
<213> Glycine max

<400> 1246
000

<210> 1247
<211> 0
<212> DNA
<213> Glycine max

<400> 1247
000

<210> 1248
<211> 0
<212> DNA
<213> Glycine max

<400> 1248
000

<210> 1249
<211> 0
<212> DNA
<213> Glycine max

<400> 1249
000

<210> 1250
<211> 0
<212> DNA
<213> Oryza sativa

<400> 1250
000

<210> 1251
<211> 0
<212> DNA
<213> Oryza sativa

<400> 1251
000

<210> 1252
<211> 0
<212> PRT
<213> Oryza sativa

<400> 1252
000

<210> 1253
<211> 0
<212> PRT
<213> Oryza sativa

<400> 1253
000

<210> 1254
<211> 0
<212> PRT
<213> Oryza sativa

<400> 1254
000

<210> 1255
<211> 0
<212> DNA
<213> Zea mays

<400> 1255
000

<210> 1256
<211> 0
<212> DNA
<213> Zea mays

<400> 1256
000

<210> 1257
<211> 0
<212> DNA
<213> Zea mays

<400> 1257
000

<210> 1258
<211> 0
<212> DNA
<213> Zea mays

<400> 1258
000

<210> 1259
<211> 0
<212> DNA
<213> Zea mays

<400> 1259
000

<210> 1260
<211> 0
<212> DNA
<213> Zea mays

<400> 1260
000

<210> 1261
<211> 0
<212> DNA
<213> Zea mays

<400> 1261
000

<210> 1262
<211> 0
<212> DNA
<213> Glycine max

<400> 1262
000

<210> 1263
<211> 0

<212> DNA
<213> Glycine max

<400> 1263
000

<210> 1264
<211> 0
<212> DNA
<213> Glycine max

<400> 1264
000

<210> 1265
<211> 0
<212> DNA
<213> Glycine max

<400> 1265
000

<210> 1266
<211> 0
<212> DNA
<213> Glycine max

<400> 1266
000

<210> 1267
<211> 0
<212> DNA
<213> Glycine max

<400> 1267
000

<210> 1268
<211> 0
<212> DNA
<213> Glycine max

<400> 1268
000

<210> 1269
<211> 0
<212> DNA
<213> Glycine max

<400> 1269
000

<210> 1270
<211> 0
<212> DNA
<213> Glycine max

<400> 1270
000

<210> 1271
<211> 0
<212> DNA
<213> Oryza sativa

<400> 1271
000

<210> 1272
<211> 0
<212> PRT
<213> Oryza sativa

<400> 1272
000

<210> 1273
<211> 0
<212> PRT
<213> Oryza sativa

<400> 1273
000

<210> 1274
<211> 0
<212> PRT
<213> Oryza sativa

<400> 1274
000

<210> 1275
<211> 0
<212> PRT
<213> Oryza sativa

<400> 1275
000

<210> 1276
<211> 0
<212> PRT
<213> Oryza sativa

<400> 1276
000

<210> 1277
<211> 0
<212> DNA
<213> Zea mays

<400> 1277
000

<210> 1278
<211> 0
<212> DNA
<213> Zea mays

<400> 1278
000

<210> 1279
<211> 0
<212> PRT
<213> Oryza sativa

<400> 1279
000

<210> 1280
<211> 0
<212> DNA
<213> Glycine max

<400> 1280
000

<210> 1281
<211> 0
<212> DNA
<213> Glycine max

<400> 1281
000

<210> 1282
<211> 0
<212> DNA
<213> Glycine max

<400> 1282
000

<210> 1283
<211> 0
<212> DNA
<213> Glycine max

<400> 1283
000

<210> 1284
<211> 0
<212> DNA
<213> Oryza sativa

<400> 1284
000

<210> 1285
<211> 0

<212> DNA
<213> Oryza sativa

<400> 1285
000

<210> 1286
<211> 0
<212> DNA
<213> Oryza sativa

<400> 1286
000

<210> 1287
<211> 0
<212> PRT
<213> Oryza sativa

<400> 1287
000

<210> 1288
<211> 0
<212> PRT
<213> Oryza sativa

<400> 1288
000

<210> 1289
<211> 0
<212> PRT
<213> Oryza sativa

<400> 1289
000

<210> 1290
<211> 0
<212> PRT
<213> Oryza sativa

<400> 1290
000

<210> 1291
<211> 0
<212> PRT
<213> Oryza sativa

<400> 1291
000

<210> 1292
<211> 0
<212> PRT
<213> Oryza sativa

<400> 1292
000

<210> 1293
<211> 0
<212> DNA
<213> Zea mays

<400> 1293
000

<210> 1294
<211> 0
<212> DNA
<213> Zea mays

<400> 1294
000

<210> 1295
<211> 0
<212> DNA
<213> Zea mays

<400> 1295
000

<210> 1296
<211> 0
<212> DNA
<213> Zea mays

<400> 1296
000

<210> 1297
<211> 0
<212> DNA
<213> Zea mays

<400> 1297
000

<210> 1298
<211> 0
<212> DNA
<213> Zea mays

<400> 1298
000

<210> 1299
<211> 0
<212> DNA
<213> Zea mays

<400> 1299
000

<210> 1300
<211> 0
<212> DNA
<213> Oryza sativa

<400> 1300
000

<210> 1301
<211> 0
<212> PRT
<213> Oryza sativa

<400> 1301
000

<210> 1302
<211> 0
<212> DNA
<213> Zea mays

<400> 1302
000

<210> 1303
<211> 0
<212> DNA
<213> Glycine max

<400> 1303
000

<210> 1304
<211> 0
<212> DNA
<213> Glycine max

<400> 1304
000

<210> 1305
<211> 0
<212> DNA
<213> Glycine max

<400> 1305
000

<210> 1306
<211> 0
<212> DNA
<213> Oryza sativa

<400> 1306
000

<210> 1307
<211> 0

<212> DNA
<213> Glycine max

<400> 1307
000

<210> 1308
<211> 0
<212> PRT
<213> Oryza sativa

<400> 1308
000

<210> 1309
<211> 0
<212> PRT
<213> Oryza sativa

<400> 1309
000

<210> 1310
<211> 0
<212> DNA
<213> Zea mays

<400> 1310
000

<210> 1311
<211> 0
<212> DNA
<213> Zea mays

<400> 1311
000

<210> 1312
<211> 0
<212> DNA
<213> Glycine max

<400> 1312
000

<210> 1313
<211> 0
<212> DNA
<213> Zea mays

<400> 1313
000

<210> 1314
<211> 0
<212> DNA
<213> Glycine max

<400> 1314
000

<210> 1315
<211> 0
<212> PRT
<213> Oryza sativa

<400> 1315
000

<210> 1316
<211> 0
<212> DNA
<213> Zea mays

<400> 1316
000

<210> 1317
<211> 0
<212> PRT
<213> Oryza sativa

<400> 1317
000

<210> 1318
<211> 0
<212> DNA
<213> Glycine max

<400> 1318
000

<210> 1319
<211> 0
<212> DNA
<213> Glycine max

<400> 1319
000

<210> 1320
<211> 0
<212> DNA
<213> Glycine max

<400> 1320
000

<210> 1321
<211> 0
<212> DNA
<213> Glycine max

<400> 1321
000

<210> 1322
<211> 0
<212> DNA
<213> Glycine max

<400> 1322
000

<210> 1323
<211> 0
<212> DNA
<213> Glycine max

<400> 1323
000

<210> 1324
<211> 0
<212> DNA
<213> Glycine max

<400> 1324
000

<210> 1325
<211> 0
<212> DNA
<213> Glycine max

<400> 1325
000

<210> 1326
<211> 0
<212> DNA
<213> Glycine max

<400> 1326
000

<210> 1327
<211> 0
<212> DNA
<213> Oryza sativa

<400> 1327
000

<210> 1328
<211> 0
<212> DNA
<213> Glycine max

<400> 1328
000

<210> 1329
<211> 0

<212> DNA
<213> Glycine max

<400> 1329
000

<210> 1330
<211> 0
<212> DNA
<213> Glycine max

<400> 1330
000

<210> 1331
<211> 0
<212> PRT
<213> Oryza sativa

<400> 1331
000

<210> 1332
<211> 0
<212> PRT
<213> Oryza sativa

<400> 1332
000

<210> 1333
<211> 0
<212> PRT
<213> Oryza sativa

<400> 1333
000

<210> 1334
<211> 0
<212> PRT
<213> Oryza sativa

<400> 1334
000

<210> 1335
<211> 0
<212> DNA
<213> Zea mays

<400> 1335
000

<210> 1336
<211> 0
<212> DNA
<213> Zea mays

<400> 1336
000

<210> 1337
<211> 0
<212> DNA
<213> Glycine max

<400> 1337
000

<210> 1338
<211> 0
<212> DNA
<213> Glycine max

<400> 1338
000

<210> 1339
<211> 0
<212> DNA
<213> Oryza sativa

<400> 1339
000

<210> 1340
<211> 0
<212> DNA
<213> Oryza sativa

<400> 1340
000

<210> 1341
<211> 0
<212> DNA
<213> Zea mays

<400> 1341
000

<210> 1342
<211> 0
<212> DNA
<213> Zea mays

<400> 1342
000

<210> 1343
<211> 0
<212> DNA
<213> Zea mays

<400> 1343
000

<210> 1344
<211> 0
<212> DNA
<213> Zea mays

<400> 1344
000

<210> 1345
<211> 0
<212> DNA
<213> Zea mays

<400> 1345
000

<210> 1346
<211> 0
<212> DNA
<213> Oryza sativa

<400> 1346
000

<210> 1347
<211> 0
<212> DNA
<213> Glycine max

<400> 1347
000

<210> 1348
<211> 0
<212> DNA
<213> Glycine max

<400> 1348
000

<210> 1349
<211> 0
<212> DNA
<213> Arabidopsis thaliana

<400> 1349
000

<210> 1350
<211> 0
<212> PRT
<213> Arabidopsis thaliana

<400> 1350
000

<210> 1351
<211> 0

<212> DNA
<213> Arabidopsis thaliana

<400> 1351
000

<210> 1352
<211> 0
<212> PRT
<213> Arabidopsis thaliana

<400> 1352
000

<210> 1353
<211> 0
<212> DNA
<213> Arabidopsis thaliana

<400> 1353
000

<210> 1354
<211> 0
<212> PRT
<213> Arabidopsis thaliana

<400> 1354
000

<210> 1355
<211> 0
<212> DNA
<213> Arabidopsis thaliana

<400> 1355
000

<210> 1356
<211> 0
<212> PRT
<213> Arabidopsis thaliana

<400> 1356
000

<210> 1357
<211> 0
<212> DNA
<213> Arabidopsis thaliana

<400> 1357
000

<210> 1358
<211> 0
<212> PRT
<213> Arabidopsis thaliana

<400> 1358
000

<210> 1359
<211> 0
<212> DNA
<213> Arabidopsis thaliana

<400> 1359
000

<210> 1360
<211> 0
<212> PRT
<213> Arabidopsis thaliana

<400> 1360
000

<210> 1361
<211> 0
<212> DNA
<213> Arabidopsis thaliana

<400> 1361
000

<210> 1362
<211> 0
<212> PRT
<213> Arabidopsis thaliana

<400> 1362
000

<210> 1363
<211> 0
<212> DNA

<213> Arabidopsis thaliana

<400> 1363
000

<210> 1364
<211> 0
<212> PRT
<213> Arabidopsis thaliana

<400> 1364
000

<210> 1365
<211> 0
<212> DNA
<213> Arabidopsis thaliana

<400> 1365

000

<210> 1366
<211> 0
<212> PRT
<213> Arabidopsis thaliana

<400> 1366
000

<210> 1367
<211> 0
<212> DNA
<213> Arabidopsis thaliana

<400> 1367
000

<210> 1368
<211> 0
<212> PRT
<213> Arabidopsis thaliana

<400> 1368
000

<210> 1369
<211> 0
<212> DNA
<213> Arabidopsis thaliana

<400> 1369
000

<210> 1370
<211> 0
<212> PRT
<213> Arabidopsis thaliana

<400> 1370
000

<210> 1371
<211> 0
<212> DNA
<213> Arabidopsis thaliana

<400> 1371
000

<210> 1372
<211> 0
<212> PRT
<213> Arabidopsis thaliana

<400> 1372
000

<210> 1373
<211> 0
<212> DNA
<213> Arabidopsis thaliana

<400> 1373
000

<210> 1374
<211> 0
<212> PRT
<213> Arabidopsis thaliana

<400> 1374
000

<210> 1375
<211> 0
<212> DNA
<213> Arabidopsis thaliana

<400> 1375
000

<210> 1376
<211> 0
<212> PRT
<213> Arabidopsis thaliana

<400> 1376
000

<210> 1377
<211> 0
<212> DNA
<213> Arabidopsis thaliana

<400> 1377
000

<210> 1378
<211> 0
<212> PRT
<213> Arabidopsis thaliana

<400> 1378
000

<210> 1379
<211> 0
<212> DNA
<213> Arabidopsis thaliana

<400> 1379
000

<210> 1380
<211> 0

<212> PRT
<213> Arabidopsis thaliana

<400> 1380
000

<210> 1381
<211> 0
<212> DNA
<213> Arabidopsis thaliana

<400> 1381
000

<210> 1382
<211> 0
<212> PRT
<213> Arabidopsis thaliana

<400> 1382
000

<210> 1383
<211> 0
<212> DNA
<213> Arabidopsis thaliana

<400> 1383
000

<210> 1384
<211> 0
<212> PRT
<213> Arabidopsis thaliana

<400> 1384
000

<210> 1385
<211> 0
<212> DNA
<213> Arabidopsis thaliana

<400> 1385
000

<210> 1386
<211> 0
<212> PRT
<213> Arabidopsis thaliana

<400> 1386
000

<210> 1387
<211> 0
<212> DNA
<213> Arabidopsis thaliana

<400> 1387
000

<210> 1388
<211> 0
<212> PRT
<213> Arabidopsis thaliana

<400> 1388
000

<210> 1389
<211> 0
<212> DNA
<213> Arabidopsis thaliana

<400> 1389
000

<210> 1390
<211> 0
<212> PRT
<213> Arabidopsis thaliana

<400> 1390
000

<210> 1391
<211> 0
<212> DNA
<213> Arabidopsis thaliana

<400> 1391
000

<210> 1392
<211> 0
<212> PRT
<213> Arabidopsis thaliana

<400> 1392
000

<210> 1393
<211> 0
<212> DNA
<213> Arabidopsis thaliana

<400> 1393
000

<210> 1394
<211> 0
<212> PRT
<213> Arabidopsis thaliana

<400> 1394
000

<210> 1395
<211> 0
<212> DNA
<213> Arabidopsis thaliana

<400> 1395
000

<210> 1396
<211> 0
<212> PRT
<213> Arabidopsis thaliana

<400> 1396
000

<210> 1397
<211> 0
<212> DNA
<213> Arabidopsis thaliana

<400> 1397
000

<210> 1398
<211> 0
<212> PRT
<213> Arabidopsis thaliana

<400> 1398
000

<210> 1399
<211> 0
<212> DNA
<213> Arabidopsis thaliana

<400> 1399
000

<210> 1400
<211> 0
<212> PRT
<213> Arabidopsis thaliana

<400> 1400
000

<210> 1401
<211> 0
<212> DNA
<213> Arabidopsis thaliana

<400> 1401
000

<210> 1402
<211> 0

<212> PRT
<213> Arabidopsis thaliana

<400> 1402
000

<210> 1403
<211> 0
<212> DNA
<213> Arabidopsis thaliana

<400> 1403
000

<210> 1404
<211> 0
<212> PRT
<213> Arabidopsis thaliana

<400> 1404
000

<210> 1405
<211> 0
<212> DNA
<213> Arabidopsis thaliana

<400> 1405
000

<210> 1406
<211> 0
<212> PRT
<213> Arabidopsis thaliana

<400> 1406
000

<210> 1407
<211> 0
<212> DNA
<213> Arabidopsis thaliana

<400> 1407
000

<210> 1408
<211> 0
<212> PRT
<213> Arabidopsis thaliana

<400> 1408
000

<210> 1409
<211> 0
<212> DNA
<213> Arabidopsis thaliana

<400> 1409
000

<210> 1410
<211> 0
<212> PRT
<213> Arabidopsis thaliana

<400> 1410
000

<210> 1411
<211> 0
<212> DNA
<213> Arabidopsis thaliana

<400> 1411
000

<210> 1412
<211> 0
<212> PRT
<213> Arabidopsis thaliana

<400> 1412
000

<210> 1413
<211> 0
<212> DNA
<213> Arabidopsis thaliana

<400> 1413
000

<210> 1414
<211> 0
<212> PRT
<213> Arabidopsis thaliana

<400> 1414
000

<210> 1415
<211> 0
<212> DNA
<213> Arabidopsis thaliana

<400> 1415
000

<210> 1416
<211> 0
<212> PRT
<213> Arabidopsis thaliana

<400> 1416
000

<210> 1417
<211> 0
<212> DNA
<213> Arabidopsis thaliana

<400> 1417
000

<210> 1418
<211> 0
<212> PRT
<213> Arabidopsis thaliana

<400> 1418
000

<210> 1419
<211> 0
<212> DNA
<213> Arabidopsis thaliana

<400> 1419
000

<210> 1420
<211> 0
<212> PRT
<213> Arabidopsis thaliana

<400> 1420
000

<210> 1421
<211> 0
<212> DNA
<213> Arabidopsis thaliana

<400> 1421
000

<210> 1422
<211> 0
<212> PRT
<213> Arabidopsis thaliana

<400> 1422
000

<210> 1423
<211> 0
<212> DNA
<213> Arabidopsis thaliana

<400> 1423
000

<210> 1424
<211> 0

<212> PRT
<213> Arabidopsis thaliana

<400> 1424
000

<210> 1425
<211> 0
<212> DNA
<213> Arabidopsis thaliana

<400> 1425
000

<210> 1426
<211> 0
<212> PRT
<213> Arabidopsis thaliana

<400> 1426
000

<210> 1427
<211> 0
<212> DNA
<213> Arabidopsis thaliana

<400> 1427
000

<210> 1428
<211> 0
<212> PRT
<213> Arabidopsis thaliana

<400> 1428
000

<210> 1429
<211> 0
<212> DNA
<213> Arabidopsis thaliana

<400> 1429
000

<210> 1430
<211> 0
<212> PRT
<213> Arabidopsis thaliana

<400> 1430
000

<210> 1431
<211> 0
<212> DNA
<213> Arabidopsis thaliana

<400> 1431
000

<210> 1432
<211> 0
<212> PRT
<213> Arabidopsis thaliana

<400> 1432
000

<210> 1433
<211> 0
<212> DNA
<213> Arabidopsis thaliana

<400> 1433
000

<210> 1434
<211> 0
<212> PRT
<213> Arabidopsis thaliana

<400> 1434
000

<210> 1435
<211> 0
<212> DNA
<213> Arabidopsis thaliana

<400> 1435
000

<210> 1436
<211> 0
<212> PRT
<213> Arabidopsis thaliana

<400> 1436
000

<210> 1437
<211> 0
<212> DNA
<213> Arabidopsis thaliana

<400> 1437
000

<210> 1438
<211> 0
<212> PRT
<213> Arabidopsis thaliana

<400> 1438
000

<210> 1439
<211> 0
<212> DNA
<213> Arabidopsis thaliana

<400> 1439
000

<210> 1440
<211> 0
<212> PRT
<213> Arabidopsis thaliana

<400> 1440
000

<210> 1441
<211> 0
<212> DNA
<213> Arabidopsis thaliana

<400> 1441
000

<210> 1442
<211> 0
<212> PRT
<213> Arabidopsis thaliana

<400> 1442
000

<210> 1443
<211> 0
<212> DNA
<213> Arabidopsis thaliana

<400> 1443
000

<210> 1444
<211> 0
<212> PRT
<213> Arabidopsis thaliana

<400> 1444
000

<210> 1445
<211> 0
<212> DNA
<213> Arabidopsis thaliana

<400> 1445
000

<210> 1446
<211> 0

<212> PRT
<213> Arabidopsis thaliana

<400> 1446
000

<210> 1447
<211> 0
<212> DNA
<213> Arabidopsis thaliana

<400> 1447
000

<210> 1448
<211> 0
<212> PRT
<213> Arabidopsis thaliana

<400> 1448
000

<210> 1449
<211> 0
<212> DNA
<213> Arabidopsis thaliana

<400> 1449
000

<210> 1450
<211> 0
<212> PRT
<213> Arabidopsis thaliana

<400> 1450
000

<210> 1451
<211> 0
<212> DNA
<213> Arabidopsis thaliana

<400> 1451
000

<210> 1452
<211> 0
<212> PRT
<213> Arabidopsis thaliana

<400> 1452
000

<210> 1453
<211> 0
<212> DNA
<213> Arabidopsis thaliana

<400> 1453
000

<210> 1454
<211> 0
<212> PRT
<213> Arabidopsis thaliana

<400> 1454
000

<210> 1455
<211> 0
<212> DNA
<213> Arabidopsis thaliana

<400> 1455
000

<210> 1456
<211> 0
<212> PRT
<213> Arabidopsis thaliana

<400> 1456
000

<210> 1457
<211> 0
<212> DNA
<213> Arabidopsis thaliana

<400> 1457
000

<210> 1458
<211> 0
<212> PRT
<213> Arabidopsis thaliana

<400> 1458
000

<210> 1459
<211> 0
<212> DNA
<213> Arabidopsis thaliana

<400> 1459
000

<210> 1460
<211> 0
<212> PRT
<213> Arabidopsis thaliana

<400> 1460
000

<210> 1461
<211> 0
<212> DNA
<213> Arabidopsis thaliana

<400> 1461
000

<210> 1462
<211> 0
<212> PRT
<213> Arabidopsis thaliana

<400> 1462
000

<210> 1463
<211> 0
<212> DNA
<213> Arabidopsis thaliana

<400> 1463
000

<210> 1464
<211> 0
<212> PRT
<213> Arabidopsis thaliana

<400> 1464
000

<210> 1465
<211> 0
<212> DNA
<213> Arabidopsis thaliana

<400> 1465
000

<210> 1466
<211> 0
<212> PRT
<213> Arabidopsis thaliana

<400> 1466

000

<210> 1467
<211> 0
<212> DNA
<213> Arabidopsis thaliana

<400> 1467
000

<210> 1468

<211> 0
<212> PRT
<213> Arabidopsis thaliana

<400> 1468
000

<210> 1469
<211> 0
<212> DNA
<213> Arabidopsis thaliana

<400> 1469
000

<210> 1470
<211> 0
<212> PRT
<213> Arabidopsis thaliana

<400> 1470
000

<210> 1471
<211> 0
<212> DNA
<213> Arabidopsis thaliana

<400> 1471
000

<210> 1472
<211> 0
<212> PRT
<213> Arabidopsis thaliana

<400> 1472
000

<210> 1473

<211> 0
<212> DNA
<213> Arabidopsis thaliana

<400> 1473
000

<210> 1474
<211> 0
<212> PRT
<213> Arabidopsis thaliana

<400> 1474
000

<210> 1475
<211> 0

<212> DNA
<213> Arabidopsis thaliana

<400> 1475
000

<210> 1476
<211> 0
<212> PRT
<213> Arabidopsis thaliana

<400> 1476
000

<210> 1477
<211> 0
<212> DNA
<213> Arabidopsis thaliana

<400> 1477
000

<210> 1478
<211> 0
<212> PRT
<213> Arabidopsis thaliana

<400> 1478
000

<210> 1479
<211> 0
<212> DNA
<213> Arabidopsis thaliana

<400> 1479
000

<210> 1480
<211> 0
<212> PRT
<213> Arabidopsis thaliana

<400> 1480
000

<210> 1481
<211> 0
<212> DNA
<213> Arabidopsis thaliana

<400> 1481
000

<210> 1482
<211> 0
<212> PRT
<213> Arabidopsis thaliana

<400> 1482
000

<210> 1483
<211> 0
<212> DNA
<213> Arabidopsis thaliana

<400> 1483
000

<210> 1484
<211> 0
<212> PRT
<213> Arabidopsis thaliana

<400> 1484
000

<210> 1485
<211> 0
<212> DNA
<213> Arabidopsis thaliana

<400> 1485
000

<210> 1486
<211> 0
<212> PRT
<213> Arabidopsis thaliana

<400> 1486
000

<210> 1487
<211> 0
<212> DNA
<213> Arabidopsis thaliana

<400> 1487
000

<210> 1488
<211> 0
<212> PRT
<213> Arabidopsis thaliana

<400> 1488
000

<210> 1489
<211> 0
<212> DNA
<213> Arabidopsis thaliana

<400> 1489
000

<210> 1490
<211> 0
<212> PRT
<213> Arabidopsis thaliana

<400> 1490
000

<210> 1491
<211> 0
<212> DNA
<213> Arabidopsis thaliana

<400> 1491
000

<210> 1492
<211> 0
<212> PRT
<213> Arabidopsis thaliana

<400> 1492
000

<210> 1493
<211> 0
<212> DNA
<213> Arabidopsis thaliana

<400> 1493
000

<210> 1494
<211> 0
<212> PRT
<213> Arabidopsis thaliana

<400> 1494
000

<210> 1495
<211> 0
<212> DNA
<213> Arabidopsis thaliana

<400> 1495
000

<210> 1496
<211> 0
<212> PRT
<213> Arabidopsis thaliana

<400> 1496
000

<210> 1497
<211> 0

<212> DNA
<213> Arabidopsis thaliana

<400> 1497
000

<210> 1498
<211> 0
<212> PRT
<213> Arabidopsis thaliana

<400> 1498
000

<210> 1499
<211> 0
<212> DNA
<213> Arabidopsis thaliana

<400> 1499
000

<210> 1500
<211> 0
<212> PRT
<213> Arabidopsis thaliana

<400> 1500
000

<210> 1501
<211> 0
<212> DNA
<213> Arabidopsis thaliana

<400> 1501
000

<210> 1502
<211> 0
<212> PRT
<213> Arabidopsis thaliana

<400> 1502
000

<210> 1503
<211> 0
<212> DNA
<213> Arabidopsis thaliana

<400> 1503
000

<210> 1504
<211> 0
<212> PRT
<213> Arabidopsis thaliana

<400> 1504
000

<210> 1505
<211> 0
<212> DNA
<213> Arabidopsis thaliana

<400> 1505
000

<210> 1506
<211> 0
<212> PRT
<213> Arabidopsis thaliana

<400> 1506
000

<210> 1507
<211> 0
<212> DNA
<213> Arabidopsis thaliana

<400> 1507
000

<210> 1508
<211> 0
<212> PRT
<213> Arabidopsis thaliana

<400> 1508
000

<210> 1509
<211> 0
<212> DNA
<213> Arabidopsis thaliana

<400> 1509
000

<210> 1510
<211> 0
<212> PRT
<213> Arabidopsis thaliana

<400> 1510
000

<210> 1511
<211> 0
<212> DNA
<213> Arabidopsis thaliana

<400> 1511
000

<210> 1512
<211> 0
<212> PRT
<213> Arabidopsis thaliana

<400> 1512
000

<210> 1513
<211> 0
<212> DNA
<213> Arabidopsis thaliana

<400> 1513
000

<210> 1514
<211> 0
<212> PRT
<213> Arabidopsis thaliana

<400> 1514
000

<210> 1515
<211> 0
<212> DNA
<213> Arabidopsis thaliana

<400> 1515
000

<210> 1516
<211> 0
<212> PRT
<213> Arabidopsis thaliana

<400> 1516
000

<210> 1517
<211> 0
<212> DNA
<213> Arabidopsis thaliana

<400> 1517
000

<210> 1518
<211> 0
<212> PRT
<213> Arabidopsis thaliana

<400> 1518
000

<210> 1519
<211> 0

<212> DNA
<213> Arabidopsis thaliana

<400> 1519
000

<210> 1520
<211> 0
<212> PRT
<213> Arabidopsis thaliana

<400> 1520
000

<210> 1521
<211> 0
<212> DNA
<213> Arabidopsis thaliana

<400> 1521
000

<210> 1522
<211> 0
<212> PRT
<213> Arabidopsis thaliana

<400> 1522
000

<210> 1523
<211> 0
<212> DNA
<213> Arabidopsis thaliana

<400> 1523
000

<210> 1524
<211> 0
<212> PRT
<213> Arabidopsis thaliana

<400> 1524
000

<210> 1525
<211> 0
<212> DNA
<213> Arabidopsis thaliana

<400> 1525
000

<210> 1526
<211> 0
<212> PRT
<213> Arabidopsis thaliana

<400> 1526
000

<210> 1527
<211> 0
<212> DNA
<213> Arabidopsis thaliana

<400> 1527
000

<210> 1528
<211> 0
<212> PRT
<213> Arabidopsis thaliana

<400> 1528
000

<210> 1529
<211> 0
<212> DNA
<213> Arabidopsis thaliana

<400> 1529
000

<210> 1530
<211> 0
<212> PRT
<213> Arabidopsis thaliana

<400> 1530
000

<210> 1531
<211> 0
<212> DNA
<213> Arabidopsis thaliana

<400> 1531
000

<210> 1532
<211> 0
<212> PRT
<213> Arabidopsis thaliana

<400> 1532
000

<210> 1533
<211> 0
<212> DNA
<213> Arabidopsis thaliana

<400> 1533
000

<210> 1534
<211> 0
<212> PRT
<213> Arabidopsis thaliana

<400> 1534
000

<210> 1535
<211> 0
<212> DNA
<213> Arabidopsis thaliana

<400> 1535
000

<210> 1536
<211> 0
<212> PRT
<213> Arabidopsis thaliana

<400> 1536
000

<210> 1537
<211> 0
<212> DNA
<213> Arabidopsis thaliana

<400> 1537
000

<210> 1538
<211> 0
<212> PRT
<213> Arabidopsis thaliana

<400> 1538
000

<210> 1539
<211> 0
<212> DNA
<213> Arabidopsis thaliana

<400> 1539
000

<210> 1540
<211> 0
<212> PRT
<213> Arabidopsis thaliana

<400> 1540
000

<210> 1541
<211> 0

<212> DNA
<213> Arabidopsis thaliana

<400> 1541
000

<210> 1542
<211> 0
<212> PRT
<213> Arabidopsis thaliana

<400> 1542
000

<210> 1543
<211> 0
<212> DNA
<213> Arabidopsis thaliana

<400> 1543
000

<210> 1544
<211> 0
<212> PRT
<213> Arabidopsis thaliana

<400> 1544
000

<210> 1545
<211> 0
<212> DNA
<213> Arabidopsis thaliana

<400> 1545
000

<210> 1546
<211> 0
<212> PRT
<213> Arabidopsis thaliana

<400> 1546
000

<210> 1547
<211> 0
<212> DNA
<213> Arabidopsis thaliana

<400> 1547
000

<210> 1548
<211> 0
<212> PRT
<213> Arabidopsis thaliana

<400> 1548
000

<210> 1549
<211> 0
<212> DNA
<213> Arabidopsis thaliana

<400> 1549
000

<210> 1550
<211> 0
<212> PRT
<213> Arabidopsis thaliana

<400> 1550
000

<210> 1551
<211> 0
<212> DNA
<213> Arabidopsis thaliana

<400> 1551
000

<210> 1552
<211> 0
<212> PRT
<213> Arabidopsis thaliana

<400> 1552
000

<210> 1553
<211> 0
<212> DNA
<213> Arabidopsis thaliana

<400> 1553
000

<210> 1554
<211> 0
<212> PRT
<213> Arabidopsis thaliana

<400> 1554
000

<210> 1555
<211> 0
<212> DNA
<213> Arabidopsis thaliana

<400> 1555
000

<210> 1556
<211> 0
<212> PRT
<213> Arabidopsis thaliana

<400> 1556
000

<210> 1557
<211> 0
<212> DNA
<213> Oryza sativa

<400> 1557
000

<210> 1558
<211> 0
<212> DNA
<213> Oryza sativa

<400> 1558
000

<210> 1559
<211> 0
<212> DNA
<213> Oryza sativa

<400> 1559
000

<210> 1560
<211> 0
<212> DNA
<213> Oryza sativa

<400> 1560
000

<210> 1561
<211> 0
<212> DNA
<213> Oryza sativa

<400> 1561
000

<210> 1562
<211> 0
<212> DNA
<213> Oryza sativa

<400> 1562
000

<210> 1563
<211> 0

<212> DNA
<213> Oryza sativa

<400> 1563
000

<210> 1564
<211> 0
<212> DNA
<213> Oryza sativa

<400> 1564
000

<210> 1565
<211> 0
<212> DNA
<213> Oryza sativa

<400> 1565
000

<210> 1566
<211> 0
<212> DNA
<213> Oryza sativa
<400> 1566
000

<210> 1567
<211> 0
<212> DNA
<213> Oryza sativa

<400> 1567
000

<210> 1568
<211> 0
<212> DNA
<213> Oryza sativa

<400> 1568
000

<210> 1569
<211> 0
<212> DNA
<213> Oryza sativa

<400> 1569
000

<210> 1570
<211> 0
<212> DNA
<213> Oryza sativa

<400> 1570
000

<210> 1571
<211> 0
<212> DNA
<213> Oryza sativa

<400> 1571
000

<210> 1572
<211> 0
<212> DNA
<213> Oryza sativa

<400> 1572
000

<210> 1573
<211> 0
<212> DNA
<213> Oryza sativa

<400> 1573
000

<210> 1574
<211> 0
<212> DNA
<213> Oryza sativa

<400> 1574
000

<210> 1575
<211> 0
<212> DNA
<213> Oryza sativa

<400> 1575
000

<210> 1576
<211> 0
<212> DNA
<213> Oryza sativa

<400> 1576
000

<210> 1577
<211> 0
<212> DNA
<213> Oryza sativa

<400> 1577
000

<210> 1578
<211> 0
<212> DNA
<213> Oryza sativa

<400> 1578
000

<210> 1579
<211> 0
<212> DNA
<213> Oryza sativa

<400> 1579
000

<210> 1580
<211> 0
<212> DNA
<213> Oryza sativa

<400> 1580
000

<210> 1581
<211> 0
<212> DNA
<213> Oryza sativa

<400> 1581
000

<210> 1582
<211> 0
<212> DNA
<213> Oryza sativa

<400> 1582
000

<210> 1583
<211> 0
<212> DNA
<213> Oryza sativa

<400> 1583
000

<210> 1584
<211> 0
<212> DNA
<213> Oryza sativa

<400> 1584
000

<210> 1585
<211> 0

<212> DNA
<213> Oryza sativa

<400> 1585
000

<210> 1586
<211> 0
<212> DNA
<213> Oryza sativa

<400> 1586
000

<210> 1587
<211> 0
<212> DNA
<213> Oryza sativa

<400> 1587
000

<210> 1588
<211> 0
<212> DNA
<213> Oryza sativa

<400> 1588
000

<210> 1589
<211> 0
<212> DNA
<213> Oryza sativa

<400> 1589
000

<210> 1590
<211> 0
<212> DNA
<213> Oryza sativa

<400> 1590
000

<210> 1591
<211> 0
<212> DNA
<213> Oryza sativa

<400> 1591
000

<210> 1592
<211> 0
<212> DNA
<213> Oryza sativa

<400> 1592
000

<210> 1593
<211> 0
<212> DNA
<213> Oryza sativa

<400> 1593
000

<210> 1594
<211> 0
<212> DNA
<213> Oryza sativa

<400> 1594
000

<210> 1595
<211> 0
<212> DNA
<213> Oryza sativa

<400> 1595
000

<210> 1596
<211> 0
<212> DNA
<213> Oryza sativa

<400> 1596
000

<210> 1597
<211> 0
<212> DNA
<213> Oryza sativa

<400> 1597
000

<210> 1598
<211> 0
<212> DNA
<213> Oryza sativa

<400> 1598
000

<210> 1599
<211> 0
<212> DNA
<213> Oryza sativa

<400> 1599
000

<210> 1600
<211> 0
<212> DNA
<213> Oryza sativa

<400> 1600
000

<210> 1601
<211> 516
<212> DNA
<213> Oryza sativa

<220>

<221> misc_feature
<222> (337)..(337)
<223> n is a, c, g, or t

<220>
<221> misc_feature
<222> (449)..(449)
<223> n is a, c, g, or t

<220>
<221> misc_feature
<222> (495)..(495)
<223> n is a, c, g, or t

<400> 1601

```
ttaaaagcta ggctccaaaa tttctcaaat cgttattaca caagacaaaa taatacacca      60
acaattaatt acctccatat atatatataa aagaaaagaa aagaagaagc aacgaattgc     120
atatcgcaca ccctgcaaaa ccttgaattt taacttttgc atgctgtaat taagtagttc     180
tagaactctt aatcatctgg ccgtaccaaa taatgcgctt aaataaaata ccaaattgat     240
cacaacctta aacacacacc tgaaaatgga acaacaaacg aagttattag aatttttttt     300
aatttgttct aacgtaccag ccttattgga gtagggntta ttgtctcttg taattgatca     360
aatcatccct ggcttccaga ggtgagggaa gcttcttgct gagctcagtg tgagctgccc     420
ttgcgagtgg tcgaggctat ctacagggna ctgctcggct gcagcctctt tcagggggat     480
ccgcggcttc cgggngtgga gcggcctgtg gtgagc                               516
```

<210> 1602
<211> 0
<212> DNA
<213> Oryza sativa

<400> 1602
000

<210> 1603
<211> 0
<212> DNA

<213> Oryza sativa

<400> 1603
000

<210> 1604
<211> 0
<212> DNA
<213> Oryza sativa

<400> 1604
000

<210> 1605
<211> 0
<212> DNA
<213> Oryza sativa

<400> 1605
000

<210> 1606
<211> 0
<212> DNA
<213> Oryza sativa

<400> 1606
000

<210> 1607
<211> 0
<212> DNA
<213> Oryza sativa

<400> 1607
000

<210> 1608
<211> 0
<212> DNA
<213> Oryza sativa

<400> 1608
000

<210> 1609
<211> 0
<212> DNA
<213> Oryza sativa

<400> 1609
000

<210> 1610
<211> 0
<212> DNA
<213> Oryza sativa

<400> 1610
000

<210> 1611
<211> 0
<212> DNA
<213> Oryza sativa

<400> 1611
000

<210> 1612
<211> 0
<212> DNA
<213> Oryza sativa

<400> 1612
000

<210> 1613
<211> 0
<212> DNA
<213> Oryza sativa

<400> 1613
000

<210> 1614
<211> 0
<212> DNA
<213> Oryza sativa

<400> 1614
000

<210> 1615
<211> 0
<212> DNA
<213> Oryza sativa

<400> 1615
000

<210> 1616
<211> 0
<212> DNA
<213> Oryza sativa

<400> 1616
000

<210> 1617
<211> 0
<212> DNA
<213> Oryza sativa

<400> 1617
000

<210> 1618
<211> 0
<212> DNA
<213> Oryza sativa

<400> 1618
000

<210> 1619
<211> 0
<212> DNA
<213> Oryza sativa

<400> 1619
000

<210> 1620
<211> 0
<212> DNA
<213> Oryza sativa

<400> 1620
000

<210> 1621
<211> 0
<212> DNA
<213> Oryza sativa

<400> 1621
000

<210> 1622
<211> 0
<212> DNA
<213> Oryza sativa

<400> 1622
000

<210> 1623
<211> 0
<212> DNA
<213> Oryza sativa

<400> 1623
000

<210> 1624
<211> 0
<212> DNA
<213> Oryza sativa

<400> 1624
000

<210> 1625
<211> 0

<212> DNA
<213> Oryza sativa

<400> 1625
000

<210> 1626
<211> 0
<212> DNA
<213> Oryza sativa

<400> 1626
000

<210> 1627
<211> 0
<212> DNA
<213> Oryza sativa

<400> 1627
000

<210> 1628
<211> 0
<212> DNA
<213> Oryza sativa

<400> 1628
000

<210> 1629
<211> 0
<212> DNA
<213> Oryza sativa

<400> 1629
000

<210> 1630
<211> 0
<212> DNA
<213> Oryza sativa

<400> 1630
000

<210> 1631
<211> 0
<212> DNA
<213> Oryza sativa

<400> 1631
000

<210> 1632
<211> 0
<212> DNA
<213> Glycine max

<400> 1632
000

<210> 1633
<211> 0
<212> DNA
<213> Glycine max

<400> 1633
000

<210> 1634
<211> 0
<212> DNA
<213> Glycine max

<400> 1634
000

<210> 1635
<211> 0
<212> DNA
<213> Glycine max

<400> 1635
000

<210> 1636
<211> 0
<212> DNA
<213> Glycine max

<400> 1636
000

<210> 1637
<211> 0
<212> DNA
<213> Glycine max

<400> 1637
000

<210> 1638
<211> 0
<212> DNA
<213> Glycine max

<400> 1638
000

<210> 1639
<211> 0
<212> DNA
<213> Glycine max

<400> 1639
000

<210> 1640
<211> 0
<212> DNA
<213> Glycine max

<400> 1640
000

<210> 1641
<211> 0
<212> DNA
<213> Glycine max

<400> 1641
000

<210> 1642
<211> 0
<212> DNA
<213> Glycine max

<400> 1642
000

<210> 1643
<211> 0
<212> DNA
<213> Glycine max

<400> 1643
000

<210> 1644
<211> 0
<212> DNA
<213> Glycine max

<400> 1644
000

<210> 1645
<211> 0
<212> DNA
<213> Glycine max

<400> 1645
000

<210> 1646
<211> 0
<212> DNA
<213> Glycine max

<<400> 1646
000

<210> 1647
<211> 0

<212> DNA
<213> Glycine max

<400> 1647
000

<210> 1648
<211> 0
<212> DNA
<213> Glycine max

<400> 1648
000

<210> 1649
<211> 0
<212> DNA
<213> Glycine max

<400> 1649
000

<210> 1650
<211> 0
<212> DNA
<213> Glycine max

<400> 1650
000

<210> 1651
<211> 0
<212> DNA
<213> Glycine max

<400> 1651
000

<210> 1652
<211> 0
<212> DNA
<213> Glycine max

<400> 1652
000

<210> 1653
<211> 0
<212> DNA
<213> Glycine max

<400> 1653
000

<210> 1654
<211> 0
<212> DNA
<213> Glycine max

<400> 1654
000

<210> 1655
<211> 0
<212> DNA
<213> Glycine max

<400> 1655
000

<210> 1656
<211> 0
<212> DNA
<213> Glycine max

<400> 1656
000

<210> 1657
<211> 0
<212> DNA
<213> Glycine max

<400> 1657
000

<210> 1658
<211> 0
<212> DNA
<213> Glycine max

<400> 1658
000

<210> 1659
<211> 0
<212> DNA
<213> Glycine max

<400> 1659
000

<210> 1660
<211> 0
<212> DNA
<213> Glycine max

<400> 1660
000

<210> 1661
<211> 0
<212> DNA
<213> Glycine max

<400> 1661
000

<210> 1662
<211> 0
<212> DNA
<213> Glycine max

<400> 1662
000

<210> 1663
<211> 0
<212> DNA
<213> Glycine max

<400> 1663
000

<210> 1664
<211> 0
<212> DNA
<213> Glycine max

<400> 1664
000

<210> 1665
<211> 0
<212> DNA
<213> Glycine max

<400> 1665
000

<210> 1666
<211> 0
<212> DNA
<213> Glycine max

<400> 1666
000

<210> 1667
<211> 0
<212> DNA
<213> Glycine max

<400> 1667
000

<210> 1668
<211> 0
<212> DNA
<213> Glycine max

<400> 1668
000

<210> 1669

<211> 0
<212> DNA
<213> Glycine max

<400> 1669
000

<210> 1670
<211> 0
<212> DNA
<213> Glycine max

<400> 1670
000

<210> 1671
<211> 0
<212> DNA
<213> Glycine max

<400> 1671
000

<210> 1672
<211> 0
<212> DNA
<213> Glycine max

<400> 1672
000

<210> 1673
<211> 0
<212> DNA
<213> Glycine max

<400> 1673
000

<210> 1674
<211> 0
<212> DNA
<213> Glycine max

<400> 1674
000

<210> 1675
<211> 0
<212> DNA
<213> Glycine max

<400> 1675
000

<210> 1676
<211> 0
<212> DNA

<213> Glycine max

<400> 1676
000

<210> 1677
<211> 0
<212> DNA
<213> Glycine max

<400> 1677
000

<210> 1678
<211> 0
<212> DNA
<213> Glycine max

<400> 1678
000

<210> 1679
<211> 0
<212> DNA
<213> Glycine max

<400> 1679
000

<210> 1680
<211> 0
<212> DNA
<213> Glycine max

<400> 1680
000

<210> 1681
<211> 0
<212> DNA
<213> Glycine max

<400> 1681
000

<210> 1682
<211> 0
<212> DNA
<213> Glycine max

<400> 1682
000

<210> 1683
<211> 0
<212> DNA
<213> Glycine max

<400> 1683
000

<210> 1684
<211> 0
<212> DNA
<213> Glycine max

<400> 1684
000

<210> 1685
<211> 0
<212> DNA
<213> Glycine max

<400> 1685
000

<210> 1686
<211> 0
<212> DNA
<213> Glycine max

<400> 1686
000

<210> 1687
<211> 0
<212> DNA
<213> Glycine max

<400> 1687
000

<210> 1688
<211> 0
<212> DNA
<213> Glycine max

<400> 1688
000

<210> 1689
<211> 0
<212> DNA
<213> Glycine max

<400> 1689
000

<210> 1690
<211> 0
<212> DNA
<213> Medicago truncatula

<400> 1690
000

<210> 1691
<211> 0
<212> DNA
<213> Medicago truncatula

<400> 1691
000

<210> 1692
<211> 0
<212> DNA
<213> Medicago truncatula

<400> 1692
000

<210> 1693
<211> 0
<212> DNA
<213> Medicago truncatula

<400> 1693
000

<210> 1694
<211> 0
<212> DNA
<213> Medicago truncatula

<400> 1694
000

<210> 1695
<211> 0
<212> DNA
<213> Medicago truncatula

<400> 1695
000

<210> 1696
<211> 0
<212> DNA
<213> Medicago truncatula

<400> 1696
000

<210> 1697
<211> 0
<212> DNA
<213> Medicago truncatula

<400> 1697
000

<210> 1698
<211> 0

<212> DNA
<213> Medicago truncatula

<400> 1698
000

<210> 1699
<211> 0
<212> DNA
<213> Medicago truncatula

<400> 1699
000

<210> 1700
<211> 0
<212> DNA
<213> Medicago truncatula

<400> 1700
000

<210> 1701
<211> 0
<212> DNA
<213> Medicago truncatula

<400> 1701
000

<210> 1702
<211> 0
<212> DNA
<213> Medicago truncatula
<400> 1702
000

<210> 1703
<211> 0
<212> DNA
<213> Medicago truncatula

<400> 1703
000

<210> 1704
<211> 0
<212> DNA
<213> Medicago truncatula

<400> 1704
000

<210> 1705
<211> 0
<212> DNA
<213> Medicago truncatula

<400> 1705
000

<210> 1706
<211> 0
<212> DNA
<213> Medicago truncatula

<400> 1706
000

<210> 1707
<211> 0
<212> DNA
<213> Medicago truncatula

<400> 1707
000

<210> 1708
<211> 0
<212> DNA
<213> Medicago truncatula

<400> 1708
000

<210> 1709
<211> 0
<212> DNA
<213> Medicago truncatula

<400> 1709
000

<210> 1710
<211> 0
<212> DNA
<213> Medicago truncatula

<400> 1710
000

<210> 1711
<211> 0
<212> DNA
<213> Medicago truncatula

<400> 1711
000

<210> 1712
<211> 0
<212> DNA
<213> Medicago truncatula

<400> 1712
000

<210> 1713
<211> 0
<212> DNA
<213> Medicago truncatula

<400> 1713
000

<210> 1714
<211> 0
<212> DNA
<213> Medicago truncatula

<400> 1714
000

<210> 1715
<211> 0
<212> DNA
<213> Medicago truncatula

<400> 1715
000

<210> 1716
<211> 0
<212> DNA
<213> Medicago truncatula

<400> 1716
000

<210> 1717
<211> 0
<212> DNA
<213> Medicago truncatula

<400> 1717
000

<210> 1718
<211> 0
<212> DNA
<213> Hordeum vulgare

<400> 1718
000

<210> 1719
<211> 0
<212> DNA
<213> Hordeum vulgare

<400> 1719
000

<210> 1720
<211> 0

<212> DNA
<213> Hordeum vulgare

<400> 1720
000

<210> 1721
<211> 0
<212> DNA
<213> Hordeum vulgare

<400> 1721
000

<210> 1722
<211> 0
<212> DNA
<213> Hordeum vulgare

<400> 1722
000

<210> 1723
<211> 0
<212> DNA
<213> Hordeum vulgare

<400> 1723
000

<210> 1724
<211> 0
<212> DNA
<213> Hordeum vulgare

<400> 1724
000

<210> 1725
<211> 0
<212> DNA
<213> Hordeum vulgare

<400> 1725
000

<210> 1726
<211> 0
<212> DNA
<213> Hordeum vulgare

<400> 1726
000

<210> 1727
<211> 0
<212> DNA
<213> Hordeum vulgare

<400> 1727
000

<210> 1728
<211> 0
<212> DNA
<213> Hordeum vulgare

<400> 1728
000

<210> 1729
<211> 0
<212> DNA
<213> Hordeum vulgare

<400> 1729
000

<210> 1730
<211> 0
<212> DNA
<213> Hordeum vulgare

<400> 1730
000

<210> 1731
<211> 0
<212> DNA
<213> Hordeum vulgare

<400> 1731
000

<210> 1732
<211> 0
<212> DNA
<213> Hordeum vulgare

<400> 1732
000

<210> 1733
<211> 0
<212> DNA
<213> Hordeum vulgare

<400> 1733
000

<210> 1734
<211> 0
<212> DNA
<213> Hordeum vulgare

<400> 1734
000

<210> 1735
<211> 0
<212> DNA
<213> Hordeum vulgare

<400> 1735
000

<210> 1736
<211> 0
<212> DNA
<213> Hordeum vulgare

<400> 1736
000

<210> 1737
<211> 0
<212> DNA
<213> Hordeum vulgare

<400> 1737
000

<210> 1738
<211> 0
<212> DNA
<213> Hordeum vulgare

<400> 1738
000

<210> 1739
<211> 0
<212> DNA
<213> Hordeum vulgare

<400> 1739
000

<210> 1740
<211> 0
<212> DNA
<213> Hordeum vulgare

<400> 1740
000

<210> 1741
<211> 0
<212> DNA
<213> Hordeum vulgare

<400> 1741
000

<210> 1742
<211> 0

<212> DNA
<213> Hordeum vulgare

<400> 1742
000

<210> 1743
<211> 0
<212> DNA
<213> Hordeum vulgare

<400> 1743
000

<210> 1744
<211> 0
<212> DNA
<213> Hordeum vulgare

<400> 1744
000

<210> 1745
<211> 0
<212> DNA
<213> Hordeum vulgare

<400> 1745
000

<210> 1746
<211> 0
<212> DNA
<213> Hordeum vulgare

<400> 1746
000

<210> 1747
<211> 0
<212> DNA
<213> Hordeum vulgare

<400> 1747
000

<210> 1748
<211> 0
<212> DNA
<213> Hordeum vulgare

<400> 1748
000

<210> 1749
<211> 0
<212> DNA
<213> Hordeum vulgare

<400> 1749
000

<210> 1750
<211> 0
<212> DNA
<213> Hordeum vulgare

<400> 1750

000

<210> 1751
<211> 0
<212> DNA
<213> Hordeum vulgare

<400> 1751
000

<210> 1752
<211> 0
<212> DNA
<213> Hordeum vulgare

<400> 1752
000

<210> 1753
<211> 0
<212> DNA
<213> Hordeum vulgare

<400> 1753
000

<210> 1754
<211> 0
<212> DNA
<213> Zea mays

<400> 1754
000

<210> 1755
<211> 0
<212> DNA
<213> Zea mays

<400> 1755
000

<210> 1756
<211> 0
<212> DNA
<213> Zea mays

<400> 1756

000

<210> 1757
<211> 0
<212> DNA
<213> Zea mays

<400> 1757
000

<210> 1758
<211> 0
<212> DNA
<213> Zea mays

<400> 1758
000

<210> 1759
<211> 0
<212> DNA
<213> Zea mays

<400> 1759
000

<210> 1760
<211> 0
<212> DNA
<213> Zea mays

<400> 1760
000

<210> 1761
<211> 0
<212> DNA
<213> Zea mays

<400> 1761
000

<210> 1762
<211> 0
<212> DNA
<213> Zea mays

<400> 1762
000

<210> 1763
<211> 0
<212> DNA
<213> Zea mays

<400> 1763
000

<210> 1764
<211> 0
<212> DNA
<213> Zea mays

<400> 1764
000

<210> 1765
<211> 0
<212> DNA
<213> Zea mays

<400> 1765
000

<210> 1766
<211> 0
<212> DNA
<213> Zea mays

<400> 1766
000

<210> 1767
<211> 0
<212> DNA
<213> Zea mays

<400> 1767
000

<210> 1768
<211> 0
<212> DNA
<213> Zea mays

<400> 1768
000

<210> 1769
<211> 0
<212> DNA
<213> Zea mays

<400> 1769
000

<210> 1770
<211> 0
<212> DNA
<213> Zea mays

<400> 1770
000

<210> 1771
<211> 0

<212> DNA
<213> Zea mays

<400> 1771
000

<210> 1772
<211> 0
<212> DNA
<213> Zea mays

<400> 1772
000

<210> 1773
<211> 0
<212> DNA
<213> Zea mays

<400> 1773
000

<210> 1774
<211> 0
<212> DNA
<213> Zea mays

<400> 1774
000

<210> 1775
<211> 0
<212> DNA
<213> Zea mays

<400> 1775
000

<210> 1776
<211> 0
<212> DNA
<213> Zea mays

<400> 1776
000

<210> 1777
<211> 0
<212> DNA
<213> Zea mays

<400> 1777
000

<210> 1778
<211> 0
<212> DNA
<213> Zea mays

<400> 1778
000

<210> 1779
<211> 0
<212> DNA
<213> Zea mays

<400> 1779
000

<210> 1780
<211> 0
<212> DNA
<213> Zea mays

<400> 1780
000

<210> 1781
<211> 0
<212> DNA
<213> Zea mays

<400> 1781
000

<210> 1782
<211> 0
<212> DNA
<213> Zea mays

<400> 1782
000

<210> 1783
<211> 0
<212> DNA
<213> Zea mays

<400> 1783
000

<210> 1784
<211> 0
<212> DNA
<213> Zea mays

<400> 1784
000

<210> 1785
<211> 0
<212> DNA
<213> Zea mays

<400> 1785
000

<210> 1786
<211> 0
<212> DNA
<213> Zea mays

<400> 1786
000

<210> 1787
<211> 0
<212> DNA
<213> Zea mays

<400> 1787
000

<210> 1788
<211> 0
<212> DNA
<213> Zea mays

<400> 1788
000

<210> 1789
<211> 0
<212> DNA
<213> Zea mays

<400> 1789
000

<210> 1790
<211> 0
<212> DNA
<213> Zea mays

<400> 1790
000

<210> 1791
<211> 0
<212> DNA
<213> Zea mays

<400> 1791
000

<210> 1792
<211> 0
<212> DNA
<213> Zea mays

<400> 1792
000

<210> 1793
<211> 0

<212> DNA
<213> Zea mays

<400> 1793
000

<210> 1794
<211> 0
<212> DNA
<213> Zea mays

<400> 1794
000

<210> 1795
<211> 0
<212> DNA
<213> Zea mays

<400> 1795
000

<210> 1796
<211> 0
<212> DNA
<213> Zea mays

<400> 1796
000

<210> 1797
<211> 0
<212> DNA
<213> Zea mays

<400> 1797
000

<210> 1798
<211> 0
<212> DNA
<213> Zea mays

<400> 1798
000

<210> 1799
<211> 0
<212> DNA
<213> Zea mays

<400> 1799
000

<210> 1800
<211> 0
<212> DNA
<213> Zea mays

<400> 1800
000

<210> 1801
<211> 0
<212> DNA
<213> Zea mays

<400> 1801
000

<210> 1802
<211> 0
<212> DNA
<213> Zea mays

<400> 1802
000

<210> 1803
<211> 0
<212> DNA
<213> Zea mays

<400> 1803
000

<210> 1804
<211> 0
<212> DNA
<213> Zea mays

<400> 1804
000

<210> 1805
<211> 0
<212> DNA
<213> Zea mays

<400> 1805
000

<210> 1806
<211> 0
<212> DNA
<213> Zea mays

<400> 1806
000

<210> 1807
<211> 0
<212> DNA
<213> Zea mays

<400> 1807
000

<210> 1808
<211> 0
<212> DNA
<213> Zea mays

<400> 1808
000

<210> 1809
<211> 0
<212> DNA
<213> Zea mays

<400> 1809
000

<210> 1810
<211> 0
<212> DNA
<213> Zea mays

<400> 1810
000

<210> 1811
<211> 0
<212> DNA
<213> Zea mays

<400> 1811
000

<210> 1812
<211> 0
<212> DNA
<213> Zea mays

<400> 1812
000

<210> 1813
<211> 0
<212> DNA
<213> Zea mays

<400> 1813
000

<210> 1814
<211> 0
<212> DNA
<213> Zea mays

<400> 1814
000

<210> 1815
<211> 0

<212> DNA
<213> Zea mays

<400> 1815
000

<210> 1816
<211> 0
<212> DNA
<213> Zea mays

<400> 1816
000

<210> 1817
<211> 0
<212> DNA
<213> Zea mays

<400> 1817
000

<210> 1818
<211> 0
<212> DNA
<213> Zea mays

<400> 1818
000

<210> 1819
<211> 0
<212> DNA
<213> Zea mays

<400> 1819
000

<210> 1820
<211> 0
<212> DNA
<213> Zea mays

<400> 1820
000

<210> 1821
<211> 0
<212> DNA
<213> Zea mays

<400> 1821
000

<210> 1822
<211> 0
<212> DNA
<213> Zea mays

<400> 1822
000

<210> 1823
<211> 0
<212> DNA
<213> Zea mays

<400> 1823
000

<210> 1824
<211> 0
<212> DNA
<213> Zea mays

<400> 1824
000

<210> 1825
<211> 0
<212> DNA
<213> Zea mays

<400> 1825
000

<210> 1826
<211> 0
<212> DNA
<213> Zea mays

<400> 1826
000

<210> 1827
<211> 0
<212> DNA
<213> Zea mays

<400> 1827
000

<210> 1828
<211> 0
<212> DNA
<213> Zea mays

<400> 1828
000

<210> 1829
<211> 0
<212> DNA
<213> Zea mays

<400> 1829
000

<210> 1830
<211> 0
<212> DNA
<213> Zea mays

<400> 1830
000

<210> 1831
<211> 0
<212> DNA
<213> Zea mays

<400> 1831
000

<210> 1832
<211> 0
<212> DNA
<213> Zea mays

<400> 1832
000

<210> 1833
<211> 0
<212> DNA
<213> Zea mays

<400> 1833
000

<210> 1834
<211> 0
<212> DNA
<213> Triticum aestivum

<400> 1834
000

<210> 1835
<211> 0
<212> DNA
<213> Triticum aestivum

<400> 1835
000

<210> 1836
<211> 0
<212> DNA
<213> Triticum aestivum

<400> 1836
000

<210> 1837
<211> 0

<212> DNA
<213> Triticum aestivum

<400> 1837
000

<210> 1838
<211> 0
<212> DNA
<213> Triticum aestivum

<400> 1838
000

<210> 1839
<211> 0
<212> DNA
<213> Triticum aestivum

<400> 1839
000

<210> 1840
<211> 0
<212> DNA
<213> Triticum aestivum

<400> 1840
000

<210> 1841
<211> 0
<212> DNA
<213> Triticum aestivum

<400> 1841
000

<210> 1842
<211> 0
<212> DNA
<213> Triticum aestivum

<400> 1842
000

<210> 1843
<211> 0
<212> DNA
<213> Triticum aestivum

<400> 1843
000

<210> 1844
<211> 0
<212> DNA
<213> Triticum aestivum

<400> 1844
000

<210> 1845
<211> 0
<212> DNA
<213> Triticum aestivum

<400> 1845
000

<210> 1846
<211> 0
<212> DNA
<213> Triticum aestivum

<400> 1846
000

<210> 1847
<211> 0
<212> DNA
<213> Triticum aestivum

<400> 1847
000

<210> 1848
<211> 0
<212> DNA
<213> Triticum aestivum

<400> 1848
000

<210> 1849
<211> 0
<212> DNA
<213> Triticum aestivum

<400> 1849
000

<210> 1850
<211> 0
<212> DNA
<213> Triticum aestivum

<400> 1850
000

<210> 1851
<211> 0
<212> DNA
<213> Triticum aestivum

<400> 1851
000

<210> 1852
<211> 0
<212> DNA
<213> Triticum aestivum

<400> 1852
000

<210> 1853
<211> 0
<212> DNA
<213> Triticum aestivum

<400> 1853
000

<210> 1854
<211> 0
<212> DNA
<213> Triticum aestivum

<400> 1854
000

<210> 1855
<211> 0
<212> DNA
<213> Triticum aestivum

<400> 1855
000

<210> 1856
<211> 0
<212> DNA
<213> Triticum aestivum

<400> 1856
000

<210> 1857
<211> 0
<212> DNA
<213> Triticum aestivum

<400> 1857
000

<210> 1858
<211> 0
<212> DNA
<213> Triticum aestivum

<400> 1858
000

<210> 1859
<211> 0

<212> DNA
<213> Triticum aestivum

<400> 1859
000

<210> 1860
<211> 0
<212> DNA
<213> Triticum aestivum

<400> 1860
000

<210> 1861
<211> 0
<212> DNA
<213> Triticum aestivum

<400> 1861
000

<210> 1862
<211> 0
<212> DNA
<213> Triticum aestivum

<400> 1862
000

<210> 1863
<211> 0
<212> DNA
<213> Triticum aestivum

<400> 1863
000

<210> 1864
<211> 0
<212> DNA
<213> Triticum aestivum

<400> 1864
000

<210> 1865
<211> 0
<212> DNA
<213> Triticum aestivum
<400> 1865
000

<210> 1866
<211> 0
<212> DNA
<213> Triticum aestivum

<400> 1866
000

<210> 1867
<211> 0
<212> DNA
<213> Triticum aestivum

<400> 1867
000

<210> 1868
<211> 0
<212> DNA
<213> Triticum aestivum

<400> 1868
000

<210> 1869
<211> 0
<212> DNA
<213> Triticum aestivum

<400> 1869
000

<210> 1870
<211> 0
<212> DNA
<213> Triticum aestivum

<400> 1870
000

<210> 1871
<211> 0
<212> DNA
<213> Triticum aestivum

<400> 1871
000

<210> 1872
<211> 0
<212> DNA
<213> Triticum aestivum

<400> 1872
000

<210> 1873
<211> 0
<212> DNA
<213> Triticum aestivum

<400> 1873
000

<210> 1874
<211> 0
<212> DNA
<213> Triticum aestivum

<400> 1874
000

<210> 1875
<211> 0
<212> DNA
<213> Triticum aestivum

<400> 1875
000

<210> 1876
<211> 0
<212> DNA
<213> Triticum aestivum

<400> 1876
000

<210> 1877
<211> 0
<212> DNA
<213> Triticum aestivum

<400> 1877
000

<210> 1878
<211> 0
<212> DNA
<213> Triticum aestivum

<400> 1878
000

<210> 1879
<211> 0
<212> DNA
<213> Triticum aestivum

<400> 1879
000

<210> 1880
<211> 0
<212> DNA
<213> Triticum aestivum

<400> 1880
000

<210> 1881
<211> 0

<212> DNA
<213> Triticum aestivum

<400> 1881
000

<210> 1882
<211> 0
<212> DNA
<213> Triticum aestivum

<400> 1882
000

<210> 1883
<211> 0
<212> DNA
<213> Triticum aestivum

<400> 1883
000

<210> 1884
<211> 0
<212> DNA
<213> Triticum aestivum

<400> 1884
000

<210> 1885
<211> 0
<212> DNA
<213> Triticum aestivum

<400> 1885
000

<210> 1886
<211> 0
<212> DNA
<213> Triticum aestivum

<400> 1886
000

<210> 1887
<211> 0
<212> DNA
<213> Triticum aestivum

<400> 1887
000

<210> 1888
<211> 0
<212> DNA
<213> Triticum aestivum

<400> 1888
000

<210> 1889
<211> 0
<212> DNA
<213> Triticum aestivum

<400> 1889
000

<210> 1890
<211> 0
<212> DNA
<213> Triticum aestivum

<400> 1890
000

<210> 1891
<211> 0
<212> DNA
<213> Triticum aestivum

<400> 1891
000

<210> 1892
<211> 0
<212> DNA
<213> Triticum aestivum

<400> 1892
000

<210> 1893
<211> 0
<212> DNA
<213> Triticum aestivum

<400> 1893
000

<210> 1894
<211> 0
<212> DNA
<213> Triticum aestivum

<400> 1894
000

<210> 1895
<211> 0
<212> DNA
<213> Triticum aestivum

<400> 1895
000

<210> 1896
<211> 0
<212> DNA
<213> Triticum aestivum

<400> 1896
000

<210> 1897
<211> 0
<212> DNA
<213> Triticum aestivum

<400> 1897
000

<210> 1898
<211> 0
<212> DNA
<213> Triticum aestivum

<400> 1898
000

<210> 1899
<211> 0
<212> DNA
<213> Triticum aestivum

<400> 1899
000

<210> 1900
<211> 0
<212> DNA
<213> Triticum aestivum

<400> 1900
000

<210> 1901
<211> 0
<212> DNA
<213> Triticum aestivum

<400> 1901
000

<210> 1902
<211> 0
<212> DNA
<213> Triticum aestivum

<400> 1902
000

<210> 1903
<211> 0

<212> DNA
<213> Triticum aestivum

<400> 1903
000

<210> 1904
<211> 0
<212> DNA
<213> Triticum aestivum

<400> 1904
000

<210> 1905
<211> 0
<212> DNA
<213> Triticum aestivum

<400> 1905
000

<210> 1906
<211> 0
<212> DNA
<213> Triticum aestivum

<400> 1906
000

<210> 1907
<211> 0
<212> DNA
<213> Triticum aestivum

<400> 1907
000

<210> 1908
<211> 0
<212> DNA
<213> Triticum aestivum

<400> 1908
000

<210> 1909
<211> 0
<212> DNA
<213> Triticum aestivum

<400> 1909
000

<210> 1910
<211> 0
<212> DNA
<213> Triticum aestivum

<400> 1910
000

<210> 1911
<211> 0
<212> DNA
<213> Triticum aestivum

<400> 1911
000

<210> 1912
<211> 0
<212> DNA
<213> Triticum aestivum

<400> 1912
000

<210> 1913
<211> 0
<212> DNA
<213> Triticum aestivum

<400> 1913
000

<210> 1914
<211> 0
<212> DNA
<213> Triticum aestivum

<400> 1914
000

<210> 1915
<211> 0
<212> DNA
<213> Triticum aestivum

<400> 1915
000

<210> 1916
<211> 0
<212> DNA
<213> Triticum aestivum

<400> 1916
000

<210> 1917
<211> 0
<212> DNA
<213> Triticum aestivum

<400> 1917
000

<210> 1918
<211> 0
<212> DNA
<213> Triticum aestivum

<400> 1918
000

<210> 1919
<211> 0
<212> DNA
<213> Triticum aestivum

<400> 1919
000

<210> 1920
<211> 0
<212> DNA
<213> Triticum aestivum

<400> 1920
000

<210> 1921
<211> 0
<212> DNA
<213> Triticum aestivum

<400> 1921
000

<210> 1922
<211> 0
<212> DNA
<213> Triticum aestivum

<400> 1922
000

<210> 1923
<211> 0
<212> DNA
<213> Triticum aestivum

<400> 1923
000

<210> 1924
<211> 0
<212> DNA
<213> Triticum aestivum

<400> 1924
000

<210> 1925
<211> 0

<212> DNA
<213> Lycopersicon esculentum

<400> 1925
000

<210> 1926
<211> 0
<212> DNA
<213> Lycopersicon esculentum

<400> 1926
000

<210> 1927
<211> 0
<212> DNA
<213> Lycopersicon esculentum

<400> 1927
000

<210> 1928
<211> 0
<212> DNA
<213> Lycopersicon esculentum

<400> 1928
000

<210> 1929
<211> 0
<212> DNA
<213> Lycopersicon esculentum

<400> 1929
000

<210> 1930
<211> 0
<212> DNA
<213> Lycopersicon esculentum

<400> 1930
000

<210> 1931
<211> 0
<212> DNA
<213> Lycopersicon esculentum

<400> 1931
000

<210> 1932
<211> 0
<212> DNA
<213> Lycopersicon esculentum

<400> 1932
000

<210> 1933
<211> 0
<212> DNA
<213> Lycopersicon esculentum

<400> 1933
000

<210> 1934
<211> 0
<212> DNA
<213> Lycopersicon esculentum

<400> 1934
000

<210> 1935
<211> 0
<212> DNA
<213> Lycopersicon esculentum

<400> 1935
000

<210> 1936
<211> 0
<212> DNA
<213> Lycopersicon esculentum

<400> 1936
000

<210> 1937
<211> 0
<212> DNA
<213> Lycopersicon esculentum

<400> 1937
000

<210> 1938
<211> 0
<212> DNA
<213> Lycopersicon esculentum

<400> 1938
000

<210> 1939
<211> 0
<212> DNA
<213> Lycopersicon esculentum

<400> 1939
000

<210> 1940
<211> 0
<212> DNA
<213> Lycopersicon esculentum

<400> 1940
000

<210> 1941
<211> 0
<212> DNA
<213> Lycopersicon esculentum

<400> 1941
000

<210> 1942
<211> 0
<212> DNA
<213> Lycopersicon esculentum

<400> 1942
000

<210> 1943
<211> 0
<212> DNA
<213> Lycopersicon esculentum

<400> 1943
000

<210> 1944
<211> 0
<212> DNA
<213> Lycopersicon esculentum

<400> 1944
000

<210> 1945
<211> 0
<212> DNA
<213> Lycopersicon esculentum

<400> 1945
000

<210> 1946
<211> 0
<212> DNA
<213> Lycopersicon esculentum

<400> 1946
000

<210> 1947
<211> 0

<212> DNA
<213> Lycopersicon esculentum

<400> 1947
000

<210> 1948
<211> 0
<212> DNA
<213> Lycopersicon esculentum

<400> 1948
000

<210> 1949
<211> 0
<212> DNA
<213> Lycopersicon esculentum

<400> 1949
000

<210> 1950
<211> 0
<212> DNA
<213> Lycopersicon esculentum

<400> 1950
000

<210> 1951
<211> 0
<212> DNA
<213> Lycopersicon esculentum

<400> 1951
000

<210> 1952
<211> 0
<212> DNA
<213> Lycopersicon esculentum

<400> 1952
000

<210> 1953
<211> 0
<212> DNA
<213> Lycopersicon esculentum

<400> 1953
000

<210> 1954
<211> 0
<212> DNA
<213> Lycopersicon esculentum

<400> 1954
000

<210> 1955
<211> 0
<212> DNA
<213> Lycopersicon esculentum

<400> 1955
000

<210> 1956
<211> 0
<212> DNA
<213> Lycopersicon esculentum

<400> 1956
000

<210> 1957
<211> 0
<212> DNA
<213> Lycopersicon esculentum

<400> 1957
000

<210> 1958
<211> 0
<212> DNA
<213> Lycopersicon esculentum

<400> 1958
000

<210> 1959
<211> 0
<212> DNA
<213> Lycopersicon esculentum

<400> 1959
000

<210> 1960
<211> 0
<212> DNA
<213> Lycopersicon esculentum

<400> 1960
000

<210> 1961
<211> 0
<212> DNA
<213> Lycopersicon esculentum

<400> 1961
000

<210> 1962
<211> 0
<212> DNA
<213> Lycopersicon esculentum

<400> 1962
000

<210> 1963
<211> 0
<212> DNA
<213> Lycopersicon esculentum

<400> 1963
000

<210> 1964
<211> 0
<212> DNA
<213> Lycopersicon esculentum

<400> 1964
000

<210> 1965
<211> 0
<212> DNA
<213> Lycopersicon esculentum

<400> 1965
000

<210> 1966
<211> 0
<212> DNA
<213> Lycopersicon esculentum

<400> 1966
000

<210> 1967
<211> 0
<212> DNA
<213> Lycopersicon esculentum

<400> 1967
000

<210> 1968
<211> 0
<212> DNA
<213> Lycopersicon esculentum

<400> 1968
000

<210> 1969
<211> 0

<212> DNA
<213> Lycopersicon esculentum

<400> 1969
000

<210> 1970
<211> 0
<212> DNA
<213> Lycopersicon esculentum

<400> 1970
000

<210> 1971
<211> 0
<212> DNA
<213> Lycopersicon esculentum

<400> 1971
000

<210> 1972
<211> 0
<212> DNA
<213> Lycopersicon esculentum

<400> 1972
000

<210> 1973
<211> 0
<212> DNA
<213> Lycopersicon esculentum

<400> 1973
000

<210> 1974
<211> 0
<212> DNA
<213> Lycopersicon esculentum

<400> 1974
000

<210> 1975
<211> 0
<212> DNA
<213> Lycopersicon esculentum

<400> 1975
000

<210> 1976
<211> 0
<212> DNA
<213> Lycopersicon esculentum

<400> 1976
000

<210> 1977
<211> 0
<212> DNA
<213> Lycopersicon esculentum

<400> 1977
000

<210> 1978
<211> 0
<212> DNA
<213> Lycopersicon esculentum

<400> 1978
000

<210> 1979
<211> 0
<212> DNA
<213> Lycopersicon esculentum

<400> 1979
000

<210> 1980
<211> 0
<212> DNA
<213> Lycopersicon esculentum

<400> 1980
000

<210> 1981
<211> 0
<212> DNA
<213> Lycopersicon esculentum

<400> 1981
000

<210> 1982
<211> 0
<212> DNA
<213> Lycopersicon esculentum

<400> 1982
000

<210> 1983
<211> 0
<212> DNA
<213> Lycopersicon esculentum

<400> 1983
000

<210> 1984
<211> 0
<212> DNA
<213> Lycopersicon esculentum

<400> 1984
000

<210> 1985
<211> 0
<212> DNA
<213> Lycopersicon esculentum

<400> 1985
000

<210> 1986
<211> 0
<212> DNA
<213> Lycopersicon esculentum

<400> 1986
000

<210> 1987
<211> 0
<212> DNA
<213> Lycopersicon esculentum

<400> 1987
000

<210> 1988
<211> 0
<212> DNA
<213> Lycopersicon esculentum

<400> 1988
000

<210> 1989
<211> 0
<212> DNA
<213> Lycopersicon esculentum

<400> 1989
000

<210> 1990
<211> 0
<212> DNA
<213> Lycopersicon esculentum

<400> 1990
000

<210> 1991
<211> 0

<212> DNA
<213> Lycopersicon esculentum

<400> 1991
000

<210> 1992
<211> 0
<212> DNA
<213> Lycopersicon esculentum

<400> 1992
000

<210> 1993
<211> 0
<212> DNA
<213> Lycopersicon esculentum

<400> 1993
000

<210> 1994
<211> 0
<212> DNA
<213> Lycopersicon esculentum

<400> 1994
000

<210> 1995
<211> 0
<212> DNA
<213> Lycopersicon esculentum

<400> 1995
000

<210> 1996
<211> 0
<212> DNA
<213> Lycopersicon esculentum

<400> 1996
000

<210> 1997
<211> 0
<212> DNA
<213> Lycopersicon esculentum

<400> 1997
000

<210> 1998
<211> 0
<212> DNA
<213> Lycopersicon esculentum

<400> 1998
000

<210> 1999
<211> 0
<212> DNA
<213> Lycopersicon esculentum

<400> 1999
000

<210> 2000
<211> 0
<212> DNA
<213> Lycopersicon esculentum

<400> 2000
000

<210> 2001
<211> 0
<212> DNA
<213> Lycopersicon esculentum

<400> 2001
000

<210> 2002
<211> 0
<212> DNA
<213> Lycopersicon esculentum

<400> 2002
000

<210> 2003
<211> 0
<212> DNA
<213> Lycopersicon esculentum

<400> 2003
000

<210> 2004
<211> 0
<212> DNA
<213> Lycopersicon esculentum

<400> 2004
000

<210> 2005
<211> 0
<212> DNA
<213> Lycopersicon esculentum

<400> 2005
000

<210> 2006
<211> 0
<212> DNA
<213> Lycopersicon esculentum

<400> 2006
000

<210> 2007
<211> 0
<212> DNA
<213> Lycopersicon esculentum

<400> 2007
000

<210> 2008
<211> 0
<212> DNA
<213> Lycopersicon esculentum

<400> 2008
000

<210> 2009
<211> 0
<212> DNA
<213> Lycopersicon esculentum

<400> 2009
000

<210> 2010
<211> 0
<212> DNA
<213> Lycopersicon esculentum

<400> 2010
000

<210> 2011
<211> 0
<212> DNA
<213> Lycopersicon esculentum

<400> 2011
000

<210> 2012
<211> 0
<212> DNA
<213> Lycopersicon esculentum

<400> 2012
000

<210> 2013
<211> 0

<212> DNA
<213> Lycopersicon esculentum

<400> 2013
000

<210> 2014
<211> 0
<212> DNA
<213> Lycopersicon esculentum

<400> 2014
000

<210> 2015
<211> 0
<212> DNA
<213> Lycopersicon esculentum

<400> 2015
000

<210> 2016
<211> 0
<212> DNA
<213> Lycopersicon esculentum

<400> 2016
000

<210> 2017
<211> 0
<212> DNA
<213> Lycopersicon esculentum

<400> 2017
000

<210> 2018
<211> 0
<212> DNA
<213> Lycopersicon esculentum

<400> 2018
000

<210> 2019
<211> 0
<212> DNA
<213> Lycopersicon esculentum

<400> 2019
000

<210> 2020
<211> 0
<212> DNA
<213> Lycopersicon esculentum

<400> 2020
000

<210> 2021
<211> 0
<212> DNA
<213> Lycopersicon esculentum

<400> 2021
000

<210> 2022
<211> 0
<212> DNA
<213> Lycopersicon esculentum

<400> 2022
000

<210> 2023
<211> 0
<212> DNA
<213> Lycopersicon esculentum

<400> 2023
000

<210> 2024
<211> 0
<212> DNA
<213> Lycopersicon esculentum

<400> 2024
000

<210> 2025
<211> 0
<212> DNA
<213> Lycopersicon esculentum

<400> 2025
000

<210> 2026
<211> 0
<212> DNA
<213> Lycopersicon esculentum

<400> 2026
000

<210> 2027
<211> 0
<212> DNA
<213> Lycopersicon esculentum

<400> 2027
000

<210> 2028
<211> 0
<212> DNA
<213> Lycopersicon esculentum

<400> 2028
000

<210> 2029
<211> 0
<212> DNA
<213> Lycopersicon esculentum

<400> 2029
000

<210> 2030
<211> 0
<212> DNA
<213> Lycopersicon esculentum

<400> 2030
000

<210> 2031
<211> 0
<212> DNA
<213> Lycopersicon esculentum

<400> 2031
000

<210> 2032
<211> 0
<212> DNA
<213> Lycopersicon esculentum

<400> 2032
000

<210> 2033
<211> 0
<212> DNA
<213> Lycopersicon esculentum

<400> 2033
000

<210> 2034
<211> 0
<212> DNA
<213> Lycopersicon esculentum

<400> 2034
000

<210> 2035
<211> 0

<212> DNA
<213> Lycopersicon esculentum

<400> 2035
000

<210> 2036
<211> 0
<212> DNA
<213> Lycopersicon esculentum

<400> 2036
000

<210> 2037
<211> 0
<212> DNA
<213> Lycopersicon esculentum

<400> 2037
000

<210> 2038
<211> 0
<212> DNA
<213> Lycopersicon esculentum

<400> 2038
000

<210> 2039
<211> 0
<212> DNA
<213> Lycopersicon esculentum

<400> 2039
000

<210> 2040
<211> 0
<212> DNA
<213> Lycopersicon esculentum

<400> 2040
000

<210> 2041
<211> 0
<212> DNA
<213> Lycopersicon esculentum

<400> 2041
000

<210> 2042
<211> 0
<212> DNA
<213> Lycopersicon esculentum

<400> 2042
000

<210> 2043
<211> 0
<212> DNA
<213> Lycopersicon esculentum

<400> 2043
000

<210> 2044

<211> 0
<212> DNA
<213> Lycopersicon esculentum

<400> 2044
000

<210> 2045
<211> 625
<212> DNA
<213> Lycopersicon esculentum

<400> 2045

```
ctcattcctc ttcctcctat atcttttctc tccgccccat tttcactatc acaaatcaaa      60
gcttccaaaa tttagaaatt gtatacaaaa atggaacttc tgtcctctaa actctgtgag     120
ctttgcaatg atcaagctgc tctgttttgt ccatctgatt cagcttttct ctgttttcac     180
tgtgatgcta aagttcatca ggctaatttc cttgttgctc gccaccttcg tcttactctt     240
tgctctcact gtaactccct tacgaaaaaa cgtttttccc cttgttcacc gccgcctcct     300
gctctttgtc cttcctgttc ccggaattcg tctggtgatt ccgatctccg ttctgtttca     360
acgacgtcgt cgtcgtcttc gtcgacttgt gtttccagca cgcagtccag tgctattact     420
caaaaaatta acataatctc ttcaaatcga aagcaatttc cggacagcga ctctaacggt     480
gaagtcaatt ctggcagatg taatttagta cgatccagaa gtgtgaaatt gcgagatcca     540
agagcggcga cttgtgtgtt catgcattgg tgcacaaagc ttcaaatgaa ccgcgaggaa     600
cgtgtggtgc aaacggcttg tagtg                                            625
```

<210> 2046
<211> 0
<212> DNA
<213> Lycopersicon esculentum

<400> 2046
000

<210> 2047
<211> 0
<212> DNA

<213> Lycopersicon esculentum

<400> 2047
000

<210> 2048
<211> 0
<212> DNA
<213> Lycopersicon esculentum

<400> 2048
000

<210> 2049
<211> 0
<212> DNA
<213> Lycopersicon esculentum

<400> 2049
000

<210> 2050
<211> 0
<212> DNA
<213> Lycopersicon esculentum

<400> 2050
000

<210> 2051
<211> 0
<212> DNA
<213> Lycopersicon esculentum

<400> 2051
000

<210> 2052
<211> 0
<212> DNA
<213> Lycopersicon esculentum

<400> 2052
000

<210> 2053
<211> 0
<212> DNA
<213> Lycopersicon esculentum

<400> 2053
000

<210> 2054
<211> 0
<212> DNA
<213> Lycopersicon esculentum

<400> 2054
000

<210> 2055
<211> 0
<212> DNA
<213> Lycopersicon esculentum

<400> 2055
000

<210> 2056
<211> 0
<212> DNA
<213> Lycopersicon esculentum

<400> 2056
000

<210> 2057
<211> 0
<212> DNA
<213> Lycopersicon esculentum

<400> 2057
000

<210> 2058
<211> 0
<212> DNA
<213> Lycopersicon esculentum

<400> 2058
000

<210> 2059
<211> 0
<212> DNA
<213> Lycopersicon esculentum

<400> 2059
000

<210> 2060
<211> 0
<212> DNA
<213> Lycopersicon esculentum

<400> 2060
000

<210> 2061
<211> 0
<212> DNA
<213> Lycopersicon esculentum

<400> 2061
000

<210> 2062
<211> 0
<212> DNA
<213> Lycopersicon esculentum

<400> 2062
000

<210> 2063
<211> 0
<212> DNA
<213> Lycopersicon esculentum

<400> 2063
000

<210> 2064
<211> 0
<212> DNA
<213> Lycopersicon esculentum

<400> 2064
000

<210> 2065
<211> 0
<212> DNA
<213> Lycopersicon esculentum

<400> 2065
000

<210> 2066
<211> 0
<212> DNA
<213> Lycopersicon esculentum

<400> 2066
000

<210> 2067
<211> 0
<212> DNA
<213> Lycopersicon esculentum

<400> 2067
000

<210> 2068
<211> 0
<212> DNA
<213> Lycopersicon esculentum

<400> 2068
000

<210> 2069
<211> 0

<212> DNA
<213> Lycopersicon esculentum

<400> 2069
000

<210> 2070
<211> 0
<212> DNA
<213> Lycopersicon esculentum

<400> 2070
000

<210> 2071
<211> 0
<212> DNA
<213> Lycopersicon esculentum

<400> 2071
000

<210> 2072
<211> 0
<212> DNA
<213> Lycopersicon esculentum

<400> 2072
000

<210> 2073
<211> 0
<212> DNA
<213> Lycopersicon esculentum

<400> 2073
000

<210> 2074
<211> 0
<212> DNA
<213> Lycopersicon esculentum

<400> 2074
000

<210> 2075
<211> 0
<212> DNA
<213> Lycopersicon esculentum

<400> 2075
000

<210> 2076
<211> 0
<212> DNA
<213> Lycopersicon esculentum

<400> 2076
000

<210> 2077
<211> 0
<212> DNA
<213> Lycopersicon esculentum

<400> 2077
000

<210> 2078
<211> 0
<212> DNA
<213> Lycopersicon esculentum

<400> 2078
000

<210> 2079
<211> 0
<212> DNA
<213> Lycopersicon esculentum

<400> 2079
000

<210> 2080
<211> 0
<212> DNA
<213> Lycopersicon esculentum

<400> 2080
000

<210> 2081
<211> 0
<212> DNA
<213> Lycopersicon esculentum

<400> 2081
000

<210> 2082
<211> 0
<212> DNA
<213> Lycopersicon esculentum

<400> 2082
000

<210> 2083
<211> 0
<212> DNA
<213> Lycopersicon esculentum

<400> 2083
000

<210> 2084
<211> 0
<212> DNA
<213> Lycopersicon esculentum

<400> 2084
000

<210> 2085
<211> 0
<212> DNA
<213> Lycopersicon esculentum

<400> 2085
000

<210> 2086
<211> 0
<212> DNA
<213> Lycopersicon esculentum

<400> 2086
000

<210> 2087
<211> 0
<212> DNA
<213> Lycopersicon esculentum

<400> 2087
000

<210> 2088
<211> 0
<212> DNA
<213> Lycopersicon esculentum

<400> 2088
000

<210> 2089
<211> 0
<212> DNA
<213> Lycopersicon esculentum

<400> 2089
000

<210> 2090
<211> 0
<212> DNA
<213> Lycopersicon esculentum

<400> 2090
000

<210> 2091
<211> 0

<212> DNA
<213> Lycopersicon esculentum

<400> 2091
000

<210> 2092
<211> 0
<212> DNA
<213> Lycopersicon esculentum

<400> 2092
000

<210> 2093
<211> 0
<212> DNA
<213> Lycopersicon esculentum

<400> 2093
000

<210> 2094
<211> 0
<212> DNA
<213> Lycopersicon esculentum

<400> 2094
000

<210> 2095
<211> 0
<212> DNA
<213> Lycopersicon esculentum

<400> 2095
000

<210> 2096
<211> 0
<212> DNA
<213> Lycopersicon esculentum

<400> 2096
000

<210> 2097
<211> 0
<212> DNA
<213> Lycopersicon esculentum

<400> 2097
000

<210> 2098
<211> 0
<212> DNA
<213> Lycopersicon esculentum

<400> 2098
000

<210> 2099
<211> 0
<212> DNA
<213> Lycopersicon esculentum

<400> 2099
000

<210> 2100
<211> 0
<212> DNA
<213> Lycopersicon esculentum

<400> 2100
000

<210> 2101
<211> 0
<212> DNA
<213> Lycopersicon esculentum

<400> 2101
000

<210> 2102
<211> 0
<212> DNA
<213> Arabidopsis thaliana

<400> 2102
000

<210> 2103
<211> 0
<212> DNA
<213> Arabidopsis thaliana

<400> 2103
000

<210> 2104
<211> 0
<212> DNA
<213> Arabidopsis thaliana

<400> 2104
000

<210> 2105
<211> 0
<212> DNA
<213> Arabidopsis thaliana

<400> 2105
000

<210> 2106
<211> 0
<212> DNA
<213> Arabidopsis thaliana

<400> 2106
000

<210> 2107
<211> 0
<212> DNA
<213> Arabidopsis thaliana

<400> 2107
000

<210> 2108
<211> 0
<212> DNA
<213> Arabidopsis thaliana

<400> 2108
000

<210> 2109
<211> 0
<212> DNA
<213> Arabidopsis thaliana

<400> 2109
000

<210> 2110
<211> 0
<212> DNA
<213> Arabidopsis thaliana

<400> 2110
000

<210> 2111
<211> 0
<212> DNA
<213> Arabidopsis thaliana

<400> 2111
000

<210> 2112
<211> 0
<212> DNA
<213> Arabidopsis thaliana

<400> 2112
000

<210> 2113
<211> 0

<212> DNA
<213> Arabidopsis thaliana

<400> 2113
000

<210> 2114
<211> 0
<212> DNA
<213> Arabidopsis thaliana

<400> 2114
000

<210> 2115
<211> 0
<212> DNA
<213> Arabidopsis thaliana

<400> 2115
000

<210> 2116
<211> 0
<212> DNA
<213> Arabidopsis thaliana

<400> 2116
000

<210> 2117
<211> 0
<212> DNA
<213> Arabidopsis thaliana

<400> 2117
000

<210> 2118
<211> 0
<212> DNA
<213> Arabidopsis thaliana

<400> 2118
000

<210> 2119
<211> 0
<212> DNA
<213> Arabidopsis thaliana

<400> 2119
000

<210> 2120
<211> 0
<212> DNA
<213> Arabidopsis thaliana

<400> 2120
000

<210> 2121
<211> 0
<212> DNA
<213> Arabidopsis thaliana

<400> 2121
000

<210> 2122
<211> 0
<212> DNA
<213> Arabidopsis thaliana

<400> 2122
000

<210> 2123
<211> 0
<212> DNA
<213> Arabidopsis thaliana

<400> 2123
000

<210> 2124
<211> 0
<212> DNA
<213> Oryza sativa

<400> 2124
000

<210> 2125
<211> 0
<212> PRT
<213> Oryza sativa

<400> 2125
000

<210> 2126
<211> 0
<212> DNA
<213> Oryza sativa

<400> 2126
000

<210> 2127
<211> 0
<212> PRT
<213> Oryza sativa

<400> 2127
000

<210> 2128
<211> 0
<212> DNA
<213> Oryza sativa

<400> 2128
000

<210> 2129
<211> 0
<212> PRT
<213> Oryza sativa

<400> 2129
000

<210> 2130
<211> 0
<212> DNA
<213> Oryza sativa

<400> 2130
000

<210> 2131
<211> 0
<212> PRT
<213> Oryza sativa

<400> 2131
000

<210> 2132
<211> 0
<212> DNA
<213> Oryza sativa

<400> 2132
000

<210> 2133
<211> 0
<212> PRT
<213> Oryza sativa

<400> 2133
000

<210> 2134
<211> 0
<212> DNA
<213> Oryza sativa

<400> 2134
000

<210> 2135
<211> 0

<212> PRT
<213> Oryza sativa

<400> 2135
000

<210> 2136
<211> 0
<212> DNA
<213> Oryza sativa

<400> 2136
000

<210> 2137
<211> 0
<212> PRT
<213> Oryza sativa

<400> 2137
000

<210> 2138
<211> 0
<212> DNA
<213> Oryza sativa

<400> 2138
000

<210> 2139
<211> 0
<212> PRT
<213> Oryza sativa

<400> 2139
000

<210> 2140
<211> 0
<212> DNA
<213> Oryza sativa

<400> 2140
000

<210> 2141
<211> 0
<212> PRT
<213> Oryza sativa

<400> 2141
000

<210> 2142
<211> 0
<212> DNA
<213> Oryza sativa

<400> 2142
000

<210> 2143
<211> 0
<212> PRT
<213> Oryza sativa

<400> 2143
000

<210> 2144
<211> 0
<212> DNA
<213> Oryza sativa

<400> 2144
000

<210> 2145
<211> 0
<212> PRT
<213> Oryza sativa

<400> 2145
000

<210> 2146
<211> 0
<212> DNA
<213> Zea mays

<400> 2146
000

<210> 2147
<211> 0
<212> PRT
<213> Zea mays

<400> 2147
000

<210> 2148
<211> 0
<212> DNA
<213> zea mays

<400> 2148
000

<210> 2149
<211> 0
<212> PRT
<213> Zea mays

<400> 2149
000

<210> 2150
<211> 0
<212> DNA
<213> Zea mays

<400> 2150
000

<210> 2151
<211> 0
<212> PRT
<213> Zea mays

<400> 2151
000

<210> 2152
<211> 0
<212> DNA
<213> Zea mays

<400> 2152
000

<210> 2153
<211> 0
<212> PRT
<213> Zea mays

<400> 2153
000

<210> 2154
<211> 0
<212> DNA
<213> Zea mays

<400> 2154
000

<210> 2155
<211> 0
<212> PRT
<213> Zea mays

<400> 2155
000

<210> 2156
<211> 0
<212> DNA
<213> Zea mays

<400> 2156
000

<210> 2157
<211> 0

<212> PRT
<213> Zea mays

<400> 2157
000

<210> 2158
<211> 0
<212> DNA
<213> Glycine max

<400> 2158
000

<210> 2159
<211> 0
<212> PRT
<213> Glycine max

<400> 2159
000

<210> 2160
<211> 0
<212> DNA
<213> Glycine max

<400> 2160
000

<210> 2161
<211> 0
<212> PRT
<213> Glycine max

<400> 2161
000

<210> 2162
<211> 0
<212> DNA
<213> Glycine max

<400> 2162
000

<210> 2163
<211> 0
<212> PRT
<213> Glycine max

<400> 2163
000

<210> 2164
<211> 0
<212> DNA
<213> Glycine max

<400> 2164
000

<210> 2165
<211> 0
<212> PRT
<213> Glycine max

<400> 2165
000

<210> 2166
<211> 0
<212> DNA
<213> Glycine max

<400> 2166
000

<210> 2167
<211> 0
<212> PRT
<213> Glycine max

<400> 2167
000

<210> 2168
<211> 0
<212> DNA
<213> Glycine max

<400> 2168
000

<210> 2169
<211> 0
<212> PRT
<213> Glycine max

<400> 2169
000

<210> 2170
<211> 0
<212> DNA
<213> Glycine max

<400> 2170
000

<210> 2171
<211> 0
<212> PRT
<213> Glycine max

<400> 2171
000

<210> 2172
<211> 0
<212> DNA
<213> Glycine max

<400> 2172
000

<210> 2173
<211> 0
<212> PRT
<213> Glycine max

<400> 2173
000

<210> 2174
<211> 0
<212> DNA
<213> Glycine max

<400> 2174
000

<210> 2175
<211> 0
<212> PRT
<213> Glycine max

<400> 2175
000

<210> 2176
<211> 0
<212> DNA
<213> Glycine max

<400> 2176
000

<210> 2177
<211> 0
<212> PRT
<213> Glycine max

<400> 2177
000

<210> 2178
<211> 0
<212> DNA
<213> Glycine max

<400> 2178
000

<210> 2179
<211> 0

<212> PRT
<213> Glycine max

<400> 2179
000

<210> 2180
<211> 0
<212> DNA
<213> Glycine max

<400> 2180
000

<210> 2181
<211> 0
<212> PRT
<213> Glycine max

<400> 2181
000

<210> 2182
<211> 0
<212> DNA
<213> Glycine max

<400> 2182
000

<210> 2183
<211> 0
<212> PRT
<213> Glycine max

<400> 2183
000

<210> 2184
<211> 0
<212> DNA
<213> Glycine max

<400> 2184
000

<210> 2185
<211> 0
<212> PRT
<213> Glycine max

<400> 2185
000

<210> 2186
<211> 0
<212> DNA
<213> Glycine max

<400> 2186
000

<210> 2187
<211> 0
<212> PRT
<213> Glycine max

<400> 2187
000

<210> 2188
<211> 0
<212> DNA
<213> Oryza sativa

<400> 2188
000

<210> 2189
<211> 0
<212> PRT
<213> Oryza sativa

<400> 2189
000

<210> 2190
<211> 0
<212> DNA
<213> Glycine max

<400> 2190
000

<210> 2191
<211> 0
<212> PRT
<213> Glycine max

<400> 2191
000

<210> 2192
<211> 0
<212> DNA
<213> Glycine max

<400> 2192
000

<210> 2193
<211> 0
<212> PRT
<213> Glycine max

<400> 2193
000

<210> 2194
<211> 0
<212> DNA
<213> Zea mays

<400> 2194
000

<210> 2195
<211> 0
<212> PRT
<213> Zea mays

<400> 2195
000

<210> 2196
<211> 0
<212> DNA
<213> Zea mays

<400> 2196
000

<210> 2197
<211> 0
<212> PRT
<213> Zea mays

<400> 2197
000

<210> 2198
<211> 0
<212> DNA
<213> Zea mays

<400> 2198
000

<210> 2199
<211> 0
<212> PRT
<213> Zea mays

<400> 2199
000

<210> 2200
<211> 0
<212> DNA
<213> Oryza sativa

<400> 2200
000

<210> 2201
<211> 0

<212> PRT
<213> Oryza sativa

<400> 2201
000

<210> 2202
<211> 0
<212> DNA
<213> Glycine max

<400> 2202
000

<210> 2203
<211> 0
<212> PRT
<213> Glycine max

<400> 2203
000

<210> 2204
<211> 0
<212> DNA
<213> Glycine max

<400> 2204
000

<210> 2205
<211> 0
<212> PRT
<213> Glycine max

<400> 2205
000

<210> 2206
<211> 0
<212> DNA
<213> Glycine max

<400> 2206
000

<210> 2207
<211> 0
<212> PRT
<213> Glycine max

<400> 2207
000

<210> 2208
<211> 0
<212> DNA
<213> Oryza sativa

<400> 2208
000

<210> 2209
<211> 0
<212> PRT
<213> Oryza sativa

<400> 2209
000

<210> 2210
<211> 0
<212> DNA
<213> Zea mays

<400> 2210
000

<210> 2211
<211> 0
<212> PRT
<213> Zea mays

<400> 2211
000

<210> 2212
<211> 0
<212> DNA
<213> Zea mays

<400> 2212
000

<210> 2213
<211> 0
<212> PRT
<213> Zea mays

<400> 2213
000

<210> 2214
<211> 0
<212> DNA
<213> Glycine max

<400> 2214
000

<210> 2215
<211> 0
<212> PRT
<213> Glycine max

<400> 2215
000

<210> 2216
<211> 0
<212> DNA
<213> Glycine max

<400> 2216
000

<210> 2217
<211> 0
<212> PRT
<213> Glycine max

<400> 2217
000

<210> 2218
<211> 0
<212> DNA
<213> Glycine max

<400> 2218
000

<210> 2219
<211> 0
<212> PRT
<213> Glycine max

<400> 2219
000

<210> 2220
<211> 0
<212> DNA
<213> Glycine max

<400> 2220
000

<210> 2221
<211> 0
<212> PRT
<213> Glycine max

<400> 2221
000

<210> 2222
<211> 0
<212> DNA
<213> Glycine max

<400> 2222
000

<210> 2223
<211> 0

<212> PRT
<213> Glycine max

<400> 2223
000

<210> 2224
<211> 0
<212> DNA
<213> Glycine max

<400> 2224
000

<210> 2225
<211> 0
<212> PRT
<213> Glycine max

<400> 2225
000

<210> 2226
<211> 0
<212> DNA
<213> Oryza sativa

<400> 2226
000

<210> 2227
<211> 0
<212> PRT
<213> Oryza sativa

<400> 2227
000

<210> 2228
<211> 0
<212> DNA
<213> Brassica rapa

<400> 2228
000

<210> 2229
<211> 0
<212> PRT
<213> Brassica rapa

<400> 2229
000

<210> 2230
<211> 0
<212> DNA
<213> Brassica oleracea

<400> 2230
000

<210> 2231
<211> 0
<212> PRT
<213> Brassica oleracea

<400> 2231
000

<210> 2232
<211> 0
<212> DNA
<213> zinnia elegans

<400> 2232
000

<210> 2233
<211> 0
<212> PRT
<213> zinnia elegans

<400> 2233
000

<210> 2234
<211> 0
<212> DNA
<213> Oryza sativa

<400> 2234
000

<210> 2235
<211> 0
<212> PRT
<213> Oryza sativa

<400> 2235
000

<210> 2236
<211> 0
<212> DNA
<213> Oryza sativa

<400> 2236
000

<210> 2237
<211> 0
<212> PRT
<213> Oryza sativa

<400> 2237
000

<210> 2238
<211> 929
<212> DNA
<213> Arabidopsis thaliana

<400> 2238

```
cttgaaaaag aatctacctg aaaagaaaaa aaagagagag agatataaat agctttacca      60
agacagatat actatctttt attaatccaa aaagactgag aactctagta actacgtact     120
acttaaacct tatccagttt cttgaaacag agtactctga tcaatgaact cattttcagc     180
tttttctgaa atgtttggct ccgattacga gcctcaaggc ggagattatt gtccgacgtt     240
ggccacgagt tgtccgaaga aaccggcggg ccgtaagaag tttcgtgaga ctcgtcaccc     300
aatttacaga ggagttcgtc aaagaaactc cggtaagtgg gtttctgaag tgagagagcc     360
aaacaagaaa accaggattt ggctcgggac tttccaaacc gctgagatgg cagctcgtgc     420
tcacgacgtc gctgcattag ccctccgtgg ccgatcagca tgtctcaact tcgctgactc     480
ggcttggcgg ctacgaatcc cggagtcaac atgcgccaag gatatccaaa aagcggctgc     540
tgaagcggcg ttggcttttc aagatgagac gtgtgatacg acgaccacga atcatggcct     600
ggacatggag gagacgatgg tggaagctat ttatacaccg gaacagagcg aaggtgcgtt     660
ttatatggat gaggagacaa tgtttgggat gccgactttg ttggataata tggctgaagg     720
catgctttta ccgccgccgt ctgttcaatg gaatcataat tatgacggcg aaggagatgg     780
tgacgtgtcg ctttggagtt actaatattc gatagtcgtt tccatttttg tactatagtt     840
tgaaaatatt ctagttcctt tttttagaat ggttccttca ttttatttta ttttattgtt     900
gtagaaacga gtggaaaata attcaatac                                       929
```

<210> 2239
<211> 213
<212> PRT
<213> Arabidopsis thaliana

<400> 2239

```
Met Asn Ser Phe Ser Ala Phe Ser Glu Met Phe Gly Ser Asp Tyr Glu
1               5                   10                  15

Pro Gln Gly Gly Asp Tyr Cys Pro Thr Leu Ala Thr Ser Cys Pro Lys
            20                  25                  30

Lys Pro Ala Gly Arg Lys Lys Phe Arg Glu Thr Arg His Pro Ile Tyr
        35                  40                  45

Arg Gly Val Arg Gln Arg Asn Ser Gly Lys Trp Val Ser Glu Val Arg
    50                  55                  60

Glu Pro Asn Lys Lys Thr Arg Ile Trp Leu Gly Thr Phe Gln Thr Ala
65                  70                  75                  80

Glu Met Ala Ala Arg Ala His Asp Val Ala Ala Leu Ala Leu Arg Gly
                85                  90                  95

Arg Ser Ala Cys Leu Asn Phe Ala Asp Ser Ala Trp Arg Leu Arg Ile
            100                 105                 110

Pro Glu Ser Thr Cys Ala Lys Asp Ile Gln Lys Ala Ala Ala Glu Ala
        115                 120                 125

Ala Leu Ala Phe Gln Asp Glu Thr Cys Asp Thr Thr Thr Thr Asn His

    130                 135                 140

Gly Leu Asp Met Glu Glu Thr Met Val Glu Ala Ile Tyr Thr Pro Glu
145                 150                 155                 160

Gln Ser Glu Gly Ala Phe Tyr Met Asp Glu Glu Thr Met Phe Gly Met
                165                 170                 175

Pro Thr Leu Leu Asp Asn Met Ala Glu Gly Met Leu Leu Pro Pro Pro
            180                 185                 190

Ser Val Gln Trp Asn His Asn Tyr Asp Gly Glu Gly Asp Gly Asp Val
        195                 200                 205

Ser Leu Trp Ser Tyr
    210
```

<210> 2240
<211> 803
<212> DNA
<213> Arabidopsis thaliana

<400> 2240

```
ctgatcaatg aactcatttt ctgccttttc tgaaatgttt ggctccgatt acgagtctcc      60
ggtttcctca ggcggtgatt acagtccgaa gcttgccacg agctgcccca agaaaccagc     120
gggaaggaag aagtttcgtg agactcgtca cccaatttac agaggagttc gtcaaagaaa     180
ctccggtaag tgggtgtgtg agttgagaga gccaaacaag aaaacgagga tttggctcgg     240
gactttccaa accgctgaga tggcagctcg tgctcacgac gtcgccgcca tagctctccg     300
tggcagatct gcctgtctca atttcgctga ctcggcttgg cggctacgaa tcccggaatc     360
aacctgtgcc aaggaaatcc aaaaggcggc ggctgaagcc gcgttgaatt ttcaagatga     420
gatgtgtcat atgacgacgg atgctcatgg tcttgacatg gaggagacct tggtggaggc     480
tatttatacg ccggaacaga gccaagatgc gttttatatg gatgaagagg cgatgttggg     540
gatgtctagt ttgttggata acatggccga agggatgctt ttaccgtcgc cgtcggttca     600
atggaactat aattttgatg tcgagggaga tgatgacgtg tccttatgga gctattaaaa     660
ttcgattttt atttccattt ttggtattat agctttttat acatttgatc ctttttttaga     720
atggatcttc ttcttttttt ggttgtgaga aacgaatgta aatggtaaaa gttgttgtca     780
aatgcaaatg tttttgagtg cag                                             803
```

<210> 2241
<211> 207
<212> PRT
<213> Arabidopsis thaliana

<400> 2241

```
Met Phe Gly Ser Asp Tyr Glu Ser Pro Val Ser Ser Gly Gly Asp Tyr
1               5                   10                  15

Ser Pro Lys Leu Ala Thr Ser Cys Pro Lys Lys Pro Ala Gly Arg Lys
            20                  25                  30

Lys Phe Arg Glu Thr Arg His Pro Ile Tyr Arg Gly Val Arg Gln Arg
        35                  40                  45
```

Asn Ser Gly Lys Trp Val Cys Glu Leu Arg Glu Pro Asn Lys Lys Thr
50 55 60

Arg Ile Trp Leu Gly Thr Phe Gln Thr Ala Glu Met Ala Ala Arg Ala
65 70 75 80

His Asp Val Ala Ala Ile Ala Leu Arg Gly Arg Ser Ala Cys Leu Asn
85 90 95

Phe Ala Asp Ser Ala Trp Arg Leu Arg Ile Pro Glu Ser Thr Cys Ala
100 105 110

Lys Glu Ile Gln Lys Ala Ala Ala Glu Ala Ala Leu Asn Phe Gln Asp
115 120 125

Glu Met Cys His Met Thr Thr Asp Ala His Gly Leu Asp Met Glu Glu
130 135 140

Thr Leu Val Glu Ala Ile Tyr Thr Pro Glu Gln Ser Gln Asp Ala Phe
145 150 155 160

Tyr Met Asp Glu Glu Ala Met Leu Gly Met Ser Ser Leu Leu Asp Asn
165 170 175

Met Ala Glu Gly Met Leu Leu Pro Ser Pro Ser Val Gln Trp Asn Tyr
180 185 190

Asn Phe Asp Val Glu Gly Asp Asp Asp Val Ser Leu Trp Ser Tyr
195 200 205

<210> 2242
<211> 908
<212> DNA
<213> Arabidopsis thaliana

<220>
<221> misc_feature
<222> (851)..(851)
<223> n is a, c, g, or t

<400> 2242

```
cctgaactag aacagaaaga gagagaaact attatttcag caaaccatac caacaaaaaa      60
gacagagatc ttttagttac cttatccagt ttcttgaaac agagtactct tctgatcaat     120
gaactcattt tctgcttttt ctgaaatgtt tggctccgat tacgagtctt cggtttcctc     180
aggcggtgat tatattccga cgcttgcgag cagctgcccc aagaaaccgg cgggtcgtaa     240
gaagtttcgt gagactcgtc acccaatata cagaggagtt cgtcggagaa actccggtaa     300
gtgggtttgt gaggttagag aaccaaacaa gaaaacaagg atttggctcg gaacatttca     360
aaccgctgag atggcagctc gagctcacga cgttgccgct ttagcccttc gtggccgatc     420
agcctgtctc aatttcgctg actcggcttg gagactccga atcccggaat caacttgcgc     480
taaggacatc caaaaggcgg cggctgaagc tgcgttggcg tttcaggatg agatgtgtga     540
tgcgacgacg gatcatggct tcgacatgga ggagacgttg gtggaggcta tttacacggc     600
ggaacagagc gaaaatgcgt tttatatgca cgatgaggcg atgtttgaga tgccgagttt     660
gttggctaat atggcagaag ggatgctttt gccgcttccg tccgtacagt ggaatcataa     720
tcatgaagtc gacggcgatg atgacgacgt atcgttatgg agttattaaa actcagatta     780
ttatttccat ttttagtacg atacttttta ttttattatt atttttagat cctttttttag     840
aatggaatct ncattatgtt tgtaaaactg agaaacgagt gtaaattaaa ttgattcagt     900
ttcagtat                                                              908
```

<210> 2243
<211> 216
<212> PRT
<213> Arabidopsis thaliana

<400> 2243

Met Asn Ser Phe Ser Ala Phe Ser Glu Met Phe Gly Ser Asp Tyr Glu
1               5                   10                  15

Ser Ser Val Ser Ser Gly Gly Asp Tyr Ile Pro Thr Leu Ala Ser Ser
            20                  25                  30

Cys Pro Lys Lys Pro Ala Gly Arg Lys Lys Phe Arg Glu Thr Arg His
        35                  40                  45

Pro Ile Tyr Arg Gly Val Arg Arg Arg Asn Ser Gly Lys Trp Val Cys
    50                  55                  60

Glu Val Arg Glu Pro Asn Lys Lys Thr Arg Ile Trp Leu Gly Thr Phe
65                  70                  75                  80

Gln Thr Ala Glu Met Ala Ala Arg Ala His Asp Val Ala Ala Leu Ala
                85                  90                  95

Leu Arg Gly Arg Ser Ala Cys Leu Asn Phe Ala Asp Ser Ala Trp Arg
            100                 105                 110

Leu Arg Ile Pro Glu Ser Thr Cys Ala Lys Asp Ile Gln Lys Ala Ala
        115                 120                 125

Ala Glu Ala Ala Leu Ala Phe Gln Asp Glu Met Cys Asp Ala Thr Thr
    130                 135                 140

Asp His Gly Phe Asp Met Glu Glu Thr Leu Val Glu Ala Ile Tyr Thr
145                 150                 155                 160

Ala Glu Gln Ser Glu Asn Ala Phe Tyr Met His Asp Glu Ala Met Phe
                165                 170                 175

Glu Met Pro Ser Leu Leu Ala Asn Met Ala Glu Gly Met Leu Leu Pro
            180                 185                 190

Leu Pro Ser Val Gln Trp Asn His Asn His Glu Val Asp Gly Asp Asp
        195                 200                 205

Asp Asp Val Ser Leu Trp Ser Tyr
    210                 215

<210> 2244
<211> 632
<212> DNA
<213> Brassica napus

<400> 2244

cacccgatat accggggagt tcgtctgaga aagtcaggta agtgggtgtg tgaagtgagg 60

gaaccaaaca agaaatctag aatttggctt ggaactttca aaacagctga gatggcagct 120

```
cgtgctcacg acgtcgctgc cctagccctc cgtggaagag gcgcctgcct caattatgcg     180
gactcggctt ggcggctccg catcccggag acaacctgcc acaaggatat ccagaaggct     240
gctgctgaag ccgcattggc ttttgaggct gagaaaagtg atgtgacgat gcaaaatggc     300
cagaacatgg aggagacgac ggcggtggct tctcaggctg aagtgaatga cacgacgaca     360
gaacatggca tgaacatgga ggaggcaacg gcagtggctt ctcaggctga ggtgaatgac     420
acgacgacgg atcatggcgt agacatggag gagacaatgg tggaggctgt ttttactggg     480
gaacaaagtg aagggtttaa catggcgaag gagtcgacgg tggaggctgc tgttgttacg     540
gaggaaccga gcaaaggatc ttacatggac gaggagtgga tgctcgagat gccgaccttg     600
ttggctgata tggcagaagg gatgctcctg cc                                   632
```

<210> 2245
<211> 208
<212> PRT
<213> Brassica napus

<400> 2245

```
His Pro Ile Tyr Arg Gly Val Arg Leu Arg Lys Ser Gly Lys Trp Val
1               5                   10                  15

Cys Glu Val Arg Glu Pro Asn Lys Lys Ser Arg Ile Trp Leu Gly Thr
            20                  25                  30

Phe Lys Thr Ala Glu Met Ala Ala Arg Ala His Asp Val Ala Ala Leu
        35                  40                  45

Ala Leu Arg Gly Arg Gly Ala Cys Leu Asn Tyr Ala Asp Ser Ala Trp
    50                  55                  60

Arg Leu Arg Ile Pro Glu Thr Thr Cys His Lys Asp Ile Gln Lys Ala
65                  70                  75                  80

Ala Ala Glu Ala Ala Leu Ala Phe Glu Ala Glu Lys Ser Asp Val Thr
                85                  90                  95

Met Gln Asn Gly Gln Asn Met Glu Glu Thr Thr Ala Val Ala Ser Gln
            100                 105                 110

Ala Glu Val Asn Asp Thr Thr Thr Glu His Gly Met Asn Met Glu Glu
        115                 120                 125

Ala Thr Ala Val Ala Ser Gln Ala Glu Val Asn Asp Thr Thr Thr Asp
    130                 135                 140

His Gly Val Asp Met Glu Glu Thr Met Val Glu Ala Val Phe Thr Gly
145                 150                 155                 160

Glu Gln Ser Glu Gly Phe Asn Met Ala Lys Glu Ser Thr Val Glu Ala
                165                 170                 175

Ala Val Val Thr Glu Glu Pro Ser Lys Gly Ser Tyr Met Asp Glu Glu
            180                 185                 190

Trp Met Leu Glu Met Pro Thr Leu Leu Ala Asp Met Ala Glu Gly Met
        195                 200                 205
```

<210> 2246
<211> 20
<212> DNA
<213> artificial sequence

<220>
<223> Artificial Sequence

<220>
<221> misc_feature
<222> (3)..(18)
<223> y = c or t; n = a, c g or t; h = a, c or t; m = a or c

<400> 2246
cayccnatht aymgnggngt 20

<210> 2247
<211> 21
<212> DNA
<213> artificial sequence

<220>
<223> Artificial Sequence

<220>
<221> misc_feature
<222> (3)..(18)

<223> y = c or t; n = a, c g or t; h = a, c or t; m = a or c; r = a or g

<220>
<221> misc_feature
<222> (3)..(18)
<223> y = c or t; n = a, c g or t; h = a, c or t; m = a or c; r = a or g

<400> 2247
ggnarnarca tnccytcngc c 21